# EUROPEAN PATENT APPLICATION

(11) **EP 4 467 661 A1**
(43) Date of publication of application: **27.11.2024**
(21) Application number: 24187512.9
(22) Date of filing: 21.09.2020
(51) Int. Cl.: C12Q 1/6855

(54) **COMPOSITIONS AND METHODS FOR TEMPLATE-FREE DOUBLE STRANDED GEOMETRIC ENZYMATIC NUCLEIC ACID SYNTHESIS**

(30) Priority: 19.09.2019 US 201962902729 P; 21.10.2019 US 201962923920 P
(62) Divisional of application: 20786190.7
(71) Applicant: Camena Bioscience Limited, Saffron Walden, Essex CB10 1XL (GB)
(72) Inventor: STEMPLE, Derek, Newton, 02460 (US); BELL, Neil, Saffron Walden, CB10 1XL (GB); MANKOWSKA, Sylwia, Saffron Walden, CB10 1XL (GB); HARVEY, Steven, Saffron Walden, CB10 1XL (GB); FRASER, Andrew, Saffron Walden, CB10 1XL (GB)
(74) Representative: Cooley (UK) LLP

(57) **Abstract**

The present disclosure provides compositions and methods for template-free double stranded geometric enzymatic nucleic acid synthesis of arbitrarily programmed nucleic acid sequences.

## Description

### RELATED APPLICATIONS

This application claims priority to and the benefit of U.S. Provisional Application No. 62/902,729, filed September 19, 2019, and U.S. Provisional Application No. 62/923,920, filed October 21, 2019. The contents of each of the aforementioned patent applications are incorporated herein by reference in their entireties.

### SEQUENCE LISTING

The instant application contains a Sequence Listing which has been submitted in ASCII format via EFS-Web and is hereby incorporated by reference in its entirety. Said ASCII copy, created on September 21, 2020, is named "DNWR-007_001WO_SeqList.txt" and is about 73.3 KB in size.

### BACKGROUND

Over the last decade there has been an increase in demand for synthetic DNA molecules, which are used in a range of molecular biology applications. This increase has, in part, been driven by advances in DNA sequencing technology. However, while there have been significant developments in DNA sequencing technology, DNA synthesis technology has not progressed at a comparable pace and consequently the state-of-the-art technology does not satisfy the current market needs. The present disclosure provides compositions and methods for template-free double-stranded geometric DNA synthesis that provides a solution to the unmet need in the art for the production of long, error-free, inexpensive DNA sequences having the superior accuracy and speed of synthesis demonstrated by the compositions and methods of the present disclosure.

### SUMMARY

The present disclosure provides compositions comprising a first partially double-stranded nucleic acid molecule and an at least second partially double-stranded nucleic acid molecule, wherein the first partially double-stranded nucleic acid molecule comprises a first 5' overhang and a second 5' overhang, wherein the at least second partially double-stranded nucleic acid molecule comprises a third 5' overhang and fourth 5' overhang, wherein the second 5' overhang and third 5' overhang are complementary to each other, wherein the first 5' overhang, the third 5' overhang and the fourth 5' overhang each comprise one of the 4-mer sequences, or complement thereof, of a 4-mer triplet, wherein the 4-mer triplet comprises three 4-mer sequences, which yield a single fragment with at least 90% purity upon ligation of the first partially double-stranded nucleic acid molecule and the at least second partially double-stranded nucleic acid molecule, and wherein the first 5' overhang, the third 5' overhang and the fourth 5' overhang comprise a different 4-mer sequence. The 4-mer triplet can be selected from the 4-mer triplets recited in Table 1.

The present disclosure provides methods of producing a target nucleic acid molecule, the methods comprising: a) hybridizing the first and the at least second partially double-stranded nucleic acid molecules of the preceding compositions by hybridizing the second 5' overhang of first partially double-stranded nucleic acid molecule and the third 5' overhang of the at least second partially double-stranded nucleic acid molecule; and b) ligating the hybridized first partially double-stranded nucleic acid molecule and the at least second partially double-stranded nucleic acid molecule, thereby producing the target nucleic acid molecule. In some aspects, ligating comprises contacting the hybridized first and at least second partially double-stranded nucleic acid molecules and a ligase.

In some aspects, at least one of the first 5' overhang, the second 5' overhang, the third 5' overhang and the fourth 5' overhang can be 4 nucleotides in length. In some aspects, the first 5' overhang, the second 5' overhang, the third 5' overhang and the fourth 5' overhang can each be 4 nucleotides in length.

In some aspects, the first and the at least second double-stranded nucleic acid molecules can comprise RNA, XNA, DNA or a combination thereof. In some aspects, the first and the at least second double-stranded nucleic acid molecules can comprise DNA.

In some aspects, at least one of the first double-stranded nucleic acid molecule and the at least second double-stranded nucleic acid molecule can comprise at least one modified nucleic acid.

In some aspects, at least one of the first double-stranded nucleic acid molecule and the at least second double-stranded nucleic acid molecule can be at least about 15 nucleotides in length. In some aspects, at least one of the first double-stranded nucleic acid molecule and the at least second double-stranded nucleic acid molecule can comprises a double-stranded portion that is at least 30 bp in length, or at least 250 bp in length.

The present disclosure provides compositions comprising a first partially double-stranded nucleic acid molecule, a second partially double-stranded nucleic acid molecule, a third partially double-stranded nucleic acid molecule and an at least fourth partially double-stranded nucleic acid molecule, wherein the first partially double-stranded nucleic acid molecule comprises a first 5' overhang and a second 5' overhang, wherein the second partially double-stranded nucleic acid molecule comprises a third 5' overhang and fourth 5' overhang, wherein the third partially double-stranded nucleic acid molecule comprises a fifth 5' overhang and a sixth 5' overhang, wherein the at least fourth partially double-stranded nucleic acid molecule comprises a seventh 5' overhang and an eighth 5' overhang, wherein the second 5' overhang and third 5' overhang are complementary to each other, wherein the fourth 5' overhang and the fifth 5' overhang are complementary to each other, wherein the sixth 5' overhang and the seventh 5' overhang are complementary to each other, wherein the first 5' overhang, the third 5' overhang, the fifth 5' overhang, the seventh 5' overhang and the eighth 5' overhang each comprise one of the 4-mer sequences, or complement thereof, of a 4-mer quintuplet, wherein the 4-mer quintuplet comprises five 4-mer sequences, which yield a single fragment with at least 90% purity upon ligation of the first, second, third and at least fourth partially double-stranded nucleic acid molecules, and wherein the first 5' overhang, the third 5' overhang, the fifth 5' overhang, the seventh 5' overhang and the eighth 5' overhang comprise a different 4-mer sequence. The 4-mer quintuplet can be selected from the 4-mer quintuplets recited in Table 2.

The present disclosure provides methods of producing a target nucleic acid molecule, the methods comprising: a) hybridizing the first and the at least second partially double-stranded nucleic acid fragments of the preceding compositions by hybridizing the second 5' overhang of the first partially double-stranded nucleic acid fragment and the third 5' overhang of the second partially double-stranded nucleic acid fragment; b) ligating the hybridized first partially double-stranded nucleic acid fragment and the second partially double-stranded nucleic acid fragment to produce a first ligation product; c) hybridizing the third and the at fourth second partially double-stranded nucleic acid fragments of the preceding compositions by hybridizing the sixth 5' overhang of third partially double-stranded nucleic acid fragment and the seventh 5' overhang of the at least fourth partially double-stranded nucleic acid fragment; d) ligating the hybridized third partially double-stranded nucleic acid fragment and the at least fourth partially double-stranded nucleic acid fragment to produce a second ligation product; e) hybridizing the first ligation product from step (b) and the second ligation product of step (d) by hybridizing the fourth 5' overhang and the fifth 5' overhang; and f) ligating the hybridized first ligation product and second ligation product, thereby producing the target nucleic acid molecule. In some aspects, ligating can comprise contacting the hybridized molecules and a ligase.

In some aspects, at least one of the first 5' overhang, the second 5' overhang, the third 5' overhang, the fourth 5' overhang, the fifth 5' overhang, the sixth 5' overhang, the seventh 5' overhang and the eighth 5' overhang can be 4 nucleotides in length. In some aspects, the first 5' overhang, the second 5' overhang, the third 5' overhang, the fourth 5' overhang, the fifth 5' overhang, the sixth 5' overhang, the seventh 5' overhang and the eighth 5' overhang can each be 4 nucleotides in length.

In some aspects, the first, the second, the third and the at least fourth partially double-stranded nucleic acid molecules can comprise RNA, XNA, DNA or a combination thereof. In some aspects, the first, the second, the third and the at least fourth partially double-stranded nucleic acid molecules comprise DNA.

In some aspects, at least one of the first partially double-stranded nucleic acid molecule, the second partially double-stranded nucleic acid molecule, the third partially double-stranded nucleic acid molecule and the fourth partially double-stranded nucleic acid molecule can comprise at least one modified nucleic acid.

In some aspects, at least one of the first partially double-stranded nucleic acid molecule, the second partially double-stranded nucleic acid molecule, the third partially double-stranded nucleic acid molecule and the at least fourth partially double-stranded nucleic acid molecule can be at least about 15 nucleotides in length. In some aspects, at least one of the first partially double-stranded nucleic acid molecule, the second partially double-stranded nucleic acid molecule, the third partially double-stranded nucleic acid molecule and the at least fourth partially double-stranded nucleic acid molecule comprises a double-stranded portion can be at least 20 bp in length, or at least 250 bp in length.

The present disclosure provides methods of synthesizing a target double-stranded nucleic acid molecule comprising a target nucleic acid sequence, the methods comprising: a) determining an assembly map of the desired double-stranded nucleic acid molecule, wherein the assembly map divides the target doable-stranded nucleic acid molecule into a plurality of double-stranded nucleic acid fragments, wherein the double-stranded nucleic acid fragments comprise at least two 5' overhangs, wherein nucleic acid fragments that are adjacent within the target nucleic acid sequence comprise 5' overhangs that are complementary, wherein the 5' overhangs of at least one pair of nucleic acid fragments that are adjacent within the target nucleic acid sequence each comprise one of the 4-mer sequences, or complement thereof, of a 4-mer triplet, wherein the 4-mer triplet comprises three 4-mer sequences, which yield a single fragment with at least 90% purity upon ligation of the at least one pair of adjacent nucleic acid fragments; b) providing the double-stranded nucleic acid fragments determined in step (a); c) hybridizing a first pair of double-stranded nucleic acid fragments that are adjacent within the target nucleic acid via their complementary 5' overhangs; d) ligating the hybridized nucleic acid fragments from step (c) to form a double-stranded nucleic acid fragment; e) hybridizing a second pair of double-stranded nucleic acid fragments that are adjacent within the target nucleic acid via their complementary 5' overhangs; f) ligating the hybridized nucleic acid fragments from step (e) to form a double-stranded nucleic acid fragment, such that the double-stranded nucleic acid fragment is adjacent within the target nucleic acid sequence to the double-stranded nucleic acid formed in step (d); g) repeating steps (c)-(f) using the ligation products such that the target double-stranded nucleic acid molecule is synthesized. In some aspects, the 4-mer triplet can selected from the 4-mer triplets recited in Table 1.

The present disclosure provides methods of synthesizing a target double-stranded nucleic acid molecule comprising a target nucleic acid sequence, the methods comprising: a) determining an assembly map of the desired double-stranded nucleic acid molecule, wherein the assembly map divides the target double-stranded nucleic acid molecule into a plurality of double-stranded nucleic acid fragments, wherein the double-stranded nucleic acid fragments comprise at least two 5' overhangs, wherein nucleic acid fragments that are adjacent within the target nucleic acid sequence comprise 5' overhangs that are complementary, wherein the 5' overhangs of at least one set of four nucleic acid fragments that are adjacent within the target nucleic acid sequence each comprise one of the 4-mer sequences, or complement thereof, of a 4-mer quintuplet, wherein the 4-mer quintuplet comprises five 4-mer sequences, which yield a single fragment with at least 90% purity upon ligation of the at least one set of four nucleic acid fragments; b) providing the double-stranded nucleic acid fragments determined in step (a); c) hybridizing a first pair of double-stranded nucleic acid fragments that are adjacent within the target nucleic acid via their complementary 5' overhangs; d) ligating the hybridized nucleic acid fragments from step (c) to form a double-stranded nucleic acid fragment; e) hybridizing a second pair of double-stranded nucleic acid fragments that are adjacent within the target nucleic acid via their complementary 5' overhangs; f) ligating the hybridized nucleic acid fragments from step (e) to form a double-stranded nucleic acid fragment, such that the double-stranded nucleic acid fragment is adjacent within the target nucleic acid sequence to the double-stranded nucleic acid formed in step (d); and g) repeating steps (c)-(f) using the ligation products such that the target double-stranded nucleic acid molecule is synthesized. In some aspects, the 4-mer quintuplet can be selected from the 4-mer quintuplets recited in Table 2.

In some aspects, an assembly map can divide the target double-stranded nucleic acid molecule into at least 4 double-stranded nucleic acid fragments, or at least 50 double-stranded nucleic acid fragments, or at least 100 double-stranded nucleic acid fragments.

In some aspects, the target double-stranded nucleic acid molecule can be at least 1000 nucleotides in length, or at least 2000 nucleotides in length, or least 3000 nucleotides in length.

In some aspects, the target double-stranded nucleic acid can comprise at least one homopolymeric sequence. A homopolymeric sequence can be 10 nucleotides in length. In some aspects, a target double-stranded nucleic acid molecule can have a GC content that is at least about 50%.

In some aspects of the preceding methods, at least one of the double-stranded nucleic acid fragments that corresponds to at least one of the termini of the target double-stranded nucleic acid molecule comprises a hairpin sequence.

In some aspects, the preceding methods can further comprise after step (g): h) incubating the ligation products with at least one exonuclease. In some aspects, a hairpin sequence can comprise at least one deoxyuridine base. In some aspects, a hairpin sequence can comprise at least one restriction endonuclease site.

In some aspects, the preceding methods can further comprise: i) removing the at least one exonuclease; and j) incubating the products of the exonuclease incubation with at least one enzyme that cleaves the at least one deoxyuridine base, thereby cleaving the hairpin sequence.

In some aspects, the preceding methods can further comprise: i) removing the at least one exonuclease; and j) incubating the products of the exonuclease incubation with at least one enzyme that cleaves the at least one restriction endonuclease site, thereby cleaving the hairpin sequence.

In some aspects of the preceding methods, a synthesized target double-stranded nucleic acid molecule can have a purity of at least 80% or at least 90%.

Any of the above aspects can be combined with any other aspect.

Unless otherwise defined, all technical and scientific terms used herein have the same meaning as commonly understood by one of ordinary skill in the art to which this disclosure belongs. In the Specification, the singular forms also include the plural unless the context clearly dictates otherwise; as examples, the terms "a," "an," and "the" are understood to be singular or plural and the term "or" is understood to be inclusive. By way of example, "an element" means one or more element. Throughout the specification the word "comprising," or variations such as "comprises" or "comprising," will be understood to imply the inclusion of a stated element, integer or step, or group of elements, integers or steps, but not the exclusion of any other element, integer or step, or group of elements, integers or steps. About can be understood as within 10%, 9%, 8%, 7%, 6%, 5%, 4%, 3%, 2%, 1%, 0.5%, 0.1%, 0.05%, or 0.01% of the stated value. Unless otherwise clear from the context, all numerical values provided herein are modified by the term "about."

Although methods and materials similar or equivalent to those described herein can be used in the practice or testing of the present disclosure, suitable methods and materials are described below. All publications, patent applications, patents, and other references mentioned herein are incorporated by reference in their entirety. The references cited herein are not admitted to be prior art to the claimed invention. In the case of conflict, the present Specification, including definitions, will control. In addition, the materials, methods, and examples are illustrative only and are not intended to be limiting. Other features and advantages of the disclosure will be apparent from the following detailed description and claim.

### BRIEF DESCRIPTION OF THE DRAWINGS

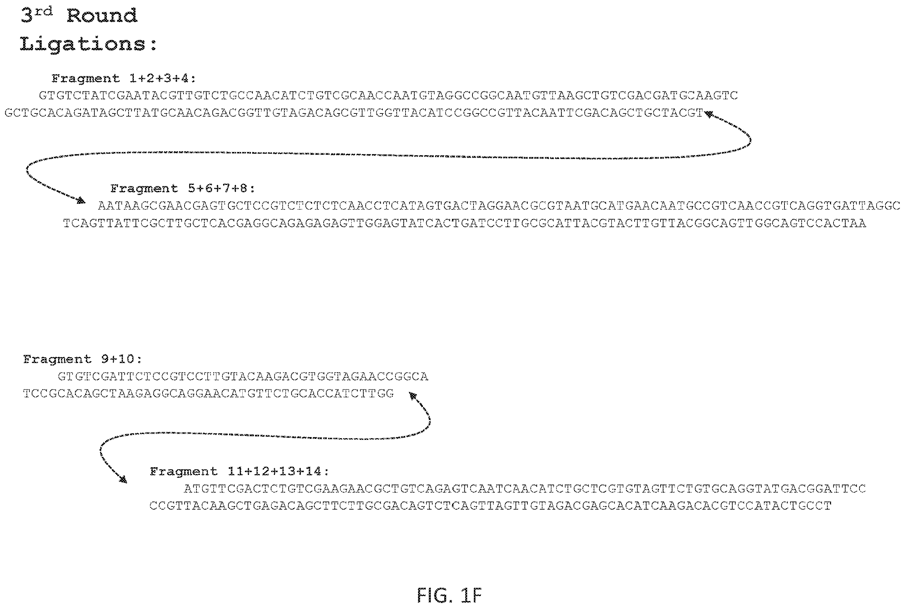

The above and further features will be more clearly appreciated from the following detailed description when taken in conjunction with the accompanying drawings.

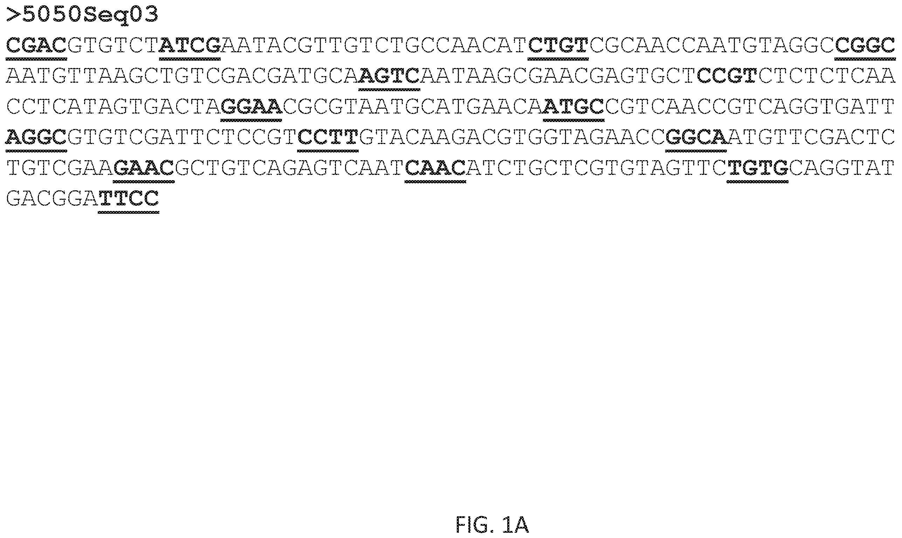

FIG. 1A-1F is a schematic overview of double-stranded geometric synthesis (gSynth) of the present disclosure.
FIG. 1A is a sequence that is to he synthesized using the double-stranded gSynth methods of the present disclosure. Parts of the sequence that are in bold and underlined correspond to 4-mer overhangs that have been selected, thus defining the fragments that will be used to synthesize the entire sequence. The sequence shown in FIG. 1A corresponds to SEQ ID NO: 2.

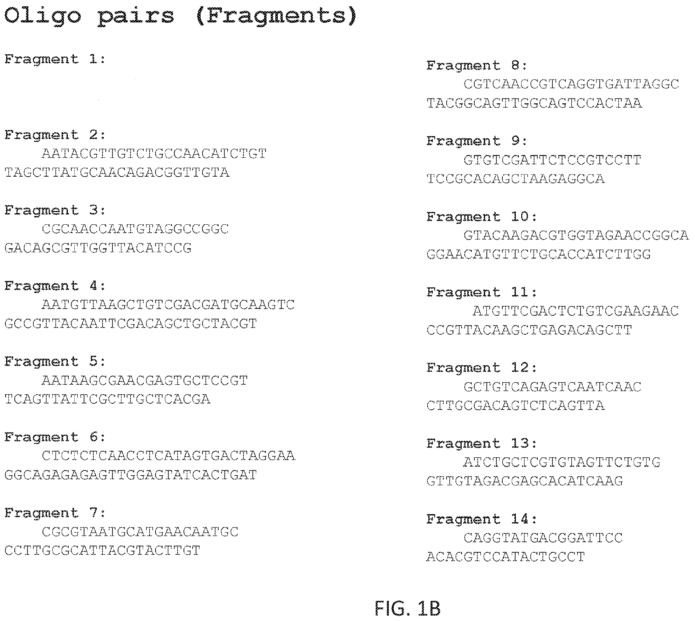

FIG. 1B shows the individual double-stranded nucleic acid fragments of the sequence shown in FIG. 1A that will be used in the double-stranded gSynth methods of the present disclosure to construct the sequence shown in FIG. 1A. These fragments are chosen based on the sites selected in FIG. 1A. The sequences shown in FIG. 1B correspond to SEQ ID NOs: 3-30.

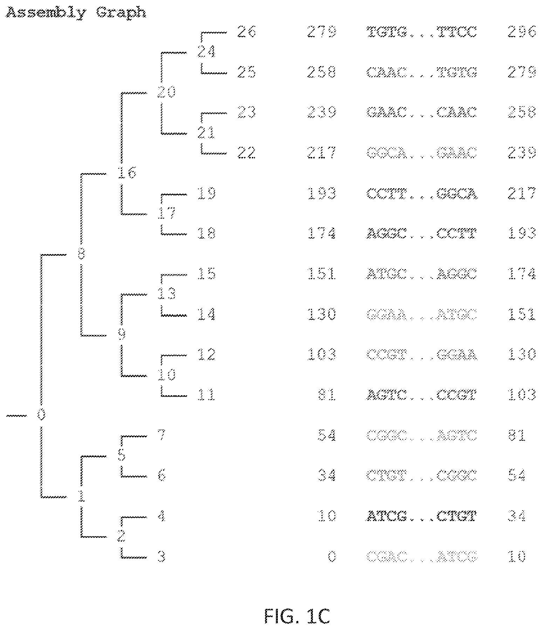

FIG. 1C is a schematic of a binary tree that shows the order in which the fragments in FIG. 1B are to be assembled to generate the sequence shown in FIG. 1A.
FIG. ID is a schematic of the first round of ligations in the double-stranded gSynth method to synthesize the sequence shown in FIG. 1A. In the first ligation round, Fragments 1 and 2, Fragments 3 and 4, Fragments 5 and 6, Fragments 7 and 8, Fragments 9 and 10, Fragments 11 and 12, and Fragments 13 and 14 are hybridized via their complementary 5' overhangs and then ligated together to create Fragment 1+2, Fragment 3+4, Fragment 5+6, Fragment 7+8, Fragment 9+10, Fragment 11+12, and Fragment 13+14. The sequences shown in FIG. ID correspond to SEQ ID NOs: 3-30.

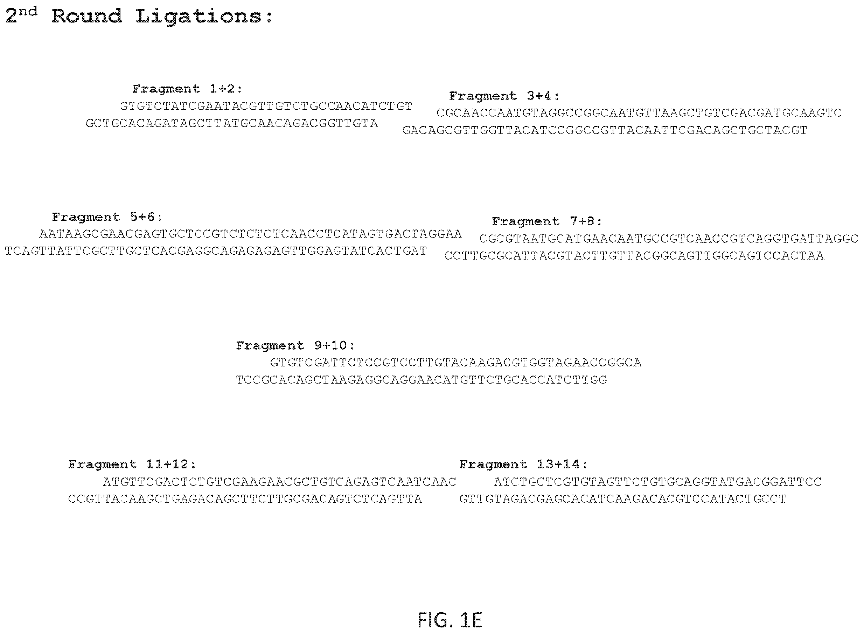

FIG. 1E is a schematic of the second round of ligations in the double-stranded gSynth method to synthesize the sequence shown in FIG. 1A. In the second ligation round, Fragments 1+2 and 3+4, Fragments 5+6 and 7+8, and Fragments 11+12 and 1 3-1-1 4 are hybridized via their complementary 5' overhangs and then ligated together to create Fragment 1+2+3+4, Fragment 5+6+7+8, and Fragment 11+12+13+14. The sequences shown in FIG. 1E correspond to SEQ ID NOs: 31-44.
FIG. IF is a schematic of the third round of ligations in the double-stranded gSynth method to synthesize the sequence shown in FIG. 1A, In the third ligation round, Fragments 1+2+3+4 and 5+6+7+8, and Fragments 9-1-10 and 11+12+13+14 are hybridized via their complementary 5' overhangs and then ligated together to create Fragment 1+2+3+4+5+6+7+8 and Fragment 9+10+11+12+13+14. The sequences shown in FIG. 1F correspond to SEQ ID NOs: 45-52.
FIG. 1G is a schematic of the fourth and final round of ligations in the double-stranded gSynth method to synthesize the sequence shown in FIG. 1A. In the fourth ligation round, Fragments 1+2+3+4+5+6+7+8 and 9+10+11+12+13+14 are hybridized via their complementary 5' overhangs and ligated together, thereby producing the sequence shown in FIG. 1A. The sequences shown in FIG. 1G correspond to SEQ ID NOs: 53-56.
FIG. 2 is an image of a DNA-gel analysis of the results of a double-stranded gSynth assembly reaction products compared to the products of hybridization and elongation (HAE) assembly reactions.
FIG. 3 is an image of a DNA-gel analysis of the results of a double-stranded gSynth assembly reaction where the terminal fragments of the sequence to be synthesized were capped with hairpins. The products of the double-stranded gSynth reaction were then analyzed before and after exonuclease digestion. The sequences shown in FIG. 3 correspond to SEQ ID NOs: 57-60.
FIG. 4 shows an overview of a V-gSynth reaction used to create a large plurality of emGFP variants.
FIGs. 5A-5D show schematics of the assembly of the p.[Y66X; T203X] IVTT and InDel libraries by V-gSynth methods of the present disclosure.

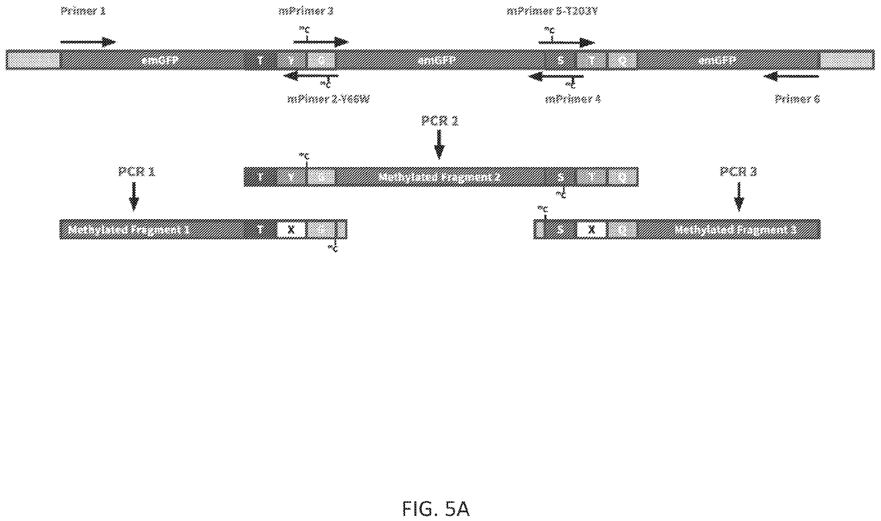

FIG. 5A shows the preparation of overlapping Methylated Fragments 1-Y66X, 2 and 3-T203X

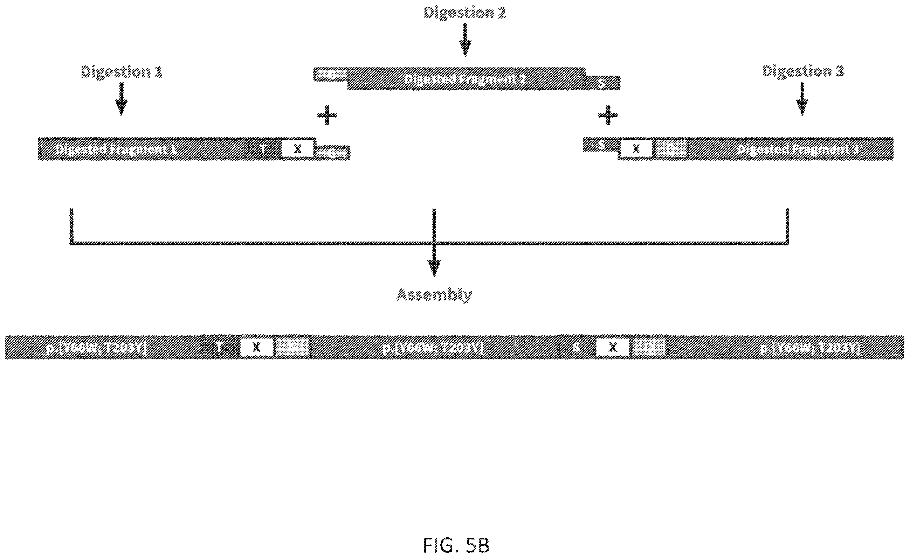

FIG. 5B shows removal of the original Y66 and T203 sequence by FSPEI digestion and production of Digested Fragment 2. The position of the 5-Methylcytosine in Methylated Fragment 1-Y66X and 3-T203X, means that desired Y66X and T203X sequence variations remain within Digested Fragment 1-Y66X and 3-T203X, respectively. The FspEI digestion also leaves compatible four-nucleotide overhangs for assembly. The bottom panel of FIG. 5B shows T7 DNA ligase assembly of Digested Fragments 1-YG6X, 2 and 3-T203X into the p.[Y66X; T203X] IVTT library.

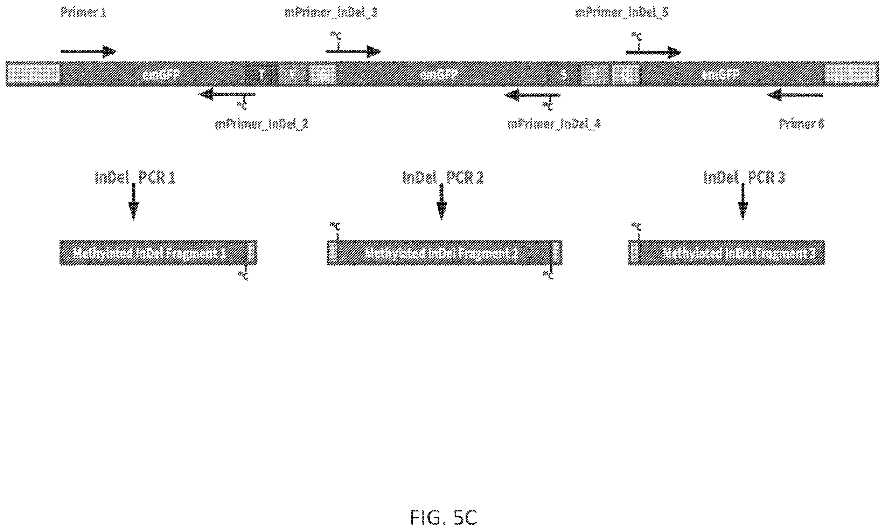

FIG. 5C shows the preparation of non-overlapping Methylated InDei Fragments 1, 2 and, during which codons T65_Y66_G67 and T202-Y203-G204 are deleted between Methylated InDel Fragments 1 and 2, and Methylated InDel Fragments 2 and 3, respectively.

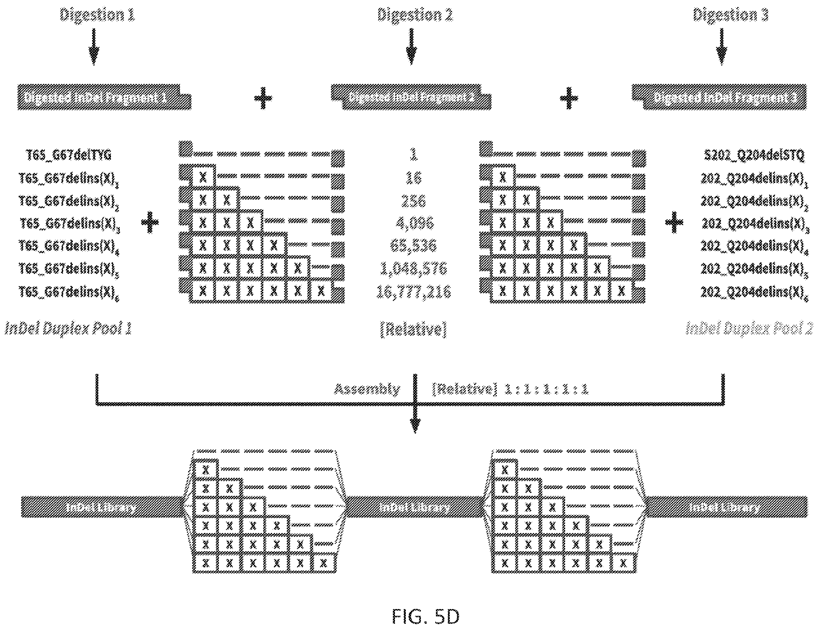

FIG. 5D shows the removal of a further 12/16 nucleotides from the 5- methylcytosine FspEI Digestion. The sequences removed by the FspEI digestion are replaced by the 3' and 5' flanking regions of the InDel Duplexes, while also inserting the 0 to 6 consecutive X codons, via the repetitive N1N2C3 nucleotide sequence, to generate the InDel library. FspEI digestion also leaves compatible four-nucleotide overhangs for assembly The bottom panel of FIG. 5D shows T7 DNA ligase-mediated assembly of Digested InDel Fragments 1, 2 and 3 and the two InDel Duplex Pools into the InDel library.

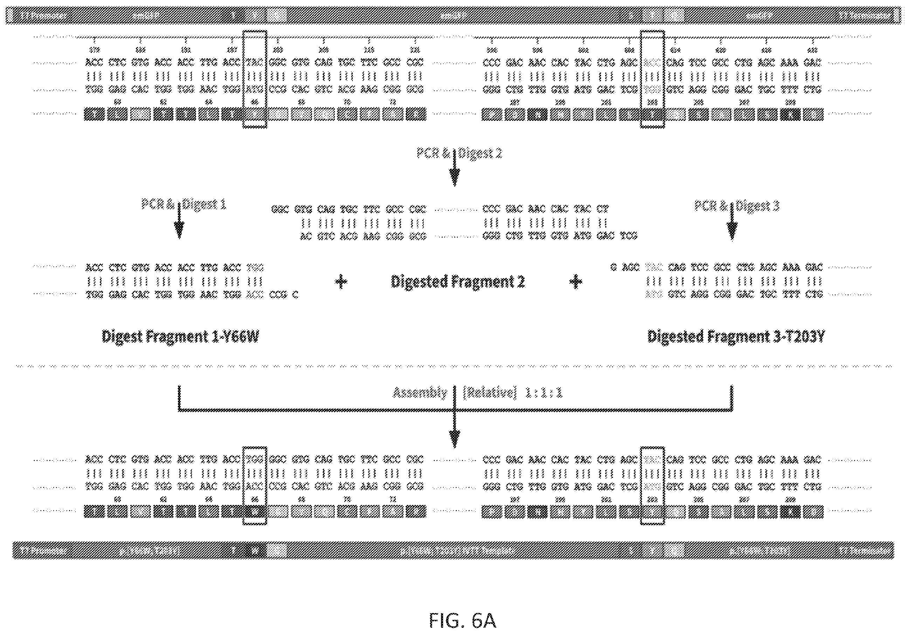

FIGs. 6A-6C show the assembly of the wild-type, p.Y66W, p.T203Y and p.[Y66W; T203Y] IVTT templates using the V-gSynth methods of the present disc.lsoure.
The top panel of FIG. 6A shows VgSynth assembly of the p. [Y66W; T203Y] IVTT template, highlighting Digested Fragment 1-Y66W and Digested Fragment 3-T203Y containing the codon substitution Y66W and T-203Y, as well as the four nucleotide overhangs generated by FspEI digestion Methylated Fragments 1-Y66W, 2 and 3-T203Y. The bottom panel of FIG. 6A shows T7 DNA ligase-mediated assembly of the Digested Fragments 1-Y66W, 2 and 3-T203Y into the p. [Y66X; T203X] IVTT library. The sequences shown in FIG. 6A correspond to SEQ ID NOs: 61-80.

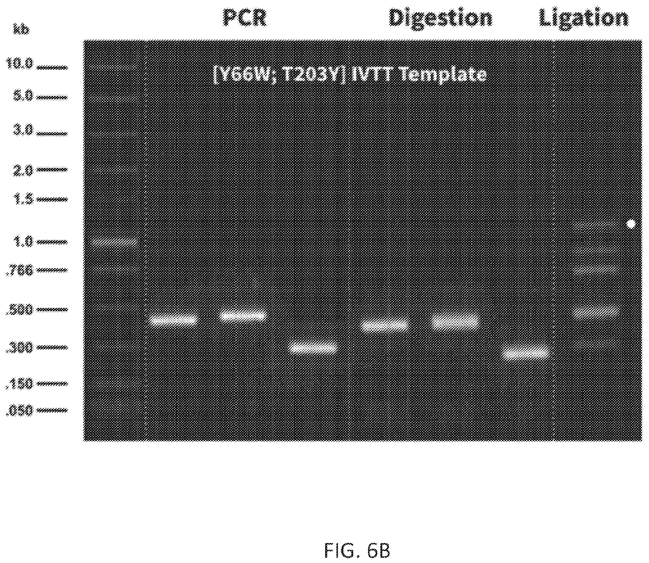

FIG. 6B is an image of agarose gel analysis of the preparation of the Methylated Fragments, their subsequent FspEI digestion and then T7 DNA ligase assembly into the p. [Y66W; T203Y] IVTT template; Ladder (lane 1), Methylated Fragment 1-Y66W (lane 2), Methylated Fragment η (lane 3), Methylated Fragment 3-T203Y (lane 4), Digested Fragment 1-Y66W (lane 5), Digested Fragment 2 (lane 6), Digested Fragment 3-T203Y (lane 7), p.[Y66W; T203Y] IVTT template (lane 8, white dot).

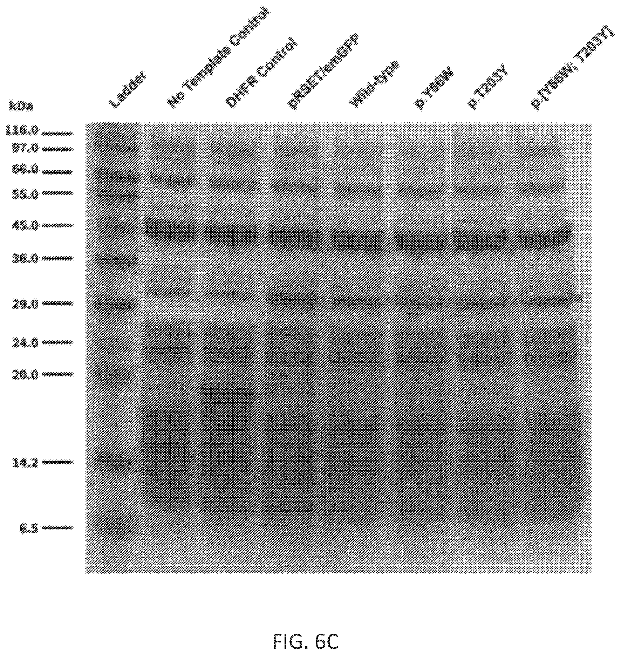

FIG. 6C is an image of SDS-PAGE gel showing the protein expression of the assembled IVTT templates, the desired proteins are indicated by the dots. Ladder (lane 1), No Template Control (Lane 2), DHFR Control (Lane 3), pRSET/emGFP Plasmid (Lane 4), wild-type (Lane 5), p.Y66W (Lane 6), p.T203Y (Lane 7) and p.[Y66W; T203Y] (Lane 8).

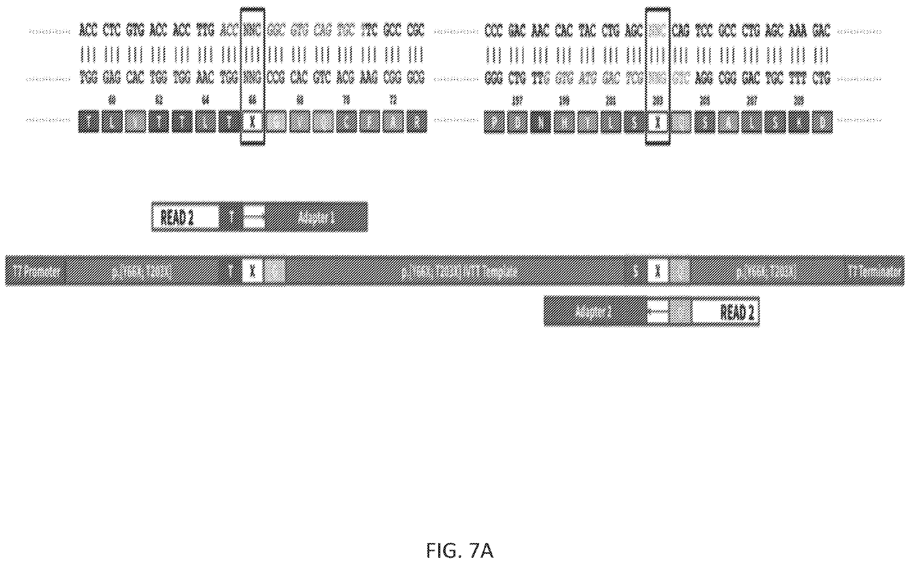

FIGs 7A-7D show the assembly of the on-bead, monoclonal p. [Y66X; T203X] IVTT Library.
FIG. 7A is a schematic of the V-gSynth assembly of the p. [Y66X; T203X] IVTT Library. The sequences shown in FIG. 7A correspond to SEQ ID NOs: 81-86.

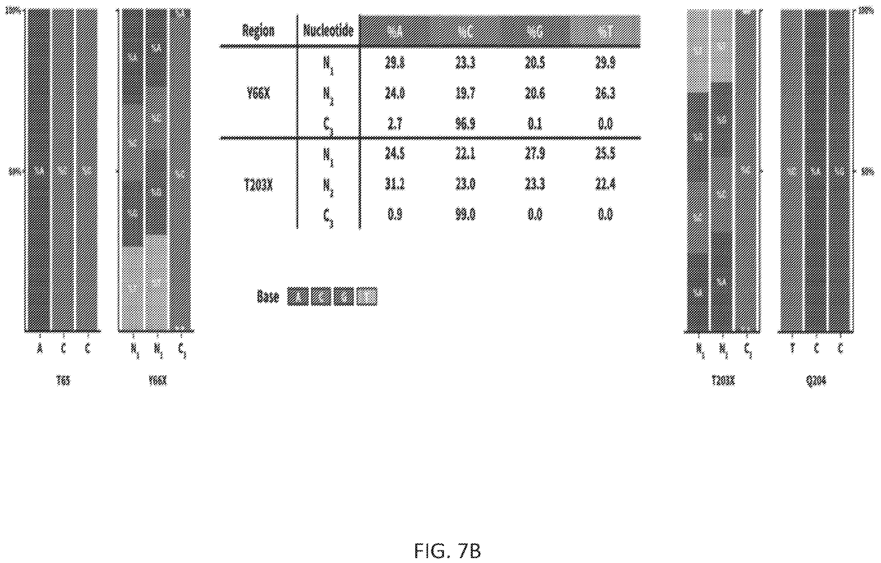

FIG. 7B shows the nucleotide distribution of the monoclonal p.[Y66X; T203X] IVTT library derived from the NGS data.

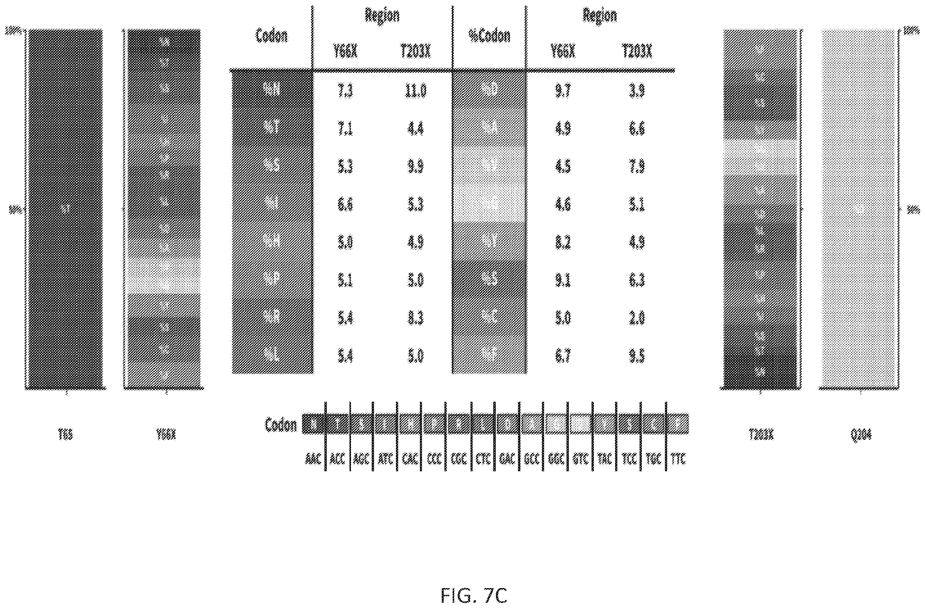

FIG. 7C shows the codon distribution of the monoclonal p.[Y66X; T203X] IVTT library derived from the NGS data.

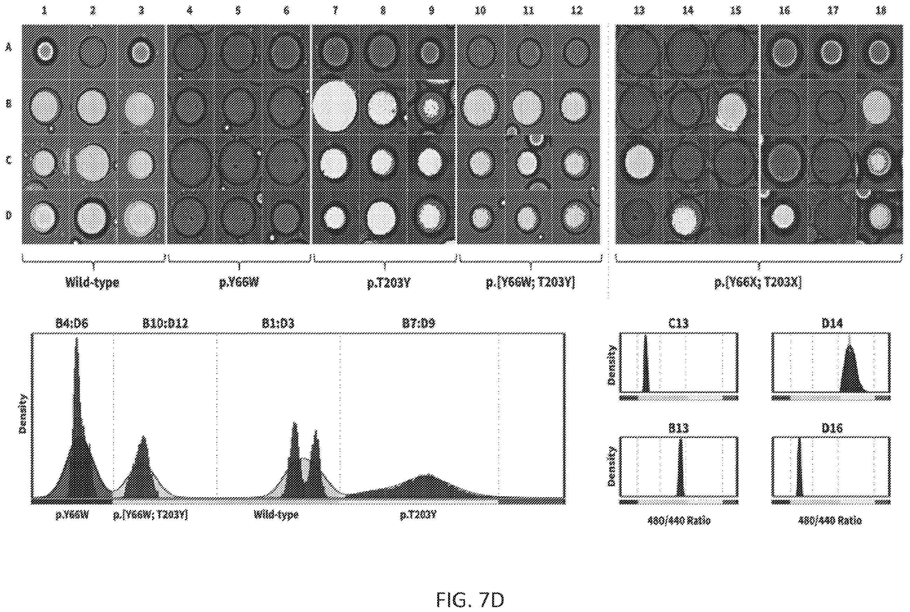

FIG. 7D shows fluorescent imaging results for the on-head wild-type, Y66W, T203Y and [Y66W; T203Y] IVTT template controls as well as the on-bead, monoclonal p.[Y66X; T203X] IVTT library as a ratio of the 480/440 nm excitation.

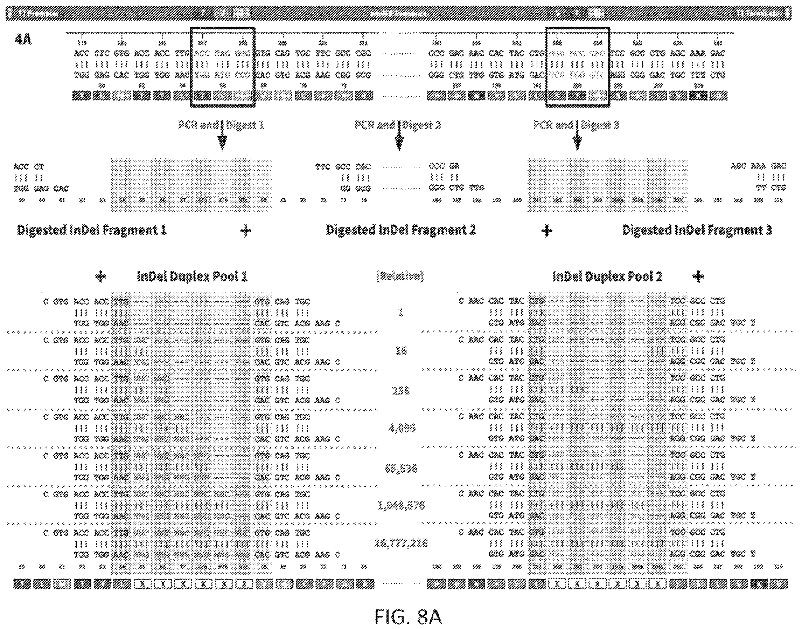

FIG. 8A-8D show the assembly of the forty-nine InDel combinations to generate an InDei library using the V-gSynth methods of the present disclosure.
FIG 8A is a schematic of the V-gSynth assembly of the InDei Library. The sequences shown in FIG. 8A correspond to SEQ ID NOs: 87-123.

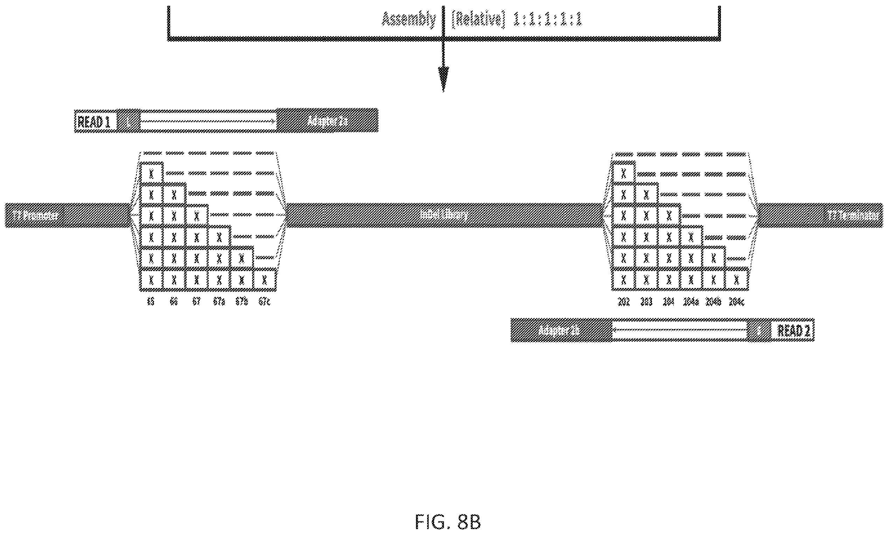

FIG. 8B is a schematic of the T7 DNA ligase assembley of the Digested InDel Fragments 1, 2 and 3 and the two InDel Duplex Pools into the InDel library.

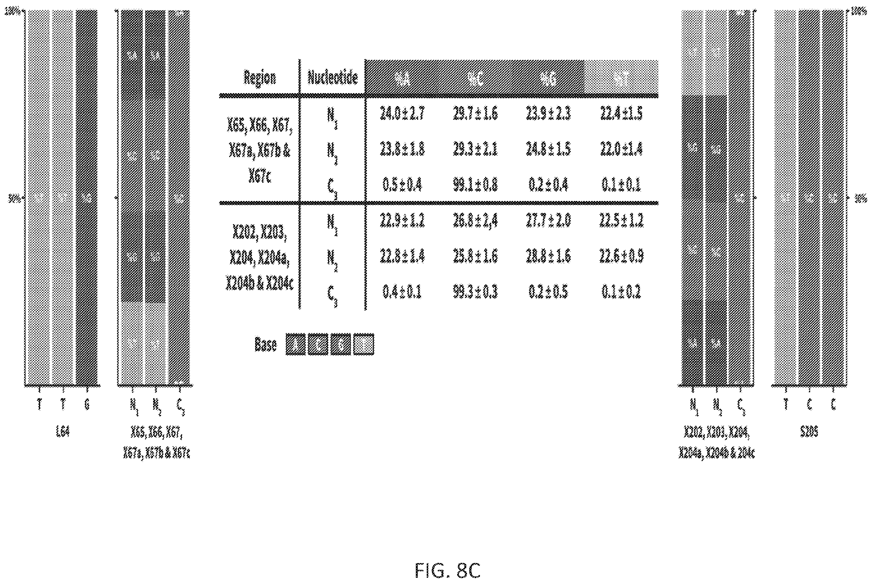

FIG. 8C shows the nucleotide distributions for the InDei library derived from the NGS data.

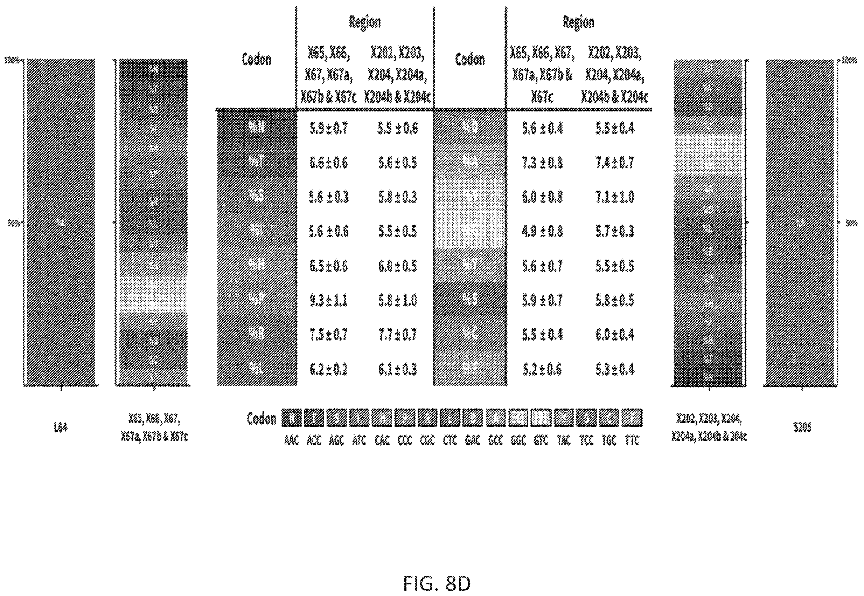

FIG. 8D shows the codon distributions for the InDel library derived from the NGS data.

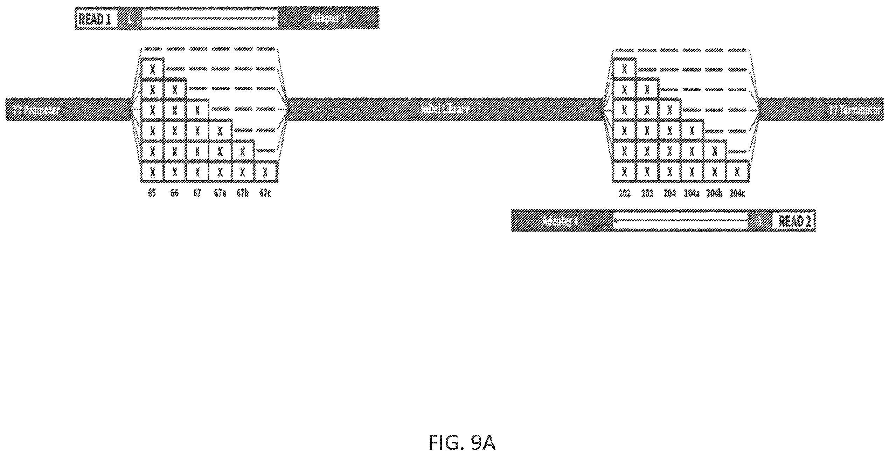

FIGs. 9A-9B show an in-depth analysis of the InDels Library NGS data.
FIG. 9A shows a schematic of the design of the NGS InDel Library, showing codon L64 and Adapter 1b in Read 1, as well as codon S205 and Adapter 2b in Read 2.

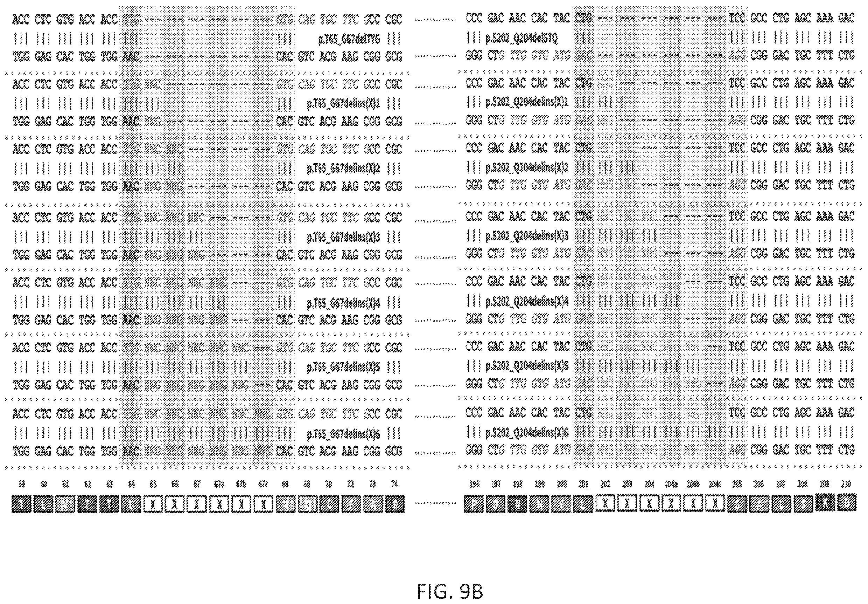

FIG. 9B shows the distribution of the degenerate X codons (nucleotide sequence N₁N₂C₃) introduced by InDel Duplex Pools 1 and InDei Duplex Pool 2. The sequences shown in FIG. 9B correspond to SEQ ID NOs: 124-153.

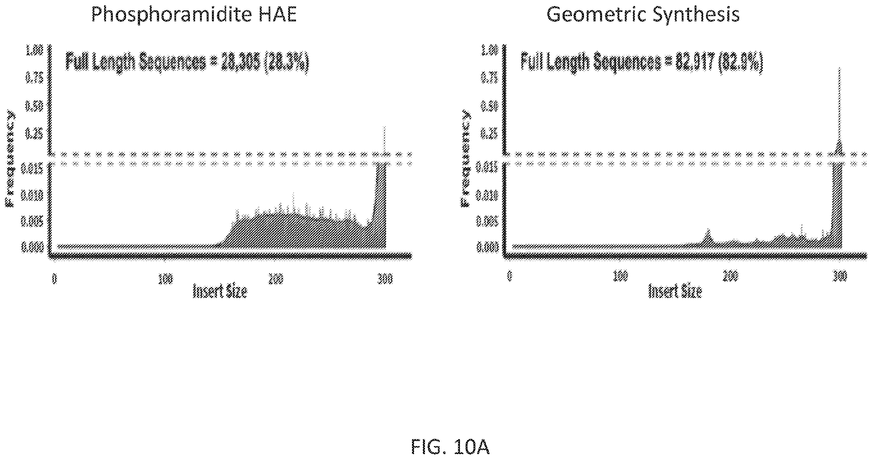

FIG. 10A is a series of graphs showing the distribution of product sizes in phosphoramidite HAE synthesis reactions and geometric synthesis reactions of the present disclosure.

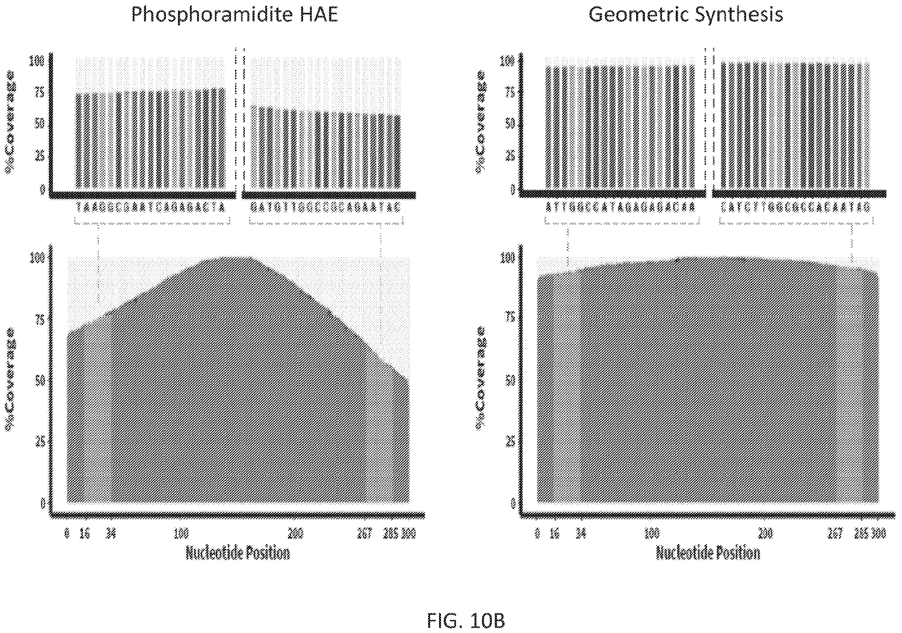

FIG. 10B is a series of graphs showing target sequence coverage in phosphoramidite HAE synthesis reactions and geometric synthesis reactions of the present disclosure.

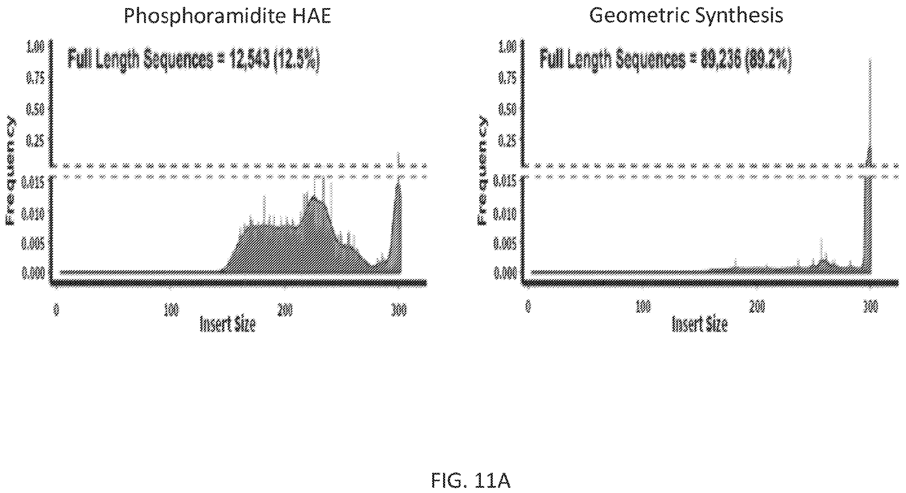

FIG. 11A is a series of graphs showing the distribution of product sizes in phosphoramidite HAE synthesis reactions and geometric synthesis reactions of the present disclosure.

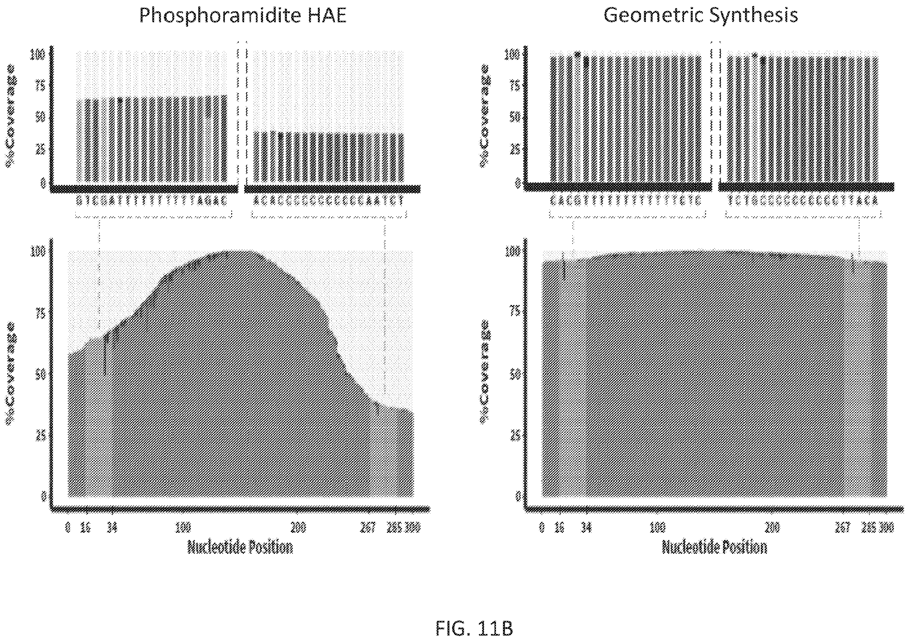

FIG. 11B is a series of graphs showing target sequence coverage in phosphoramidite HAE synthesis reactions and geometric synthesis reactions of the present disclosure. The sequences shown in FIG. 11B correspond to SEQ ID NOs: 154-157.

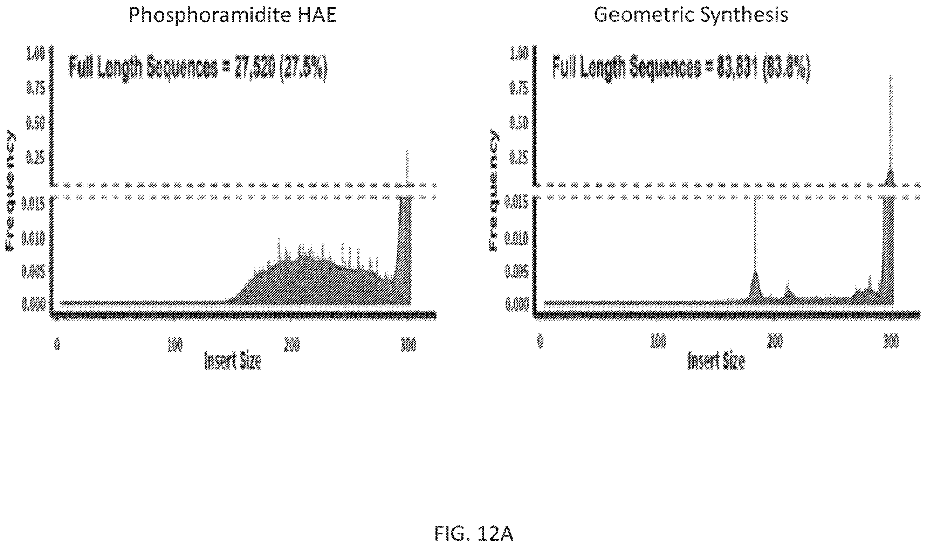

FIG. 12A is a series of graphs showing the distribution of product sizes in phosphoramidite HAE synthesis reactions and geometric synthesis reactions of the present disclosure.

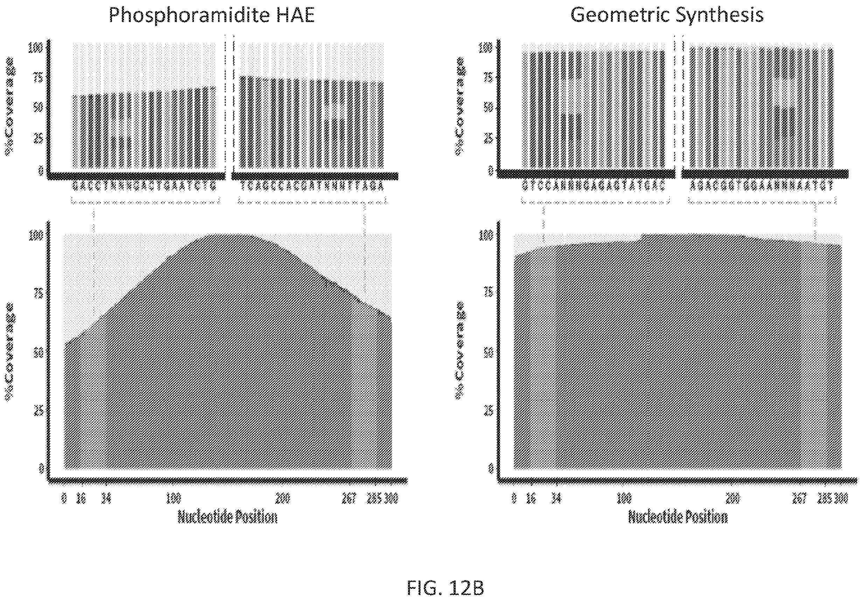

FIG. 12B is a series of graphs showing target sequence coverage in phosphoramidite HAE synthesis reactions and geometric synthesis reactions of the present disclosure. The sequences shown in FIG. 12B correspond to SEQ ID NOs: 158-161.

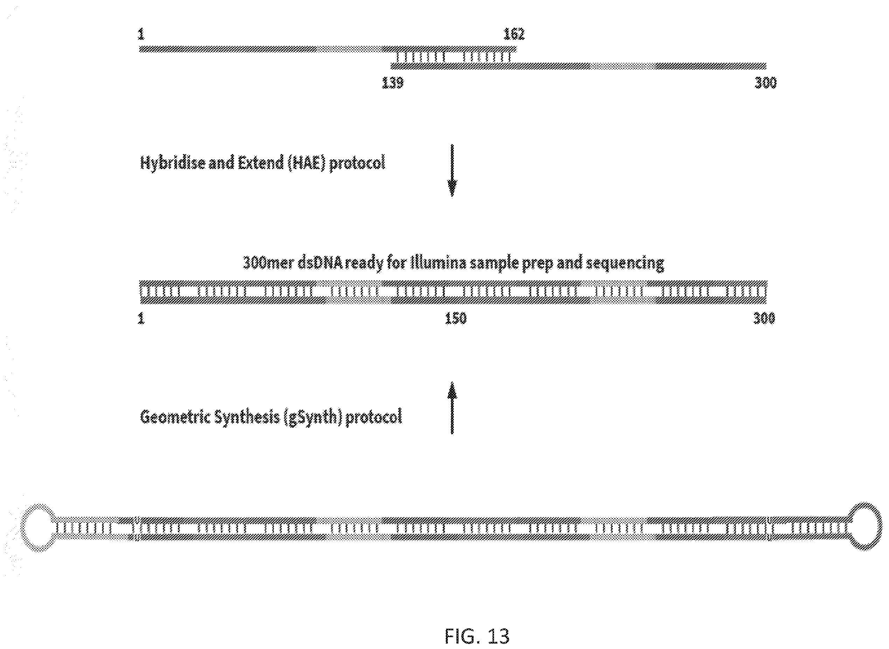

FIG. 13 is a schematic diagram of the phosphoramidite synthesis reactions described herein.

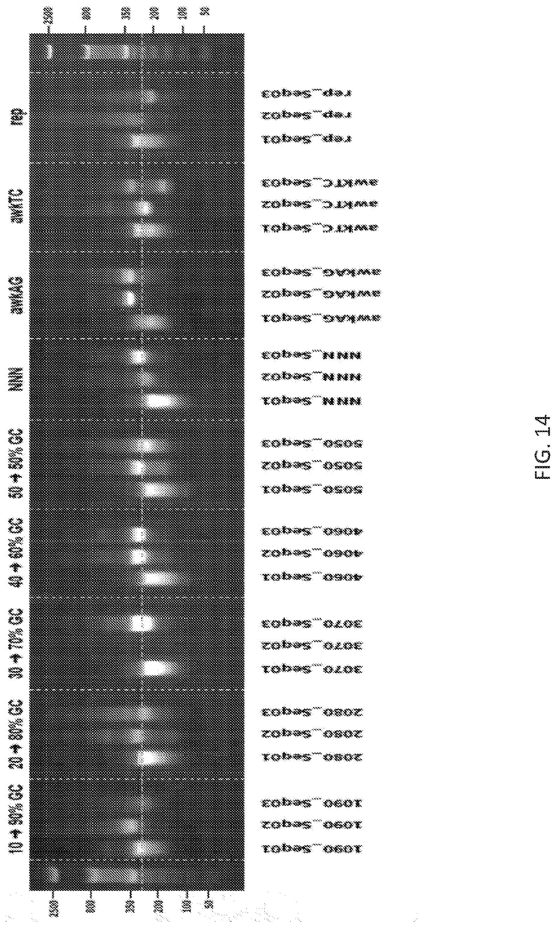

FIG. 14 is an image of agarose gel analysis of phosphoramidite synthesis reactions and geometric synthesis reactions of the present disclosure.

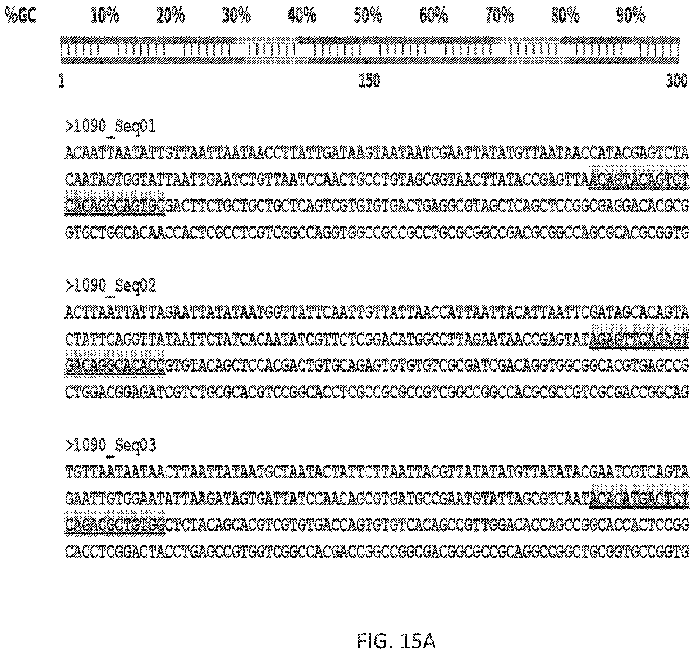

FIG. 15A shows a schematic diagram of a target nucleic acid (top), sequences of the target nucleic acids (right) and a series of graphs depicting GC content along the length of the length of the target nucleic acid as calculated by a sliding window of 50 nucleotides. The target nucleic acids were synthesized using either phosphoramidite synthesis or geometric synthesis methods of the present disclosure. The sequences shown in FIG. 15A correspond to SEQ ID NOs: 162-164.

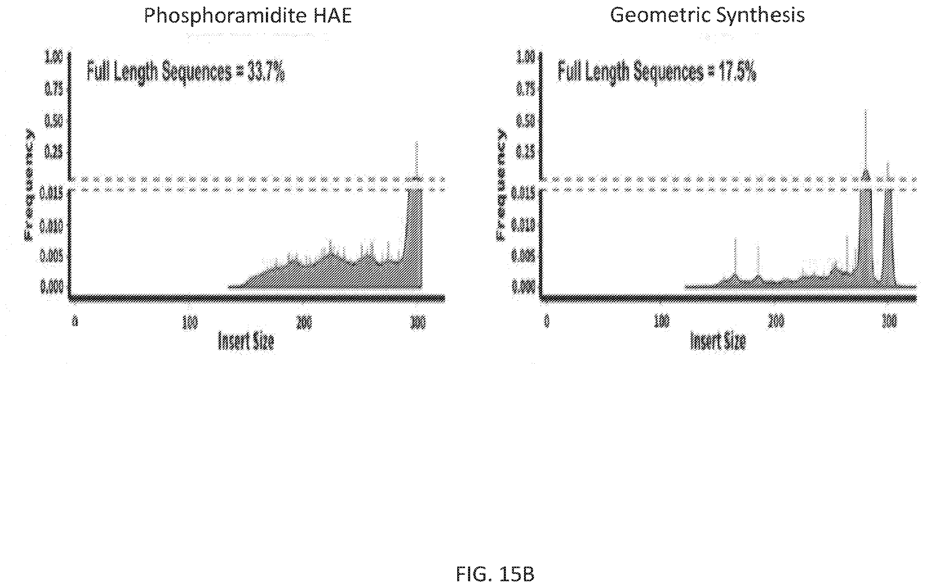

FIG. 15B is a series of graphs showing the distribution of product sizes in phosphoramidite HAE synthesis reactions and geometric synthesis reactions of the present disclosure.

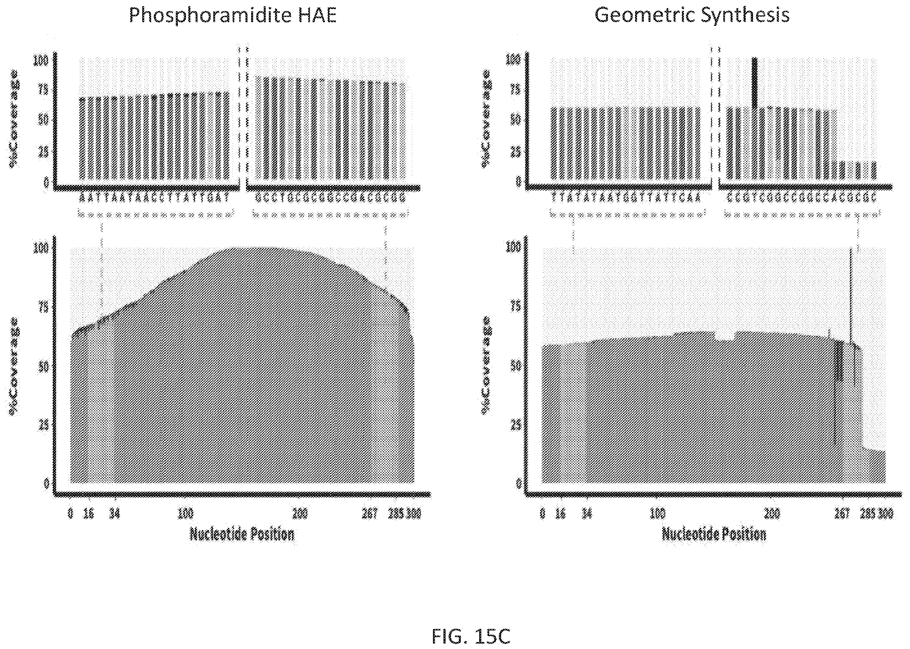

FIG. 15C is a series of graphs showing target sequence coverage in phosphoramidite HAE synthesis reactions and geometric synthesis reactions of the present disclosure. The sequences shown in FIG. 15C correspond to SEQ ID NOs: 165-1 68.

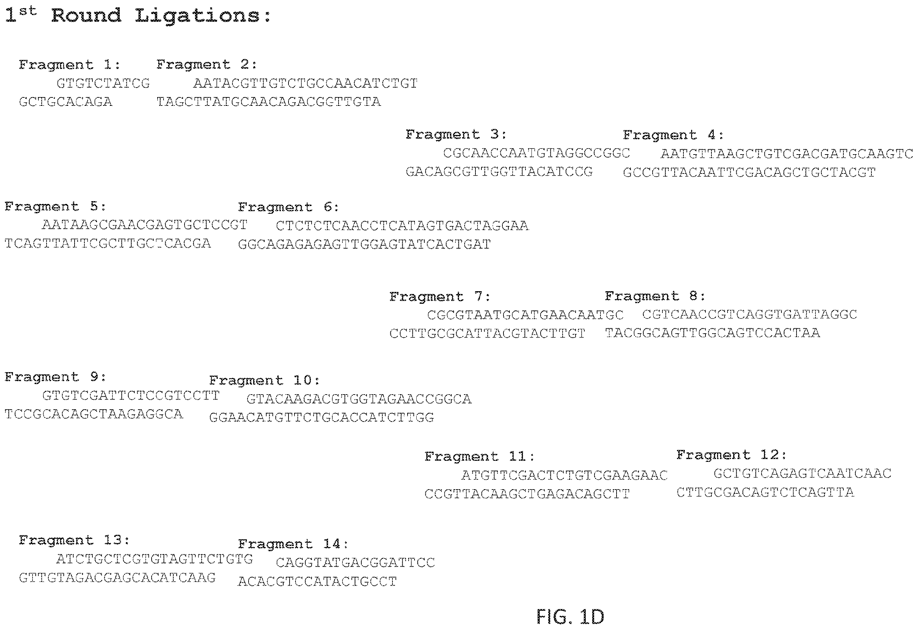

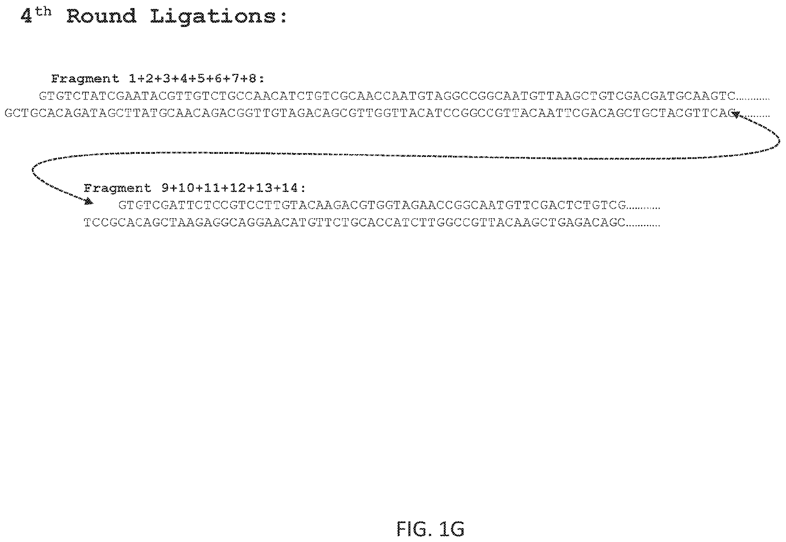

FIG. 1 5D is a series of graphs showing the distribution of product sizes in phosphoramidite HAE synthesis reactions and geometric synthesis reactions of the present disclosure.

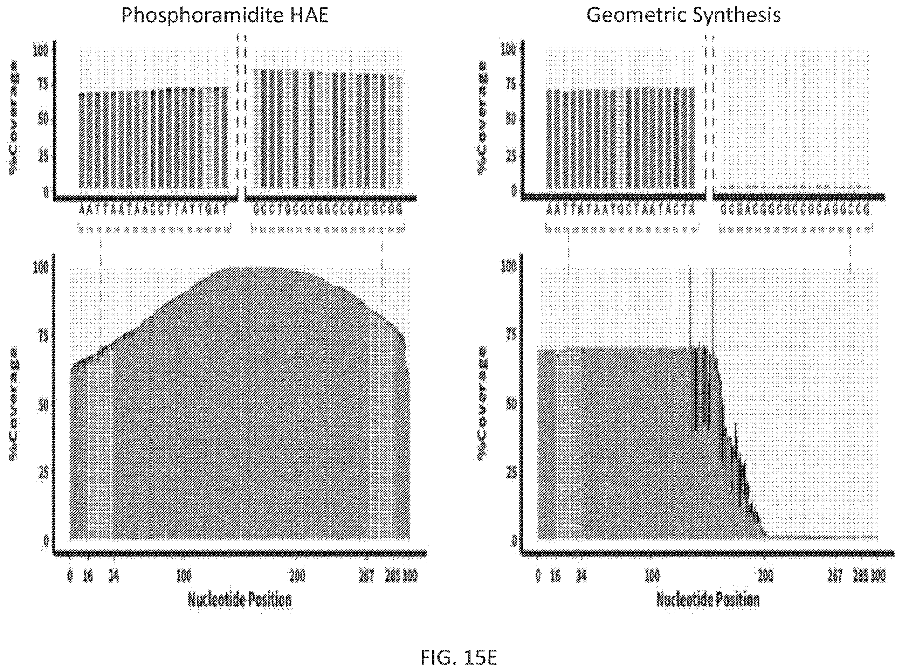

FIG. 15E is a series of graphs showing target sequence coverage in phosphoramidite HAE. synthesis reactions and geometric synthesis reactions of the present disclosure. The sequences shown in FIG. 15E correspond to SEQ ID NOs: 169-172.

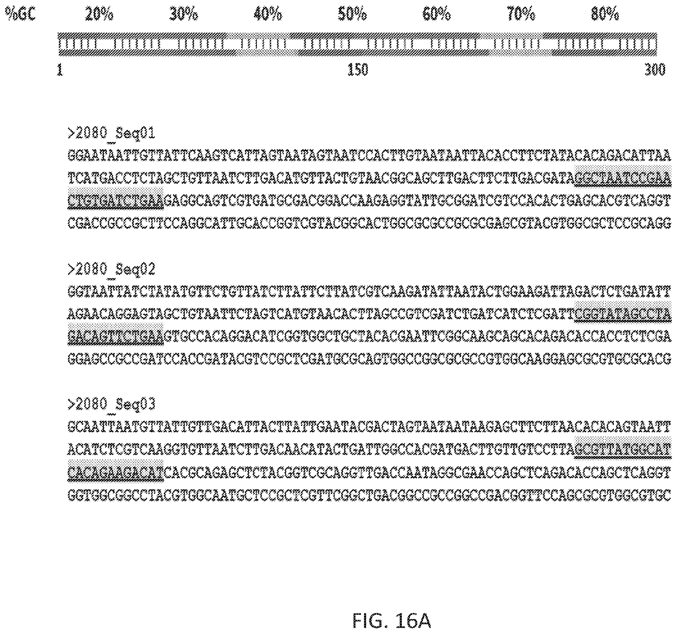

FIG. 16A shows a schematic diagram of a target nucleic acid (top), sequences of the target nucleic acids (right) and a series of graphs depicting GC content along the length of the length of the target nucleic acid as calculated by a sliding window of 50 nucleotides. The target nucleic acids were synthesized using either phosphoramidite synthesis or geometric synthesis methods of the present disclosure. The sequences shown in FIG. 16A correspond to SEQ ID NOs: 173-175.

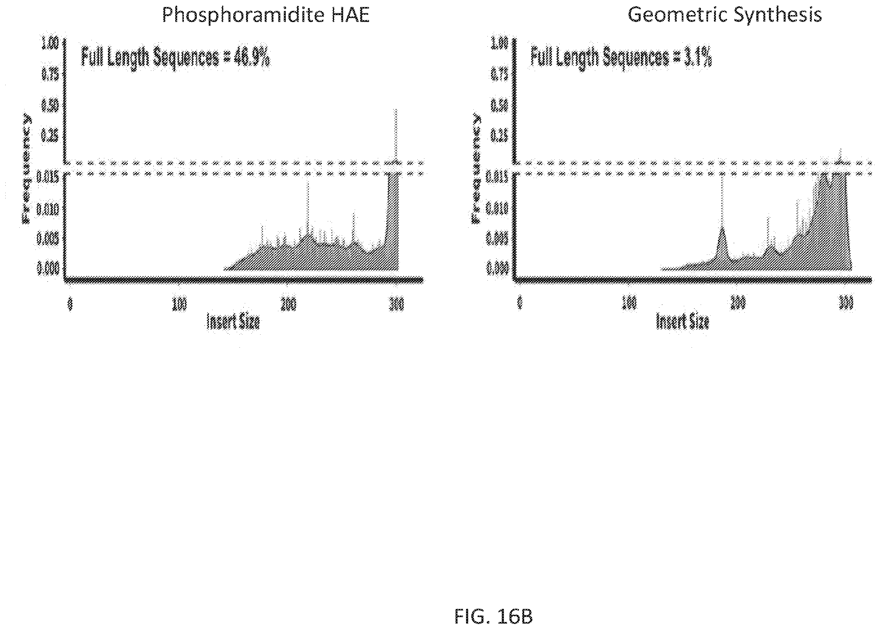

FIG. 16B is a series of graphs showing the distribution of product sizes in phosphoramidite HAE synthesis reactions and geometric synthesis reactions of the present disclosure.

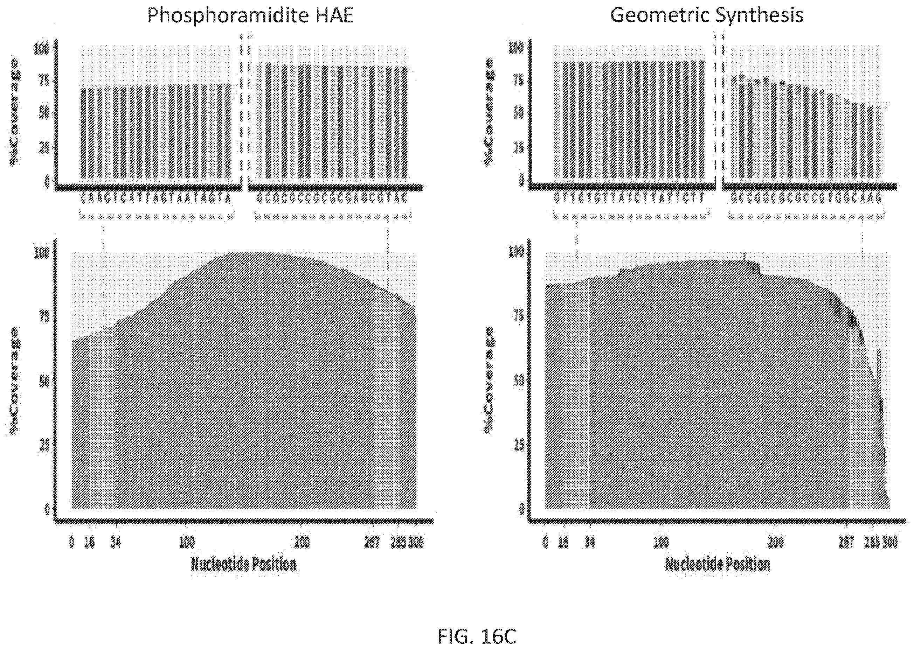

FIG. 16C is a series of graphs showing target sequence coverage in phosphoramidite HAE synthesis reactions and geometric synthesis reactions of the present disclosure. The sequences shown in FIG. 16A correspond to SEQ ID NOs: 176-179.

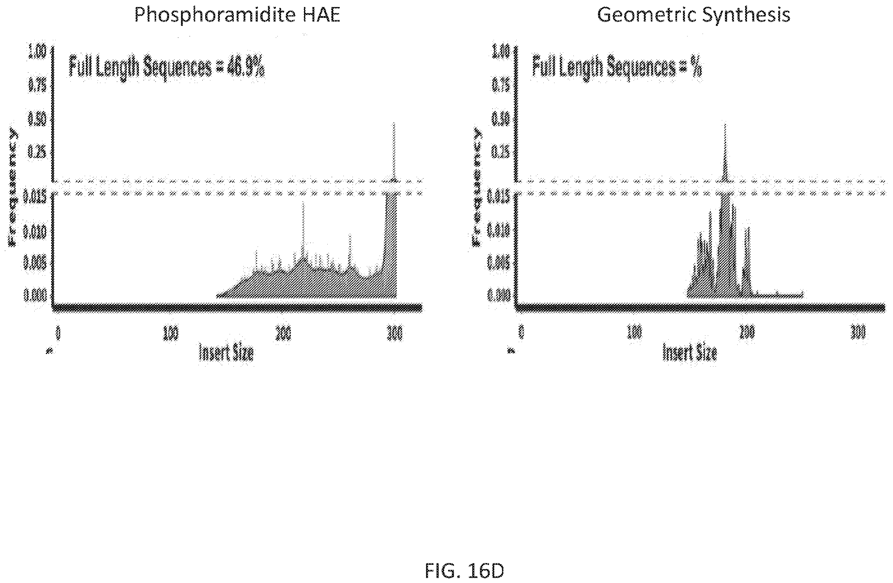

FIG. 16D is a series of graphs showing the distribution of product sizes in phosphoramidite HAE synthesis reactions and geometric synthesis reactions of the present disclosure.

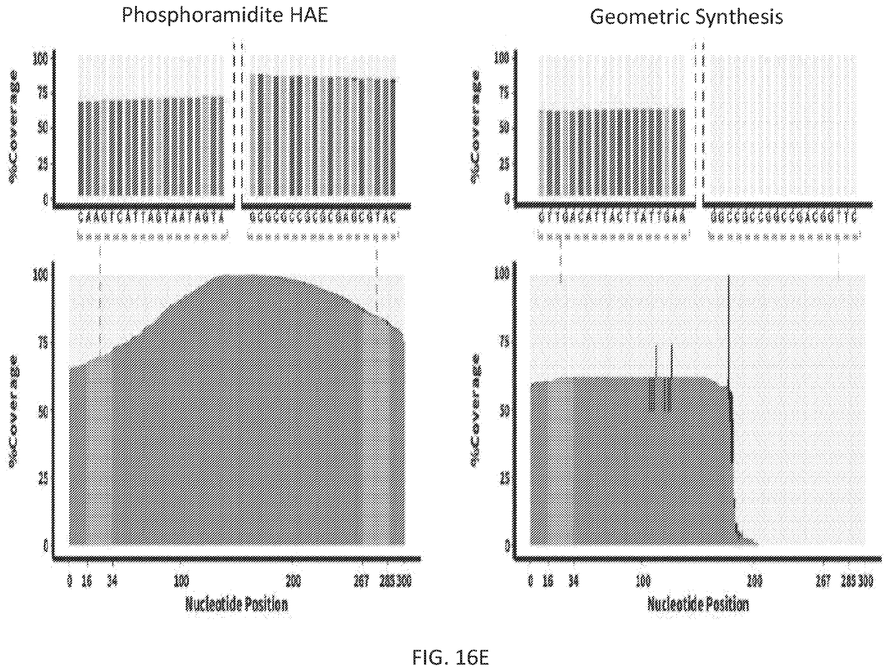

FIG. 16E is a series of graphs showing target sequence coverage in phosphoramidite HAE synthesis reactions and geometric synthesis reactions of the present disclosure. The sequences shown in FIG. 16E correspond to SEQ ID NOs: 180-183

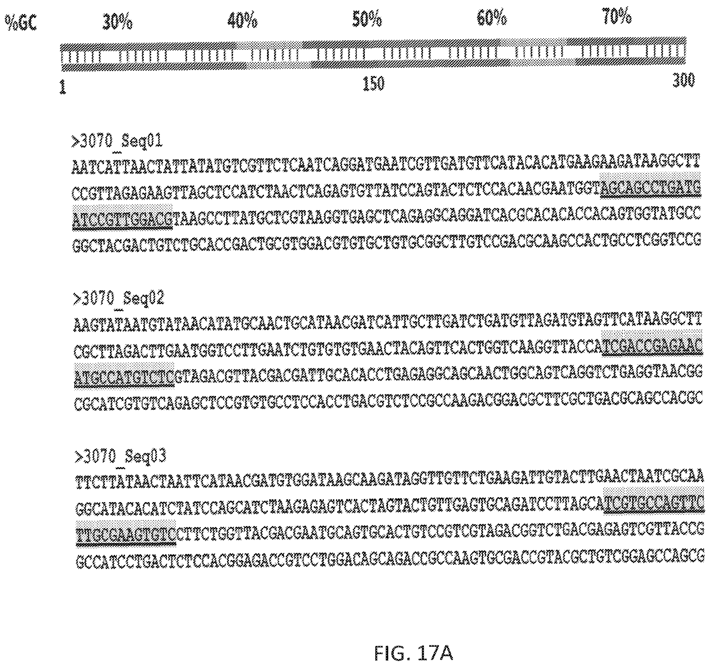

FIG. 17A shows a schematic diagram of a target nucleic acid (top), sequences of the target nucleic acids (right) and a series of graphs depicting GC content along the length of the length of the target nucleic acid as calculated by a sliding window of 50 nucleotides. The target nucleic acids were synthesized using either phosphoramidite synthesis or geometric synthesis methods of the present disclosure. The sequences shown in FIG. 17A correspond to SEQ ID NOs: 184-186.

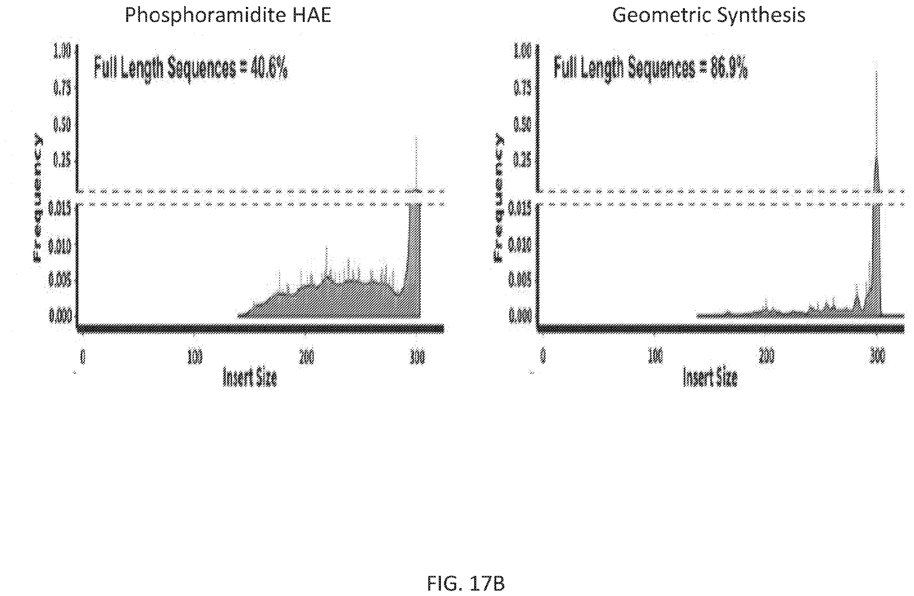

FIG. 17B is a series of graphs showing the distribution of product sizes in phosphoramidite HAE synthesis reactions and geometric synthesis reactions of the present disclosure.

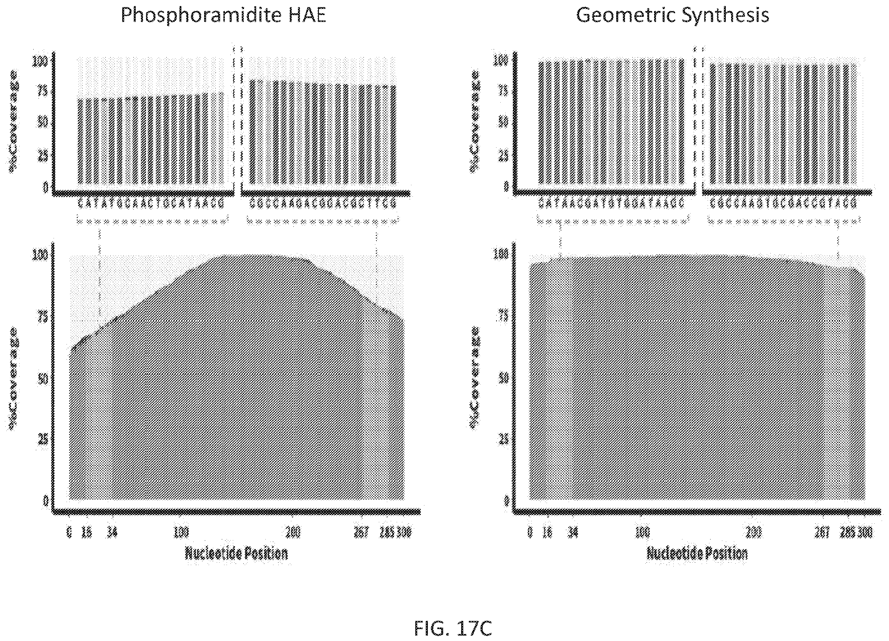

FIG. 1 7C is a series of graphs showing target sequence coverage in phosphoramidite HAE synthesis reactions and geometric synthesis reactions of the present disclosure. The sequences shown in FIG. 17C correspond to SEQ ID NOs: 187-190.

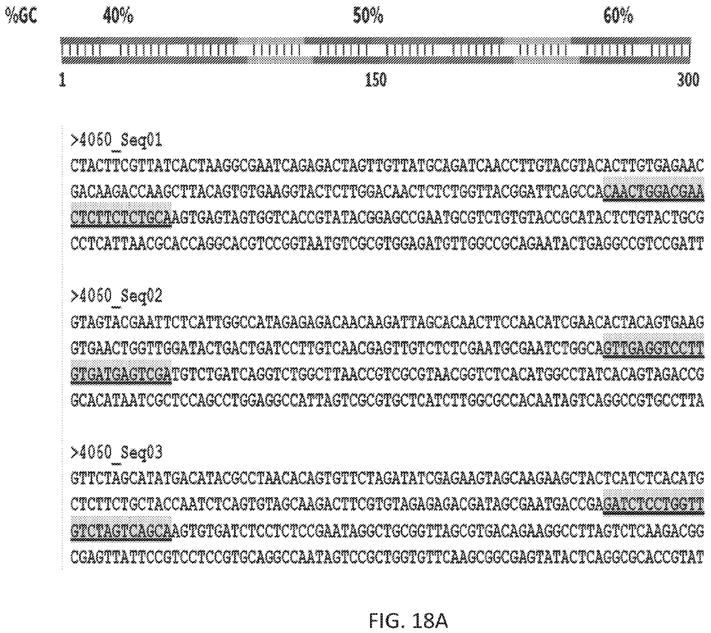

FIG. 18A shows a schematic diagram of a target nucleic acid (top), sequences of the target nucleic acids (right) and a series of graphs depicting GC content along the length of the length of the target nucleic acid as calculated by a sliding window of 50 nucleotides. The target nucleic acids were synthesized using either phosphoramidite synthesis or geometric synthesis methods of the present disclosure. The sequences shown in FIG. 18A correspond to SEQ ID NOs: 191-193.

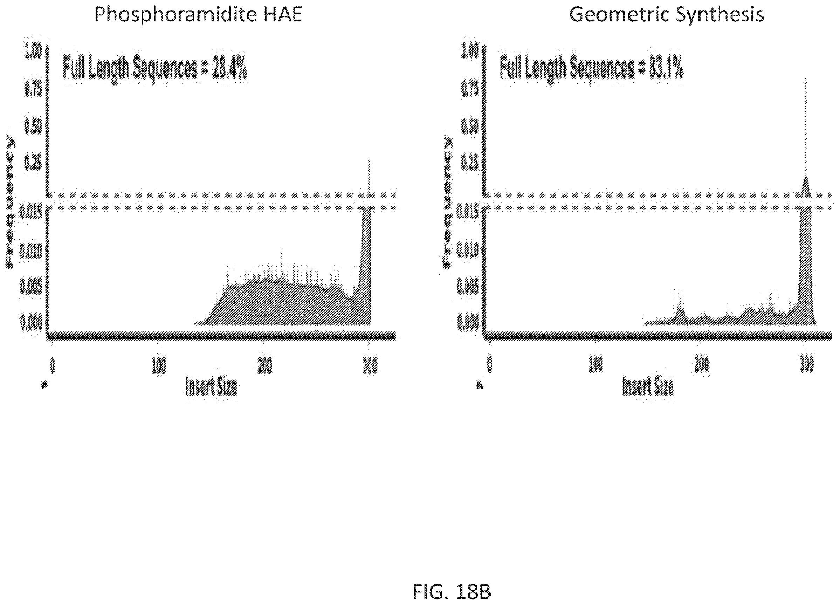

FIG. 18B is a series of graphs showing the distribution of product sizes in phosphoramidite HAE synthesis reactions and geometric synthesis reactions of the present disclosure.

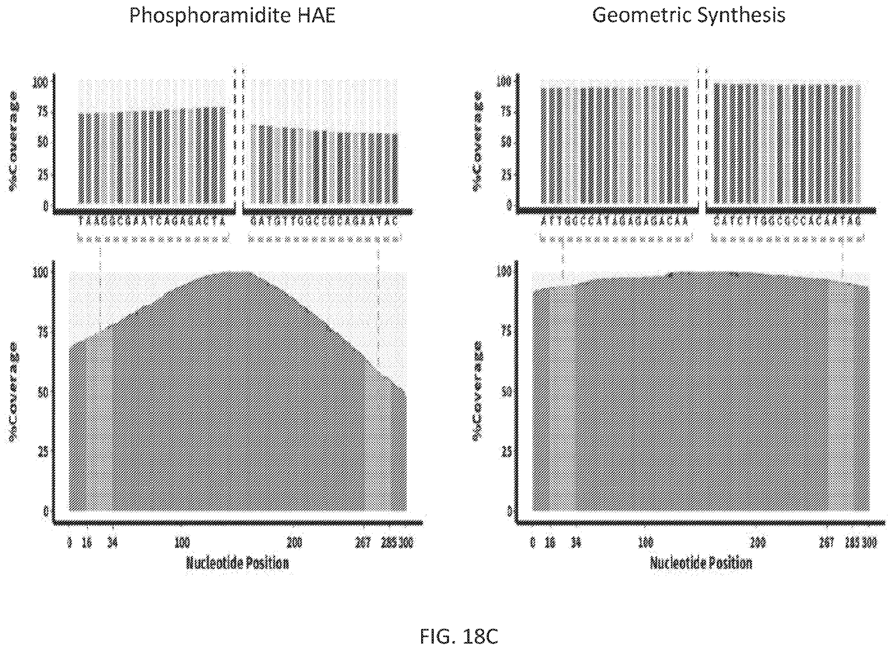

FIG 18C is a series of graphs showing target sequence coverage in phosphoramidite HAE synthesis reactions and geometric synthesis reactions of the present disclosure. The sequences shown in FIG. 18C correspond to SEQ ID NOs: 194-197,

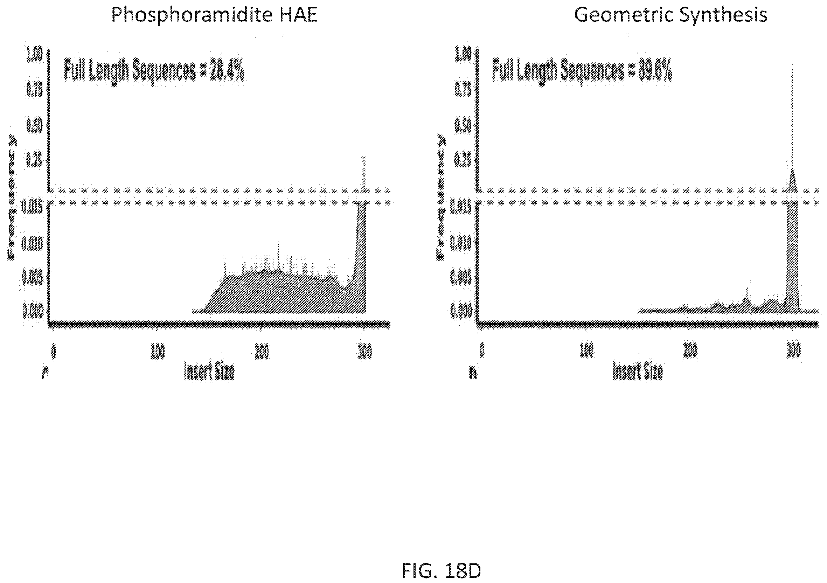

FIG. 18D is a series of graphs showing the distribution of product sizes in phosphoramidite HAE synthesis reactions and geometric synthesis reactions of the present disclosure.

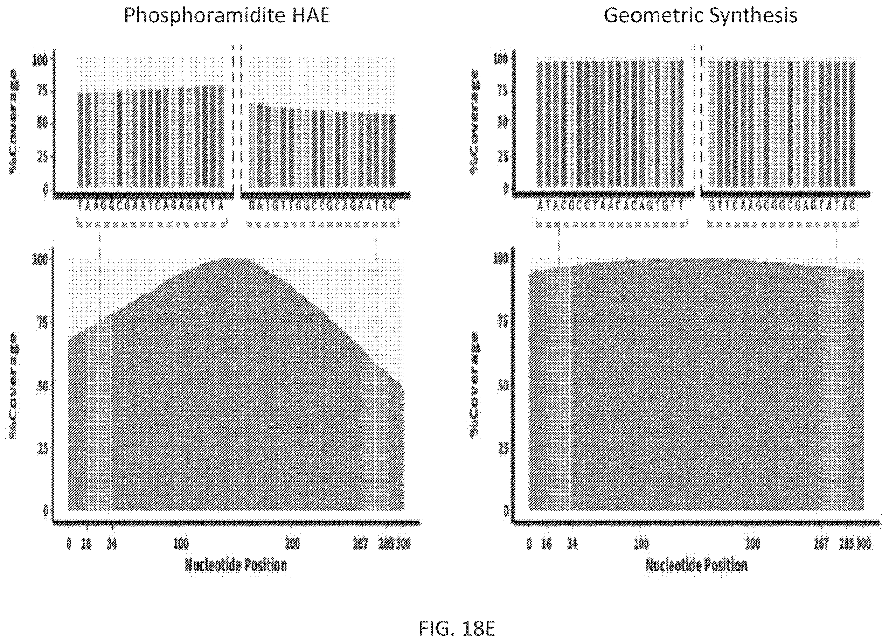

FIG. 18E is a series of graphs showing target sequence coverage in phosphoramidite HAE synthesis reactions and geometric synthesis reactions of the present disclosure. The sequences shown in FIG. 18E correspond to SEQ ID NOs: 198-201.

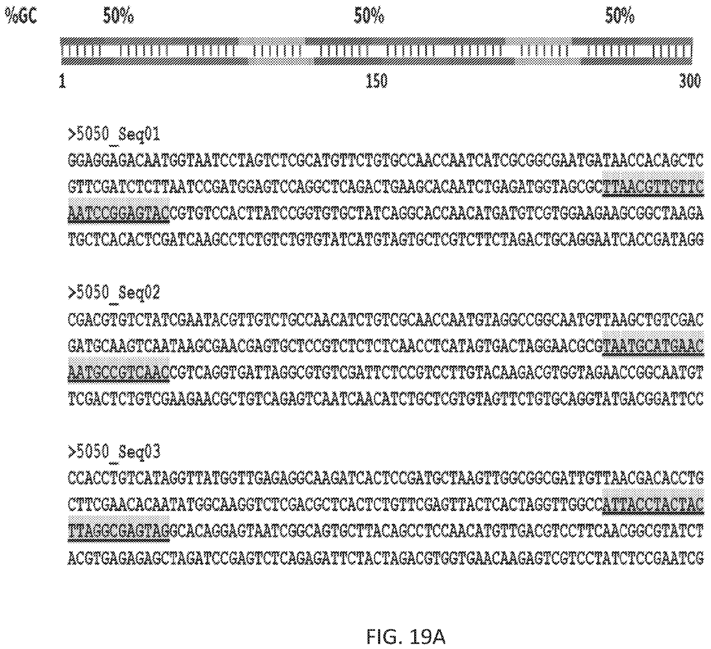

FIG. 19A shows a schematic diagram of a target nucleic acid (top), sequences of the target nucleic acids (right) and a series of graphs depicting GC content along the length of the length of the target nucleic acid as calculated by a sliding window of 50 nucleotides. The target nucleic acids were synthesized using either phosphoramidite synthesis or geometric synthesis methods of the present disclosure. The sequences shown in FIG. 19A correspond to SEQ ID NOs: 202-204.

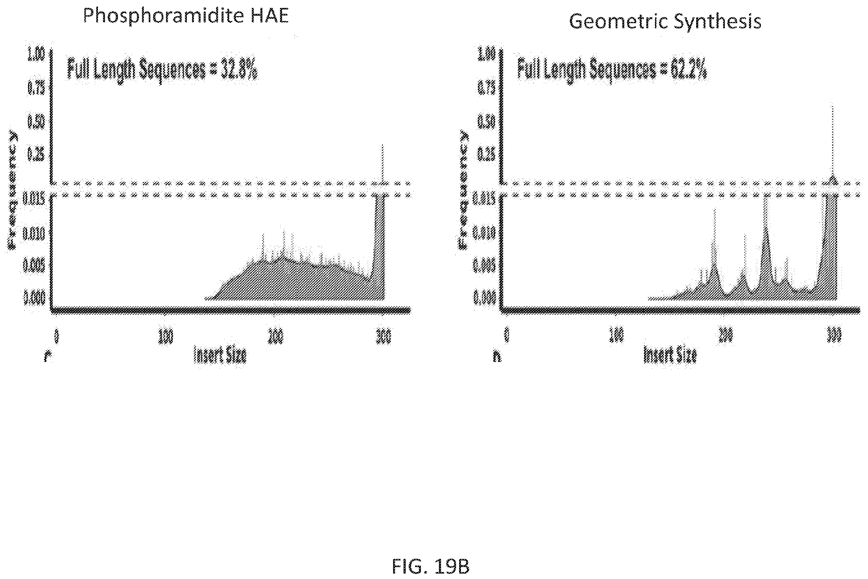

FIG. 19B is a series of graphs showing the distribution of product sizes in phosphoramidite HAE synthesis reactions and geometric synthesis reactions of the present disclosure.

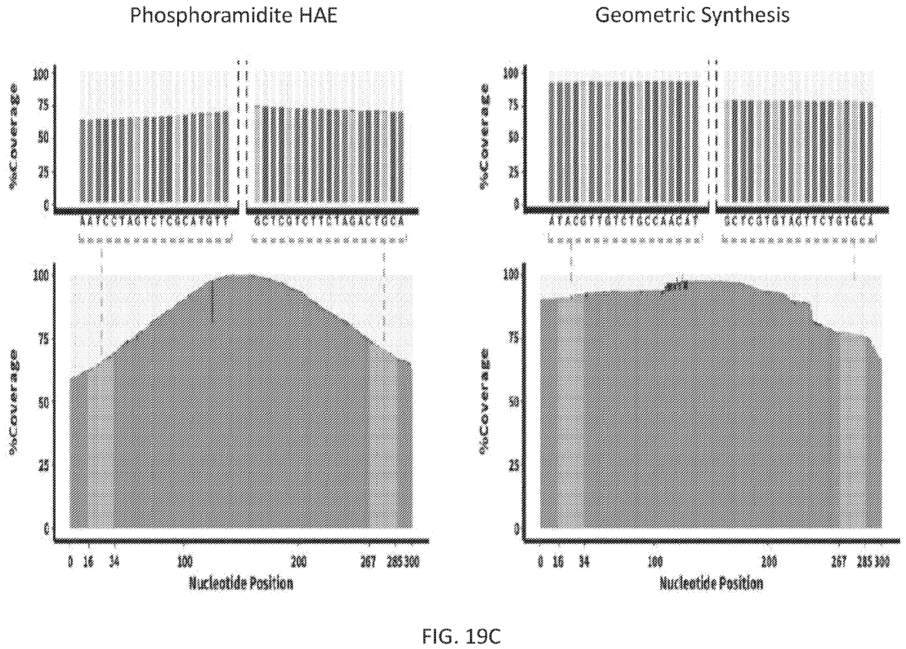

FIG. 19C is a series of graphs showing target sequence coverage in phosphoramidite HAE synthesis reactions and geometric synthesis reactions of the present disclosure. The sequences shown in FIG. 19C correspond to SEQ ID NOs: 205-208.

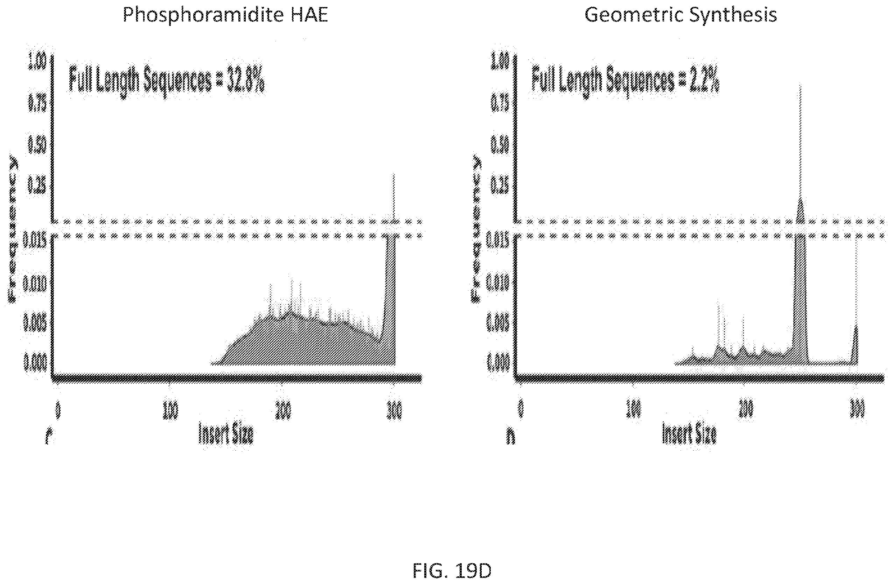

FIG. 19D is a series of graphs showing the distribution of product sizes in phosphoramidite HAE synthesis reactions and geometric synthesis reactions of the present disclosure.

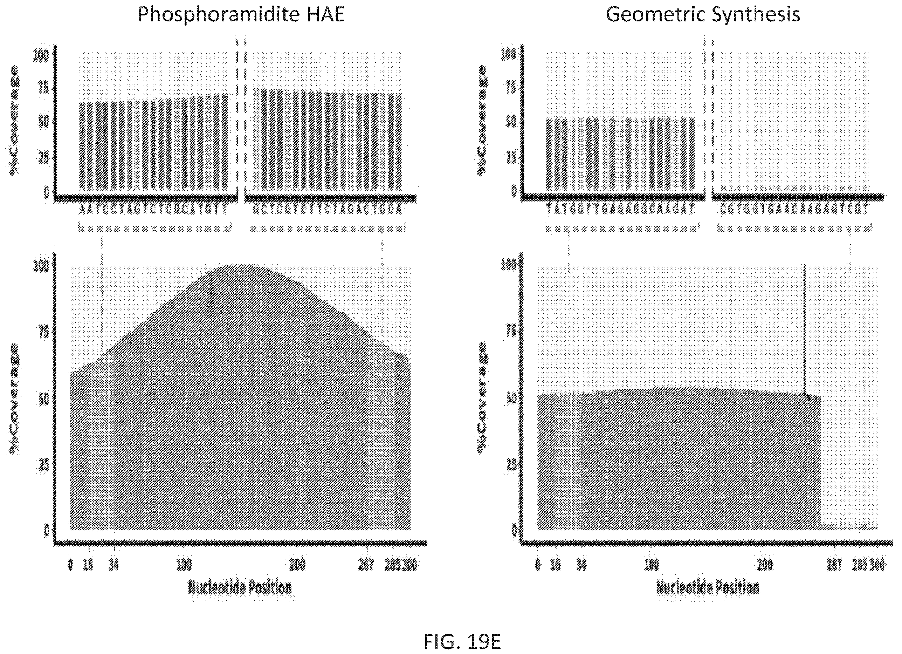

FIG. 19F is a series of graphs showing target sequence coverage in phosphoramidite HAE synthesis reactions and geometric synthesis reactions of the present disclosure. The sequences shown in FIG. 19E correspond to SEQ ID NOs: 209-212.

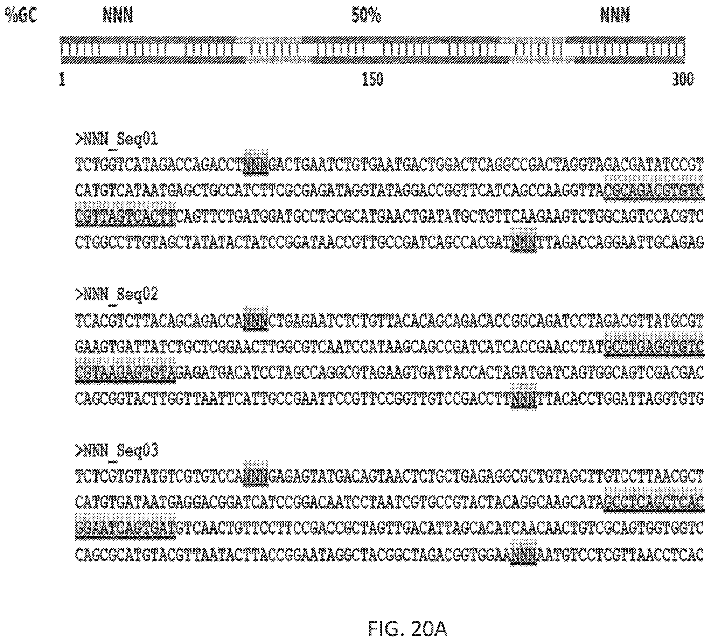

FIG. 20A shows a schematic diagram of a target nucleic acid (top), sequences of the target nucleic acids (right) and a series of graphs depicting GC content along the length of the length of the target nucleic acid as calculated by a sliding window of 50 nucleotides. The target nucleic acids were synthesized using either phosphoramidite synthesis or geometric synthesis methods of the present disclosure. The sequences shown in FIG. 20A correspond to SEQ ID NOs: 213-215.

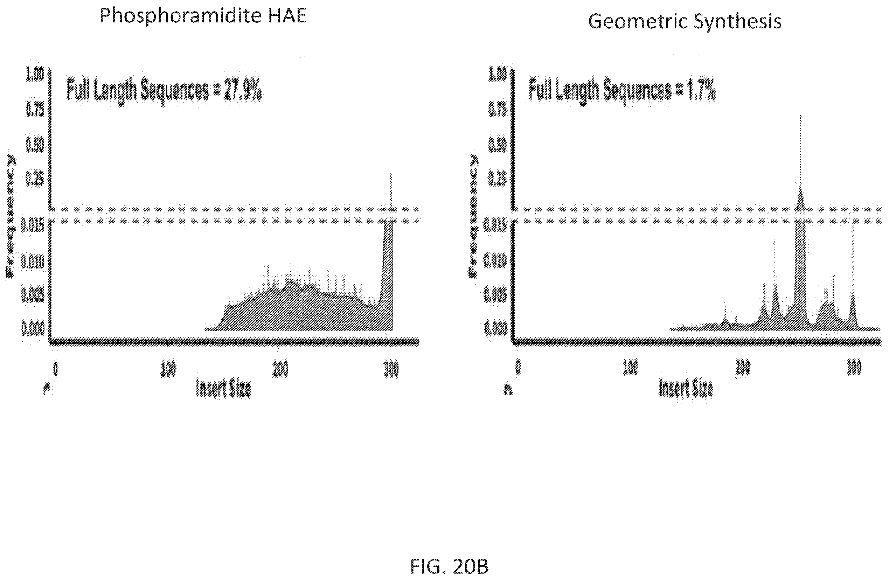

FIG. 20B is a series of graphs showing the distribution of product sizes in phosphoramidite HAE synthesis reactions and geometric synthesis reactions of the present disclosure.

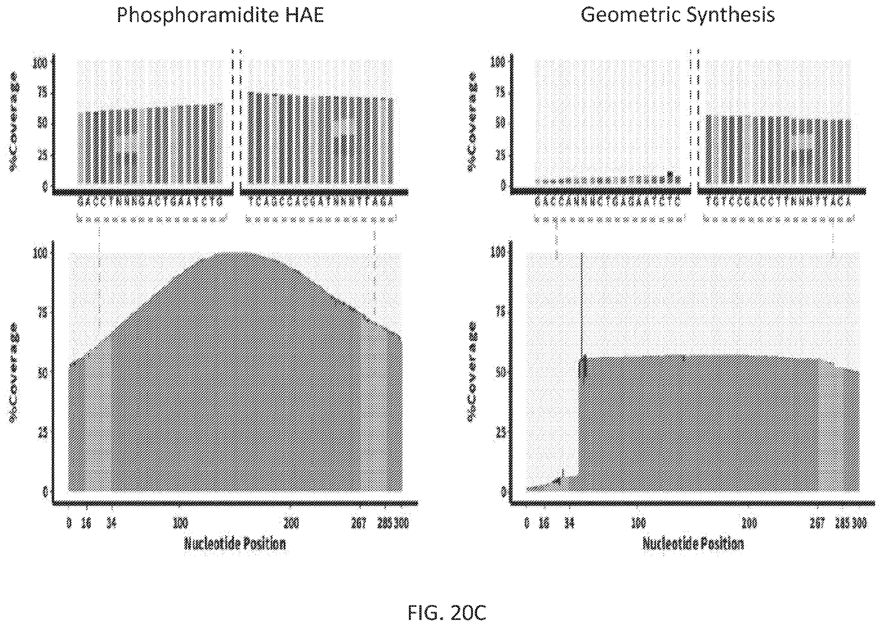

FIG. 20C is a series of graphs showing target sequence coverage in phosphoramidite HAE synthesis reactions and geometric synthesis reactions of the present disclosure. The sequences shown in FIG. 20C correspond to SEQ ID NOs: 216-219.

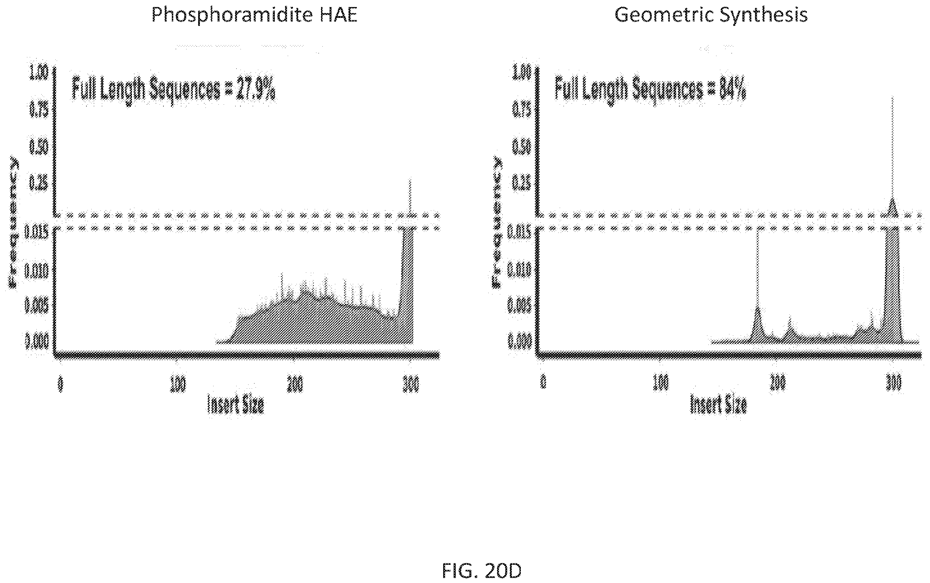

FIG. 20D is a series of graphs showing the distribution of product sizes in phosphoramidite HAE synthesis reactions and geometric synthesis reactions of the present disclosure.

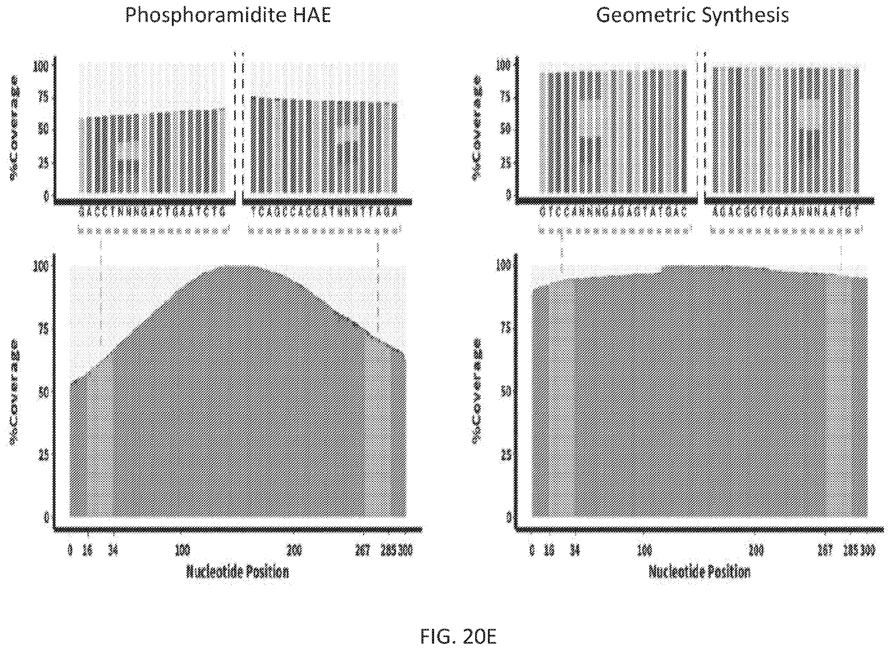

FIG. 20E is a series of graphs showing target sequence coverage in phosphoramidite HAE synthesis reactions and geometric synthesis reactions of the present disclosure. The sequences shown in FIG. 20E correspond to SEQ ID NOs: 220-223.

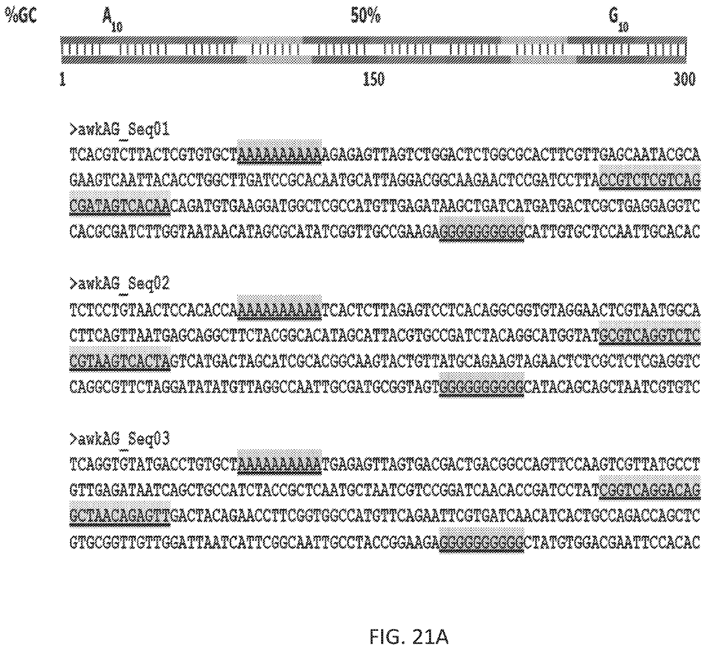

FIG. 21A shows a schematic diagram of a target nucleic acid (top), sequences of the target nucleic acids (right) and a series of graphs depicting GC content along the length of the length of the target nucleic acid as calculated by a sliding window of 50 tiucleotides. The target nucleic acids were synthesized using either phosphoramidite synthesis or geometric synthesis methods of the present disclosure. The sequences shown in FIG. 21A correspond to SEQ ID NOs: 224-226.

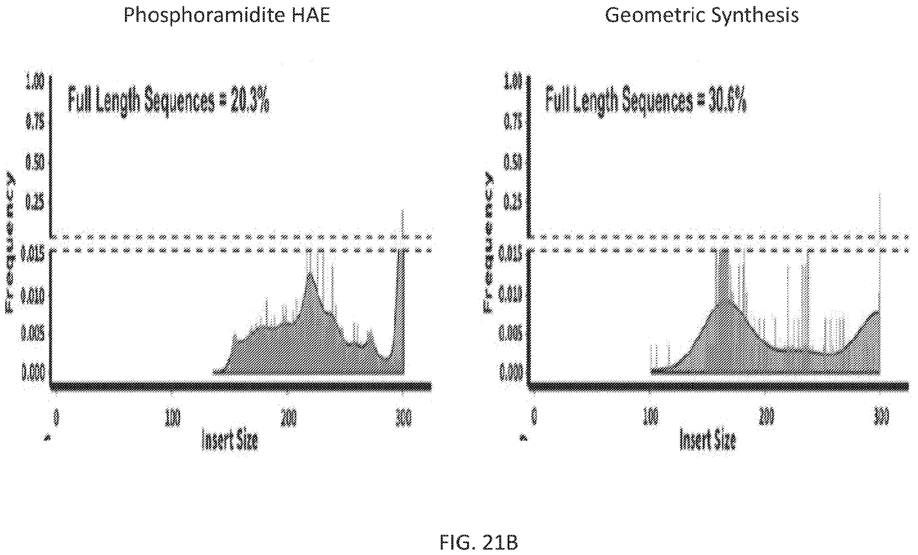

FIG. 21B is a series of graphs showing the distribution of product sizes in phosphoramidite HAE synthesis reactions and geometric synthesis reactions of the present disclosure.

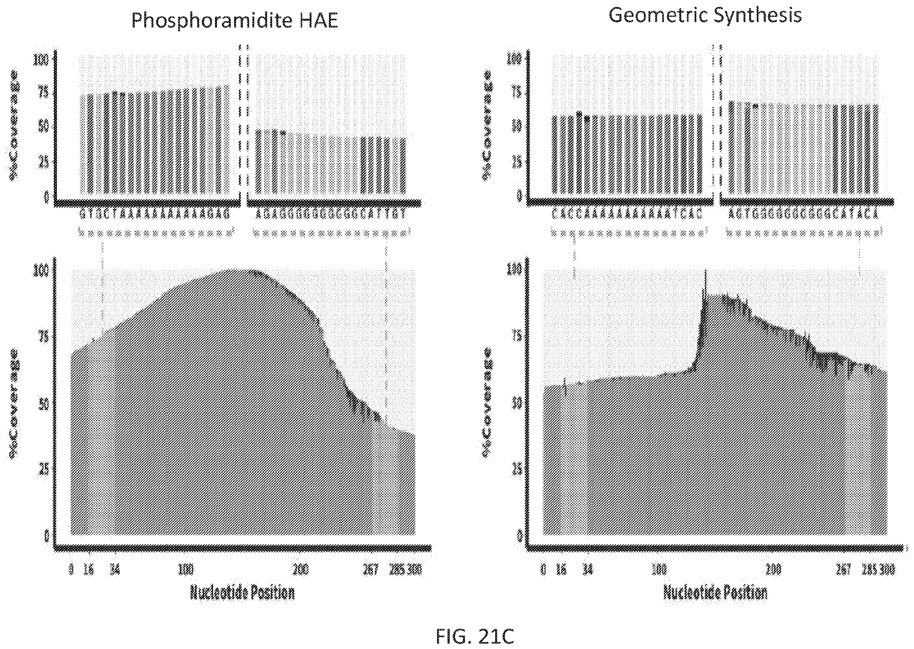

FIG. 21C is a series of graphs showing target sequence coverage in phosphoramidite HAE synthesis reactions and geometric synthesis reactions of the present disclosure. The sequences shown in FIG. 21C correspond to SEQ ID NOs: 227-230.

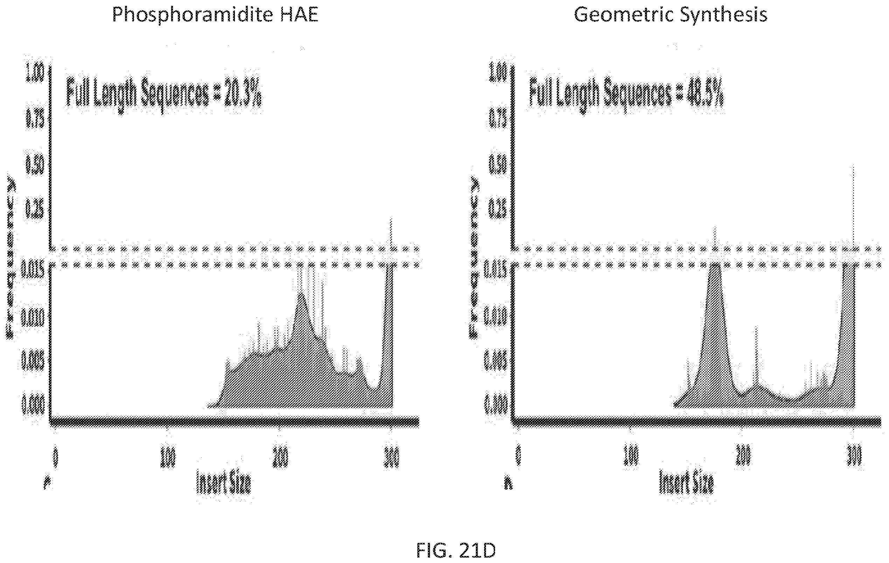

FIG. 21D is a series of graphs showing the distribution of product sizes in phosphoramidite HAE synthesis reactions and geometric synthesis reactions of the present disclosure.

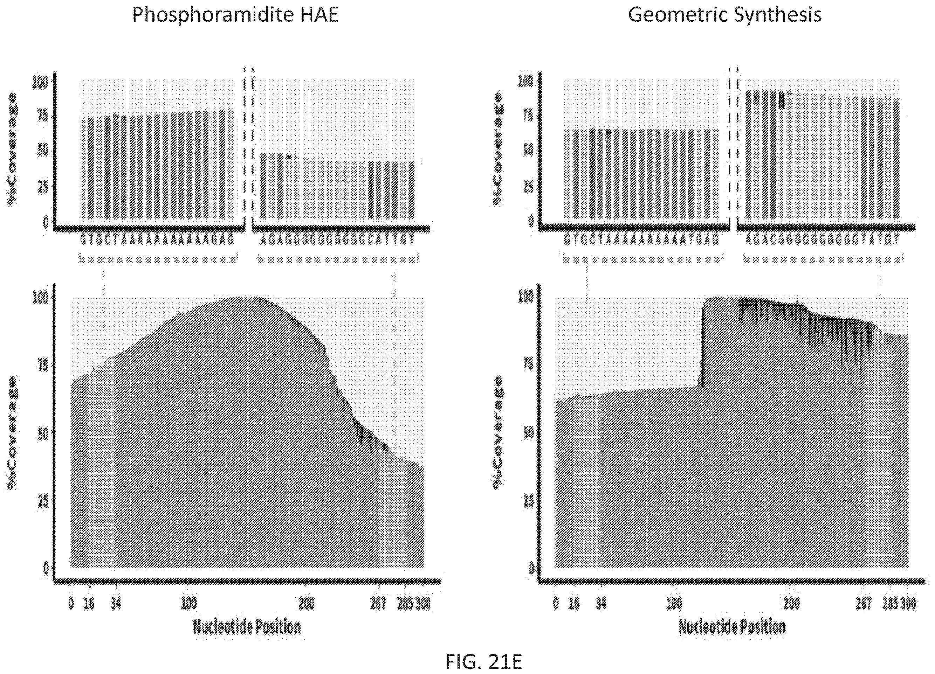

FIG. 21E is a series of graphs showing target sequence coverage in phosphoramidite HAE synthesis reactions and geometric synthesis reactions of the present disclosure. The sequences shown in FIG. 21E correspond to SEQ ID NOs: 231-234.

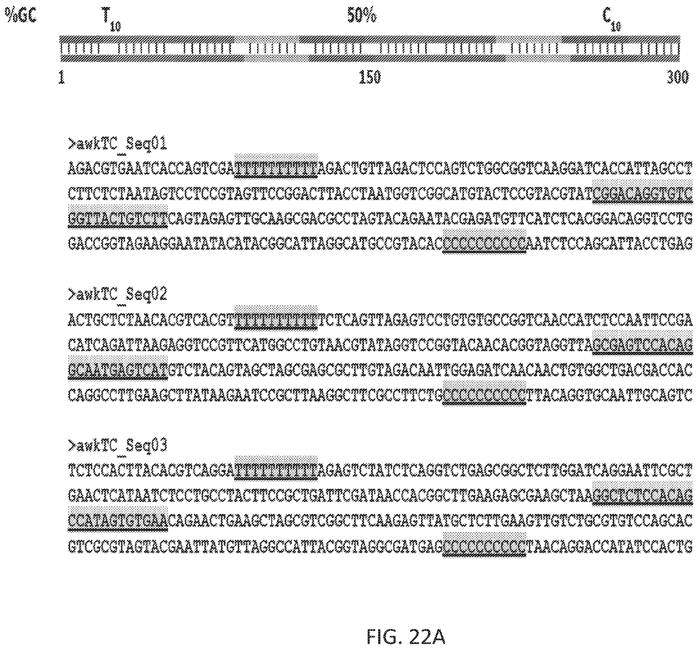

FIG. 22A shows a schematic diagram of a target nucleic acid (top), sequences of the target nucleic acids (right) and a series of graphs depicting GC content along the length of the length of the target nucleic acid as calculated by a sliding window of 50 nucleotides. The target nucleic acids were synthesized using either phosphoramidite synthesis or geometric synthesis methods of the present disclosure. The sequences shown in FIG. 22A correspond to SEQ ID NOs: 235-237.

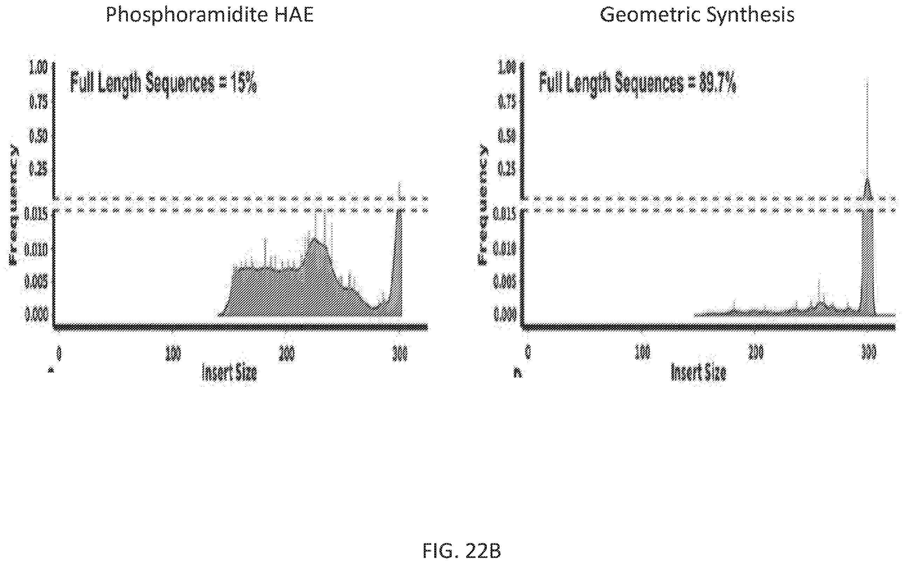

FIG. 22B is a series of graphs showing the distribution of product sizes in phosphoramidite HAE synthesis reactions and geometric synthesis reactions of the present disclosure.

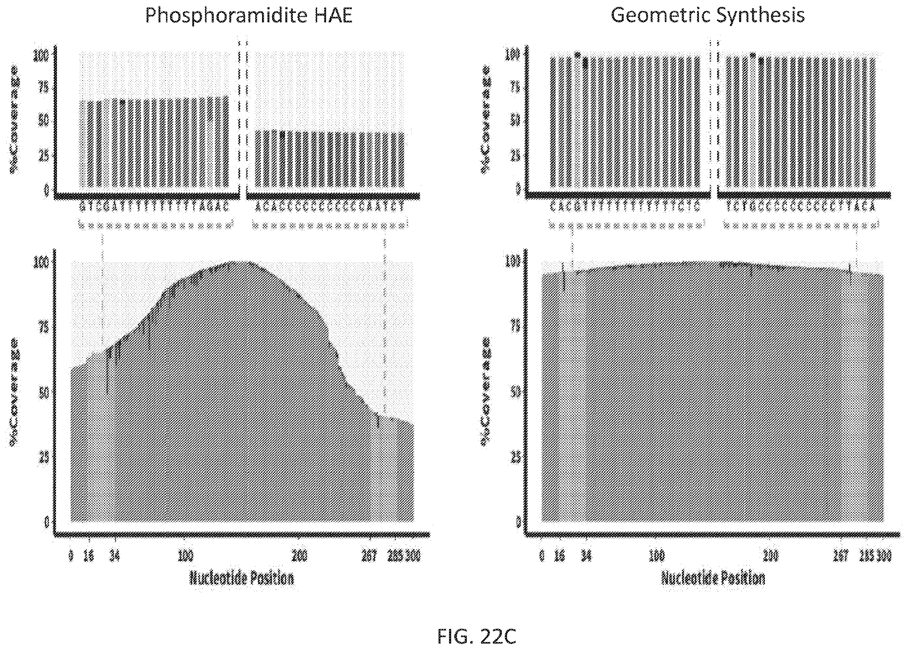

FIG. 22C is a series of graphs showing target sequence coverage in phosphoramidite HAE synthesis reactions and geometric synthesis reactions of the present disclosure. The sequences shown in FIG. 22C correspond to SEQ ID NOs: 238-241.

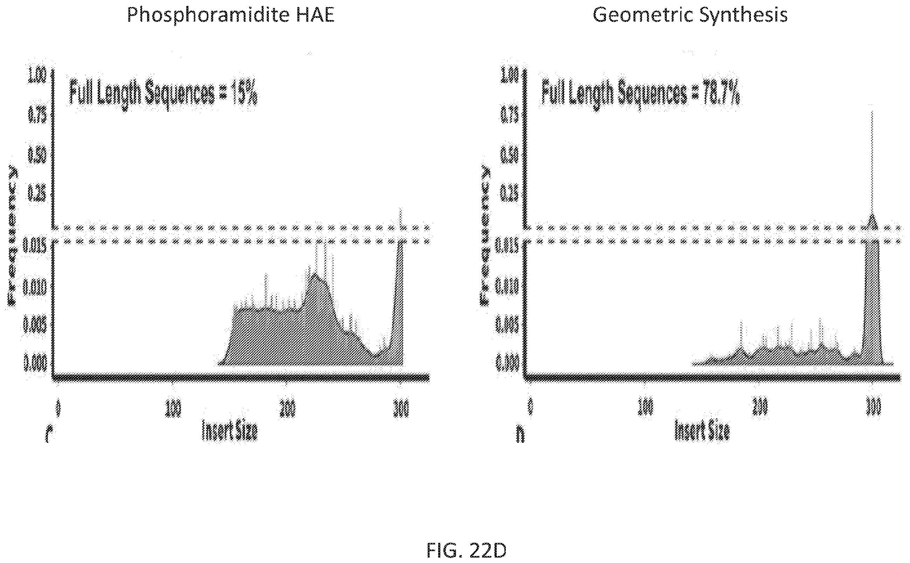

FIG. 22D is a series of graphs showing the distribution of product sizes in phosphoramidite HAE synthesis reactions and geometric synthesis reactions of the present disclosure.

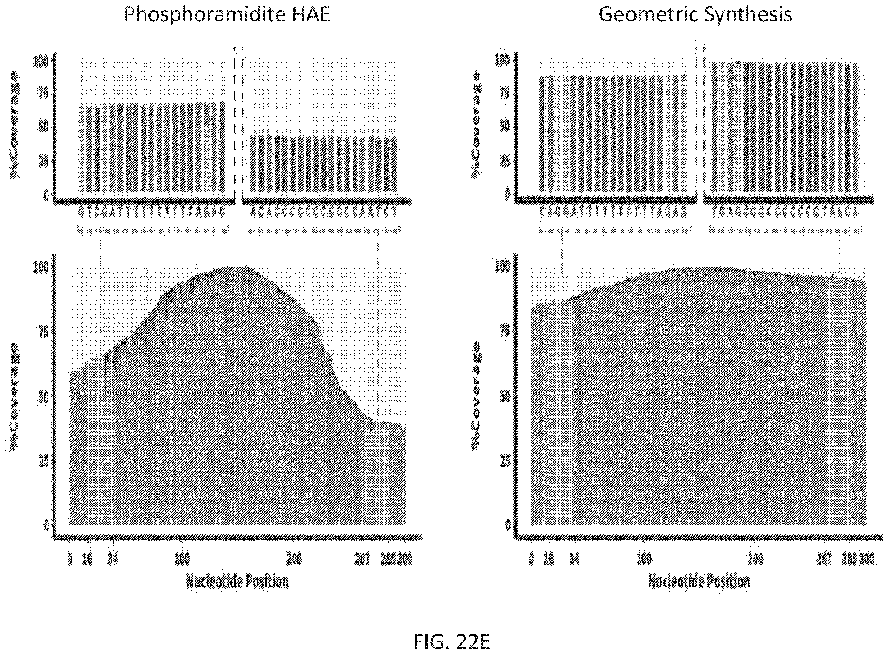

FIG. 22E is a series of graphs showing target sequence coverage in phosphoramidite HAE synthesis reactions and geometric synthesis reactions of the present disclosure. The sequences shown in FIG. 22E correspond to SEQ ID NOs: 242-245.

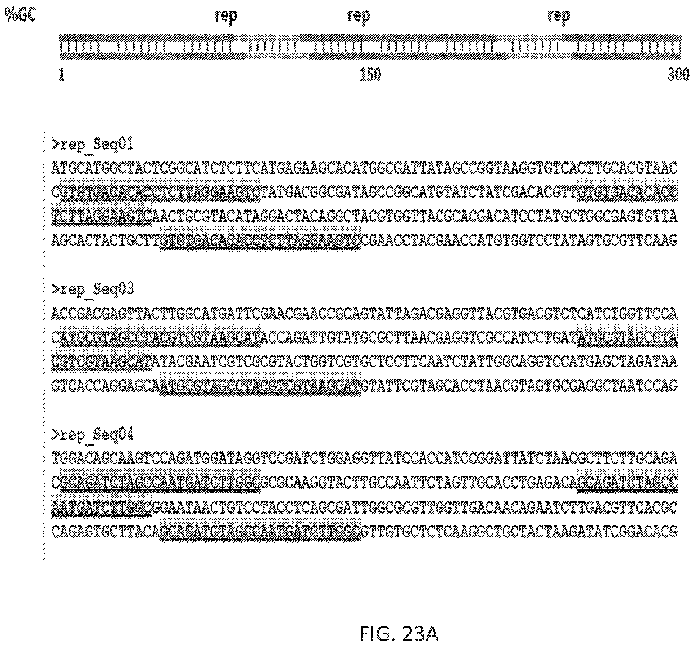

FIG. 23A shows a schematic diagram of a target nucleic acid (top), sequences of the target nucleic acids (right) and a series of graphs depicting GC content along the length of the length of the target nucleic acid as calculated by a sliding window of 50 nucleotides. The target nucleic acids were synthesized using either phosphoramidite synthesis or geometric synthesis methods of the present disclosure. The sequences shown in FIG. 23A correspond to SEQ ID NOs: 246-248.

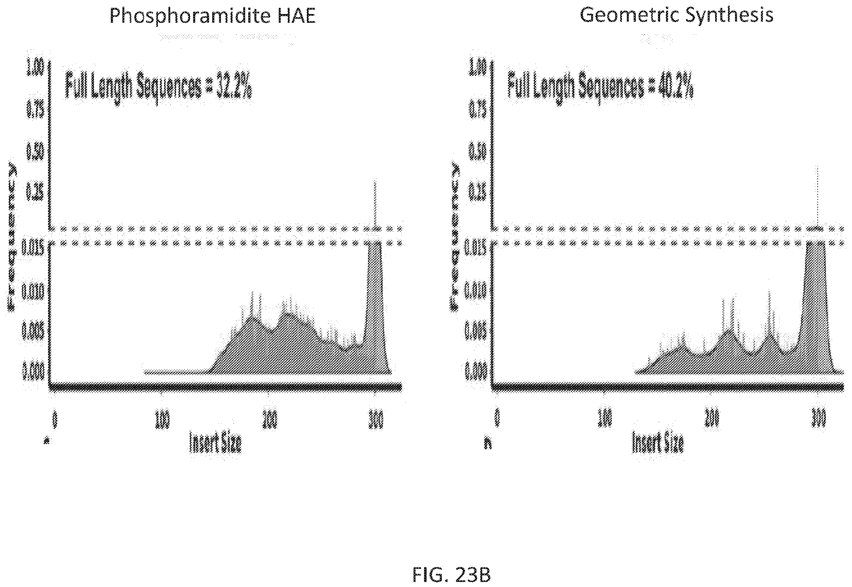

FIG. 23B is a series of graphs showing the distribution of product sizes in phosphoramidite HAE synthesis reactions and geometric synthesis reactions of the present disclosure.

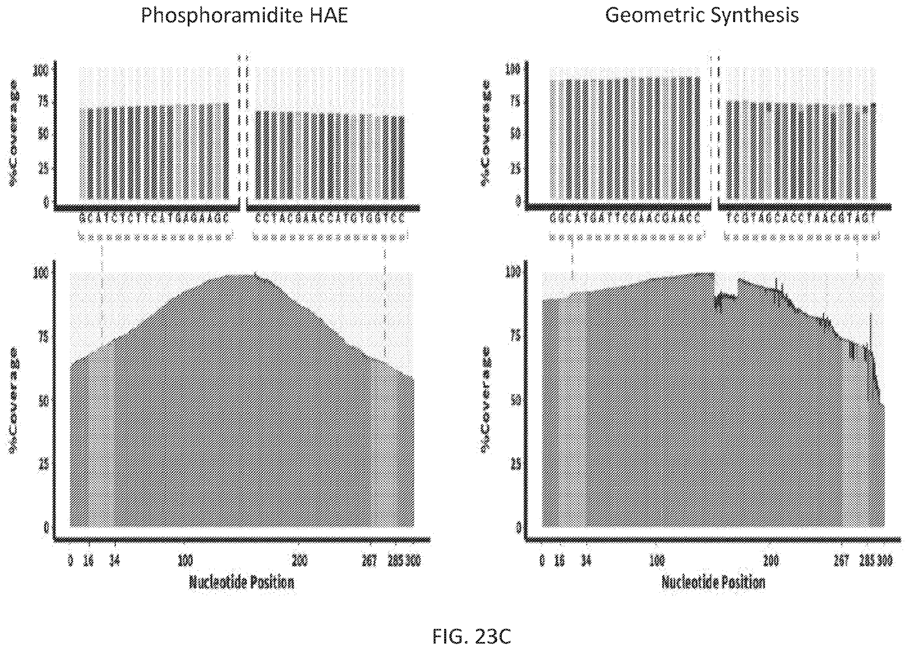

FIG 23C is a series of graphs showing target sequence coverage in phosphoramidite HAE synthesis reactions and geometric synthesis reactions of the present disclosure. The sequences shown in FIG. 23C correspond to SEQ ID NOs: 249-252.

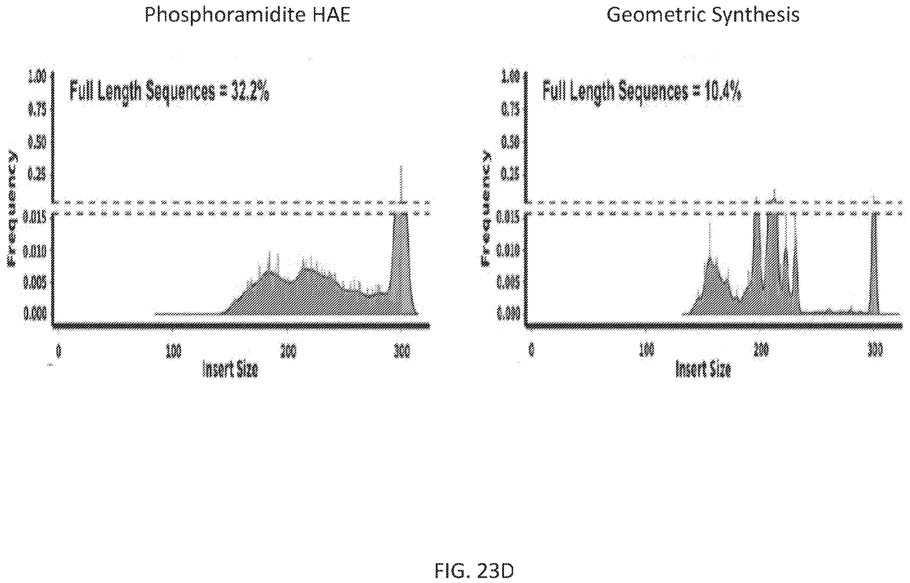

FIG. 23D is a series of graphs showing the distribution of product sizes in phosphoramidite HAE synthesis reactions and geometric synthesis reactions of the present disclosure.

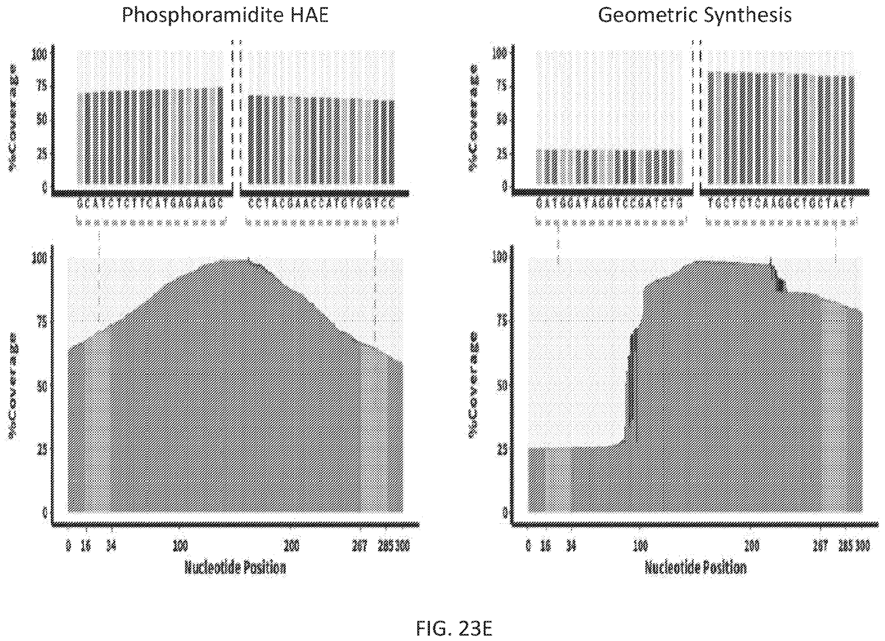

FIG. 23E is a series of graphs showing target sequence coverage in phosphoramidite HAE synthesis reactions and geometric synthesis reactions of the present disclosure. The sequences shown in FIG. 23E correspond to SEQ ID NOs: 253-256.

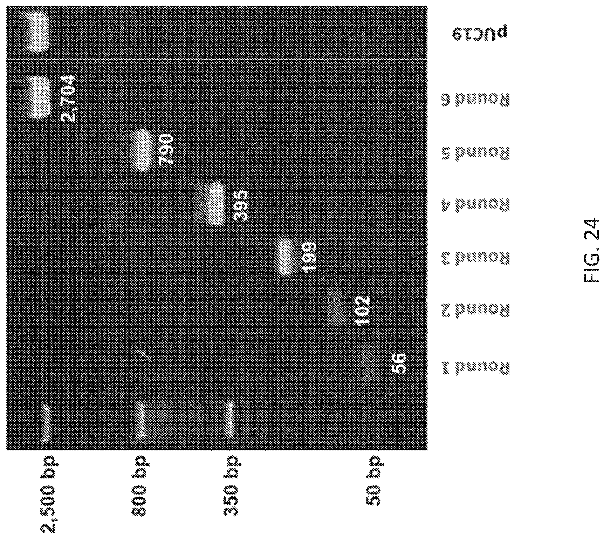

FIG. 24 shows the results of agarose gel analysis of the products of sequential rounds of a double-stranded geometric synthesis reaction for the synthesis of the pUC19 plasmid.

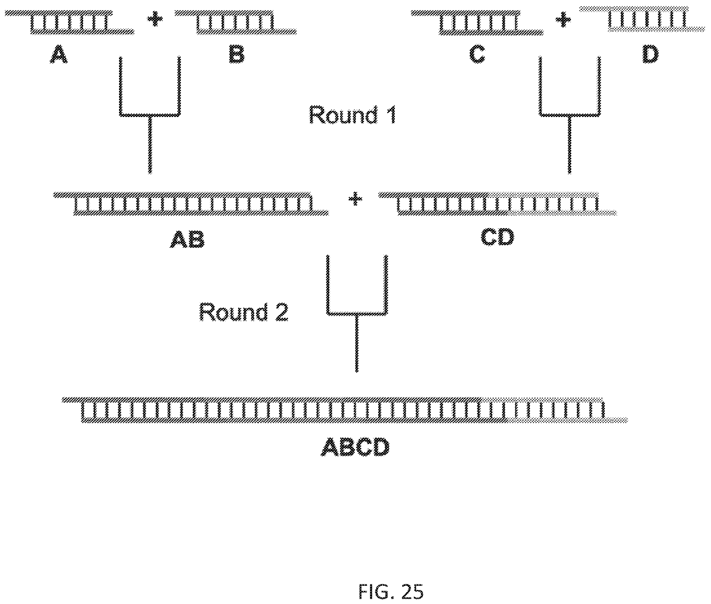

FIG. 25 shows a schematic of two rounds of ligation in a double-stranded geometric assembly reaction of the present disclosure.

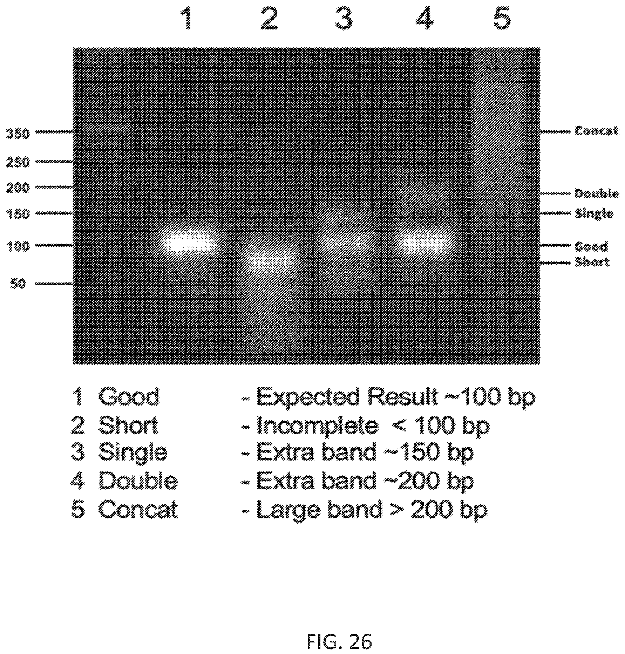

FIG. 26 is an exemplary agarose gel analysis of the products of the two rounds of ligation shown in FIG. 25.

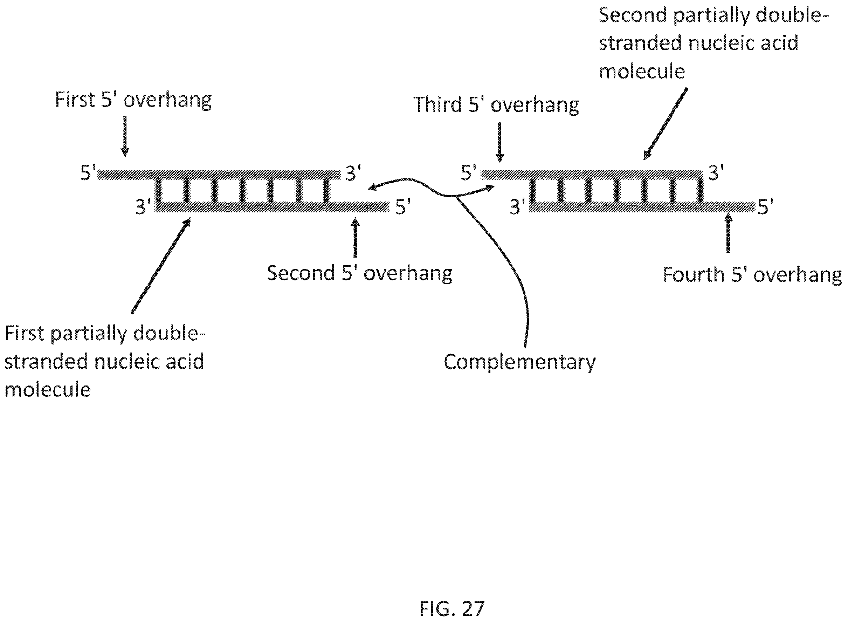

FIG. 27 is a schematic of a composition of the present disclosure comprising two partially double-stranded nucleic acid molecules.

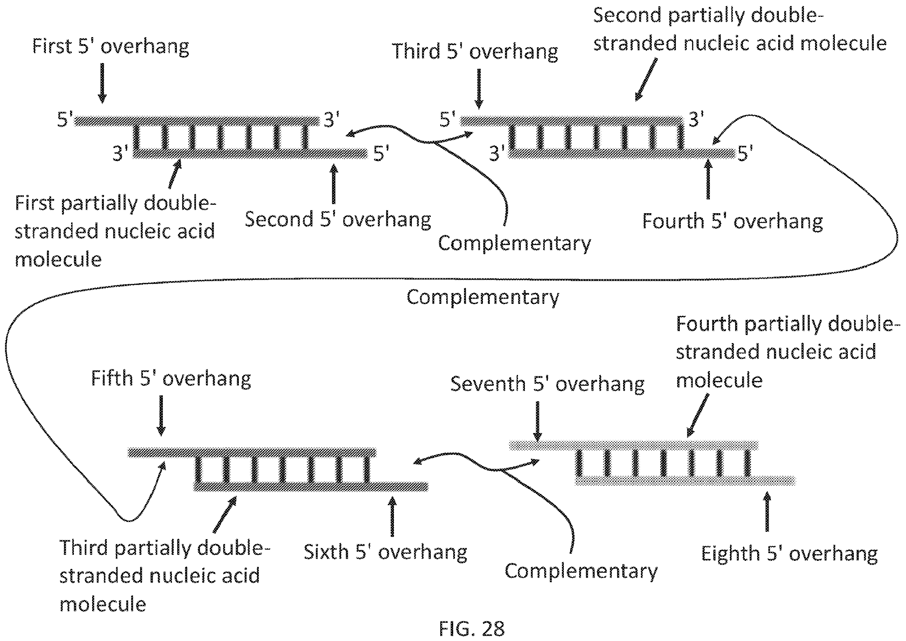

FIG. 28 is a schematic of a composition of the present disclosure comprising four partially double-stranded nucleic acid molecules.

### DETAILED DESCRIPTION

The present disclosure provides a DNA assembly methodology entitled "double-stranded geometric synthesis (gSynth)" and compositions related thereto for the synthesis of long, arbitrary double-stranded nucleic acid sequences. In a double-stranded gSynth assembly reaction, the target sequence (*i.e.* the sequence that is to be synthesized) is computationally broken into a sets of adjacent, double-stranded nucleic acid fragments, These adjacent double-stranded nucleic acid fragments are then ligated together in one-pair at-a-time ligation reactions in a systematic assembly method. These fragments possess 3' and/or 5' overhanging single-stranded N-mer sites, with three key properties. 1) The N-mer sites are not self-hybridizing or self-reactive in ligation reactions. 2) The N-mer site at one end of the fragment does not cross-hybridize or cross-react with the N-mer site at the other end. Finally, 3) there is one N-mer site on each fragment of an adjacent pair of fragments in that will hybridize and ligate with the adjacent fragment in a ligation reaction leading to a new, longer double-stranded fragment. The present disclosure provides preferred N-mer sites that facilitate more efficient and accurate ligation reactions, thereby allowing the double-stranded gSynth methods of the present disclosure to be used to synthesize nucleic acid sequences of unprecedented lengths that are not achievable using existing nucleic acid assembly and synthesis techniques. The double-stranded fragments of the present disclosure can be generated using conventional phosphoramidite chemical synthesis, single-stranded geometric synthesis (WO2019140353A1), or conventional molecular cloning, for example from a restriction digest of a plasmid.

FIGs. 1A-1F illustrate a non-limiting example of a double-stranded gSynth assembly reaction. FIG. 1A shows a target sequence (entitled "5050Seq03") that is to be synthesized using the double-stranded gSynth methods of the present disclosure. Parts of the sequence that are in bold and underlined correspond to 4-mer overhangs that have been selected, thus defining the fragments that will be used to synthesize the entire sequence. FIG. 1B shows the individual double-stranded nucleic acid fragments of the sequence shown in FIG. 1 A that will be used in the double-stranded gSynth methods of the present disclosure to construct 5050Scq03. FIG. 1D is a schematic of the first round of ligations in the double-stranded gSynth method to synthesize the sequence shown in FIG. 1A. In the first ligation round, Fragments 1 and 2, Fragments 3 and 4, Fragments 5 and 6, Fragments 7 and 8, Fragments 9 and 10, Fragments 11 and 12, and Fragments 13 and 14 are hybridized via their complementary 5' overhangs and then ligated together to create Fragment 1+2, Fragment 3+4, Fragment 5+6, Fragment 7+8, Fragment 9+10, Fragment 11+12, and Fragment 13+14. FIG. 1E is a schematic of the second round of ligations in the double-stranded gSynth method to synthesize the sequence shown in FIG. 1A. In the second ligation round, Fragments 1+2 and 3+4, Fragments 5-1--6 and 7+8, and Fragments 11+12 and 13+14 are hybridized via their complementary 5' overhangs and then ligated together to create Fragment 1+2+3+4, Fragment 5+6+7+8, and Fragment 11 +12+13+14. FIG. 1F is a schematic of the third round of ligations in the double-stranded gSynth method to synthesize the sequence shown in FIG. 1A. In the third ligation round, Fragments 1+2+3+4 and 5+6+7+8, and Fragments 9+10 and 11+12+13+14 are hybridized via their complementary 5' overhangs and then ligated together to create Fragment 1+2+3+4+5+6+7+8 and Fragment 9+10+11+12+13+14. FIG. 1G is a schematic of the fourth and final round of ligations in the double-stranded gSynth method to synthesize the sequence shown in FIG. 1A. In the fourth ligation round, Fragments 1+2+3+4+5+6+7+8 and 9+10+11+12+13+14 are hybridized via their complementary 5' overhangs and ligated together, thereby producing the sequence shown in FIG. 1A.

### Compositions of the Present Disclosure

The present disclosure provides compositions comprising a first partially double-stranded nucleic acid molecule and an at least second partially double-stranded nucleic acid molecule, wherein the first partially double-stranded nucleic acid molecule comprises a first 5' overhang and a second 5' overhang, wherein the at least second partially double-stranded nucleic acid molecule comprises a third 5' overhang and fourth 5' overhang, wherein the second 5' overhang and third 5' overhang are complementary to each other, wherein the first 5' overhang, the third 5' overhang and the fourth 5' overhang each comprise one of the 4-mer sequences, or complement thereof, of a 4-mer triplet,wherein the 4-mer triplet comprises three 4-mer sequences, which yield a single fragment with at least 90% purity upon ligation of the first partially double-stranded nucleic acid molecule and the at least second partially double-stranded nucleic acid molecule, and wherein the first 5' overhang, the third 5' overhang and the fourth 5' overhang comprise a different 4-mer sequence. A schematic of a representative composition of the present disclosure is shown in FIG. 27. In some aspects, the 4-mer triplet can be selected from the 4-mer triplets recited in Table 1.

The present disclosure provides compositions comprising a first partially double-stranded nucleic acid molecule and an at least second partially double-stranded nucleic acid molecule, wherein the first partially double-stranded nucleic acid molecule comprises a first 5' overhang and a second 5' overhang, wherein the at least second partially double-stranded nucleic acid molecule comprises a third 5' overhang and fourth 5' overhang, wherein the second 5' overhang and third 5' overhang are complementary to each other, wherein the first 5' overhang, the third 5' overhang and the fourth 5' overhang each comprise one of the 4-mer sequences, or complement thereof, of a 4-mer triplet,wherein the 4-mer triplet comprises three 4-mer sequences, which yield a single fragment upon ligation of the first partially double-stranded nucleic acid molecule and the at least second partially double-stranded nucleic acid molecule, and wherein the first 5' overhang, the third 5' overhang and the fourth 5' overhang comprise a different 4-mer sequence. A schematic of a representative composition of the present disclosure is shown in FIG. 27. In some aspects, the 4-mer triplet can be selected from the 4-mer triplets recited in Table 1.

The present disclosure provides compositions comprising a first partially double-stranded nucleic acid molecule and an at least second partially double-stranded nucleic acid molecule, wherein the first partially double-stranded nucleic acid molecule comprises a first 5' overhang and a second 5' overhang, wherein the at least second partially double-stranded nucleic acid molecule comprises a third 5' overhang and fourth 5' overhang, wherein the second 5' overhang and third 5' overhang are complementary to each other, wherein the first 5' overhang, the third 5' overhang and the fourth 5' overhang each comprise one of the 4-mer sequences, or complement thereof, of a 4-mer triplet, wherein the 4-mer triplet is selected from the 4-mer triplets recited in Table 1, and wherein the first 5' overhang, the third 5' overhang and the fourth 5' overhang comprise a different 4-mer sequence. A schematic of a representative composition of the present disclosure is shown in FIG. 27.

**Table 1. 4-mer triplets**

| | **4-mer #1 of triplet** | **4-mer #2 of triplet** | **4-mer #3 of triplet** |
|---|---|---|---|
| **4-mer Triplet #1** | AAAA | CACC | CGCC |
| **4-mer Triplet #2** | AAAA | GACA | CGCC |
| **4-mer Triplet #3** | AAAC | AAGG | TCAC |
| **4-mer Triplet #4** | AAAC | TGAC | GTAG |
| **4-mer Triplet #5** | AACG | CACC | CGCC |
| **4-mer Triplet #6** | AACG | GACA | CGCC |
| **4-mer Triplet #7** | ACCG | CCGA | GAGG |
| **4-mer Triplet #8** | ACGC | CGTT | CTGG |
| **4-mer Triplet #9** | ACGC | CTGG | CGCA |
| **4-mer Triplet #10** | AGCC | CACC | GCAA |
| **4-mer Triplet #11** | AGCC | GACA | GCAA |
| **4-mer Triplet #12** | AGCC | GCAA | TCCC |
| **4-mer Triplet #13** | AGTT | TGAT | TGTG |
| **4-mer Triplet #14** | ATCC | ACCG | GAGG |
| **4-mer Triplet #15** | ATCC | ATGC | AAGG |
| **4-mer Triplet #16** | ATCC | TACC | ACCG |
| **4-mer Triplet #17** | ATGT | TTGA | GGTC |
| **4-mer Triplet #18** | CAAC | TGAT | TGAC |
| **4-mer Triplet #19** | CAAC | TTTT | TGAT |
| **4-mer Triplet #20** | CGAG | AACA | AGTT |
| **4-mer Triplet #21** | CGAG | AGTT | TGTG |
| **4-mer Triplet #22** | CGGT | TTGC | ATCC |
| **4-mer Triplet #23** | CGTT | CTGG | CGCA |
| **4-mer Triplet #24** | CGTT | CTGG | GGAA |
| **4-mer Triplet #25** | CGTT | GGAA | CGCA |
| **4-mer Triplet #26** | CTGC | TCTT | ACGA |
| **4-mer Triplet #27** | CTGC | TGTC | ACGA |
| **4-mer Triplet #28** | CTGG | GGAA | CGCA |
| **4-mer Triplet #29** | GAGG | ACGC | CGTT |
| **4-mer Triplet #30** | GAGG | ACGC | CTGG |
| **4-mer Triplet #31** | GAGG | CCCA | TGGC |
| **4-mer Triplet #32** | GAGG | CGTT | CGCA |
| **4-mer Triplet #33** | GAGG | CGTT | GGAA |
| **4-mer Triplet #34** | GAGG | CTGG | CGCA |
| **4-mer Triplet #35** | GAGG | TGGC | TCAC |
| **4-mer Triplet #36** | GCAA | ACTG | TCCC |
| **4-mer Triplet #37** | GCAA | TGGC | TCCC |
| **4-mer Triplet #38** | GCTC | ATGG | CGGT |
| **4-mer Triplet #39** | GCTC | CGGT | ATCC |
| **4-mer Triplet #40** | GGAA | ATCC | AAGG |
| **4-mer Triplet #41** | GGAA | GTTT | ATCC |
| **4-mer Triplet #42** | GTAG | CTGC | ACGA |
| **4-mer Triplet #43** | GTAG | TCTG | CTGC |
| **4-mer Triplet #44** | GTAG | TGCT | CTGC |
| **4-mer Triplet #45** | GTTT | ATGA | ATGT |
| **4-mer Triplet #46** | GTTT | ATGT | GGTC |
| **4-mer Triplet #47** | TCAC | AAAA | CGCC |
| **4-mer Triplet #48** | TCAC | AACG | CGCC |
| **4-mer Triplet #49** | TCAC | TCTG | AACG |
| **4-mer Triplet #50** | TCAC | TGCT | AAAA |
| **4-mer Triplet #51** | TCAC | TGCT | AACG |
| **4-mer Triplet #52** | TGAC | TCGT | GTAG |
| **4-mer Triplet #53** | TGAT | ATGT | TGAC |
| **4-mer Triplet #54** | TGGC | CTCC | TCAC |

As used herein, the term "4-mer" refers to a nucleic acid sequence consisting of 4 nucleotides.

As used herein the term "4-mer triplet" refers to a set of three distinct 4-mer sequences. These three distinct 4-mer sequences together provide superior and unexpected results in that when the three sequences, or complements thereof are used in the 5' overhangs of a pair of partially double-stranded nucleic acid molecules, the pair of partially double-stranded nucleic acid molecules can be ligated together with high efficiency and/or high fidelity.

In some aspects, the three distinct 4-mer sequences, or complements thereof, of a 4-mer triplet, when used in the 5' overhangs of a pair of partially double-stranded nucleic acid molecules, can allow for the ligation of the partially double-stranded nucleic acid molecule such that the resulting ligation product has a purity of at least 80%, or at least 90%, or at least 95%, or at least 99%. In some aspects, the three distinct 4-mer sequences, or complements thereof, of a 4-mer triplet, when used in the 5' overhangs of a pair of partially double-stranded nucleic acid molecules, can allow for the ligation of the partially double-stranded nucleic acid molecule such that the resulting ligation product has a purity of at least 90%. In some aspects, purity refers to the percentage of the total ligation products that were formed as part of a ligation reaction (or multiple rounds of ligation reactions) that correspond to the correct/desired ligation product. Thus, in a non-limiting example, the three-distinct 4-mer sequences, or complements thereof, of a 4-mer triplet, when used in the 5' overhangs of a pair of partially double-stranded nucleic acid molecules, can allow for the ligation of the partially double-stranded nucleic acid molecules such that when a ligation reaction comprising a plurality of the pair of partially double-stranded nucleic acid molecules is performed, 90% of the resulting ligation products correspond to the correct/desired ligation product.

to the percentage of the total ligation products that were formed as part of a single ligation reaction, or multiple rounds of ligation reactions, that correspond to the correct/desired ligation product Without wishing to be bound by theory, the methods of the present disclosure comprising the ligation of nuclei acid molecules produce can produce plurality of ligation products, some of which correspond to the correct/desired ligation product, and some that are undesired (side-reactions, incorrect ligations, etc.). The purity of a ligation product, or a target molecule that is being synthesized, can be expressed as a percentage, which corresponds to the percentage of the total ligation products formed which correspond to the correct/desired ligation product.

In some aspects, the three distinct 4-mer sequences of a 4-mer triplet can he experimentally determined. In some aspects, the three distinct 4-mer sequences of a 4-mer triplet can be experimentally determined using the methods described in Example 5.

Non-limiting examples of preferred 4-mer triplets are shown in Table 1.

In a non-limiting example of the preceding compositions, wherein the triplet selected from Table 1 is 4-mer triplet #1, the first 5' overhang can comprise either 4-mer #1 of the triplet (AAAA), 4-mer #2 of the triplet (CACC) or 4-mer #3 of the triplet (CGCC), or the complements thereof. If the first 5' overhang comprises 4-mer #1 of the triplet (AAAA), then the third 5' overhang can comprise either 4-mer #2 of the triplet (CACC) or 4-mer #3 of the triplet (CGCC). If the first 5' overhang comprises 4-mer #1 of the triplet (AAAA) and the third 5' overhang comprises 4-mer #2 of the triplet (CACC), then the fourth 5' overhang will comprise 4-mer #3 of the triplet (CGCC). That is, one of the first, third and fourth 5' overhangs will comprise the 4-mer of the second column of a single row of Table 1, one of the first, third and fourth 5' overhangs will comprise the 4-mer of the third column of the same row of Table 1, and one of the first, third and fourth 5' overhangs will comprise the 4-mer of the fourth column of Table 1, wherein the first, third and fourth 5' overhangs comprise a different 4-mer sequence.

In some aspects, a double-stranded nucleic acid fragment or a double-stranded nucleic acid molecule can be a partially double-stranded nucleic acid molecule or a partially double-stranded nucleic acid fragment. As used herein, the terms "partially double-stranded nucleic acid molecule" and "partially double-stranded nucleic acid fragment" also refers to a nucleic acid molecule comprised of two polynucleotide strands, wherein at least a portion of the two strands are hybridized (*i.e.* base-paired) to each other such that the nucleic acid molecule comprises at least one portion that is double-stranded and at least one portion that is single-stranded (*i.e.* not base-paired with the other strand). In some aspects, only one of the strands has a single-stranded portion. In some aspects, both of the strands has a single-stranded portion. As used herein, the terms "nucleic acid molecule" and "nucleic acid fragment" are used interchangeably.

The present disclosure provides compositions comprising a first partially double-stranded nucleic acid molecule, a second partially double-stranded nucleic acid molecule, a third partially double-stranded nucleic acid molecule and an at least fourth partially double-stranded nucleic acid molecule, wherein the first partially double-stranded nucleic acid molecule comprises a first 5' overhang and a second 5' overhang, wherein the second partially double-stranded nucleic acid molecule comprises a third 5' overhang and fourth 5' overhang, wherein the third partially double-stranded nucleic acid molecule comprises a fifth 5' overhang and a sixth 5' overhang, wherein the at least fourth partially double-stranded nucleic acid molecule comprises a seventh 5' overhang and an eighth 5' overhang, wherein the second 5' overhang and third 5' overhang are complementary to each other, wherein the fourth 5' overhang and the fifth 5' overhang are complementary to each other, wherein the sixth 5' overhang and the seventh 5' overhang are complementary to each other, wherein the first 5' overhang, the third 5' overhang, the fifth 5' overhang, the seventh 5' overhang and the eighth 5' overhang each comprise one of the 4-mer sequences, or complement thereof, of a 4-mer quintuplet, wherein the 4-mer quintuplet comprises five 4-mer sequences, which yield a single fragment with at least 90% purity upon ligation of the first, second, third and at least fourth partially double-stranded nucleic acid molecules, and wherein the first 5' overhang, the third 5' overhang, the fifth 5' overhang, the seventh 5' overhang and the eighth 5' overhang comprise a different 4-mer sequence.

The present disclosure provides compositions comprising a first partially double-stranded nucleic acid molecule, a second partially double-stranded nucleic acid molecule, a third partially double-stranded nucleic acid molecule and an at least fourth partially double-stranded nucleic acid molecule, wherein the first partially double-stranded nucleic acid molecule comprises a first 5' overhang and a second 5' overhang, wherein the second partially double-stranded nucleic acid molecule comprises a third 5' overhang and fourth 5' overhang, wherein the third partially double-stranded nucleic acid molecule comprises a fifth 5' overhang and a sixth 5' overhang, wherein the at least fourth partially double-stranded nucleic acid molecule comprises a seventh 5' overhang and an eighth 5' overhang, wherein the second 5' overhang and third 5' overhang are complementary to each other, wherein the fourth 5' overhang and the fifth 5' overhang are complementary to each other, wherein the sixth 5' overhang and the seventh 5' overhang are complementary to each other, wherein the first 5' overhang, the third 5' overhang, the fifth 5' overhang, the seventh 5' overhang and the eighth 5' overhang each comprise one of the 4-mer sequences, or complement thereof, of a 4-mer quintuplet, wherein the 4-mer quintuplet comprises five 4-mer sequences, which yield only one fragment upon ligation of the first, second, third and at least fourth partially double-stranded nucleic acid molecules, and wherein the first 5' overhang, the third 5' overhang, the fifth 5' overhang, the seventh 5' overhang and the eighth 5' overhang comprise a different 4-mer sequence. A schematic of a representative composition of the present disclosure is shown in FIG. 28. In some aspects, the 4-mer quintuplet can be selected from the 4-mer quintuplets recited in Table 2.

The present disclosure provides compositions comprising a first double-stranded nucleic acid fragment, a second double-stranded nucleic acid fragment, a third double-stranded nucleic acid fragment and an at least fourth double-stranded nucleic acid fragment, wherein the first double-stranded nucleic acid fragment comprises a first 5' overhang and a second 5' overhang, wherein the second double-stranded nucleic acid fragment comprises a third 5' overhang and fourth 5' overhang, wherein the third double-stranded nucleic acid fragment comprises a fifth 5' overhang and a sixth 5' overhang, wherein the at least fourth double-stranded nucleic acid fragment comprises a seventh 5' overhang and an eighth 5' overhang, wherein the second 5' overhang and third 5' overhang are complementary to each other, wherein the fourth 5' overhang and the fifth 5' overhang are complementary to each other, wherein the sixth 5' overhang and the seventh 5' overhang are complementary to each other, wherein the first 5' overhang, the third 5' overhang, the fifth 5' overhang, the seventh 5' overhang and the eighth 5' overhang each comprise one of the 4-mer sequences, or complement thereof, of a 4-mer quintuplet, wherein the 4-mer quintuplet is selected from the 4-mer quintuplets recited in Table 2, and wherein the first 5' overhang, the third 5' overhang, the fifth 5' overhang, the seventh 5' overhang and the eighth 5' overhang comprise a different 4-mer sequence. A schematic of a representative composition of the present disclosure is shown in FIG. 28.

**Table 2. 4-mer quintuplets**

| | **4-mer #1 of quintuplet** | **4-mer #2 of quintuplet** | **4-mer #3 of quintuplet** | **4-mer #4 of quintuplet** | **4-mer #5 of quintuplet** |
|---|---|---|---|---|---|
| **4-mer Quintuplet #1** | AAAC | AAGG | TGAC | TCGT | GTAG |
| **4-mer Quintuplet #2** | ACGC | CGTT | CTGG | GGAA | CGCA |
| **4-mer Quintuplet #3** | AGCC | CACC | GCAA | ACTG | TCCC |
| **4-mer Quintuplet #4** | AGCC | CACC | GCAA | TGGC | TCCC |
| **4-mer Quintuplet #5** | AGCC | GACA | GCAA | ACTG | TCCC |
| **4-mer Quintuplet #6** | AGCC | GACA | GCAA | TGGC | TCCC |
| **4-mer Quintuplet #7** | ATCC | TACC | ACCG | CCGA | GAGG |
| **4-mer Quintuplet #8** | CAAC | TTTT | TGAT | ATGT | TGAC |
| **4-mer Quintuplet #3** | CGAG | AACA | AGTT | TGAT | TGTG |
| **4-mer Quintuplet #10** | GAGG | ACGC | CGTT | CTGG | CGCA |
| **4-mer Quintuplet #11** | GAGG | ACGC | CGTT | CTGG | GGAA |
| **4-mer Quintuplet #12** | GAGG | ACGC | CGTT | GGAA | CGCA |
| **4-mer Quintuplet #13** | GAGG | ACGC | CTGG | GGAA | CGCA |
| **4-mer Quintuplet #14** | GAGG | CCCA | TGGC | CTCC | TCAC |
| **4-mer Quintuplet #15** | GCTC | ATGG | CGGT | TTGC | ATCC |
| **4-mer Quintuplet #16** | GGAA | GTTT | ATCC | ATCC | AAGG |
| **4-mer Quintuplet #17** | GTAG | TCTG | CTGC | TCTT | ACGA |
| **4-mer Quintuplet #18** | GTAG | TCTG | CTGC | TGTC | ACGA |
| **4-mer Quintuplet #19** | GTAG | TGCT | CTGC | TCTT | ACGA |
| **4-mer Quintuplet #20** | GTAG | TGCT | CTGC | TGTC | ACGA |
| **4-mer Quintuplet #21** | GTTT | ATGA | ATGT | TTGA | GGTC |
| **4-mer Quintuplet #22** | TCAC | TCTG | AACG | CACC | CGCC |
| **4-mer Quintuplet #23** | TCAC | TCTG | AACG | GACA | CGCC |
| **4-mer Quintuplet #24** | TCAC | TGCT | AAAA | CACC | CGCC |
| **4-mer Quintuplet #25** | TCAC | TGCT | AAAA | GACA | CGCC |
| **4-mer Quintuplet #26** | TCAC | TGCT | AACG | CACC | CGCC |
| **4-mer Quintuplet #27** | TCAC | TGCT | AACG | GACA | CGCC |

As used herein the term "4-mer quintuplet" refers to a set of five distinct 4-mer sequences. These five distinct 4-mer sequences together provide superior and unexpected results in that when the five sequences are used as in the 5' overhangs of a set of four partially double-stranded nucleic acid molecules, the four partially double-stranded nucleic acid molecules can be ligated together in a step wise assembly reaction with high efficiency and/or high fidelity.

In some aspects, the five distinct 4-mer sequences, or complements thereof, of a 4-mer quintuplet, when used in the 5' overhangs of a set of four partially double-stranded nucleic acid molecules, can allow for the ligation of the four partially double-stranded nucleic acid molecules such that the resulting ligation product has a purity of at least 80%, or at least 90%, or at least 95%, or at least 99%. In some aspects, the five distinct 4-mer sequences, or complements thereof, of a 4-mer quintuplet, when used in the 5' overhangs of a set of four partially double-stranded nucleic acid molecules, can allow for the ligation of the four partially double-stranded nucleic acid molecules such that the resulting ligation product has a purity of at least 90%.

In some aspects, purity refers to the percentage of the total ligation products that were formed as part of a ligation reaction (or multiple rounds of ligation reactions) that correspond to the correct/desired ligation product. Thus, in a non-limiting example, the five distinct 4-mer sequences, or complements thereof, of a 4-mer quintuplet, when used in the 5' overhangs of a set of four partially double-stranded nucleic acid molecules, can allow for the ligation of the four partially double-stranded nucleic acid molecules such that when a ligation reaction (or two or more consecutive rounds of ligation reactions) comprising a plurality of the set of four partially double-stranded nucleic acid molecules is performed, 90% of the resulting ligation products correspond to the correct/desired ligation product.

In some aspects, the five distinct 4-mer sequences of a 4-mer quintuplet can be experimentally determined. In some aspects, the five distinct 4-mer sequences of a 4-mer quintuplet can be experimentally determined using the methods described in Example 5.

Non-limiting examples of preferred 4-mer triplets are shown in Table 2.

In a non-limiting example of the preceding compositions, wherein the quintuplet selected from Table 2 is 4-mer quintuplet #1, one of the first 5' overhang, the third 5' overhang, the fifth 5' overhang, the seventh 5' overhang and the eighth 5' overhang will comprise 4-mer #1 of the quintuplet (AAAC), or the complement thereof, another one of the first 5' overhang, the third 5' overhang, the fifth 5' overhang, the seventh 5' overhang and the eighth 5' overhang will comprise 4-mer #2 of the quintuplet (AAGG), or the complement thereof, another one of the first 5' overhang, the third 5' overhang, the fifth 5' overhang, the seventh 5' overhang and the eighth 5' overhang will comprise 4-mer #3 of the quintuplet (TGAC), or the complement thereof, another one of the first 5' overhang, the third 5' overhang, the fifth 5' overhang, the seventh 5' overhang and the eighth 5' overhang will comprise 4-mer #4 of the quintuplet (TCGT), or the complement thereof, and another one of the first 5' overhang, the third 5' overhang, the fifth 5' overhang, the seventh 5' overhang and the eighth 5' overhang will comprise 4-mer #5 of the quintuplet (GTAG), or the complement thereof, and each of the overhangs comprise a different 4-mer sequence.

In some aspects, the double-stranded portion of a partially double-stranded nucleic acid molecule can comprise at least about 5 base-pairs (bp), or at least about 6 bp, or at least about 7 bp, or at least about 8 bp, or at least about 9 bp, or at least about 10 bp, or at least about I 1 bp, or at least about 12 bp, or at least about 13 bp, or at least about 14 bp, or at least about 15 bp, or at least about 16 bp, or at least about 17 bp, or at least about 18 bp, or at least about 19 bp, or at least about 20 bp, or at least about 21 bp, or at least about 22 bp, or at least about 23 bp, or at least about 24 bp, or at least about 25 bp, or at least about 26 bp, or at least about 27 bp, or at least about 28 bp, or at least about 29 bp, or at least about 30 bp, or at least about 31 bp, or at least about 32 bp, or at least about 33 bp, or at least about 34 bp, or at least about 35 bp, or at least about 36 bp, or at least about 37 bp, or at least about 38 bp, or at least about 39 bp, or at least about 40 bp in length.

In some aspects, the double-stranded portion of a partially double-stranded nucleic acid molecule is about 5 bp to about 40 bp in length. In some aspects, the double-stranded portion of a partially double-stranded nucleic acid molecule is about 10 bp to about 35 bp in length. In some aspects, the double-stranded portion of a partially double-stranded nucleic acid molecule is about 20 bp to about 30 bp in length.

In some aspects, a partially double-stranded nucleic acid molecule can be at least about 5 nucleotides, or at least about 10 nucleotides, or at least about 15 nucleotides, or at least about 20 nucleotides, or at least about 25 nucleotides, or at least about 30 nucleotides, or at least about 35 nucleotides, or at least about 40 nucleotides in length.

As used herein, the term 5' overhang is used to refer to a single-stranded portion of a partially double-stranded nucleic acid molecule that is located at the 5' terminus of one of the strands. An illustrative example of 5' overhangs are shown in FIG. 27.

As used herein, the term 3' overhang is used to refer to a single-stranded portion of a partially double-stranded nucleic acid molecule that is located at the 3' terminus of one of the strands.

In some aspects of the compositions of the present disclosure, a 5' overhang can comprise one of the 4-mers of one of the 4-mer triplets recited in Table 1. In some aspects of the compositions of the present disclosure, a 5' overhang can consist of one of the 4-mer triplets recited in Table 1.

In some aspects of the compositions of the present disclosure, a 5' overhang can comprise one of the 4-mers of one of the 4-mer quintuplets recited in Table 2. In some aspects of the compositions of the present disclosure, a 5' overhang can consist of one of the 4-mers of one of the 4-mer quintuplets recited in Table 2.

In some aspects of the compositions of the present disclosure a 5' overhang can be about 4 nucleotides in length. In some aspects of the compositions of the present disclosure a 5' overhang can be at least about 4 nucleotides, or at least about 5 nucleotides, or at least about 6 nucleotides, or at least about 7 nucleotides, or at least about 8 nucleotides, or at least about 9 nucleotides, or at least about 10 nucleotides in length.

In some aspects, a 5' overhang is no more than 4, or no more than 5, or no more than 6, or no more than 7, or no more than 8, or no more than 9, or no more than 10 nucleotides, or no more than 11 nucleotides, or no more than 12 nucleotides, or no more than 13 nucleotide, or no more than 14 nucleotides, or no more than 15 nucleotides, or no more than 16 nucleotides, or no more than 17 nucleotides, or no more than 18 nucleotides, or no more than 19 nucleotides, or no more than 20 nucleotides in length.

In some aspects of the compositions of the present disclosure a 3' overhang can be about 4 nucleotides in length. In some aspects of the compositions of the present disclosure a 3' overhang can be at least about 4 nucleotides, or at least about 5 nucleotides, or at least about 6 nucleotides, or at least about 7 nucleotides, or at least about 8 nucleotides, or at least about 9 nucleotides, or at least about 10 nucleotides in length.

In some aspects, a 3' overhang is no more than 4, or no more than 5, or no more than 6, or no more than 7, or no more than 8, or no more than 9, or no more than 10 nucleotides, or no more than 11 nucleotides, or no more than 12 nucleotides, or no more than 13 nucleotide, or no more than 14 nucleotides, or no more than 15 nucleotides, or no more than 16 nucleotides, or no more than 17 nucleotides, or no more than 18 nucleotides, or no more than 19 nucleotides, or no more than 20 nucleotides in length.

In some aspects of the compositions of the present disclosure, a 5' overhang can comprise at least one of the nucleic acid sequences, or the complement thereof, selected from AAAA, CACC, CGCC, AAAA, GACA, CGCC, AAAC, AAGG, TGAC, AAAC, TGAC, GTAG, AACG, CACC, CGCC, AACG, GACA, CGCC, ACCG, CCGA, GAGG, ACGC, CGTT, CTGG, ACGC, CTGG, CGCA, AGCC, CACC, GCAA, AGCC, GACA, GCAA, AGCC, GCAA, TCCC, AGTT, TGAT, TGTG, ATCC, ACCG, GAGG, ATCC, ATGC, AAGG, ATCC, TACC, ACCG, ATGT, TTGA, GGTC, CAAC, TGAT, TGAC, CAAC, TTTT, TGAT, CGAG, AACA, AGTT, CGAG, AGTT, TGTG, CGGT, TTGC, ATCC, CGTT, CTGG, CGCA, CGTT, CTGG, GGAA, CGTT, GGAA, CGCA, CTGC, TCTT, ACGA, CTGC, TGTC, ACGA, CTGG, GGAA, CGCA, GAGG, ACGC, CGTT, GAGG, ACGC, CTGG, GAGG, CCCA, TGGC, GAGG, CGTT, CGCA, GAGG, CGTT, GGAA, GAGG, CTGG, CGCA, GAGG, TGGC, TCAC, GCAA, ACTG, TCCC, GCAA, TGGC, TCCC, GCTC, ATGG, CGGT, GCTC, CGGT, ATCC, GGAA, ATCC, AAGG, GGAA, GTTT, ATCC, GTAG, CTGC, ACGA, GTAG, TCTG, CTGC, GTAG, TGCT, CTGC, GTTT, ATGA, ATGT, GTTT, ATGT, GGTC, TCAC, AAAA, CGCC, TCAC, AACG, CGCC, TCAC, TCTG, AACG, TCAC, TGCT, AAAA, TCAC, TGCT, AACG, TGAC, TCGT, GTAG, TGAT, ATGT, TGAC, TGGC, CTCC and TCAC.

In some aspects of the compositions of the present disclosure, a 5' overhang can consist of at least one of the sequences, or the complement thereof, selected from AAAA, CACC, CGCC, AAAA, GACA, CGCC, AAAC, AAGG, TGAC, AAAC, TGAC, GTAG, AACG, CACC, CGCC, AACG, GACA, CGCC, ACCG, CCGA, GAGG, ACGC, CGTT, CTGG, ACGC, CTGG, CGCA, AGCC, CACC, GCAA, AGCC, GACA, GCAA, AGCC, GCAA, TCCC, AGTT, TGAT, TGTG, ATCC, ACCG, GAGG, ATCC, ATGC, AAGG, ATCC, TACC, ACCG, ATGT, TTGA, GGTC, CAAC, TGAT, TGAC, CAAC, TTTT, TGAT, CGAG, AACA, AGTT, CGAG, AGTT, TGTG, CGGT, TTGC, ATCC, CGTT, CTGG, CGCA, CGTT, CTGG, GGAA, CGTT, GGAA, CGCA, CTGC, TCTT, ACGA, CTGC, TGTC, ACGA, CTGG, GGAA, CGCA, GAGG, ACGC, CGTT, GAGG, ACGC, CTGG, GAGG, CCCA, TGGC, GAGG, CGTT, CGCA, GAGG, CGTT, GGAA, GAGG, CTGG, CGCA, GAGG, TGGC, TCAC, GCAA, ACTG, TCCC, GCAA, TGGC, TCCC, GCTC, ATGG, CGGT, GCTC, CGGT, ATCC, GGAA, ATCC, AAGG, GGAA, GTTT, ATCC, GTAG, CTGC, ACGA, GTAG, TCTG, CTGC, GTAG, TGCT, CTGC, GTTT, ATGA, ATGT, GTTT, ATGT, GGTC, TCAC, AAAA, CGCC, TCAC, AACG, CGCC, TCAC, TCTG, AACG, TCAC, TGCT, AAAA, TCAC, TGCT, AACG, TGAC, TCGT, GTAG, TGAT, ATGT, TGAC, TGGC, CTCC and TCAC.

In some aspects of the compositions of the present disclosure, a 5' overhang can comprise at least one of the nucleic acid sequences, or the complement thereof, selected from AAAC, AAGG, TGAC, TCGT, GTAG, ACGC, CGTT, CTGG, GGAA, CGCA, AGCC, CACC, GCAA, ACTG, TCCC, AGCC, CACC, GCAA, TGGC, TCCC, AGCC, GACA, GCAA, ACTG, TCCC, AGCC, GACA, GCAA, TGGC, TCCC, ATCC, TACC, ACCG, CCGA, GAGG, CAAC, TTTT, TGAT, ATGT, TGAC, CGAG, AACA, AGTT, TGAT, TGTG, GAGG, ACGC, CGTT, CTGG, CGCA, GAGG, ACGC, CGTT, CTGG, GGAA, GAGG, ACGC, CGTT, GGAA, CGCA, GAGG, ACGC, CTGG, GGAA, CGCA, GAGG, CCCA, TGGC, CTCC, TCAC, GCTC, ATGG, CGGT, TTGC, ATCC, GGAA, GTTT, ATCC, ATGC, AAGG, GTAG, TCTG, CTGC, TCTT, ACGA, GTAG, TCTG, CTGC, TGTC, ACGA, GTAG, TGCT, CTGC, TCTT, ACGA, GTAG, TGCT, CTGC, TGTC, ACGA, GTTT, ATGA, ATGT, TTGA, GGTC, TCAC, TCTG, AACG, CACC, CGCC, TCAC, TCTG, AACG, GACA, CGCC, TCAC, TGCT, AAAA, CACC, CGCC, TCAC, TGCT, AAAA, GACA, CGCC, TCAC, TGCT, AACG, CACC, CGCC, TCAC, TGCT, AACG, GACA, and CGCC

In some aspects of the compositions of the present disclosure, a 5' overhang can consist of at least one of the sequences, or the complement thereof, selected from AAAC, AAGG, TGAC, TCGT, GTAG, ACGC, CGTT, CTGG, GGAA, CGCA, AGCC, CACC, GCAA, ACTG, TCCC, AGCC, CACC, GCAA, TGGC, TCCC, AGCC, GACA, GCAA, ACTG, TCCC, AGCC, GACA, GCAA, TGGC, TCCC, ATCC, TACC, ACCG, CCGA, GAGG, CAAC, TTTT, TGAT, ATGT, TGAC, CGAG, AACA, AGTT, TGAT, TGTG, GAGG, ACGC, CGTT, CTGG, CGCA, GAGG, ACGC, CGTT, CTGG, GGAA, GAGG, ACGC, CGTT, GGAA, CGCA, GAGG, ACGC, CTGG, GGAA, CGCA, GAGG, CCCA, TGGC, CTCC, TCAC, GCTC, ATGG, CGGT, TTGC, ATCC, GGAA, GTTT, ATCC, ATGC, AAGG, GTAG, TCTG, CTGC, TCTT, ACGA, GTAG, TCTG, CTGC, TGTC, ACGA, GTAG, TGCT, CTGC, TCTT, ACGA, GTAG, TGCT, CTGC, TGTC, ACGA, GTTT, ATGA, ATGT, TTGA, GGTC, TCAC, TCTG, AACG, CACC, CGCC, TCAC, TCTG, AACG, GACA, CGCC, TCAC, TGCT, AAAA, CACC, CGCC, TCAC, TGCT, AAAA, GACA, CGCC, TCAC, TGCT, AACG, CACC, CGCC, TCAC, TGCT, AACG, GACA, and CGCC.

In some aspects of the compositions of the present disclosure, a 3' overhang can comprise at least one of the nucleic acid sequences, or the complement thereof, selected from AAAA, CACC, CGCC, AAAA, GACA, CGCC, AAAC, AAGG, TGAC, AAAC, TGAC, GTAG, AACG, CACC, CGCC, AACG, GACA, CGCC, ACCG, CCGA, GAGG, ACGC, CGTT, CTGG, ACGC, CTGG, CGCA, AGCC, CACC, GCAA, AGCC, GACA, GCAA, AGCC, GCAA, TCCC, AGTT, TGAT, TGTG, ATCC, ACCG, GAGG, ATCC, ATGC, AAGG, ATCC, TACC, ACCG, ATGT, TTGA, GGTC, CAAC, TGAT, TGAC, CAAC, TTTT, TGAT, CGAG, AACA, AGTT, CGAG, AGTT, TGTG, CGGT, TTGC, ATCC, CGTT, CTGG, CGCA, CGTT, CTGG, GGAA, CGTT, GGAA, CGCA, CTGC, TCTT, ACGA, CTGC, TGTC, ACGA, CTGG, GGAA, CGCA, GAGG, ACGC, CGTT, GAGG, ACGC, CTGG, GAGG, CCCA, TGGC, GAGG, CGTT, CGCA, GAGG, CGTT, GGAA, GAGG, CTGG, CGCA, GAGG, TGGC, TCAC, GCAA, ACTG, TCCC, GCAA, TGGC, TCCC, GCTC, ATGG, CGGT, GCTC, CGGT, ATCC, GGAA, ATCC, AAGG, GGAA, GTTT, ATCC, GTAG, CTGC, ACGA, GTAG, TCTG, CTGC, GTAG, TGCT, CTGC, GTTT, ATGA, ATGT, GTTT, ATGT, GGTC, TCAC, AAAA, CGCC, TCAC, AACG, CGCC, TCAC, TCTG, AACG, TCAC, TGCT, AAAA, TCAC, TGCT, AACG, TGAC, TCGT, GTAG, TGAT, ATGT, TGAC, TGGC, CTCC and TCAC.

In some aspects of the compositions of the present disclosure, a 3' overhang can consist of at least one of the sequences, or the complement thereof, selected from AAAA, CACC, CGCC, AAAA, GACA, CGCC, AAAC, AAGG, TGAC, AAAC, TGAC, GTAG, AACG, CACC, CGCC, AACG, GACA, CGCC, ACCG, CCGA, GAGG, ACGC, CGTT, CTGG, ACGC, CTGG, CGCA, AGCC, CACC, GCAA, AGCC, GACA, GCAA, AGCC, GCAA, TCCC, AGTT, TGAT, TGTG, ATCC, ACCG, GAGG, ATCC, ATGC, AAGG, ATCC, TACC, ACCG, ATGT, TTGA, GGTC, CAAC, TGAT, TGAC, CAAC, TTTT, TGAT, CGAG, AACA, AGTT, CGAG, AGTT, TGTG, CGGT, TTGC, ATCC, CGTT, CTGG, CGCA, CGTT, CTGG, GGAA, CGTT, GGAA, CGCA, CTGC, TCTT, ACGA, CTGC, TGTC, ACGA, CTGG, GGAA, CGCA, GAGG, ACGC, CGTT, GAGG, ACGC, CTGG, GAGG, CCCA, TGGC, GAGG, CGTT, CGCA, GAGG, CGTT, GGAA, GAGG, CTGG, CGCA, GAGG, TGGC, TCAC, GCAA, ACTG, TCCC, GCAA, TGGC, TCCC, GCTC, ATGG, CGGT, GCTC, CGGT, ATCC, GGAA, ATCC, AAGG, GGAA, GTTT, ATCC, GTAG, CTGC, ACGA, GTAG, TCTG, CTGC, GTAG, TGCT, CTGC, GTTT, ATGA, ATGT, GTTT, ATGT, GGTC, TCAC, AAAA, CGCC, TCAC, AACG, CGCC, TCAC, TCTG, AACG, TCAC, TGCT, AAAA, TCAC, TGCT, AACG, TGAC, TCGT, GTAG, TGAT, ATGT, TGAC, TGGC, CTCC and TCAC.

In some aspects of the compositions of the present disclosure, a 3' overhang can comprise at least one of the nucleic acid sequences, or the complement thereof, selected from AAAC, AAGG, TGAC, TCGT, GTAG, ACGC, CGTT, CTGG, GGAA, CGCA, AGCC, CACC, GCAA, ACTG, TCCC, AGCC, CACC, GCAA, TGGC, TCCC, AGCC, GACA, GCAA, ACTG, TCCC, AGCC, GACA, GCAA, TGGC, TCCC, ATCC, TACC, ACCG, CCGA, GAGG, CAAC, TTTT, TGAT, ATGT, TGAC, CGAG, AACA, AGTT, TGAT, TGTG, GAGG, ACGC, CGTT, CTGG, CGCA, GAGG, ACGC, CGTT, CTGG, GGAA, GAGG, ACGC, CGTT, GGAA, CGCA, GAGG, ACGC, CTGG, GGAA, CGCA, GAGG, CCCA, TGGC, CTCC, TCAC, GCTC, ATGG, CGGT, TTGC, ATCC, GGAA, GTTT, ATCC, ATGC, AAGG, GTAG, TCTG, CTGC, TCTT, ACGA, GTAG, TCTG, CTGC, TGTC, ACGA, GTAG, TGCT, CTGC, TCTT, ACGA, GTAG, TGCT, CTGC, TGTC, ACGA, GTTT, ATGA, ATGT, TTGA, GGTC, TCAC, TCTG, AACG, CACC, CGCC, TCAC, TCTG, AACG, GACA, CGCC, TCAC, TGCT, AAAA, CACC, CGCC, TCAC, TGCT, AAAA, GACA, CGCC, TCAC, TGCT, AACG, CACC, CGCC, TCAC, TGCT, AACG, GACA, and CGCC

In some aspects of the compositions of the present disclosure, a 3' overhang can consist of at least one of the sequences, or the complement thereof, selected from AAAC, AAGG, TGAC, TCGT, GTAG, ACGC, CGTT, CTGG, GGAA, CGCA, AGCC, CACC, GCAA, ACTG, TCCC, AGCC, CACC, GCAA, TGGC, TCCC, AGCC, GACA, GCAA, ACTG, TCCC, AGCC, GACA, GCAA, TGGC, TCCC, ATCC, TACC, ACCG, CCGA, GAGG, CAAC, TTTT, TGAT, ATGT, TGAC, CGAG, AACA, AGTT, TGAT, TGTG, GAGG, ACGC, CGTT, CTGG, CGCA, GAGG, ACGC, CGTT, CTGG, GGAA, GAGG, ACGC, CGTT, GGAA, CGCA, GAGG, ACGC, CTGG, GGAA, CGCA, GAGG, CCCA, TGGC, CTCC, TCAC, GCTC, ATGG, CGGT, TTGC, ATCC, GGAA, GTTT, ATCC, ATGC, AAGG, GTAG, TCTG, CTGC, TCTT, ACGA, GTAG, TCTG, CTGC, TGTC, ACGA, GTAG, TGCT, CTGC, TCTT, ACGA, GTAG, TGCT, CTGC, TGTC, ACGA, GTTT, ATGA, ATGT, TTGA, GGTC, TCAC, TCTG, AACG, CACC, CGCC, TCAC, TCTG, AACG, GACA, CGCC, TCAC, TGCT, AAAA, CACC, CGCC, TCAC, TGCT, AAAA, GACA, CGCC, TCAC, TGCT, AACG, CACC, CGCC, TCAC, TGCT, AACG, GACA, and CGCC.

In some aspects of the compositions of the present disclosure, any description and/or characteristic of a 5' overhang can be applied to a 3' overhang.

The present disclosure provides compositions comprising a first partially double-stranded nucleic acid molecule and an at least second partially double-stranded nucleic acid molecule, wherein the first partially double-stranded nucleic acid molecule comprises a first 5' overhang and a second 5' overhang, wherein the at least second partially double-stranded nucleic acid molecule comprises a third 5' overhang and fourth 5' overhang, wherein the second 5' overhang and third 5' overhang are complementary to each other, wherein at least one of the first 5' overhang, the third 5' overhang and the fourth 5' overhang comprises one of the 4-mer sequences, or complement thereof, recited in Table 3, and wherein the first 5' overhang, the third 5' overhang and the fourth 5' overhang comprise a different 4-mer sequence. In some aspects of the preceding compositions, at least two of the first 5' overhang, the third 5' overhang and the fourth 5' overhang comprises one of the 4-mer sequences, or complement thereof, recited in Table 3. In some aspects of the preceding compositions, each of the first 5' overhang, the third 5' overhang and the fourth 5' overhang comprises one of the 4-mer sequences, or complement thereof, recited in Table 3.

**Table 3.**

| |
|---|
| AATG |
| ACTA |
| AGAT |
| AGCG |
| ATGG |
| CGAA |
| CTCC |
| CTTA |
| GGTA |
| TCCA |

The present disclosure provides compositions comprising a first double-stranded nucleic acid fragment, a second double-stranded nucleic acid fragment, a third double-stranded nucleic acid fragment and an at least fourth double-stranded nucleic acid fragment, wherein the first double-stranded nucleic acid fragment comprises a first 5' overhang and a second 5' overhang, wherein the second double-stranded nucleic acid fragment comprises a third 5' overhang and fourth 5' overhang, wherein the third double-stranded nucleic acid fragment comprises a fifth 5' overhang and a sixth 5' overhang, wherein the at least fourth double-stranded nucleic acid fragment comprises a seventh 5' overhang and an eighth 5' overhang, wherein the second 5' overhang and third 5' overhang are complementary to each other, wherein the fourth 5' overhang and the fifth 5' overhang are complementary to each other, wherein the sixth 5' overhang and the seventh 5" overhang are complementary to each other, wherein at least one of the first 5' overhang, the third 5' overhang, the fifth 5' overhang, the seventh 5' overhang and the eighth 5' comprises one of the 4-mer sequences, or complement thereof, recited in Table 3, and wherein the first 5' overhang, the third 5' overhang, the fifth 5' overhang, the seventh 5' overhang and the eighth 5' overhang comprise a different 4-mer sequence.

In some aspects of the preceding composition, at least two of the first 5' overhang, the third 5' overhang, the fifth 5' overhang, the seventh 5' overhang and the eighth 5' comprises one of the 4-mer sequences, or complement thereof, recited in Table 3. In some aspects of the preceding compositions, at least three of the first 5' overhang, the third 5' overhang, the fifth 5' overhang, the seventh 5' overhang and the eighth 5' comprises one of the 4-mer sequences, or complement thereof, recited in Table 3. In some aspects of the preceding compositions, at least four of the first 5' overhang, the third 5' overhang, the fifth 5' overhang, the seventh 5' overhang and the eighth 5' comprises one of the 4-mer sequences, or complement thereof, recited in Table 3. In some aspects of the preceding compositions, each of the first 5' overhang, the third 5' overhang, the fifth 5' overhang, the seventh 5' overhang and the eighth 5' comprises one of the 4-mer sequences, or complement thereof, recited in Table 3.

The present disclosure provides compositions comprising a first partially double-stranded nucleic acid molecule and an at least second partially double-stranded nucleic acid molecule, wherein the first partially double-stranded nucleic acid molecule comprises a first 5' overhang and a second 5' overhang, wherein the at least second partially double-stranded nucleic acid molecule comprises a third 5' overhang and fourth 5' overhang, wherein the second 5' overhang and third 5' overhang are complementary to each other, wherein at least one of the first 5' overhang, the third 5' overhang and the fourth 5' overhang comprises one of the 4-mer sequences, or complement thereof, recited in Table 4, and wherein the first 5' overhang, the third 5' overhang and the fourth 5' overhang comprise a different 4-mer sequence. In some aspects of the preceding compositions, at least two of the first 5' overhang, the third 5' overhang and the fourth 5' overhang comprises one of the 4-mer sequences, or complement thereof, recited in Table 4 In some aspects of the preceding compositions, each of the first 5' overhang, the third 5' overhang and the fourth 5' overhang comprises one of the 4-mer sequences, or complement thereof, recited in Table 4.

**Table 4.**

| | | |
|---|---|---|
| AACT | AGGG | CGTA |
| AAGA | ATAG | CTCA |
| AAGC | ATCC | CTTC |
| AATC | ATGA | GAAC |
| ACAT | ATTA | GACA |
| ACCG | CAAA | GCAA |
| ACGA | CAGA | GGGA |
| ACTC | CCAC | GTAA |
| AGAA | CCAG | |
| AGAC | CCGA | |
| AGCA | CCTA | |

The present disclosure provides compositions comprising a first double-stranded nucleic acid fragment, a second double-stranded nucleic acid fragment, a third double-stranded nucleic acid fragment and an at least fourth double-stranded nucleic acid fragment, wherein the first double-stranded nucleic acid fragment comprises a first 5' overhang and a second 5' overhang, wherein the second double-stranded nucleic acid fragment comprises a third 5' overhang and fourth 5' overhang, wherein the third double-stranded nucleic acid fragment comprises a fifth 5' overhang and a sixth 5' overhang, wherein the at least fourth double-stranded nucleic acid fragment comprises a seventh 5" overhang and an eighth 5' overhang, wherein the second 5' overhang and third 5' overhang are complementary to each other, wherein the fourth 5' overhang and the fifth 5' overhang are complementary to each other, wherein the sixth 5' overhang and the seventh 5' overhang are complementary to each other, wherein at least one of the first 5' overhang, the third 5' overhang, the fifth 5' overhang, the seventh 5' overhang and the eighth 5' comprises one of the 4-mer sequences, or complement thereof, recited in Table 4, and wherein the first 5' overhang, the third 5' overhang, the fifth 5' overhang, the seventh 5' overhang and the eighth 5' overhang comprise a different 4-mer sequence.

In some aspects of the preceding compositions, at least two of the first 5' overhang, the third 5' overhang, the fifth 5' overhang, the seventh 5" overhang and the eighth 5' comprises one of the 4-mer sequences, or complement thereof, recited in Table 4. In some aspects of the preceding compositions, at least three of the first 5' overhang, the third 5' overhang, the fifth 5' overhang, the seventh 5' overhang and the eighth 5' comprises one of the 4-mer sequences, or complement thereof, recited in Table 4. In some aspects of the preceding compositions, at least four of the first 5' overhang, the third 5' overhang, the fifth 5' overhang, the seventh 5' overhang and the eighth 5' comprises one of the 4-mer sequences, or complement thereof, recited in Table 4. In some aspects of the preceding compositions, each of the first 5' overhang, the third 5' overhang, the fifth 5' overhang, the seventh 5' overhang and the eighth 5' comprises one of the 4-mer sequences, or complement thereof, recited in Table 4.

The present disclosure provides compositions comprising a first partially double-stranded nucleic acid molecule and an at least second partially double-stranded nucleic acid molecule, wherein the first partially double-stranded nucleic acid molecule comprises a first 5' overhang and a second 5' overhang, wherein the at least second partially double-stranded nucleic acid molecule comprises a third 5' overhang and fourth 5' overhang, wherein the second 5' overhang and third 5' overhang are complementary to each other, wherein at least one of the first 5' overhang, the third 5' overhang and the fourth 5" overhang comprises one of the 4-mer sequences, or complement thereof, recited in Table 5, and wherein the first 5' overhang, the third 5' overhang and the fourth 5' overhang comprise a different 4-mer sequence. In some aspects of the preceding compositions, at least two of the first 5' overhang, the third 5' overhang and the fourth 5' overhang comprises one of the 4-mer sequences, or complement thereof, recited in Table 5. In some aspects of the preceding compositions, each of the first 5' overhang, the third 5' overhang and the fourth 5' overhang comprises one of the 4-mer sequences, or complement thereof, recited in Table 5.

**Table 5.**

| | | | | | |
|---|---|---|---|---|---|
| AAAC | CACA | CGCC | GAGT | GTAG | TGCC |
| AACC | CACC | CGCT | GATG | GTCA | TGCG |
| AACG | CACG | CGGA | GCAC | GTCC | TGGC |
| AAGG | CAGC | CGGC | GCAG | GTCG | TGTC |
| ACAC | CAGG | CGGG | GCAT | GTCT | TGTG |
| ACAG | CATC | CGGT | GCCA | GTGA | TTCC |
| ACCA | CCAT | CGTC | GCCC | GTGC | TTGC |
| ACCC | CCCA | CGTG | GCCG | GTGT | GTAT |
| ACCT | CCCC | CGTT | GCCT | GTTC | ATTT |
| ACGC | CCCG | CTAC | GCGA | GTTG | AGTA |
| ACGT | CCCT | CTCG | GCTA | GTTT | TAAT |
| AGCC | CCGC | CTGC | GCTC | TACC | GTTA |
| AGCT | CCGT | CTGG | GCTG | TAGC | |
| AGGC | CCTC | CTGT | GCTT | TCAC | |
| AGGT | CCTG | GAAG | GGAA | TCCC | |
| AGTC | CCTT | GACC | GGAC | TCCG | |
| AGTG | CGAC | GACG | GGAG | TCGC | |
| ATCG | CGAG | GACT | GGAT | TCGG | |
| STGC | CGAT | GAGC | GGCA | TCGT | |
| CAAC | CGCA | GAGG | GGTC | TGAC | |

The present disclosure provides compositions comprising a first double-stranded nucleic acid fragment, a second double-stranded nucleic acid fragment, a third double-stranded nucleic acid fragment and an at least fourth double-stranded nucleic acid fragment, wherein the first double-stranded nucleic acid fragment comprises a first 5' overhang and a second 5' overhang, wherein the second double-stranded nucleic acid fragment comprises a third 5' overhang and fourth 5' overhang, wherein the third double-stranded nucleic acid fragment comprises a fifth 5' overhang and a sixth 5' overhang, wherein the at least fourth double-stranded nucleic acid fragment comprises a seventh 5' overhang and an eighth 5' overhang, wherein the second 5' overhang and third 5' overhang are complementary to each other, wherein the fourth 5' overhang and the fifth 5' overhang are complementary to each other, wherein the sixth 5' overhang and the seventh 5' overhang are complementary to each other, wherein at least one of the first 5' overhang, the third 5' overhang, the fifth 5' overhang, the seventh 5' overhang and the eighth 5' comprises one of the 4-mer sequences, or complement thereof, recited in Table 5, and wherein the first 5' overhang, the third 5' overhang, the fifth 5' overhang, the seventh 5' overhang and the eighth 5' overhang comprise a different 4-mer sequence.

In some aspects of the preceding compositions, at least two of the first 5' overhang, the third 5' overhang, the fifth 5' overhang, the seventh 5' overhang and the eighth 5' comprises one of the 4-mer sequences, or complement thereof, recited in Table 5. In some aspects of the preceding compositions, at least three of the first 5' overhang, the third 5' overhang, the fifth 5' overhang, the seventh 5' overhang and the eighth 5' comprises one of the 4-mer sequences, or complement thereof, recited in Table 5. In some aspects of the preceding compositions, at least four of the first 5' overhang, the third 5' overhang, the fifth 5' overhang, the seventh 5' overhang and the eighth 5' comprises one of the 4-mer sequences, or complement thereof, recited in Table 5. In some aspects of the preceding compositions, each of the first 5' overhang, the third 5' overhang, the fifth 5' overhang, the seventh 5' overhang and the eighth 5' comprises one of the 4-mer sequences, or complement thereof, recited in Table 5.

The present disclosure provides compositions comprising a first partially double-stranded nucleic acid molecule and an at least second partially double-stranded nucleic acid molecule, wherein the first partially double-stranded nucleic acid molecule comprises a first 5' overhang and a second 5' overhang, wherein the at least second partially double-stranded nucleic acid molecule comprises a third 5' overhang and fourth 5' overhang, wherein the second 5' overhang and third 5' overhang are complementary to each other, wherein at least one of the first 5' overhang, the third 5' overhang and the fourth 5' overhang comprises one of the 4-mer sequences, or complement thereof, recited in Table 3 and Table 4, and wherein the first 5' overhang, the third 5' overhang and the fourth 5' overhang comprise a different 4-mer sequence. In some aspects of the preceding compositions, at least two of the first 5' overhang, the third 5' overhang and the fourth 5" overhang comprises one of the 4-mer sequences, or complement thereof, recited in Table 3 and Table 4. In some aspects of the preceding compositions, each of the first 5' overhang, the third 5' overhang and the fourth 5' overhang comprises one of the 4-mer sequences, or complement thereof, recited in Table 3 and Table 4.

The present disclosure provides compositions comprising a first double-stranded nucleic acid fragment, a second double-stranded nucleic acid fragment, a third double-stranded nucleic acid fragment and an at least fourth double-stranded nucleic acid fragment, wherein the first double-stranded nucleic acid fragment comprises a first 5' overhang and a second 5' overhang, wherein the second double-stranded nucleic acid fragment comprises a third 5' overhang and fourth 5' overhang, wherein the third double-stranded nucleic acid fragment comprises a fifth 5' overhang and a sixth 5' overhang, wherein the at least fourth double-stranded nucleic acid fragment comprises a seventh 5' overhang and an eighth 5' overhang, wherein the second 5' overhang and third 5' overhang are complementary to each other, wherein the fourth 5' overhang and the fifth 5' overhang are complementary to each other, wherein the sixth 5' overhang and the seventh 5' overhang are complementary to each other, wherein at least one of the first 5' overhang, the third 5' overhang, the fifth 5' overhang, the seventh 5' overhang and the eighth 5' comprises one of the 4-mer sequences, or complement thereof, recited in Table 3 and Table 4, and wherein the first 5' overhang, the third 5' overhang, the fifth 5' overhang, the seventh 5' overhang and the eighth 5' overhang comprise a different 4-mer sequence.

In some aspects of the preceding compositions, at least two of the first 5' overhang, the third 5' overhang, the fifth 5' overhang, the seventh 5' overhang and the eighth 5' comprises one of the 4-mer sequences, or complement thereof, recited in Table 3 and Table 4. In some aspects of the preceding compositions, at least three of the first 5' overhang, the third 5' overhang, the fifth 5' overhang, the seventh 5' overhang and the eighth 5' comprises one of the 4-mer sequences, or complement thereof, recited in Table 3 and Table 4. In some aspects of the preceding compositions, at least four of the first 5' overhang, the third 5' overhang, the fifth 5' overhang, the seventh 5' overhang and the eighth 5' comprises one of the 4-mer sequences, or complement thereof, recited in Table 3 and Table 4. In some aspects of the preceding compositions, each of the first 5' overhang, the third 5' overhang, the fifth 5' overhang, the seventh 5' overhang and the eighth 5' comprises one of the 4-mer sequences, or complement thereof, recited in Table 3 and Table 4.

The present disclosure provides compositions comprising a first partially double-stranded nucleic acid molecule and an at least second partially double-stranded nucleic acid molecule, wherein the first partially double-stranded nucleic acid molecule comprises a first 5' overhang and a second 5' overhang, wherein the at least second partially double-stranded nucleic acid molecule comprises a third 5' overhang and fourth 5' overhang, wherein the second 5' overhang and third 5' overhang are complementary to each other, wherein at least one of the first 5' overhang, the third 5' overhang and the fourth 5' overhang comprises one of the 4-mer sequences, or complement thereof, recited in Table 3 and Table 5, and wherein the first 5' overhang, the third 5' overhang and the fourth 5' overhang comprise a different 4-mer sequence. In some aspects of the preceding compositions, at least two of the first 5' overhang, the third 5' overhang and the fourth 5' overhang comprises one of the 4-mer sequences, or complement thereof, recited in Table 3 and Table 5. In some aspects of the preceding compositions, each of the first 5' overhang, the third 5' overhang and the fourth 5' overhang comprises one of the 4-mer sequences, or complement thereof, recited in Table 3 and Table 5.

The present disclosure provides compositions comprising a first double-stranded nucleic acid fragment, a second double-stranded nucleic acid fragment, a third double-stranded nucleic acid fragment and an at least fourth double-stranded nucleic acid fragment, wherein the first double-stranded nucleic acid fragment comprises a first 5' overhang and a second 5' overhang, wherein the second double-stranded nucleic acid fragment comprises a third 5' overhang and fourth 5' overhang, wherein the third double-stranded nucleic acid fragment comprises a fifth 5' overhang and a sixth 5' overhang, wherein the at least fourth double-stranded nucleic acid fragment comprises a seventh 5' overhang and an eighth 5' overhang, wherein the second 5' overhang and third 5' overhang are complementary to each other, wherein the fourth 5' overhang and the fifth 5' overhang are complementary to each other, wherein the sixth 5' overhang and the seventh 5' overhang are complementary to each other, wherein at least one of the first 5' overhang, the third 5' overhang, the fifth 5' overhang, the seventh 5' overhang and the eighth 5' comprises one of the 4-mer sequences, or complement thereof, recited in Table 3 and Table 5, and wherein the first 5' overhang, the third 5' overhang, the fifth 5' overhang, the seventh 5' overhang and the eighth 5' overhang comprise a different 4-mer sequence.

In some aspects of the preceding compositions, at least two of the first 5' overhang, the third 5' overhang, the fifth 5' overhang, the seventh 5' overhang and the eighth 5' comprises one of the 4-mer sequences, or complement thereof, recited in Table 3 and Table 5. In some aspects of the preceding compositions, at least three of the first 5' overhang, the third 5' overhang, the fifth 5' overhang, the seventh 5' overhang and the eighth 5" comprises one of the 4-mer sequences, or complement thereof, recited in Table 3 and Table 5. In some aspects of the preceding compositions, at least four of the first 5' overhang, the third 5' overhang, the fifth 5' overhang, the seventh 5' overhang and the eighth 5' comprises one of the 4-mer sequences, or complement thereof, recited in Table 3 and Table 5. In some aspects of the preceding compositions, each of the first 5' overhang, the third 5' overhang, the fifth 5' overhang, the seventh 5' overhang and the eighth 5' comprises one of the 4-mer sequences, or complement thereof, recited in Table 3 and Table 5.

The present disclosure provides compositions comprising a first partially double-stranded nucleic acid molecule and an at least second partially double-stranded nucleic acid molecule, wherein the first partially double-stranded nucleic acid molecule comprises a first 5' overhang and a second 5' overhang, wherein the at least second partially double-stranded nucleic acid molecule comprises a third 5' overhang and fourth 5' overhang, wherein the second 5' overhang and third 5' overhang are complementary to each other, wherein at least one of the first 5' overhang, the third 5' overhang and the fourth 5' overhang comprises one of the 4-mer sequences, or complement thereof, recited in Table 4 and Table 5, and wherein the first 5' overhang, the third 5' overhang and the fourth 5' overhang comprise a different 4-mer sequence. In some aspects of the preceding compositions, at least two of the first 5' overhang, the third 5' overhang and the fourth 5' overhang comprises one of the 4-mer sequences, or complement thereof, recited in Table 4 and Table 5. In some aspects of the preceding compositions, each of the first 5' overhang, the third 5' overhang and the fourth 5' overhang comprises one of the 4-mer sequences, or complement thereof, recited in Table 4 and Table 5.

The present disclosure provides compositions comprising a first double-stranded nucleic acid fragment, a second double-stranded nucleic acid fragment, a third double-stranded nucleic acid fragment and an at least fourth double-stranded nucleic acid fragment, wherein the first double-stranded nucleic acid fragment comprises a first 5' overhang and a second 5' overhang, wherein the second double-stranded nucleic acid fragment comprises a third 5' overhang and fourth 5' overhang, wherein the third double-stranded nucleic acid fragment comprises a fifth 5' overhang and a sixth 5' overhang, wherein the at least fourth double-stranded nucleic acid fragment comprises a seventh 5' overhang and an eighth 5' overhang, wherein the second 5' overhang and third 5' overhang are complementary to each other, wherein the fourth 5" overhang and the fifth 5' overhang are complementary to each other, wherein the sixth 5' overhang and the seventh 5' overhang are complementary to each other, wherein at least one of the first 5' overhang, the third 5' overhang, the fifth 5' overhang, the seventh 5' overhang and the eighth 5' comprises one of the 4-mer sequences, or complement thereof, recited in Table 4 and Table 5, and wherein the first 5' overhang, the third 5' overhang, the fifth 5' overhang, the seventh 5' overhang and the eighth 5' overhang comprise a different 4-mer sequence.

In some aspects of the preceding compositions, at least two of the first 5' overhang, the third 5' overhang, the fifth 5' overhang, the seventh 5' overhang and the eighth 5' comprises one of the 4-mer sequences, or complement thereof, recited in Table 4 and Table 5. In some aspects of the preceding compositions, at least three of the first 5' overhang, the third 5' overhang, the fifth 5' overhang, the seventh 5' overhang and the eighth 5' comprises one of the 4-mer sequences, or complement thereof, recited in Table 4 and Table 5. In some aspects of the preceding compositions, at least four of the first 5' overhang, the third 5' overhang, the fifth 5' overhang, the seventh 5' overhang and the eighth 5' comprises one of the 4-mer sequences, or complement thereof, recited in Table 4 and Table 5. In some aspects of the preceding compositions, each of the first 5' overhang, the third 5' overhang, the fifth 5' overhang, the seventh 5' overhang and the eighth 5' comprises one of the 4-mer sequences, or complement thereof, recited in Table 4 and Table 5.

The present disclosure provides compositions comprising a first partially double-stranded nucleic acid molecule and an at least second partially double-stranded nucleic acid molecule, wherein the first partially double-stranded nucleic acid molecule comprises a first 5' overhang and a second 5' overhang, wherein the at least second partially double-stranded nucleic acid molecule comprises a third 5' overhang and fourth 5' overhang, wherein the second 5' overhang and third 5' overhang are complementary to each other, wherein at least one of the first 5' overhang, the third 5' overhang and the fourth 5' overhang comprises one of the 4-mer sequences, or complement thereof, recited in Table 3, Table 4 and Table 5, and wherein the first 5' overhang, the third 5' overhang and the fourth 5' overhang comprise a different 4-mer sequence. In some aspects of the preceding compositions, at least two of the first 5' overhang, the third 5' overhang and the fourth 5' overhang comprises one of the 4-mer sequences, or complement thereof, recited in Table 3, Table 4 and Table 5. In some aspects of the preceding compositions, each of the first 5' overhang, the third 5' overhang and the fourth 5' overhang comprises one of the 4-mer sequences, or complement thereof, recited in Table 3, Table 4 and Table 5.

The present disclosure provides compositions comprising a first double-stranded nucleic acid fragment, a second double-stranded nucleic acid fragment, a third double-stranded nucleic acid fragment and an at least fourth double-stranded nucleic acid fragment, wherein the first double-stranded nucleic acid fragment comprises a first 5' overhang and a second 5' overhang, wherein the second double-stranded nucleic acid fragment comprises a third 5' overhang and fourth 5' overhang, wherein the third double-stranded nucleic acid fragment comprises a fifth 5' overhang and a sixth 5' overhang, wherein the at least fourth double-stranded nucleic acid fragment comprises a seventh 5' overhang and an eighth 5' overhang, wherein the second 5' overhang and third 5' overhang are complementary to each other, wherein the fourth 5' overhang and the fifth 5' overhang are complementary to each other, wherein the sixth 5' overhang and the seventh 5' overhang are complementary to each other, wherein at least one of the first 5' overhang, the third 5' overhang, the fifth 5' overhang, the seventh 5' overhang and the eighth 5' comprises one of the 4-mer sequences, or complement thereof, recited in Table 3, Table 4 and Table 5, and wherein the first 5' overhang, the third 5' overhang, the fifth 5' overhang, the seventh 5' overhang and the eighth 5' overhang comprise a different 4-mer sequence.

In some aspects of the preceding compositions, at least two of the first 5' overhang, the third 5' overhang, the fifth 5' overhang, the seventh 5' overhang and the eighth 5' comprises one of the 4-mer sequences, or complement thereof, recited in Table 3, Table 4 and Table 5. In some aspects of the preceding compositions, at least three of the first 5' overhang, the third 5' overhang, the fifth 5' overhang, the seventh 5' overhang and the eighth 5' comprises one of the 4-mer sequences, or complement thereof, recited in Table 3, Table 4 and Table 5. In some aspects of the preceding compositions, at least four of the first 5' overhang, the third 5' overhang, the fifth 5' overhang, the seventh 5' overhang and the eighth 5' comprises one of the 4-mer sequences, or complement thereof, recited in Table 3, Table 4 and Table 5. In some aspects of the preceding compositions, each of the first 5' overhang, the third 5' overhang, the fifth 5' overhang, the seventh 5' overhang and the eighth 5' comprises one of the 4-mer sequences, or complement thereof, recited in Table 3, Table 4 and Table 5.

In some aspects of the present disclosure, a partially double-stranded nucleic acid molecule can comprise DNA, RNA, XNA or any combination of DNA, RNA and XNA. As used herein, the term "XNA" is used to refer to xeno nucleic acids. As would be appreciated by the skilled artisan, xeno nucleic acids are synthetic nucleic acid analogues comprising a different sugar backbone than the natural nucleic acids DNA and RNA. XNAs can include, but are not limited to, 1,5-anhydrohexitol nucleic acid (HNA), Cyclohexene nucleic acid (CeNA), Threose nucleic acid (TNA), Glycol nucleic acid (GNA), Locked nucleic acid (LNA), Peptide nucleic acid (PNA) and FANA (Fluoro Arabino nucleic acid).

In some aspects, a partially double-stranded nucleic acid molecule can comprise at least one modified nucleic acid. In some aspects, a modified nucleic acid can comprise methylated cytidine. In some aspects, a modified nucleic acid can comprise 5mC (5-methylcytosine), 5hmC (5-hydromethylcytosine), 5fC (5-formylcytosine), 3mA (3-methyladenine), 5-fU (5-formyluridine), 5=hmU (5-hydroxymethyluridme), 5-hoU (5-hydroxyuridine), 7mG (7-methylguanine), 8oxoG (8-oxo-7,8-dihydroguanine), AP (apurinic/apyrimidinic sites), CPDs (Cyclobutane pyrimidine dimers), dI (deoxymosine), dR5P (deoxyribose 5'-phosphate), dU (deoxyuridine), dX (deoxyxanthosine), PA (3'-phospho-α, β-unsaturated aldehyde), rN (ribonucleotides), Tg (Thymine Glycol), TT (TT dimer) and/or Mismatches including AP:A (apurinic/apyrimidinic site base paired with adenine), DHT:A (5,6-dihydrothymine base paired with an adenine), 5-hmU:A (5-hydroxymethyluracil base paired with an adenine), 5-hmU:G (5-hydroxymethyluracil base paired with a guanine), I:T (inosine base paired with a thymine), 6-MeA:T (6-methyladenine base paired with a thymine), 8-OG:C (8-oxoguanine base paired with a cytosine), 8-OG:G (8-oxoguanine base paired with a guanine), U:A (uridine base paired with an adenine) or U:G (uridine base paired with a guanine) or any combination thereof.

In some aspects, a partially double-stranded nucleic acid molecule can comprise at least one non-hybridized sequence, at least one non-symmetrical element, at least one hairpin, at least one G-quadruplex, at least one I-motif, at least one hemi-modified site, at least on CpG or any combination thereof. The at least one non-hybridized sequence, at least one non-symmetrical element, at least one hairpin, at least one G-quadruplex, at least one I-motif, at least one hemi-modified site, at least on CpG or any combination thereof can be used to introduce at least one or at least two unique molecular identifier (UMI) regions.

In some aspects of the compositions of the present disclosure, a partially double-stranded nucleic acid molecule can be attached to at least one solid support. In some aspects of the compositions of the present disclosure, at least one partially double-stranded nucleic acid in the plurality is attached to at least one bead.

In some aspects of the compositions of the present disclosure, a partially double-stranded nucleic acid molecule can comprise at least one hairpin sequence. A hairpin sequence can comprise at least one deoxyuridine base. A hairpin sequence can comprise at least one restriction endonuclease site. The restriction endonuclease site can be a Type II S restriction endonuclease site.

The present disclosure provides compositions comprising a plurality of partially double-stranded nucleic acid molecules, wherein the plurality comprises at least two distinct species of partially double-stranded nucleic acid molecules, wherein the partially double-stranded nucleic acid molecules comprise a first 5' overhang and a second 5' overhang, wherein the first 5' overhang of one species of partially double-stranded nucleic acid molecules is complementary to only one other 5' overhang present in the plurality of double-stranded nucleic acid molecules, and wherein the other 5' overhang is present on a different species of partially double-stranded nucleic acid molecules, and wherein no 5' overhang in the plurality of partially double-stranded nucleic acid molecules is self-complementary.

The present disclosure provides compositions comprising a plurality of partially double-stranded nucleic acid molecules, wherein the plurality comprises at least two distinct species of partially double-stranded nucleic acid molecules, wherein the partially double-stranded nucleic acid molecules comprise a first 3' overhang and a second 3' overhang, wherein the first 3' overhang of one species of partially double-stranded nucleic acid molecules is complementary to only one other 3' overhang present in the plurality of double-stranded nucleic acid molecules, and wherein the other 3' overhang is present on a different species of partially double-stranded nucleic acid molecules, and wherein no 3' overhang in the plurality of partially double-stranded nucleic acid molecules is self-complementary.

In some aspects of the compositions of the present disclosure, at least one partially double-stranded nucleic acid molecule in the plurality does not comprise a first 5' overhang and instead comprises a blunt end and the second 5' overhang.

In some aspects of the compositions of the present disclosure, at least one partially double-stranded nucleic acid molecule in the plurality does not comprise a second 5' overhang and instead comprises a blunt end and the first 5' overhang.

In some aspects of the compositions of the present disclosure, at least one partially double-stranded nucleic acid molecule in the plurality does not comprise a first 3' overhang and instead comprises a blunt end and the second 3' overhang.

In some aspects of the compositions of the present disclosure, at least one partially double-stranded nucleic acid molecule in the plurality does not comprise a second 3' overhang and instead comprises a blunt end and the first 3' overhang.

In some aspects of the compositions of the present disclosure, at least one partially double-stranded nucleic acid in the plurality can comprise at least one modified nucleic acid. The at least one modified nucleic acid can comprise methylated cytidine. The at least one modified nucleic acid comprises 5mC (5-methylcytosme), 5hmC (5-hydromethylcytosme), 5fC (5-formylcytosine), 3mA (3-methyladenine), 5-fU (5-formyluridine), 5-hmU (5-hydroxymethyluridine), 5-hoU (5-hydroxyuridine), 7mG (7-methylguanine), 8oxoG (8-oxo-7,8-dihydroguanine), AP (apurinic/apyrimidinic sites), CPDs (Cyclobutane pyrimidine dimers), dI (deoxyinosine), dR5P (deoxyribose 5'-phosphate), dU (deoxyuridine), dX (deoxyxanthosine), PA (3'-phospho-α, β-unsaturated aldehyde), rN (ribonucleotides), Tg (Thymine Glycol), TT (TT dimer) and/or Mismatches including AP:A (apurinic/apyrimidinic site base paired with adenine), DHT:A (5,6-dihydrothymine base paired with an adenine), 5-hmU:A (5-hydroxymethyluracil base paired with an adenine), 5-hniU:G (5-hydroxymethyluracil base paired with a guanine), I:T (inosine base paired with a thymine), 6-MeA:T (6-methyladenine base paired with a thymine), 8-OG:C (8-oxoguanine base paired with a cytosine), 8-OG:G (8-oxoguanine base paired with a guanine), U:A (uridine base paired with an adenine) or U:G (uridine base paired with a guanine) or any combination thereof.

In some aspects of the methods of the present disclosure, at least one partially double-stranded nucleic acid in the plurality can comprise at least one non-hybridized sequence, at least one non-symmetrical element, at least one hairpin, at least one G-quadruplex, at least one I-motif, at least one hemi-modified site, at least on CpG or any combination thereof. The at least one non-hybridized sequence, at least one non-symmetrical element, at least one hairpin, at least one G-quadruplex, at least one I-motif, at least one hemi-modified site, at least on CpG or any combination thereof can be used to introduce at least one or at least two unique molecular identifier (UMI) regions.

In some aspects of the compositions of the present disclosure, at least one partially double-stranded nucleic acid in the plurality can comprise at least one nucleotide substitution, deletion, insertion or any combination thereof that causes at least one amino acid codon variation, deletion, insertion or any combination thereof as compared to a wildtype or reference sequence

In some aspects of the compositions of the present disclosure, at least one partially double-stranded nucleic acid in the plurality can be attached to at least one solid support. In some aspects of the compositions of the present disclosure, at least one partially double-stranded nucleic acid in the plurality is attached to at least one bead.

In some aspects of the compositions of the present disclosure, at least one partially double-stranded nucleic acid in the plurality can comprise at least one hairpin sequence. A hairpin sequence can comprise at least one deoxyuridine base. A hairpin sequence can comprise at least one restriction endonuclease site. The restriction endonuclease site can be a Type II S restriction endonuclease site.

In some aspects of the compositions of the present disclosure, at least one partially double-stranded nucleic acid in the plurality can comprise RNA, DNA, XNA, at least one modified nucleic acid, at least one peptide or any combination thereof.

In some aspects of the compositions of the present disclosure, at least one partially double-stranded nucleic acid in the plurality can be obtained from any source.

In some aspects of the compositions of the present disclosure, at least one partially double-stranded nucleic acid in the plurality is obtained from at least one endonuclease digestion reaction of native DNA, at least one PCR reaction, at least one Recombinase Polymerase Amplification (RPA) reaction, at least one reverse transcription reaction, at least single-stranded geometric synthesis reaction or any combination thereof. In some aspects of the compositions of the present disclosure, at least one partially double-stranded nucleic acid in the plurality can be obtained from chemical synthesis of oligonucleotides.

In some aspects of any method or composition of the present disclosure, a 5' overhang can comprise at least about 1, or at least about 2, or at least about 3, or at least about 4, or at least about 5, or at least about 6 or at least about 7, or at least about 8, or at least about 9, or at least about 10, or at least about 11, or at least about 12, or at least about 13, or at least about 14, or at least about 15, or at least about 16, or at least about 17, or at least about 18, or at least about 19, or at least about 20, or at least about 21, or at least about 22, or at least about 23, or at least about 24, or at least about 25, or at least about 26, or at least about 27, or at least about 28, or at least about 29, or at least about 30, or at least about 35, or at least about 40, or at least about 45, or at least about 50 nucleotides.

In some aspects of any method or composition of the present disclosure, a 5' overhang can consist of at least about 1, or at least about 2, or at least about 3, or at least about 4, or at least about 5, or at least about 6 or at least about 7, or at least about 8, or at least about 9, or at least about 10, or at least about 11, or at least about 12, or at least about 13, or at least about 14, or at least about 15, or at least about 16, or at least about 17, or at least about 18, or at least about 19, or at least about 20, or at least about 21, or at least about 22, or at least about 23, or at least about 24, or at least about 25, or at least about 26, or at least about 27, or at least about 28, or at least about 29, or at least about 30, or at least about 35, or at least about 40, or at least about 45, or at least about 50 nucleotides.

In some aspects of any method or composition of the present disclosure, a 3' overhang can comprise at least about 1, or at least about 2, or at least about 3, or at least about 4, or at least about 5, or at least about 6 or at least about 7, or at least about 8, or at least about 9, or at least about 10, or at least about 11, or at least about 12, or at least about 13, or at least about 14, or at least about 15, or at least about 16, or at least about 17, or at least about 18, or at least about 19, or at least about 20, or at least about 21, or at least about 22, or at least about 23, or at least about 24, or at least about 25, or at least about 26, or at least about 27, or at least about 28, or at least about 29, or at least about 30, or at least about 35, or at least about 40, or at least about 45, or at least about 50 nucleotides.

In some aspects of any method or composition of the present disclosure, a 3' overhang can consist of at least about 1, or at least about 2, or at least about 3, or at least about 4, or at least about 5, or at least about 6 or at least about 7, or at least about 8, or at least about 9, or at least about 10, or at least about 11, or at least about 12, or at least about 13, or at least about 14, or at least about 15, or at least about 16, or at least about 17, or at least about 18, or at least about 19, or at least about 20, or at least about 21, or at least about 22, or at least about 23, or at least about 24, or at least about 25, or at least about 26, or at least about 27, or at least about 28, or at least about 29, or at least about 30, or at least about 35, or at least about 40, or at least about 45, or at least about 50 nucleotides.

### Methods of the Present Disclosure

The methods of the present disclosure can comprise the use of any of the compositions described herein. As used herein in the methods of the present disclosure, a double-stranded nuclei acid fragment or a double-stranded nucleic acid molecule can be a partially double-stranded nucleic acid fragment or a partially double-stranded nucleic acid molecule.

The present disclosure provides methods of synthesizing a target double-stranded nucleic acid molecule comprising a target nucleic acid sequence, the methods comprising a) determining an assembly map of the desired double-stranded nucleic acid molecule, wherein the assembly map divides the target double-stranded nucleic acid molecule into a plurality of double-stranded nucleic acid fragments, wherein the double-stranded nucleic acid fragments comprise at least two 5' overhangs, wherein nucleic acid fragments that are adjacent within the target nucleic acid sequence comprise 5' overhangs that are complementary, wherein the 5' overhangs of at least one pair of nucleic acid fragments that are adjacent within the target nucleic acid sequence each comprise one of the 4-mer sequences, or complement thereof, of a 4-mer triplet, wherein the 4-mer triplet comprises three 4-mer sequences, which yield a single fragment with at least 90% purity upon ligation of the at least one pair of adjacent nucleic acid fragments; b) providing the double-stranded nucleic acid fragments determined in step (a); c) hybridizing a first pair of double-stranded nucleic acid fragments that are adjacent within the target nucleic acid via their complementary 5' overhangs; d) ligating the hybridized nucleic acid fragments from step (c) to form a double-stranded nucleic acid fragment; e) hybridizing a second pair of double-stranded nucleic acid fragments that are adjacent within the target nucleic acid via their complementary 5' overhangs; f) ligating the hybridized nucleic acid fragments from step (e) to form a double-stranded nucleic acid fragment, such that the double-stranded nucleic acid fragment is adjacent within the target nucleic acid sequence to the double-stranded nucleic acid formed in step (d); g) repeating steps (c)-(f) using the ligation products such that the target double-stranded nucleic acid molecule is synthesized.

The present disclosure provides methods of synthesizing a target double-stranded nucleic acid molecule comprising a target nucleic acid sequence, the methods comprising: a) determining an assembly map of the desired double-stranded nucleic acid molecule, wherein the assembly map divides the target double-stranded nucleic acid molecule into a plurality of double-stranded nucleic acid fragments, wherein the double-stranded nucleic acid fragments comprise at least two 5' overhangs, wherein nucleic acid fragments that are adjacent within the target nucleic acid sequence comprise 5' overhangs that are complementary, wherein the 5' overhangs of at least one pair of nucleic acid fragments that are adjacent within the target nucleic acid sequence each comprise one of the 4-mer sequences, or complement thereof, of a 4-mer triplet, wherein the 4-mer triplet comprises three 4-mer sequences, which yield only one fragment upon ligation of the at least one pair of adjacent nucleic acid fragments; b) providing the double-stranded nucleic acid fragments determined in step (a); c) hybridizing a first pair of double-stranded nucleic acid fragments that are adjacent within the target nucleic acid via their complementary 5' overhangs; d) ligating the hybridized nucleic acid fragments from step (c) to form a double-stranded nucleic acid fragment; e) hybridizing a second pair of double-stranded nucleic acid fragments that are adjacent within the target nucleic acid via their complementary 5' overhangs; f) ligating the hybridized nucleic acid fragments from step (e) to form a double-stranded nucleic acid fragment, such that the double-stranded nucleic acid fragment is adjacent within the target nucleic acid sequence to the double-stranded nucleic acid formed in step (d); g) repeating steps (c)-(f) using the ligation products such that the target double-stranded nucleic acid molecule is synthesized. In some aspects, the 4-mer triplet can selected from the 4-mer triplets recited in Table 1.

The present disclosure provides methods of synthesizing a target double-stranded nucleic acid molecule comprising a target nucleic acid sequence, the methods comprising: a) determining an assembly map of the desired double-stranded nucleic acid molecule, wherein the assembly map divides the target double-stranded nucleic acid molecule into a plurality of double-stranded nucleic acid fragments, wherein the double-stranded nucleic acid fragments comprise at least two 5' overhangs, wherein nucleic acid fragments that are adjacent within the target nucleic acid sequence comprise 5' overhangs that are complementary, wherein the 5' overhangs of at least one pair of nucleic acid fragments that are adjacent within the target nucleic acid sequence each comprise one of the 4-mer sequences, or complement thereof, of a 4-mer triplet, wherein the 4-mer triplet is selected from the 4-mer triplets recited in Table 1; b) providing the double-stranded nucleic acid fragments determined in step (a); c) hybridizing a first pair of double-stranded nucleic acid fragments that are adjacent within the target nucleic acid via their complementary 5' overhangs; d) ligating the hybridized nucleic acid fragments from step (c) to form a double-stranded nucleic acid fragment; e) hybridizing a second pair of double-stranded nucleic acid fragments that are adjacent within the target nucleic acid via their complementary 5' overhangs; f) ligating the hybridized nucleic acid fragments from step (e) to form a double-stranded nucleic acid fragment, such that the double-stranded nucleic acid fragment is adjacent within the target nucleic acid sequence to the double-stranded nucleic acid formed in step (d), and g) repeating steps (c)-(f) using the ligation products such that the target double-stranded nucleic acid molecule is synthesized.

The present disclosure provides methods of synthesizing a target double-stranded nucleic acid molecule comprising a target nucleic acid sequence, the methods comprising: a) determining an assembly map of the desired double-stranded nucleic acid molecule, wherein the assembly map divides the target double-stranded nucleic acid molecule into a plurality of double-stranded nucleic acid fragments, wherein the double-stranded nucleic acid fragments comprise at least two 5' overhangs, wherein nucleic acid fragments that are adjacent within the target nucleic acid sequence comprise 5' overhangs that are complementary, wherein at least one 5' overhang of at least one pair of nucleic acid fragments that are adjacent within the target nucleic acid sequence comprises at least one 4-mer, or complement thereof, recited in Table 3, Table 4, Table 5 or any combination thereof; b) providing the double-stranded nucleic acid fragments determined in step (a); c) hybridizing a first pair of double-stranded nucleic acid fragments that are adjacent within the target nucleic acid via their complementary 5' overhangs; d) ligating the hybridized nucleic acid fragments from step (c) to form a double-stranded nucleic acid fragment; e) hybridizing a second pair of double-stranded nucleic acid fragments that are adjacent within the target nucleic acid via their complementary 5' overhangs; f) ligating the hybridized nucleic acid fragments from step (e) to form a double-stranded nucleic acid fragment, such that the double-stranded nucleic acid fragment is adjacent within the target nucleic acid sequence to the double-stranded nucleic acid formed in step (d); g) repeating steps (c)-(f) using the ligation products such that the target double-stranded nucleic acid molecule is synthesized.

The present disclosure provides methods of synthesizing a target double-stranded nucleic acid molecule comprising a target nucleic acid sequence, the methods comprising: a) determining an assembly map of the desired double-stranded nucleic acid molecule, wherein the assembly map divides the target double-stranded nucleic acid molecule into a plurality of double-stranded nucleic acid fragments, wherein the double-stranded nucleic acid fragments comprise at least two 5' overhangs, wherein nucleic acid fragments that are adjacent within the target nucleic acid sequence comprise 5' overhangs that are complementary, wherein the 5' overhangs of at least one set of four nucleic acid fragments that are adjacent within the target nucleic acid sequence each comprise one of the 4-mer sequences, or complement thereof, of a 4-mer quintuplet, wherein the 4-mer quintuplet comprises five 4-mer sequences, which yield a single fragment with at least 90% purity upon ligation of the at least one set of four nucleic acid fragments; b) providing the double-stranded nucleic acid fragments determined in step (a); c) hybridizing a first pair of double-stranded nucleic acid fragments that are adjacent within the target nucleic acid via their complementary 5' overhangs; d) ligating the hybridized nucleic acid fragments from step (c) to form a double-stranded nucleic acid fragment, e) hybridizing a second pair of double-stranded nucleic acid fragments that are adjacent within the target nucleic acid via their complementary 5' overhangs; f) ligating the hybridized nucleic acid fragments from step (e) to form a double-stranded nucleic acid fragment, such that the double-stranded nucleic acid fragment is adjacent within the target nucleic acid sequence to the double-stranded nucleic acid formed in step (d);and g) repeating steps (c)-(f) using the ligation products such that the target double-stranded nucleic acid molecule is synthesized.

The present disclosure provides methods of synthesizing a target double-stranded nucleic acid molecule comprising a target nucleic acid sequence, the methods comprising: a) determining an assembly map of the desired double-stranded nucleic acid molecule, wherein the assembly map divides the target double-stranded nucleic acid molecule into a plurality of double-stranded nucleic acid fragments, wherein the double-stranded nucleic acid fragments comprise at least two 5' overhangs, wherein nucleic acid fragments that are adjacent within the target nucleic acid sequence comprise 5' overhangs that are complementary, wherein the 5' overhangs of at least one set of four nucleic acid fragments that are adjacent within the target nucleic acid sequence each comprise one of the 4-mer sequences, or complement thereof, of a 4-mer quintuplet, wherein the 4-mer quintuplet comprises five 4-mer sequences, which yield only one fragment upon ligation of the at least one set of four nucleic acid fragments; b) providing the double-stranded nucleic acid fragments determined in step (a); c) hybridizing a first pair of double-stranded nucleic acid fragments that are adjacent within the target nucleic acid via their complementary 5' overhangs; d) ligating the hybridized nucleic acid fragments from step (c) to form a double-stranded nucleic acid fragment; e) hybridizing a second pair of double-stranded nucleic acid fragments that are adjacent within the target nucleic acid via their complementary 5' overhangs; f) ligating the hybridized nucleic acid fragments from step (e) to form a double-stranded nucleic acid fragment, such that the double-stranded nucleic acid fragment is adjacent within the target nucleic acid sequence to the double-stranded nucleic acid formed in step (d); and g) repeating steps (c)-(f) using the ligation products such that the target double-stranded nucleic acid molecule is synthesized. In some aspects, the 4-mer quintuplet can be selected from the 4-mer quintuplets recited in Table 2.

The present disclosure provides methods of synthesizing a target double-stranded nucleic acid molecule comprising a target nucleic acid sequence, the methods comprising: a) determining an assembly map of the desired double-stranded nucleic acid molecule, wherein the assembly map divides the target double-stranded nucleic acid molecule into a plurality of double-stranded nucleic acid fragments, wherein the double-stranded nucleic acid fragments comprise at least two 5' overhangs, wherein nucleic acid fragments that are adjacent within the target nucleic acid sequence comprise 5' overhangs that are complementary, wherein the 5' overhangs of at least one set of four nucleic acid fragments that are adjacent within the target nucleic acid sequence each comprise one of the 4-mer sequences, or complement thereof, of a 4-mer quintuplet, wherein the 4-mer quintuplet is selected from the 4-mer quintuplets recited in Table 2; b) providing the double-stranded nucleic acid fragments determined in step (a); c) hybridizing a first pair of double-stranded nucleic acid fragments that are adjacent within the target nucleic acid via their complementary 5' overhangs; d) ligating the hybridized nucleic acid fragments from step (c) to form a double-stranded nucleic acid fragment; e) hybridizing a second pair of double-stranded nucleic acid fragments that are adjacent within the target nucleic acid via their complementary 5' overhangs; f) ligating the hybridized nucleic acid fragments from step (e) to form a double-stranded nucleic acid fragment, such that the double-stranded nucleic acid fragment is adjacent within the target nucleic acid sequence to the double-stranded nucleic acid formed in step (d); and g) repeating steps (c)-(f) using the ligation products such that the target double-stranded nucleic acid molecule is synthesized.

The present disclosure provides methods of synthesizing a target double-stranded nucleic acid molecule comprising a target nucleic acid sequence, the methods comprising: a) determining an assembly map of the desired double-stranded nucleic acid molecule, wherein the assembly map divides the target double-stranded nucleic acid molecule into a plurality of double-stranded nucleic acid fragments, wherein the double-stranded nucleic acid fragments comprise at least two 5' overhangs, wherein nucleic acid fragments that are adjacent within the target nucleic acid sequence comprise 5' overhangs that are complementary, wherein at least one 5' overhang comprises at least one 4-mer, or complement thereof, recited in Table 3, Table 4, Table 5 or any combination thereof; b) providing the double-stranded nucleic acid fragments determined in step (a); c) hybridizing a first pair of double-stranded nucleic acid fragments that are adjacent within the target nucleic acid via their complementary 5' overhangs; d) ligating the hybridized nucleic acid fragments from step (c) to form a double-stranded nucleic acid fragment; e) hybridizing a second pair of double-stranded nucleic acid fragments that are adjacent within the target nucleic acid via their complementary 5' overhangs; f) ligating the hybridized nucleic acid fragments from step (e) to form a double-stranded nucleic acid fragment, such that the double-stranded nucleic acid fragment is adjacent within the target nucleic acid sequence to the double-stranded nucleic acid formed in step (d); and g) repeating steps (c)-(f) using the ligation products such that the target double-stranded nucleic acid molecule is synthesized. In some aspects, the 4-mer quintuplet can be selected from the 4-mer quintuplets recited in Table 2.

In some aspects of the methods of the present disclosure, the assembly map divides the target double-stranded nucleic acid molecule into at least 4 double-stranded nucleic acid fragments. In some aspects of the methods of the present disclosure, the assembly map divides the target double-stranded nucleic acid molecule into at least about 10, or at least about 20, or at least about 30, or at least about 40, or at least about 50., or at least about 60, or at least about 70, or at least about 80, or at least about 90, or at least about 100, or at least about 110, or at least about 120, or at least about 130, or at least about 140, or at least about 150, or at least about 160, or at least about 170, or at least about 180, or at least about 200, or at least about 225, or at least about 250, or at least about 275, or at least about 300 double-stranded nucleic acid fragments.

In some aspects, the target double-stranded nucleic acid is at least about 100, or at least about 200, or at least about 300, or at least about 400, or at least about 500, or at least about 600, or at least about 700, or at least about 800, or at least about 900, or at least about 1000, or at least about 1100, or at least about 1200, or at least about 1300, or at least about 1400, or at least about 1500, or at least about 1600, or at least about 1700, or at least about 1800, or at least about 1900, or at least about 2000, or at least about 2100, or at least about 2200, or at least about 2300, or at least about 2400, or at least about 2500, or at least about 2600, or at least about 2700, or at least about 2800, or at least about 2900, or at least about 3000, or at least about 3500, or at least about 4000, or at least about 5000, or at least about 6000, or at least about 7000, or at least about 8000, or at least about 9000, or at least about 10000 nucleotides (base pairs) in length.

In some aspects, the target double-stranded nucleic acid molecule can comprise at least one homopolymeric sequence. As used herein, the term homopolymeric sequence is used to refer to any type of repeating nucleic acid sequence, including, but not limited to, repeats of single nucleotides or repeats of small motifs. In some aspects, a homopolymeric sequence can be at least about 10 nucleotides, or at least about 20 nucleotides, or at least about 30 nucleotides, or at least about 40 nucleotides, or at least about 50 nucleotides, or at least about 60 nucleotides, or at least about 70 nucleotides, or at least about 80 nucleotides, or at least about 90 nucleotides, or at least about 100 nucleotides in length.

In some aspects, the target double-stranded nucleic acid molecule can have a GC content of at least about 50%.

In some aspects of the preceding methods, at least one of the double-stranded nucleic acid fragments that corresponds to at least on termini of the target double-stranded nucleic acid molecule can comprise a blunt end. As used herein, the term blunt end is used to refer to the end of a double-stranded nucleic acid molecule that does not have a single stranded overhang.

In some aspects of the preceding methods, at least one of the double-stranded nucleic acid fragments that corresponds to at least on termini of the target double-stranded nucleic acid molecule can comprise a hairpin sequence. In some aspects, the hairpin sequence can comprise at least one deoxyuridine base. In some aspects, the hairpin sequence can comprise at least one restriction endonuclease site.

In some aspects of the preceding methods, the method can further comprise after step (g): h) incubating the ligation products with at least one exonuclease. In aspects wherein a hairpin sequence comprises at least one deoxyuridine base, the method can further comprise after step (h): i) removing the at least one exonuclease; and j) incubating the products of the exonuclease incubation with at least one enzyme that cleaves a deoxyuridine base, thereby cleaving the hairpin sequence. In aspects wherein a hairpin sequence comprises at least one restriction endonuclease site, the method can further comprise after step (h): i) removing the at least one exonuclease; and j) incubating the products of the exonuclease incubation with at least one enzyme that cleaves the at least one restriction endonuclease site, thereby cleaving the hairpin sequence. In some aspects, an enzyme that cleaves a deoxyuridine base can be the USER (NEB) enzyme.

In some aspects of the methods of the present disclosure, ligation can comprise the use of a ligase. Any ligase known in the art may be used. Preferably, the ligase is T7 DNA ligase. Preferably, the ligase is HiFi Tag DNA Ligase.

In some aspects of the methods of the present disclosure, the synthesized target double-stranded nucleic acid molecule has a purity of at least about 80%, or at least about 85%, or at least about 90%, or at least about 95%, or at least about 99%.

In some aspects, the purity of a synthesized target double-stranded nucleic acid molecule refers to the percentage of the total ligation products that were formed as part of a single ligation reaction, or multiple rounds of ligation reactions, that correspond to the correct/desired ligation product. Without wishing to be bound by theory, the methods of the present disclosure comprising the ligation of nuclei acid molecules produce can produce plurality of ligation products, some of which correspond to the correct/desired ligation product, and some that are undesired (side-reactions, incorrect ligations, etc.). The purity of a ligation product, or a target molecule that is being synthesized, can be expressed as a percentage, which corresponds to the percentage of the total ligation products formed which correspond to the correct/desired ligation product.

The present disclosure provides methods of producing at least one target nucleic acid molecule, the methods comprising: (a) providing a first partially double-stranded nucleic acid molecule, wherein the first double-stranded nucleic acid molecule comprises a first 5' overhang and a second 5' overhang; (b) providing a second partially double-stranded nucleic acid molecule, wherein the second partially double-stranded nucleic acid molecule comprises a third 5' overhang and a fourth 5' overhang, wherein the second 5' overhang is complementary to the third 5' overhang; (c) hybridizing the second 5' overhang and the third 5' overhang; (d) ligating the first partially double-stranded nucleic acid molecule and the second partially double-stranded nucleic acid molecule to produce a first ligated fragment, wherein the first ligated fragment comprises the first 5' overhang and the fourth 5' overhang (e) providing a third partially double-stranded nucleic acid molecule, wherein the third double-stranded nucleic acid molecule comprises a fifth 5' overhang and a sixth 5' overhang, wherein the fourth 5' overhang is complementary to the fifth 5' overhang; (f) providing at least a fourth partially double-stranded nucleic acid molecule, wherein the at least fourth partially double-stranded nucleic acid molecule comprises a seventh 5' overhang and a eighth 5' overhang, wherein the sixth 5' overhang is complementary to the seventh 5' overhang; (g) hybridizing the sixth 5' overhang and the seventh 5' overhang; (h) ligating the third partially double-stranded nucleic acid molecule and the at least fourth partially double-stranded nucleic acid molecule to produce an at least second ligated fragment, wherein the at least second ligated fragment comprises the fifth 5' overhang and the eighth 5' overhang; (i) hybridizing the fourth 5' overhang present in the first ligated fragment to the eighth 5' overhang located in the at least second ligated fragment; and (j) ligating the first ligated fragment and at least second ligated fragment to produce an at least third ligated fragment, wherein the at least third ligated fragment comprises the first 5' overhand and the eighth 5' overhang.

In some aspects, the preceding methods can further comprise:(k) Providing an at least fifth partially double-stranded nucleic acid molecule, wherein the at least fifth partially double-stranded nucleic acid molecule comprises a ninth 5' overhang and a tenth 5' overhang, wherein the ninth 5' overhang is complementary to the eighth 5' overhang; (1) hybridizing the ninth 5' overhang and the eighth 5' overhang; and (m) ligating the at least third ligated fragment and the at least fifth partially double-stranded nucleic acid molecule to produce an at least fourth ligated fragment, wherein the at least fourth ligated fragment comprises the first 5' overhang and the tenth 5' overhang.

In some aspects the preceding methods can further comprise: (k) Providing a fifth partially double-stranded nucleic acid molecule, wherein the fifth partially double-stranded nucleic acid molecule comprises a ninth 5' overhang and a tenth 5' overhang, wherein the ninth 5' overhang is complementary to the eighth 5' overhang; (i) Providing an at least sixth partially double-stranded nucleic acid molecule, wherein the at least sixth partially double-stranded nucleic acid molecule comprises an eleventh 5' overhand and a twelfth 5' overhand; wherein the tenth 5' overhang is complementary to the eleventh 5' overhang; (m) hybridizing the tenth 5' overhang and the eleventh 5' overhang; (n) ligating the fifth partially double-stranded nucleic acid molecule and the at least sixth partially double-stranded nucleic acid molecule to produce a fourth ligated fragment, wherein the fourth ligated fragment comprises the ninth 5' overhand and the twelfth 5' overhang; (o) hybridizing the eighth 5' overhand and the ninth 5' overhang; and (p) ligating the at least third ligated fragment to the at least fourth ligated fragment to produce an at least at least fifth ligated fragment, wherein the at least fifth ligated fragment comprises the first 5' overhang and the twelfth 5' overhang.

In some aspects, the preceding methods can further comprise: (k) Providing a fifth partially double-stranded nucleic acid molecule, wherein the fifth partially double-stranded nucleic acid molecule comprises a ninth 5' overhang and a tenth 5' overhang, wherein the ninth 5' overhang is complementary to the eighth 5' overhang; (i) Providing a sixth partially double-stranded nucleic acid molecule, wherein the sixth partially double-stranded nucleic acid molecule comprises an eleventh 5' overhand and a twelfth 5' overhand, wherein the tenth 5' overhang is complementary to the eleventh 5' overhang; (m) hybridizing the tenth 5' overhang and the eleventh 5' overhang; (n) ligating the fifth partially double-stranded nucleic acid molecule and the sixth partially double-stranded nucleic acid molecule to produce a fourth ligated fragment, wherein the fourth ligated fragment comprises the ninth 5' overhand and the twelfth 5' overhang; (o) providing a seventh partially double-stranded nucleic acid molecule, wherein the seventh partially double-stranded nucleic acid molecule comprises a thirteenth 5' overhang and a fourteenth 5' overhang, wherein the thirteenth 5' overhang is complementary to the twelfth 5' overhang; (p) providing an at least eighth partially double-stranded nucleic acid molecule, wherein the at least eighth partially double-stranded nucleic acid molecule comprises a fifteenth 5' overhang and sixteenth 5' overhang, wherein the fourteenth 5' overhang is complementary to the fifteenth 5' overhang; (q) hybridizing the fourteenth 5' overhang and the fifteenth 5' overhang; (r) ligating the seventh partially double-stranded nucleic acid molecule and the at least eighth partially double-stranded nucleic acid molecule to produce an at least fifth ligated fragment, wherein the at least fifth ligated fragment comprises the thirteenth 5' overhang and the sixteenth 5' overhang; (s) hybridizing the twelfth 5' overhang and the thirteenth 5' overhang; (t) ligating the fourth ligated fragment and the at least fifth ligated fragment to produce an at least sixth ligated fragment, wherein the at least sixth ligated fragment comprises the ninth 5' overhang and the sixteenth 5' overhang; (u) hybridizing the eighth 5' overhang to the ninth 5' overhang; (v) ligating the at least sixth ligated fragment and the third ligated fragment to produce an at least seventh ligated fragment, wherein the at least seventh ligated fragment comprises the first 5' overhang and the sixteenth 5' overhang.

The present disclosure provides methods of producing at least one target nucleic acid molecule, the methods comprising: (a) providing a first partially double-stranded nucleic acid molecule, wherein the first double-stranded nucleic acid molecule comprises a first 3' overhang and a second 3' overhang; (b) providing a second partially double-stranded nucleic acid molecule, wherein the second partially double-stranded nucleic acid molecule comprises a third 3' overhang and a fourth 3' overhang, wherein the second 3' overhang is complementary to the third 3' overhang; (c) hybridizing the second 3' overhang and the third 3' overhang; (d) ligating the first partially double-stranded nucleic acid molecule and the second partially double-stranded nucleic acid molecule to produce a first ligated fragment, wherein the first ligated fragment comprises the first 3' overhang and the fourth 3' overhang; (e) providing a third partially double-stranded nucleic acid molecule, wherein the third double-stranded nucleic acid molecule comprises a fifth 3' overhang and a sixth 3' overhang, wherein the fourth 3' overhang is complementary to the fifth 3' overhang; (f) providing at least a fourth partially double-stranded nucleic acid molecule, wherein the at least fourth partially double-stranded nucleic acid molecule comprises a seventh 3' overhang and a eighth 3' overhang, wherein the sixth 3' overhang is complementary to the seventh 3' overhang, and (g) hybridizing the sixth 3' overhang and the seventh 3' overhang; (h) ligating the third partially double-stranded nucleic acid molecule and the at least fourth partially double-stranded nucleic acid molecule to produce an at least second ligated fragment, wherein the at least second ligated fragment comprises the fifth 3' overhang and the eighth 3' overhang; (i) hybridizing the fourth 3' overhang present in the first ligated fragment to the eighth 3' overhang located in the at least second ligated fragment; and (j) ligating the first ligated fragment and at least second ligated fragment to produce an at least third ligated fragment, wherein the at least third ligated fragment comprises the first 3' overhand and the eighth 3' overhang.

In some aspects the preceding methods can further comprise: (k) Providing an at least fifth partially double-stranded nucleic acid molecule, wherein the at least fifth partially double-stranded nucleic acid molecule comprises a ninth 3' overhang and a tenth 3' overhang, wherein the ninth 3' overhang is complementary to the eighth 3' overhang; (1) hybridizing the ninth 3' overhang and the eighth 3' overhang; and (m) ligating the at least third ligated fragment and the at least fifth partially double-stranded nucleic acid molecule to produce an at least fourth ligated fragment, wherein the at least fourth ligated fragment comprises the first 3' overhang and the tenth 3' overhang.

In some aspects, the preceding methods can further comprise: (k) Providing a fifth partially double-stranded nucleic acid molecule, wherein the fifth partially double-stranded nucleic acid molecule comprises a ninth 3' overhang and a tenth 3' overhang, wherein the ninth 3' overhang is complementary to the eighth 3' overhang, (i) Providing an at least sixth partially double-stranded nucleic acid molecule, wherein the at least sixth partially double-stranded nucleic acid molecule comprises an eleventh 3' overhand and a twelfth 3' overhand; wherein the tenth 3' overhang is complementary to the eleventh 3' overhang; (m) hybridizing the tenth 3' overhang and the eleventh 3' overhang; (n) ligating the fifth partially double-stranded nucleic acid molecule and the at least sixth partially double-stranded nucleic acid molecule to produce a fourth ligated fragment, wherein the fourth ligated fragment comprises the ninth 3' overhand and the twelfth 3' overhang; (o) hybridizing the eighth 3' overhand and the ninth 3' overhang; and (p) ligating the at least third ligated fragment to the at least fourth ligated fragment to produce an at least at least fifth ligated fragment, wherein the at least fifth ligated fragment comprises the first 3' overhang and the twelfth 3' overhang.

In some aspects, the preceding methods can further comprise: (k) Providing a fifth partially double-stranded nucleic acid molecule, wherein the fifth partially double-stranded nucleic acid molecule comprises a ninth 3' overhang and a tenth 3' overhang, wherein the ninth 3' overhang is complementary to the eighth 3' overhang; (i) Providing a sixth partially double-stranded nucleic acid molecule, wherein the sixth partially double-stranded nucleic acid molecule comprises an eleventh 3' overhand and a twelfth 3' overhand, wherein the tenth 3' overhang is complementary to the eleventh 3' overhang; (m) hybridizing the tenth 3' overhang and the eleventh 3' overhang; (n) ligating the fifth partially double-stranded nucleic acid molecule and the sixth partially double-stranded nucleic acid molecule to produce a fourth ligated fragment, wherein the fourth ligated fragment comprises the ninth 3' overhand and the twelfth 3' overhang; (o) providing a seventh partially double-stranded nucleic acid molecule, wherein the seventh partially double-stranded nucleic acid molecule comprises a thirteenth 3' overhang and a fourteenth 3' overhang, wherein the thirteenth 3' overhang is complementary to the twelfth 3' overhang; (p) providing an at least eighth partially double-stranded nucleic acid molecule, wherein the at least eighth partially double-stranded nucleic acid molecule comprises a fifteenth 3' overhang and sixteenth 3' overhang, wherein the fourteenth 3' overhang is complementary to the fifteenth 3' overhang; (q) hybridizing the fourteenth 3' overhang and the fifteenth 3' overhang; (r) ligating the seventh partially double-stranded nucleic acid molecule and the at least eighth partially double-stranded nucleic acid molecule to produce an at least fifth ligated fragment, wherein the at least fifth ligated fragment comprises the thirteenth 3' overhang and the sixteenth 3' overhang, (s) hybridizing the twelfth 3' overhang and the thirteenth 3' overhang; (t) ligating the fourth ligated fragment and the at least fifth ligated fragment to produce an at least sixth ligated fragment, wherein the at least sixth ligated fragment comprises the ninth 3' overhang and the sixteenth 3' overhang; (u) hybridizing the eighth 3' overhang to the ninth 3' overhang; (v) ligating the at least sixth ligated fragment and the third ligated fragment to produce an at least seventh ligated fragment, wherein the at least seventh ligated fragment comprises the first 3' overhang and the sixteenth 3' overhang.

In some aspects of the methods of the present disclosure, the first 5' overhang, the fourth 5' overhang, the fifth 5' overhang, the eighth 5' overhang, ninth 5' overhang, the tenth 5' overhang, the twelfth 5' overhang, the thirteenth 5' overhang, the sixteenth 5' overhang or any combination thereof can comprise a hairpin sequence.

In some aspects of the methods of the present disclosure, the first 3' overhang, the fourth 3' overhang, the fifth 3' overhang, the eighth 3' overhang, ninth 3' overhang, the tenth 3' overhang, the twelfth 3' overhang, the thirteenth 3' overhang, the sixteenth 3' overhang or any combination thereof can comprise a hairpin sequence.

In some aspects of the methods of the present disclosure, a hairpin sequence can comprise at least one deoxyuridine base. In some aspects of the methods of the present disclosure, a hairpin sequence can comprise at least one restriction endonuclease site. The restriction endonuclease site can be a Type II S restriction endonuclease site.

In some aspects, the preceding methods can further comprise after step (d), incubating the reaction of step (d) with at least one exonuclease. In some aspects, the preceding methods can further comprise after step (h), incubating the reaction of step (h) with at least one exonuclease. In some aspects, the preceding methods can further comprise after step (j), incubating the reaction of step (j) with at least one exonuclease. In some aspects, the preceding methods can further comprise after step (m), incubating the reaction of step (m) with at least one exonuclease. In some aspects, the preceding methods can further comprise after step (n), incubating the reaction of step (n) with at least one exonuclease. In some aspects, the preceding methods can further comprise after step (p), incubating the reaction of step (p) with at least one exonuclease. In some aspects, the preceding methods can further comprise after step (r), incubating the reaction of step (r) with at least one exonuclease. In some aspects, the preceding methods can further comprise after after step (t), incubating the reaction of step (t) with at least one exonuclease. In some aspects, the preceding methods can further comprise after step (v), incubating the reaction of step (v) with at least one exonuclease. In some aspects of the methods of the present disclosure, a ligation reaction can be followed by an incubation of the ligation reaction components with at least exonuclease.

In some aspects of the methods of the present disclosure, an incubation with at least one exonuclease results in the digestion of any nucleic acid fragment not capped at both ends with a hairpin sequence.

In some aspects, the methods of the present disclosure can further comprise after incubation with the at least one exonuclease: removing the at least one exonuclease; and contacting the product of the enxonuclease incubation with at least one enzyme that cleaves at deoxyuridine, thereby removing the hairpin sequence.

In some aspects, the methods of the present disclosure can further comprise after incubation with at the at least one exonuclease: removing the at least one exonuclease; and contacting the product of the exonuclease incubation with at least one endonuclease that cleaves the at least one restriction endonuclease site in the hairpin sequence, thereby removing the hairpin sequence.

In some aspects of the preceding methods, the first partially double-stranded nucleic acid molecule does not comprise the first 5' overhang and instead comprises a blunt end and the second 5' overhang. In some aspects of the preceding methods, the fourth partially double-stranded nucleic acid molecule does not comprise the eighth 5' overhang and instead comprises a blunt end and the seventh 5' overhang. In some aspects of the preceding methods, the at least fifth partially double-stranded nucleic acid molecule does not comprise the tenth 5' overhang and instead comprises a blunt end and the ninth 5' overhang. In some aspects of the preceding methods, the at least sixth partially double-stranded nucleic acid molecule does not comprise the twelfth 5' overhang and instead comprises a blunt end and the eleventh 5' overhang. In some aspects of the preceding methods, the at least eighth partially double-stranded nucleic acid molecule does not comprise the sixteenth 5' overhang and instead comprises a blunt end and the fifteenth 5' overhang.

In some aspects of the preceding methods, the first partially double-stranded nucleic acid molecule does not comprise the first 3' overhang and instead comprises a blunt end and the second 3' overhang. In some aspects of the preceding methods, the fourth partially double-stranded nucleic acid molecule does not comprise the eighth 3' overhang and instead comprises a blunt end and the seventh 3' overhang. In some aspects of the preceding methods, the at least fifth partially double-stranded nucleic acid molecule does not comprise the tenth 3' overhang and instead comprises a blunt end and the ninth 3' overhang. In some aspects of the preceding methods, the at least sixth partially double-stranded nucleic acid molecule does not comprise the twelfth 3' overhang and instead comprises a blunt end and the eleventh 3' overhang. In some aspects of the preceding methods, the at least eighth partially double-stranded nucleic acid molecule does not comprise the sixteenth 3' overhang and instead comprises a blunt end and the fifteenth 3' overhang.

The present disclosure provide a method of producing at least one target nucleic acid molecule, the methods comprising: (a) Providing at least one template double-stranded nucleic acid molecule comprising a first template strand and a second template strand; (b) Amplifying a first portion of the at least one template double-stranded nucleic acid molecule using a first primer molecule that hybridizes to a first region on the second template strand and a second primer molecule that hybridizes to a second region on the first template strand to produce at least one first double-stranded nucleic acid fragment; (c) Amplifying a second portion of the at least one template double-stranded nucleic acid molecule using a third primer molecule that hybridizes to the third region on the second strand and a fourth primer molecule that hybridizes to a fourth region on the first template strand to produce at least one second double-stranded nucleic acid fragment; (d) Amplifying a third portion of the at least one template double-stranded nucleic acid molecule using a fifth primer molecule that hybridizes to the fifth region on the second template strand and a sixth primer molecule that hybridizes to the sixth region on the first template strand to produce at least one third double-stranded nucleic acid fragment; (c) Contacting the at least one first double-stranded nucleic acid fragment with a restriction enzyme to form a first 3' overhang; (f) Contacting the at least one second double-stranded nucleic acid fragment and a restriction enzyme to form a second 3' overhang and third 3' overhang, wherein the second 3' overhang is complementary to the first 3' overhang; (g) Contacting the at least one third double-stranded nucleic acid fragment and a restriction enzyme to form a fourth 3' overhang, wherein the fourth 3' overhang is complementary to the third 3' overhang; (h) Hybridizing the first 3' overhang to the second 3' overhang; (i) Hybridizing the third 3' overhang to the fourth 3' overhang; (j) Ligating the at least one first double-stranded nucleic acid fragment and the at least one double-stranded second fragment; (k) Ligating the at least one second double-stranded nucleic acid fragment and the at least one third double-stranded nucleic acid fragment, thereby producing the at least one target nucleic acid molecule.

The present disclosure provide a method of producing at least one target nucleic acid molecule, the methods comprising: (a) providing at least one template double-stranded nucleic acid molecule comprising a first template strand and a second template strand; (b) amplifying a first portion of the at least one template double-stranded nucleic acid molecule using a first primer molecule that hybridizes to a first region on the second template strand and a second primer molecule that hybridizes to a second region on the first template strand to produce at least one first double-stranded nucleic acid fragment; (c) amplifying a second portion of the at least one template double-stranded nucleic acid molecule using a third primer molecule that hybridizes to the third region on the second strand and a fourth primer molecule that hybridizes to a fourth region on the first template strand to produce at least one second double-stranded nucleic acid fragment; (d) amplifying a third portion of the at least one template double-stranded nucleic acid molecule using a fifth primer molecule that hybridizes to the fifth region on the second template strand and a sixth primer molecule that hybridizes to the sixth region on the first template strand to produce at least one third double-stranded nucleic acid fragment; (e) Contacting the at least one first double-stranded nucleic acid fragment with a restriction enzyme to form a first 3' overhang; (f) contacting the at least one second double-stranded nucleic acid fragment and a restriction enzyme to form a second 3' overhang and third 3' overhang; (g) contacting the at least one third double-stranded nucleic acid fragment and a restriction enzyme to form a fourth 3' overhang, wherein the fourth 3' overhang is complementary to the third 3' overhang; (h) providing at least one fourth double-stranded nucleic acid fragment, wherein the at least one fourth double-stranded nucleic acid fragment comprises a fifth 3' overhang and a sixth 3' overhang, wherein the fifth 3' overhang is complementary to the first 3' overhang and the sixth 3' overhang is complementary to the second 3' overhang; (i) hybridizing the fifth 3' overhang and the first 3' overhang; (j) Hybridizing the sixth 3' overhang and the second 3' overhang; (k) Hybridizing the third 3' overhang to the fourth 3' overhang; (l) ligating the at least one first double-stranded nucleic acid fragment and the at least one fourth double-stranded nucleic acid fragment, (m) Ligating the at least one fourth double-stranded nucleic acid fragment and the at least one second double-stranded second fragment; (n) Ligating the at least one second double-stranded nucleic acid fragment and the at least one third double-stranded nucleic acid fragment, thereby producing the at least one target nucleic acid molecule.

The present disclosure provide a method of producing at least one target nucleic acid molecule, the methods comprising: (a) Providing at least one template double-stranded nucleic acid molecule comprising a first template strand and a second template strand; (b) Amplifying a first portion of the at least one template double-stranded nucleic acid molecule using a first primer molecule that hybridizes to a first region on the second template strand and a second primer molecule that hybridizes to a second region on the first template strand to produce at least one first double-stranded nucleic acid fragment; (c) Amplifying a second portion of the at least one template double-stranded nucleic acid molecule using a third primer molecule that hybridizes to the third region on the second strand and a fourth primer molecule that hybridizes to a fourth region on the first template strand to produce at least one second double-stranded nucleic acid fragment; (d) Amplifying a third portion of the at least one template double-stranded nucleic acid molecule using a fifth primer molecule that hybridizes to the fifth region on the second template strand and a sixth primer molecule that hybridizes to the sixth region on the first template strand to produce at least one third double-stranded nucleic acid fragment; (e) Contacting the at least one first double-stranded nucleic acid fragment with a restriction enzyme to form a first 3' overhang; (f) Contacting the at least one second double-stranded nucleic acid fragment and a restriction enzyme to form a second 3' overhang and third 3' overhang, wherein the second 3' overhang is complementary to the first 3' overhang; (g) Contacting the at least one third double-stranded nucleic acid fragment and a restriction enzyme to form a fourth 3' overhang; (h) Providing at least one fourth double-stranded nucleic acid fragment, wherein the at least one fourth double-stranded nucleic acid fragment comprises a fifth 3' overhang and a sixth 3' overhang, wherein the fifth 3' overhang is complementary to the first 3' overhang and the sixth 3' overhang is complementary to the second 3' overhang; (i) Providing at least one fifth double-stranded nucleic acid fragment, wherein the at least one fifth double-stranded nucleic acid fragment comprises a seventh 3' overhang and a eighth 3' overhang, wherein the seventh 3' overhang is complementary to the third 3' overhang and the eighth 3' overhang is complementary to the fourth 3' overhang; (j) Hybridizing the fifth 3' overhang and the first 3' overhang; (k) Hybridizing the sixth 3' overhang and the second 3' overhang; (1) Hybridizing the seventh 3' overhang and the third 3' overhang; (m) Hybridizing the eighth 3' overhang and the fourth 3' overhang; (n) Ligating the at least one first double-stranded nucleic acid fragment and the at least one fourth double-stranded nucleic acid fragment; (o) Ligating the at least one fourth double-stranded nucleic acid fragment and the at least one second double-stranded nucleic acid fragment; (p) Ligating the at least one second double-stranded fragment and the at least one fifth double-stranded nucleic acid fragment; (q) Ligating the at least one fifth double-stranded nucleic acid fragment and the at least one third double-stranded nucleic acid fragment, thereby producing the at least one target nucleic acid molecule.

The present disclosure provide a method of producing at least one target nucleic acid molecule, the methods comprising: (a) Providing at least one template double-stranded nucleic acid molecule comprising a first template strand and a second template strand; (b) Amplifying a first portion of the at least one template double-stranded nucleic acid molecule using a first primer molecule that hybridizes to a first region on the second template strand and a second primer molecule that hybridizes to a second region on the first template strand to produce at least one first double-stranded nucleic acid fragment; (c) Amplifying a second portion of the at least one template double-stranded nucleic acid molecule using a third primer molecule that hybridizes to the third region on the second strand and a fourth primer molecule that hybridizes to a fourth region on the first template strand to produce at least one second double-stranded nucleic acid fragment; (d) Amplifying a third portion of the at least one template double-stranded nucleic acid molecule using a fifth primer molecule that hybridizes to the fifth region on the second template strand and a sixth primer molecule that hybridizes to the sixth region on the first template strand to produce at least one third double-stranded nucleic acid fragment; (c) Contacting the at least one first double-stranded nucleic acid fragment with a restriction enzyme to form a first 5' overhang; (f) Contacting the at least one second double-stranded nucleic acid fragment and a restriction enzyme to form a second 5' overhang and third 5' overhang, wherein the second 5' overhang is complementary to the first 5' overhang; (g) Contacting the at least one third double-stranded nucleic acid fragment and a restriction enzyme to form a fourth 5' overhang, wherein the fourth 5' overhang is complementary to the third 5' overhang; (h) Hybridizing the first 5' overhang to the second 5' overhang; (i) Hybridizing the third 5' overhang to the fourth 5' overhang; (j) Ligating the at least one first double-stranded nucleic acid fragment and the at least one double-stranded second fragment; (k) Ligating the at least one second double-stranded nucleic acid fragment and the at least one third double-stranded nucleic acid fragment, thereby producing the at least one target nucleic acid molecule.

The present disclosure provide a method of producing at least one target nucleic acid molecule, the methods comprising: (a) providing at least one template double-stranded nucleic acid molecule comprising a first template strand and a second template strand; (b) amplifying a first portion of the at least one template double-stranded nucleic acid molecule using a first primer molecule that hybridizes to a first region on the second template strand and a second primer molecule that hybridizes to a second region on the first template strand to produce at least one first double-stranded nucleic acid fragment; (c) amplifying a second portion of the at least one template double-stranded nucleic acid molecule using a third primer molecule that hybridizes to the third region on the second strand and a fourth primer molecule that hybridizes to a fourth region on the first template strand to produce at least one second double-stranded nucleic acid fragment; (d) amplifying a third portion of the at least one template double-stranded nucleic acid molecule using a fifth primer molecule that hybridizes to the fifth region on the second template strand and a sixth primer molecule that hybridizes to the sixth region on the first template strand to produce at least one third double-stranded nucleic acid fragment; (e) Contacting the at least one first double-stranded nucleic acid fragment with a restriction enzyme to form a first 5' overhang; (f) contacting the at least one second double-stranded nucleic acid fragment and a restriction enzyme to form a second 5' overhang and third 5' overhang; (g) contacting the at least one third double-stranded nucleic acid fragment and a restriction enzyme to form a fourth 5' overhang, wherein the fourth 5' overhang is complementary to the third 5' overhang; (h) providing at least one fourth double-stranded nucleic acid fragment, wherein the at least one fourth double-stranded nucleic acid fragment comprises a fifth 5' overhang and a sixth 5' overhang, wherein the fifth 5' overhang is complementary to the first 5' overhang and the sixth 5' overhang is complementary to the second 5' overhang; (i) hybridizing the fifth 5' overhang and the first 5' overhang; (j) Hybridizing the sixth 5' overhang and the second 5' overhang; (k) Hybridizing the third 5' overhang to the fourth 5' overhang; (l) ligating the at least one first double-stranded nucleic acid fragment and the at least one fourth double-stranded nucleic acid fragment, (m) Ligating the at least one fourth double-stranded nucleic acid fragment and the at least one second double-stranded second fragment; (n) Ligating the at least one second double-stranded nucleic acid fragment and the at least one third double-stranded nucleic acid fragment, thereby producing the at least one target nucleic acid molecule.

The present disclosure provide a method of producing at least one target nucleic acid molecule, the methods comprising: (a) Providing at least one template double-stranded nucleic acid molecule comprising a first template strand and a second template strand; (b) Amplifying a first portion of the at least one template double-stranded nucleic acid molecule using a first primer molecule that hybridizes to a first region on the second template strand and a second primer molecule that hybridizes to a second region on the first template strand to produce at least one first double-stranded nucleic acid fragment; (c) Amplifying a second portion of the at least one template double-stranded nucleic acid molecule using a third primer molecule that hybridizes to the third region on the second strand and a fourth primer molecule that hybridizes to a fourth region on the first template strand to produce at least one second double-stranded nucleic acid fragment; (d) Amplifying a third portion of the at least one template double-stranded nucleic acid molecule using a fifth primer molecule that hybridizes to the fifth region on the second template strand and a sixth primer molecule that hybridizes to the sixth region on the first template strand to produce at least one third double-stranded nucleic acid fragment; (e) Contacting the at least one first double-stranded nucleic acid fragment with a restriction enzyme to form a first 5' overhang; (f) Contacting the at least one second double-stranded nucleic acid fragment and a restriction enzyme to form a second 5' overhang and third 5' overhang, wherein the second 5' overhang is complementary to the first 5' overhang; (g) Contacting the at least one third double-stranded nucleic acid fragment and a restriction enzyme to form a fourth 5' overhang; (h) Providing at least one fourth double-stranded nucleic acid fragment, wherein the at least one fourth double-stranded nucleic acid fragment comprises a fifth 5' overhang and a sixth 5' overhang, wherein the fifth 5' overhang is complementary to the first 5' overhang and the sixth 5' overhang is complementary to the second 5' overhang; (i) Providing at least one fifth double-stranded nucleic acid fragment, wherein the at least one fifth double-stranded nucleic acid fragment comprises a seventh 5' overhang and a eighth 5' overhang, wherein the seventh 5' overhang is complementary to the third 5' overhang and the eighth 5' overhang is complementary to the fourth 5" overhang; (j) Hybridizing the fifth 5' overhang and the first 5' overhang; (k) Hybridizing the sixth 5' overhang and the second 5' overhang; (l) Hybridizing the seventh 5' overhang and the third 5' overhang; (m) Hybridizing the eighth 5' overhang and the fourth 5' overhang; (n) Ligating the at least one first double-stranded nucleic acid fragment and the at least one fourth double-stranded nucleic acid fragment; (o) Ligating the at least one fourth double-stranded nucleic acid fragment and the at least one second double-stranded nucleic acid fragment; (p) Ligating the at least one second double-stranded fragment and the at least one fifth double-stranded nucleic acid fragment; (q) Ligating the at least one fifth double-stranded nucleic acid fragment and the at least one third double-stranded nucleic acid fragment, thereby producing the at least one target nucleic acid molecule.

In some aspects of the preceding methods, the second region on the first template strand and the third region on the second template strand can be at least partially complementary. In some aspects of the preceding methods, the fourth region on the first template strand and the fifth region on the second template strand can be at least partially complementary.

In some aspects, the present disclosure provides methods comprising: a) Generation of an assembly map, comprising fragment designs, wherein each fragment possesses 3' and/or 5' overhangs. The 3' or 5' overhangs are selected from a set of N-mer sites, known not to inappropriately cross-hybridize or inappropriately ligate and also known to ligate efficiently with target N-mer sites on adjacent oligonucleotide pairs; b) Contacting two fragments at a time in a ligation reaction leading to a larger new fragment; c) Contacting a fragment either with a blunt ended fragment (i.e. a fragment with only one overhanging single-stranded N-mer or; d) Contacting a fragment with a nucleic acid hairpin with a complementary overhanging single-stranded N-mer.

In some aspects of the preceding methods, at least one nucleic acid molecule or at least one fragment can comprise at least one modified nucleic acid. The at least one modified nucleic acid can comprise methylated cytidine. The at least one modified nucleic acid can comprise 5mC (5-methylcytosine), 5hmC (5-hydromethylcytosine), 5fC (5-formylcytosine), 3mA (3-methyladenine), 5-fU (5-formyluridine), 5-hmU (5-hydroxymethyluridine), 5-hoU (5-hydroxyuridine), 7mG (7-methylguanine), 8oxoG (8-oxo-7,8-dihydroguanine), AP (apurinic/apyrimidinic sites), CPDs (Cyclobutane pyrimidine dimers), dI (deoxyinosine), dR5P (deoxyribose 5'-phosphate), dU (deoxyuridine), dX (deoxyxanthosine), PA (3'-phospho-α, β-unsaturated aldehyde), rN (ribonucleotides), Tg (Thymine Glycol), TT (TT dimer) and/or Mismatches including AP:A (apurinic/apyrimidinic site base paired with adenine), DHT:A (5,6-dihydrothymine base paired with an adenine), 5--hmU:A (5-hydroxymethyluracil base paired with an adenine), 5-hmU:G (5-hydroxymethyluracil base paired with a guanine), I:T (inosine base paired with a thymine), 6-MeA:T (6-methyladenine base paired with a thymine), 8-OG:C (8-oxoguanine base paired with a cytosine), 8-OG:G (8-oxoguanine base paired with a guanine), U:A (uridine base paired with an adenine) or U:G (uridine base paired with a guanine) or any combination thereof.

In some aspects of the preceding methods, at least one nucleic acid molecule or at least one fragment can comprise at least one non-hybridized sequence, at least one non-symmetrical element, at least one hairpin, at least one G-quadruplex, at least one I-motif, at least one hemi-modified site, at least on CpG or any combination thereof. The at least one non-hybridized sequence, at least one non-symmetrical element, at least one hairpin, at least one G-quadruplex, at least one I-motif, at least one hemi-modified site, at least on CpG or any combination thereof can be used to introduce at least one or at least two unique molecular identifier (UMI) regions. The at least one or at least two UMI regions can lead to increased diversity.

In some aspects of the preceding methods, the at least one target nucleic acid molecule is a plurality of target nucleic acid molecules. Thus, in some aspects, the products of the preceding methods is a plurality of target nucleic acid molecules. A plurality of target nucleic acids can comprise at least about 1, or at least about 2, or at least about 3, or at least about 4, or at least about 5, or at least about 6, or at least about 7, or at least about 8, or at least about 9, or at least about 10, or at least about 1.0 × 10², or at least about 1.0 × 10³, or at least about 1.0 × 10⁴, or at least about 1.0 × 10⁵, or at least about 1.0 × 10⁶, or at least about 1.0 × 10⁷, or at least about 1.0 × 10⁸, or at least about 1.0 × 10⁹, or at least about 1,0 × 10¹⁰, or at least about 1.0 × 10¹¹, or at least about 1.0 × 10¹², or at least about 1.0 × 10¹³, or at least about 1.0 × 10¹⁴, or at least about 1.0 × 10¹⁵, or at least about 1.0 × 10¹⁶, or at least about 1.0 × 10¹⁷, or at least about 1.0 × 10¹⁸, or at least about 1.0 × 10¹⁹, or at least about 1.0 × 10²⁰, or at least about 1.0 × 10²⁵, or at least about 1.0 × 10³⁰, or at least about 1.0 × 10³⁵, or at least about 1.0 × 10⁴⁰, or at least about 1.0 × 10¹⁰⁰ distinct target nucleic acid species, wherein each distinct target nucleic acid species comprises a different nucleic acid sequence. In some aspects, each nucleic acid species is present in the plurality in approximately the same amount

In some aspects of the preceding methods, at least one target nucleic acid can comprise at least one nucleotide substitution, deletion, insertion or any combination thereof that causes at least one amino acid codon variation, deletion, insertion or any combination thereof as compared to a wildtype or reference sequence. The distribution of variant substitutions, insertions, deletions or any combination thereof can be approximately even at multiple distal sites.

In some aspects, the products of the methods of the present disclosure can be used for the screening and/or selection of proteins and/or peptides. In some aspects, the products of the methods of the present disclosure can be used for screening and/or selection of at least one protein fusion, at least one protein-peptide fusion and/or at least one peptide-peptide fusions. In some aspects, the products of the methods of the present disclosure can be used for the screening and/or selection of differential methylated promoters, gene bodies, untranslated regions (UTRs) or any combination thereof. In some aspects, the products of the methods of the present disclosure can be used for the screening and/or selection of aptamers, siRNAs, PCR primers, sequencing adapters or any combination thereof. Screening and/or selection can performed using a cell-based assay. Screening and/or selection can performed using a cell-free assay.

In some aspects, the products of the methods of the present disclosure can be used for barcoding or unique molecular identifiers (UMIs). The barcoding or unique molecular identifiers can be used in single cell sequencing.

In some aspects, the products of the methods of the present disclosure can comprise sequences and/or modifications for the attachment of proteins onto a nucleic acid sequence.

In some aspects, the methods of the present disclosure can be performed on at least one solid support. In some aspects, the methods of the present disclosure can be performed on at least one bead. In some aspects, the products of the methods of the present disclosure can be attached to at least one solid support. In some aspects, the products of the methods of the present disclosure can be are attached to at least one bead. In some aspects, the products of the methods of the present disclosure can be attached to at least one bead such that the bead is attached to only nucleic acid molecules comprising the same sequence.

In some aspects of the methods of present disclosure, the first partially double-stranded nucleic acid molecule, the second partially double-stranded nucleic acid molecule, the third partially double-stranded nucleic acid molecule, the fourth partially double-stranded nucleic acid molecule, the fifth partially double-stranded nucleic acid molecule, the sixth partially double-stranded nucleic acid molecule, the seventh partially double-stranded nucleic acid molecule, the eighth partially double-stranded nucleic acid molecule or any combination thereof can comprise RNA, DNA, XNA, at least one modified nucleic acid, at least one peptide or any combination thereof.

In some aspects of the methods of present disclosure, the first partially double-stranded nucleic acid molecule, the second partially double-stranded nucleic acid molecule, the third partially double-stranded nucleic acid molecule, the fourth partially double-stranded nucleic acid molecule, the fifth partially double-stranded nucleic acid molecule, the sixth partially double-stranded nucleic acid molecule, the seventh partially double-stranded nucleic acid molecule, the eighth partially double-stranded nucleic acid molecule or any combination thereof can be obtained from any source.

In some aspects of the methods of present disclosure, the first partially double-stranded nucleic acid molecule, the second partially double-stranded nucleic acid molecule, the third partially double-stranded nucleic acid molecule, the fourth partially double-stranded nucleic acid molecule, the fifth partially double-stranded nucleic acid molecule, the sixth partially double-stranded nucleic acid molecule, the seventh partially double-stranded nucleic acid molecule, the eighth partially double-stranded nucleic acid molecule or any combination thereof can be obtained from at least one endonuclease digestion reaction of native DNA, at least one PCR reaction, at least one Recombinase Polymerase Amplification (RPA) reaction, at least one reverse transcription reaction, at least single-stranded geometric synthesis reaction or any combination thereof.

In some aspects of the methods of present disclosure, the first partially double-stranded nucleic acid molecule, the second partially double-stranded nucleic acid molecule, the third partially double-stranded nucleic acid molecule, the fourth partially double-stranded nucleic acid molecule, the fifth partially double-stranded nucleic acid molecule, the sixth partially double-stranded nucleic acid molecule, the seventh partially double-stranded nucleic acid molecule, the eighth partially double-stranded nucleic acid molecule or any combination thereof can be obtained from chemical synthesis of oligonucleotides.

In some aspects of the methods of present disclosure, the first primer, the second primer, the third primer, the fourth primer, the fifth primer, the sixth primer or any combination thereof can comprise at least one modified nucleic acid The at least one modified nucleic acid can comprise methylated cytidine. The at least one modified nucleic acid can comprise 5mC (5-methylcytosine), 5hmC (5-hydromethylcytosine), 5fC (5-formylcytosine), 3mA (3-methyladenine), 5-fU (5-formyluridine), 5-hmU (5-hydroxymethyluridine), 5-hoU (5-hydroxyuridine), 7mG (7-methylguanine), 8oxoG (8-oxo-7,8-dihydroguanine), AP (apurinic/apyrimidinic sites), CPDs (Cyclobutane pyrimidine dimers), dI (deoxyinosine), dR5P (deoxyribose 5'-phosphate), dU (deoxyuridine), dX (deoxyxanthosine), PA (3'-phospho-α, β-unsaturated aldehyde), rN (ribonucleotides), Tg (Thymine Glycol), TT (TT dimer) and/or Mismatches including AP:A (apurinic/apyrimidinic site base paired with adenine), DHT:A (5,6-dihydrothymme base paired with an adenine), 5-hmU:A (5-hydroxymethyluracil base paired with an adenine), 5-hmU:G (5-hydroxymethyluracil base paired with a guanine), 1:1' (inosine base paired with a thymine), 6-MeA:T (6-methyladenine base paired with a thymine), 8-OG:C (8-oxoguanine base paired with a cytosine), S-OG:G (8-oxoguanine base paired with a guanine), U:A (uridine base paired with an adenine) or U:G (uridine base paired with a guanine) or any combination thereof.

In some aspects of the methods of present disclosure, the first primer, the second primer, the third primer, the fourth primer, the fifth primer, the sixth primer or any combination thereof can comprise at least one nucleotide substitution, deletion, insertion or any combination thereof that causes at least one amino acid codon variation, deletion, insertion or any combination thereof as compared to a wildtype or reference sequence.

In some aspects of the methods of present disclosure, a restriction enzyme can be an MspJI family restriction enzyme. The restriction enzyme can be MSpJI, FspEI, LpnPI, AspBHI, RlaI, SgrTI or any combination thereof.

In some aspects of the methods of present disclosure, the at least one fourth double-stranded nucleic acid fragment, the at least one fifth double-stranded nucleic acid fragment or any combination thereof can comprise at least one nucleotide substitution, deletion, insertion or any combination thereof that causes at least one amino acid codon variation, deletion, insertion or any combination thereof as compared to a wildtype or reference sequence.

### Examples

### Example 1 - double-stranded geometric synthesis (gSynth)

### Example 1A

In this example, the results of a double-stranded gSynth assembly reactions, as described herein, were compared to the results of the existing, alternative method of hybridization and elongation (HAE) using DNA polymerase. HAE is similar to polymerase cycling assembly (PCA) reactions but does not use PCR amplification A variety of programed sequences were synthesized using bother double-stranded gSynth and HAE. These programmed sequences included sequences that had a GC content ranging from 10% to 90% along the length of the sequence, from 20% to 80% along the length of the sequence, from 30% to 70% along the length of the sequence, from 40% to 60% along the length of the sequence, and sequences that were 50% GC along the entire length of the sequence.

Briefly, the double-stranded gSynth reactions were performed as follows: each of the largely double stranded pairs of fragments were first resuspended at 10 µM in annealing buffer (10mM Tris-HCl, 50mM NaCl). The solution was then heated to 95 °C for 30 seconds on a PCR machine, then allowed to cool to room temperature. After annealing in the first ligation reaction, adjacent fragments are combined (2.5 µL each of a 10 µM solution). For fragments lacking a 5' PO₄, the ligation reaction also includes Polynucleotide Kinase (PNK). Thus, in one embodiment, the complete ligation reaction includes: 5µl oligos (2.5 µl pair A, 2.5 µl pair B) + 6 µl of 2x Buffer + 0.5 µl PNK + 0.5 µl T7 DNA ligase. In these reactions 1x Buffer is: 66 mM Tris-HCl, 10 mM MgCl2, 1 mM ATP, 1 mM DTT, 7.5% Polyethylene glycol (PEG 6000), pH 7 6 @ 25°C. Reactions are held at 25 °C. Each subsequent ligation reaction between adjacent fragments is performed by combining all of the reaction volumes of each of the two fragments together.

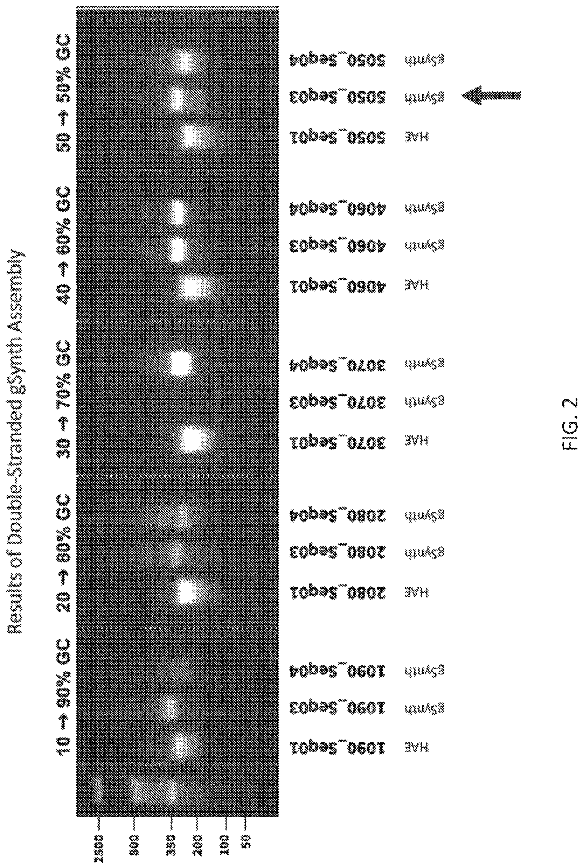

The products of the assembly reactions were analyzed via gel-seperation and the results of the analysis are shown in FIG. 2. As shown in FIG. 2, there was a consistent difference in size between the HAE assembly reaction products and the corresponding gSynth assembly reaction products, with the gSynth assembly reaction products exhibiting sizes that were closer to those that would be expected from the reaction. Additionally, the HAE assembly products showed a broader range of sizes than the gSynth assembly products. Thus, the results of this example demonstrate that the double-stranded gSynth assembly methods of the present disclosure are more accurate than existing methods such as HAE, and more consistently produce nucleic acid molecules of expected sizes.

### Example 1B

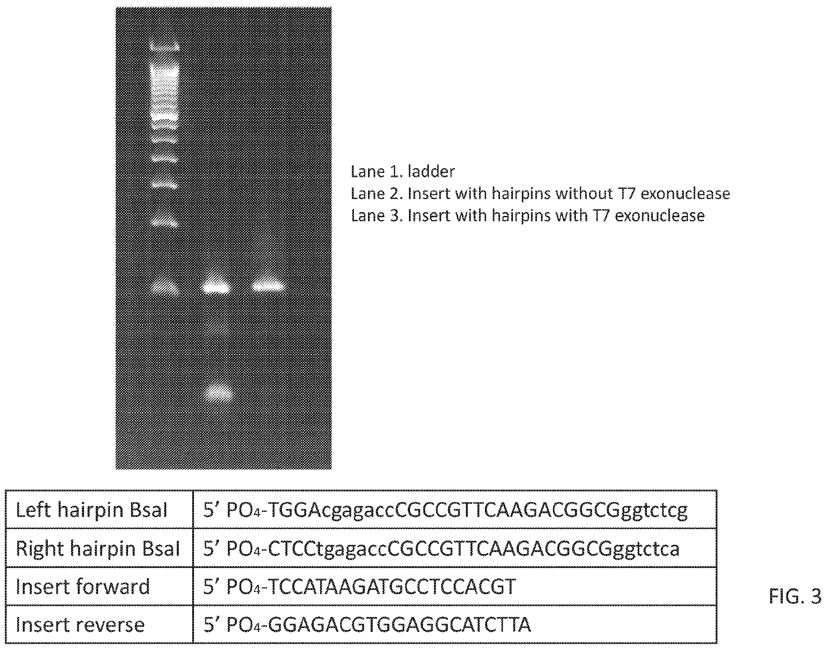

In this example, double-stranded gSynth assembly reactions were performed as described herein, where the double-stranded nucleic acid fragments corresponding to the two termini of the sequence to be synthesized were capped with hairpin structures. The products of the gSynth assembly reaction were then analyzed by gel separation before and after treatment with a T7 exonuclease . Without wishing to be bound by theory, any nucleic acid that is not capped at both ends by a hairpin should be digested by the exonuclease. That is, only desired, full-length assembly products should be present after digestion with T7 exonuclease. The results of the gel separation analysis are shown in FIG. 3. As shown in FIG. 3, only a single product corresponding to the expected molecular weight is observed after digestion with exonuclease. Thus, in the double-stranded gSynth assembly reactions of the present disclosure, terminal fragments can be capped with nucleic acid hairpins and exonuclease digestion can be used to obtain a highly pure product.

### Example 2 - Variant geometric synthesis

The following examples further describe an application of the double-stranded gSynth methods of the present disclosure entitled variant geometric synthesis (V-gSynth), a modular DNA manipulation method for generating gene variant libraries by insertion, deletion and/or substitution of codons.

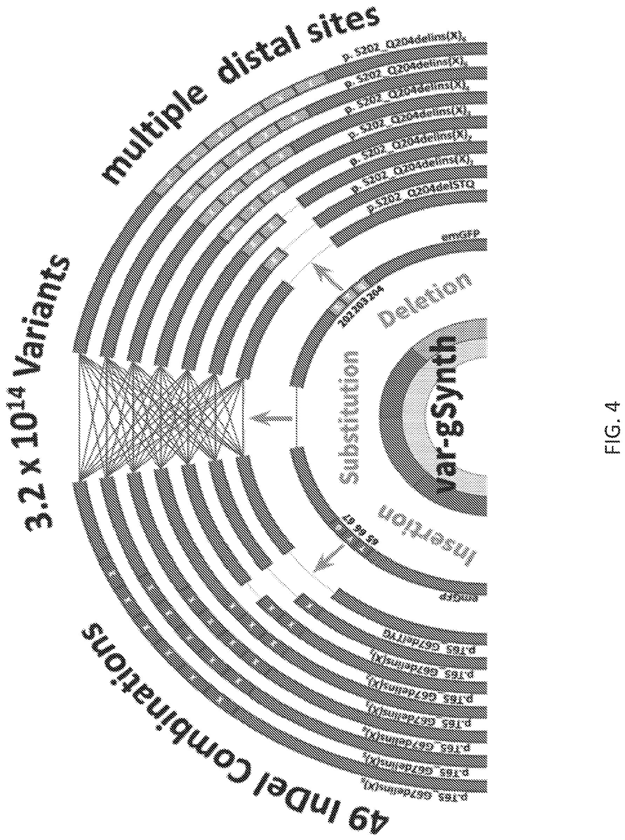

FIG. 4 shows an overview of a V-gSynth reaction used to create a large plurality of emGFP variants. FIG. 4 shows a schematic diagram that describes the possible variants of emGFP that can be synthesized using V-gSynth. As shown in FIG. 4, by combining the six InDels (p.T65_G67delTYG, p.T65_G67delins(X)1, p.T65_G67delins(X)2, p.T65_G67delins(X)3, p.T65_G67delins(X)4, p.T65_G67delins(X)5 and p.T65_G67delins(X)6) generated at positions T65, Y66 and G67, with the six InDeis (p.S202_Q204delSTQ, p.S202_Q204delins(X)1, p._Q204delins(X)2, p.S202_Q204delins(X)3, p.S202_Q204delins(X)4, p.S202_Q204delins(X)5 and p.S202_Q204delins(X)6) generated at positions S202, T203 and Q204; a possible 49 InDel combinations can be generated using Variant Geometric Synthesis. The combination of the 49 InDeis (highlighted by the dark grey lines) can generate up to 3.2 x 10¹⁴ sequence variants across the two distal sites.

To demonstrate the utility of V-gSynth, a substitution-based bead display library of functional GFP variants was constructed. These functional GFP variants exhibited altered spectral properties. Extending this proof of concept, a large variant library containing InDels, with up to 12 codon insertions and an estimated 3.2 ×10¹⁴ protein-coding variants was constructed. Sequencing analysis demonstrated an even codon distribution and extraordinary high diversity of variants that greatly exceeds previous work.

### Example 2A -Generation of variant geometric synthesis (V-gSynth) libraries

To generate diverse gene variant libraries that included insertions and deletions (InDels), a PCR approach was used that involved the preparation from the gene of interest, with primers containing the modified nucleobase 5-methylcytosine, as shown in FIG. 5A. The MspJl family of restriction enzymes (MspJI, FspEI, LpnPI, AspBHI, Rial, and SgrTI) recognize 5-methylcytosine nucleobases and cleaves both strands of the DNA, N12/N16 nucleotides from the 5-methylcytosine, generating a 3-prime, four nucleotide overhang. Without wishing to be bound by theory, this method is advantageous as, in contrast to restriction enzyme-based methods, the 5-methylcytosine base be incorporated at a desired location throughout a gene and consequently this approach can be scaled for the production of many different targeted gene variant libraries.

After preparation of the methylated fragments, FspEI was used to create four-nucleotide overhangs that can be assembled, via ligation, into the required gene, as shown in FIG. 5B and FIG. 5D. As only fragments containing the 5-methylcytosine nucleobase are digested, and not the template DNA, this approach also removes the DpnI digestion step that is used in other protocols.

To create non-synonymous mutations, codon changes can be incorporated into the oligos used to amplify the different fragments, as shown in FIG. 5A and FIG. 5B. When creating InDels variants, new oligos pairs, which have four-nucleotide overhangs, can be incorporated into the ligation as shown in FIG. 5C and FIG. 5D. Without wishing to be bound by theory, T7 DNA ligase was used for the assembly reactions, due to the increased activity of T7 DNA ligase for four-nucleotide overhangs as compared to either shorter overhangs or blunt ended DNA.

### Example 2B - Wild type, Y66W, T203Y and [Y66W; T203Y] in vitro transcription and translation (IVTT) templates

Initial V-gSynth experiments consisted of assembling four IVTT templates. The motivation to generate IVTT templates was based on the GFP variants p.Y66W, p.T203Y and p. [Y66W; T203Y] described by Sawano *et al.* (Sawano, A. "Directed Evolution of Green Fluorescent Protein by a New Versatile PCR Strategy for Site-Directed and Semi-Random Mutagenesis." Nucleic Acids Research, vol. 28, no. 16, 2000, doi:10.1093/nar/28.16.e78.). Using two excitation wavelength 488 nm and 440 nm, the four IVTT templates should be distinguishable by their 488/440 nm ratio, where the ratio of p.T203Y > wild-type > p.[Y66W; T203Y] > p.Y66W. Each of the wild-type, p.Y66W, p.T203Y and p.[Y66W; T203Y] IVTT templates were assembled from three FspEI digested, Methylated Fragments as shown in FIGs. 5A and 5B, as well as FIG. 6A. For example, the p.[Y66W;, T203Y] IVTT template required the assembly of Digested Fragment I-Y66W, Fragment 2 and Fragment 3-T203Y (FIG. 6A), with a single product for each methylated and Digested Fragment being generated (FIG. 6B). Assembly of the Digested Fragment 1-Y66W, Fragment 2 and Fragment 3-T203Y yielded the full-length p.[Y66W; T203Y] IVTT template along with the two intermediate products, from the initial ligation of Fragment 1-Y66W to Fragment 2, along with the initial ligation of Fragments 2 to 3-T203Y (FIG. 6B).

Next, the wild-type, p.Y66W, p.T203Y and p.[Y66W; T203Y] IVTT templates were evaluated within the PUREexpress system. The expression of the wild-type, p.Y66W, p.T203Y and p. [Y66W; T203Y] IVTT templates yielded emGFP variants of the same size and with comparable expression levels to the original pRSET/emGFP plasmid (FIG. 6C). Finally, Sanger sequencing provide the last piece of evidence to show the wild-type, p. Y66W, p.T203Y and p.[Y66W; T203Y] IVTT templates had been assembled in-frame.

### Example 2C - On-bead wild-type, p.Y66W, p.T203Y and p.[Y66W; T203Y] IVTT templates and p. [Y66X; T203X] IVTT library

A monoclonal, on-bead p.[Y66X; T203X] IVTT library was constructed (along with on-bead wild-type, p.Y66W, p.T203Y and p.[Y66W; T203Y] IVTT templates as controls). A combination of sixteen individual variants at position Y66 (Y66N, Y66T, Y66S, Y66I, Y66H, Y66P, Y66R, Y66L, Y66D, Y66A, Y66G, Y66V, Y66, Y66S, Y66C and Y66F) and a further sixteen individual variants at position T203 (T203N, T203, T203S, T203I, T203H, T203P, T203R, T203L, T203D, T203A, T203G, T203V, T203S, T203C and T203F) constitute the 256 members of the p.[Y66X; T203X] IVTT library (FIG. 7A). During the assembly of the p.[Y66X; T203X] IVTT library and IVTT template controls, Primer 6-azide was used to introduce the required azide modification to covalently attach the IVTT library and templates to magnetic DBCO beads.

Assembly of the on-bead p.[Y66X; T203X] IVTT library was first confirmed by NGS. Sequencing of the p.[Y66X; T203X] IVTT library generated the raw fastq files which contained 34,980 paired-end reads, from which 31,283 (89.4%) where the desired in-frame reads, which contained the Adapter 1a sequence (nucleotide position 202 to 214) and the in-frame nucleotides ACC (nucleotide position 196- 198, codon position T65) in read 1, as well as the Adapter 2a sequence (nucleotide position 609 to 597), and the in-frame nucleotides CTG (base position 615 to 613, codon position (204) in Read 2, as shown in FIG. 7A. From the remaining 31,283 (89.4%) reads the base and codon composition at positions Y66X and T203X were calculated. The wild type sequence was represented by 128 (0.40%) of the 31,283 reads, with the median and expected value, for each individual member from the 256 members of the p. [Y66X; T203X] IVTT library being 102 ± 60.2 (0.32 ± 0.19%) and 122 (0.39%), respectively.

Overall, the sequence variations introduced at positions Y66X and T203X create the degenerate nucleotide sequence N₁N₂C₃. Median A, C, G and T values for nucleotide N₁ were 27.1 ± 3.7%, 22.7 ± 0.9%, 24.2 ± 5.2% and 25.9 ± 0.6%; for nucleotide N₂ were 27.6 ± 5.1%, 21.4 ± 2.4%, 24.8 ± 2.2% and 26.2 ± 5.3 and for nucleotide C₃ were 1.8 ± 1.2%, 97.9 ± 1.5%, 0.1 ± 0.0% and 0.2 ± 0.3, respectively (FIG. 7B), with the median codon value being 5.4 ± 2.0% (FIG. 7C). Overall, the %GC for N₁, N₂ and N₃ was 47.0 ± 4.3%, 46.2 ± 0.2% and 98.0 ± 1.5%, respectively.

Once the on-bead p.[Y66X; T203X] library was confirmed, fluorescent imaging was performed. A single monoclonal bead from the p.[Y66X; T203X] library was encapsulated within a single droplet of the IVTT reaction mix. The single droplet will fluoresce according to the monoclonal variant present within the monoclonal DNA on the bead. Individual beads were placed within an emulsion using 2.0% PicoSurf-1 in HFE7500 (v/v). Three images of each emulsion were captured at 488 nm and 440 nm excitation along with the brightfield image, with the 488/440 ratio being overlaid onto the brightfield image for individual droplets containing single beads (FIG. 7D).

The 488/440 ratio for the monoclonal variant library indicates that individual droplets from the library had different spectral properties, which are consistent with the wild-type, p.Y66W, p.T203Y and p. [Y66W; T203Y] controls. Furthermore, there was an increase in droplets which contain a single bead yet did not fluoresce at either 440 or 488 nm, as many of the variants introduced eliminate the fluorescence of that particular GFP variant (FIG. 7D). These results from the production of the monoclonal variant library indicate that V-gSynth is able to faithfully construct a diverse, yet evenly distributed variant library, which when introduced onto beads can produce monoclonal beads suitable for functional assays.

### Example 2D - InDel library p.[T65_G67delTYG; S202_Q204delSTQ] to p.[T65 G67delins(X)6; S202_Q204delins(X)6]

This example demonstrates the production of a hugely diverse InDel library using the double stranded geometric synthesis methods of the present disclosure. The InDel library was an amalgamation of forty-nine InDel combinations. Initially, the three codons T65_Y66_G67 were deleted during the preparation of the Methylated InDel Fragments 1 and 2, while codons S202_T203_Q204 were deleted between Methylated InDel Fragments 2 and 3. FspEI digestion removed a further 12/16 nucleotides from the 5-methycytosine leaving four-nucleotide overhangs suitable for T% DNA ligase (FIGs. 5C-5D and FIGs. 8A-8B). Insertion of a total of fourteen InDel duplexes, seven InDel duplexes per pool for each of the deleted positions T65_Y66_G67 and S202_T203_Q204, created the forty-nine combination of InDels within the InDel library. The two InDel duplexes pools, contained a series of seven InDel duplexes at a ratio of 1 : 16 : 256 : 4096 : 65,536 : 1,048,576 : 16,777,216 which reflects the diversity of the degenerate nucleotide sequence N₁N₂C₃, introduced consecutively from zero up to six times ( FIGs. 9A and 9B). A single assembly reaction, using equimolar concentrations of the digested InDel Fragments 1, 2 and 3 and the two InDel duplex pools created the diverse InDel Library. Following assembly, an NGS library was prepared using Primer NGS Uni and Primer NGS IDX11, to add the Illumina adapter sequence and index.

Sequencing of the InDel Library generated the raw fastq files which contained 221,610,757 paired end reads, of which 188,805 (0.09%) aligned to the wildtype emGFP sequence and were removed. Wildtype reads were detected as described for the p.[Y66X; T203X] IVTT library (see above), pairs of reads which had the wildtype sequence in either Read 1(77,033), Read 2 (92,814) or both Read 1 and Read 2 (18,958) were discarded, leaving 221,421,952 paired end reads. Following the removal of the wildtype sequences, 192,331,072 (86.8%) in-frame reads were kept as they contained the desired adapter 1b sequence GTG CAG TGC TTC G (nucleotide position 205 to 217) and sequence TGG (base position 193 to 195, codon position L64) in read 1 as well as adapter 2b sequence (base position 606 to 594), and the sequence GGA (base position 616 to 618, codon, position S205) in Read 2 (FIG. 9B). Finally, due to the potential size of the library (∼3.2 x 1014), any nonunique reads were considered to be PCR duplicates, removing a further 3,585,328 (1.6%), leaving 188,745,744 (85.2%) reads as unique sequences with the potential to produce a desired, in-frame, full-length protein variant. Throughout the analysis the reads remained paired to maintain the original diversity of the InDel library, once the reads were filtered (as described above) the emGFP InDel library was analyzed to determine the composition of each InDel, nucleotide and codon.

The population of each InDel combination, was directly related to the initial InDel Duplex concentration (and therefore diversity), within the two InDel Duplex Pools. The largest population of reads 87.7% (expected, 87.9%) belonged to the most diverse combination p.[T65_G67delins(X)6; S202_Q204delins(X)6], in comparison, the least diverse combination p.[T65_G67delTYG; S202Q204delSTQ] combination contained 0.0% (expected, 0.0%) of the reads. As described for the on-bead p.[Y66X; T203X] IVTT library (see above), the sequence variations introduced at positions T65_Y66_G67 and S202_T203_Q204 were created by the degenerate nucleotide sequence N₁N₂C₃. Median A, C, G and T values for nucleotide N₁ were 23.5 ± 1.5%, 28.3 ± 2.3%, 25.8 ± 3.0% and 22.5 ± 1.3%; for nucleotide N₂ were 23.3 ± 1.5%, 27.6 ± 2.5%, 26.8 ± 2.5% and 22.3 ± 1.4 and for nucleotide C3 were 0.5 ± 0.3%, 99.2 ± 0.6%, 0.2 ± 0.4% and 0.1 ± 0.1% respectively, with the median codon value being 5.9 ± 1.7%. Overall, the %GC for N₁, N₂ and N₃ was 54.1 ± 2.3%, 54.4 ± 2.4 and 99.4 ± 0.4%.

### Discussion of Examples 2A-2D

Creating accurate and well-balanced sequence diversity, whether in the form of substitution, insertion and/or deletion, is the Keystone for many methodologies involving the use of variant libraries, nonemore so than in directed evolution. Variant library quality within directed evolution defines the library size and library diversity, therefore influences any screening strategy and size. Ultimately, the variant library quality determines the potential success (or failure) of any given directed evolution undertaking.

V-gSynth, which leverages the double-stranded geometric synthesis methods of the present disclosure, is a highly capable, flexible and user-friendly methodology which, can introduce substitutions, insertions and/or deletions, simultaneously at multiple distal sites. Hugely diverse variant libraries can be produced within a single working day, while only requiring commercially available enzymes and reagents combined with the most basic of molecular biology recourses. Furthermore, due to automation friendly nature of V-gSynth, the methodology can be parallelized and scaled as required.

IVTT templates were generated using V-gSynth, however due to the inherent flexibly of the four nucleotide overhangs generated by FspEI, V-gSynth is compatible with any cloning strategy. Likewise, while only the coding region of a single gene within a single plasmid, was targeted, nothing is stopping the targeted assembly of variants from multiple genes and from multiple sources. Furthermore, as the assembly of V-gSynth monoclonal beads is PCR-free. Thus DNA, RNA as well as modified nucleic acids (including nucleobase, sugar and/or back bone modifications) can be incorporated into the monoclonal variants bead library, extending the scope of V-gSynth from protein evolution into other areas such as aptamers, SELEX etc.

The one-pot, single step assembly approach of V-gSynth is capable of generating huge diversity, while maintaining an even distribution of that diversity, this was as exemplified by the assembly of the InDel library, which is a generated through the combination of 49 uniquie InDel combinations. An unprecedented ∼85% of all sequences generated within the InDei library were unique sequences with the potential to produce a desired, in-frame, full-length protein variant. Many of the out-of-frame reads within the InDel library, will originate from the synthetic oligos used for the InDel duplexes, in particular the N-1 error associated with phosphoramidite synthesis. By employing a more faithful oligo synthesis method and/or further purification of the synthetic oligos (such as PAGE or HPLC) these N-1 errors can be greatly reduced.

Furthermore, the slight increase of the %GC (within the InDei library) of N₁ (54.1 ± 2.3%) and N₂ (54.4 ± 2.4) above the ideal 50% for nucleotides N₁ and N₂ within the degenerate N₁N₂C₃ sequence, may be due to the melting temperatures of the InDel oligo duplexes. Duplexes with a higher %GC and therefore (on average) a higher melting temperature, will have had a greater representation within the InDel Duplex Pools. Optimisation of the InDei duplex sequence, along with the annealing conditions should create %GC of the N₁ and N₂ nucleotide more in line with the ideal 50%. The consequence of gaining an even %GC, would been seen at the protein level with, for example, within the InDel library the codon P (nucleotide sequence CCC) had the highest representation (9.0 ± 1 1%) while codon F (nucleotide sequence TTC) was represented the least (5.3 ± 0.5%). An ideal %GC would allow for a more even codon representation, regardless of the nucleotide sequence.

During the application of V-gSynth we successfully generated a monoclonal, on-bead IVTT library which contained 256 nucleotide and 225 codon variations, along with an InDel library with an estimated ~3.2 x 1014 nucleotide and -1.5 x 1014 codon variations.

### Methods for Examples 2A-2D

### Reagents

Unless otherwise stated all enzymes, buffers, dNTPs, rNTPs and the GeneJET Gel Extraction kit were supplied by New England Biolabs (NEB; Ipswich, MA, USA) and all oligonucleotides were supplied by Integrated DNA Technologies (IDT, Coralville, 1A, USA). Dibenzocyclooctyne (DBCO) Magnetic Beads (Jena Bioscience; Jena, Germany), PicoSurf-1 (Sphere Fluidics; Cambridge, UK), HFE7500 oil (Fluorochem; Hadfield, UK), Nuclease-free water, pREST/emGFP and QuBit/high sensitivity dsDNA kit (ThermoFisher; Waltham, MA, USA), Solid Phase Reversible Immobilization (SPRI) beads were made as previously described (Rohland, N., and D. Reich. "Cost-Effective, High-Throughput DNA Sequencing Libraries for Multiplexed Target Capture." Genome Research, vol. 22, no. 5, 2012, pp. 939-946., doi:10.1101/gr.128124.111.).

### emGFP reference nucleotide and codon variations nomenclature

Nucleotide and codon numbering of emGFP are from the consensus sequence of eGFP (Tsien, Roger Y. "The Green Fluorescent Protein." Annual Review of Biochemistry, vol. 67, no. 1, 1998, pp. 509-544., doi:10.1146/annurev.biochem.67.1.509.). Nomenclature used throughout this disclosure to describe the nucleotide and codon variations, are based upon recommendations by Stylianos Antonarakis and Johan den Dunnen (Dunnen, Johan T. Den, and Stylianos E. Antonarakis. "Mutation Nomenclature Extensions and Suggestions to Describe Complex Mutations: A Discussion." Human Mutation, vol. 15, no. 1, 2000, pp. 7-12., doi: 10. 1002/(sici) 1 098-1004(200001) 1 5: 13.0.co;2-n; Dunnen, Johan T. Den, et al. "HGVS Recommendations for the Description of Sequence Variants: 2016 Update." Human Mutation, vol. 37, no. 6, 2016, pp. 564-569., doi:10.1002/humu.22981.).

### Methylated primers

All methylated primers for the generation of the Methylated Fragments, contained the recognition site GCCATGCTGTCXAGGNNNNNNNNN↓NNNN↑ (SEQ ID NO: 1), where X is 5-methylcytosine and N is either A, C, G or T. The recognition site, used in our methylated primers is compatible with MspJI, FspEI and LpnPI restriction enzymes.

### Generation of the wild-type, p.Y66W, p.T203Y and p. [Y66W; T203Y] IVTT templates

The V-gSynth methodology consists of three simple steps (FIGs. 5A-5B and FIG. 6A).

### A. Preparation of Methylated Fragments

Methylated Fragments 1-Y66, 1-Y66W, 2, 3-T203 and 3-T203Y were prepared in 1x Q5 Reaction Buffer, 1x Q5 High GC Enhancer, 0.5 µM each forward and reverse primer, 0.2 mM each dNTP, 1 ng pRSET/EmGFP vector and 0.02 U/µL, Q5 DNA Polymerase. Thermocycling Conditions were 30 s at 98 °C, followed by 30 cycles of 10 s at 98 °C, 15 s at 65 °C and 45 s at 72 °C, with a final step of 2 min at 72 °C. The Methylated Fragments were purified using SPRI beads, eluted in water, quantified by Qubit and used directly within FspEI digestions.

### B. FspEI digestion of Methylated Fragments

FspEI digestion consisted of 1x CutSmart buffer, 1x Enzyme Activator, 0.01 Units/µL and 100 to 1000 ng of a Methylated Fragment (prepared as described above) and incubated at 37 °C for 30 min. The Digested Fragments were purified using SPRI beads, eluted in water and used directly within T7 Ligase Assemblies.

### C. T7 DNA ligase assembly of Digested Fragments

Assembly of the IVTT templates consisted of an equimolar mix (100 to 1000 ng total DNA) of the Digested Fragments 1-Y66, 2 and 3-T203 (wild-type), 1-Y66W, 2 and 3-T203 (p.Y66W), 1-Y66, 2 and 3-T203Y (p.T203Y) and 1-Y66W, 2 and 3-T203Y (p.[Y66W; T203Y]) in 1x T7 DNA Ligase Reaction Buffer with 150 Units/µL of T7 DNA Ligase and incubated at 25 °C for 60 min. Assembled IVTT templates were used directly (without purification) within IVTT reactions or amplified for sequencing.

### Generation of on-bead wild-type, Y66W, T203Y and [Y66W: T203Y] IVTT templates

The on-bead wild-type, p.Y66W, p.T203Y and p.[Y66W; T203Y] IVTT templates were prepared as described above with the exception that Primer 6 was replaced with Primer 6-azide. Once the IVTT templates had been assembled, the templated were covalently attached by click chemistry (Klob_2001; Best_2009; Jewett_2010) to DBCO beads by adding an equal volume of DBCO beads (1 mg/mL) in 6 mM Tris-HCl (pH 7.4), 1.2 M NaCl, 0.6 mM EDTA, 0.006% Tween and 40% DMSO was added to each individual assembly reaction, before being incubated for 2 hr at room temperature. The individual on-bead, assembled, IVTT templates were washed four times with 1x PBS/0.01% Tween, before being stored in 1x PBS/0.01% Tween (1 mg/mL) at 4° C, ready for use as controls within the emulsion based IVTT reactions (see below).

### Generation of on-bead [Y66X; Y203X] IVTT library

To generate the 256 members of the on-bead, p.[Y66X; Y203X] IVTT library, thirty-three Methylated Fragments were prepared, sixteen of the Methylated Fragments were variations on Methylated Fragment 1 and carried the sixteen codons Y66N, Y66T, Y66S, Y66I, Y66H, Y66P, Y66R, Y66L, Y66D, Y66A, Y66G, Y66V, Y66, Y66S, Y66C and Y66F (simplified to Methylated Fragment 1 -Y66X)A further sixteen Methylated Fragments were variations on Methylated Fragment 3 and carried the sixteen codons T203N, T203, T203S, T203I, T203H, T203P, T203R, T203L, T203D, T203A, T203G, T203V, T203S, T203C and T203F (simplified to Methylated Fragment 3-Y203X). Finally, Methylated Fragment 2 was identical throughout the 256 variants.

The combination of the sixteen codons at position p Y66X and p.T203X are equivalent to the nucleotide substitution c.[199_201>NNC; 700_702>NNC]. The p.Y66S codon substitution occurs twice, because the nucleotide substitutions c.199_201>AGC and c. 199_201>TCC, are equivalent at the protein level. Similarly, the p.T203S codon substitution occurs twice as the nucleotide substitutions c.700_702>AGC and c.700_702>TCC are equivalent at the protein level. FspEI digestion and T7 DNA ligase assemblies were carried out as described above using Primer 6-azide throughout to covalently attached the p. [Y66X; Y203X] library to DBCO beads. Once each of the 256 variants was individually attached to the DBCO beads, the beads were pooled and stored in 1x PBS/0.01% Tween (1 mg/mL) at 4o C, with the on-bead p.[Y66X; Y203X] library being used either for the preparation of a NGS library or for fluorescent imaging (see below).

### In-vitro transcription and translation (IVTT) reactions

In-vitro transcription and translation (TVTT) reactions used the PUREexpress system and contained 10 µL of component A, 7.5 µL component B, 250 ng of template with the reactions being adjusted to a final volume of 25 µL with nuclease-free water. IVTT reactions were incubated at 37° C for 4 hours before running on an SDS-PAGE. Emulsion based IVTT reactions contained 10 µL of component A, 7.5 µL component B, 1 µL template beads (1 mg/mL), with the reactions being adjusted to a final volume of 25 µL with nuclease-free water. The aqueous phase was mixed with 100 µL of an oil phase containing 2.0% PicoSurf-1 in HFE7500 (v/v). The emulsion was created by vortexing for 3 min at .3/4 of the maximal vortex speed, followed by incubation of the emulsions at 37° C for 4 hours before imaging.

### Fluorescence Imaging

Sawano *et al.* demonstrated that the four GFP variant, wild-type, p. Y66W, p.T203Y and p.[Y66W; T203Y] can be distinguished using the ratio, from the fluorescence of two excitation wavelengths, where p.T2035Y > wild-type > p.[Y66W; T203Y] > p.Y66W, therefore making the four GFP variant distinguishable within a mixture (Sawano_2000). This approach was used to image the emulsions, using 488 and 440 nm as the two excitation wavelengths, on an Olympus FV1000 fluorescent microscope.

### InDel Duplexes

InDei Duplex Pool 1 contained seven duplexes T65_G67delTYG, T65_G67delins(X)1, T65_G67delins(X)2, T65_G67delins(X)3, T65_G67delins(X)4, T65_G67delins(X)5, T65_G67delins(X)6; while InDel Duplex Pool 2 contained the seven duplexes S202_Q204delSTQ, 202_Q204delins(X)1, 202_Q204delins(X)2, 202_Q204delins(X)3, 202_Q204delins(X)4, 202_Q204delins(X)5, 202Q204delins(X)6 (FIGs. 5C-D, FIGs. 8A-8B, and FIGs. 9A-9B). Each individual duplex was annealed from their sense and antisense oligos in 10 mM Tris-HCl (pH 7.4), by heating to 90° C for 2 mins and cooling slowly to room temperature at -1° C/min, before being pooled. Each InDei Duplex Pool contained the series of seven duplexes at a ratio of 1 : 16 : 256 : 4096 : 65,536 : 1,048,576 : 16,777,216. This ratio is used as it reflects the diversity of each of the seven duplexes, the diversity is derived from the 0 to 6 consecutive and degenerate codons introduce by repetitive N₁N₂C₃ nucleotide sequence.

### Generation of the InDel library, containing the forty-nine InDel combinations

The highly diverse InDel library can be described as a combination of forty-nine libraries with p.[T65_G67delTYG; S202_Q204delSTQ] being the smallest library, contains only 1 member, were codons T65_Y66_G67 and S202_T203_Q204 are deleted. Library p.[T65_G67delins(X)6; S202_Q204delins(X)6] is the largest library containing ~2.8 x 1014 members, as codons T65_Y66_G67 and S202_T203_Q204 were deleted and twelve degenerate codons inserted, six degenerate codons inserted at position of T65_Y66_G67 and a further six degenerate codons inserted at position of S202_T203_Q204 FIG. 5D, FIGs. 8A-8B and FIGs. 9A-9B).

The methylated primers used to generate the Methylated InDel Fragments 1, 2 and 3 were designed to delete codons T65_Y66_G67 between Methylated InDel Fragments 1 and 2, while also deleting codons S202_T203_Q204 between Methylated InDel Fragments 2 and 3. FspEI digestion of Methylated InDel Fragments 1, 2 and 3 then removes a further 12/16 nucleotides from the 5-methylcytosine and generates the Digested InDel Fragments 1, 2 and 3. Once codons T65_Y66_G67 have been deleted, seven InDel duplexes (InDei Duplex Pool 1) are used to insert a series of 0 to 6 consecutive and degenerate codons, a further seven InDel duplexes (InDel Duplex Pool 2) are used to insert a second series of 0 to 6 consecutive and degenerate codons at the deleted S202_T203_Q204 codons (FIG 5D, FIGs. 8A-8B and FIGs 9A-9B). Each of the degenerate codons were introduce by repetitive N₁N₂C₃ nucleotide sequence. Assembly of the InDel library was from a single ligation reaction containing Digested InDei Fragments 1, 2 and 3, as well as InDel Duplex Pools 1 and 2.

### Sequencing and data analysis

Sanger sequencing was performed by Eurofins Genomics (Koln, Germany), samples were prepared by PCR using Q5 DNA Polymerase, purified using SPRI beads, eluted in water then quantified by Qubit. Sanger sequencing samples were prepared according to the manufacturer's instructions before shipping. NGS library QC and sequencing were performed by the Cambridge Genome Centre (Cambridge, UK) on an Illumina NextSeq using a NextSeq 500/550 High Output Kit v2.5 (150 Cycles). NGS Libraries were prepared by PCR using Q5 DNA Polymerase to add sequencing primers and individual barcodes. NGS Libraries were isolated on an agarose gel and purified using the GeneJET Gel Extraction kit. The NextSeq FASTQ files were quality filtered and trimmed using cutadapt with custom Adapter 1a, Adapter 1b, Adapter 2a and Adapter 2b sequences (FIG. 7A, FIG. 8B and FIGs. 9A-9B). Nucleotide and codon frequencies were determined in GNU bash, version 3.2.57 then plotted using R version 3.5.3 for Mac OS X.

### Example 3 - Comparison of the geometric synthesis methods of the present disclosure and standard phosphoramidite synthesis

To compare the geometric synthesis methods of the present disclosure to standard and widely used phosphoramidite synthesis methods, the geometric synthesis methods of the present disclosure and phosphoramidite synthesis methods were used to synthesize a series of 300 nucleotide-long target nucleic acid molecules. Different target nucleic acid molecules were designed with different characteristics to determine the impact that the target nucleic acid sequence has on the efficiency and accuracy of both the geometric synthesis methods of the present disclosure and standard phosphoramidite methods.

The products synthesized by both methods were analyzed using next-generation sequencing methods. The analysis of the next-generation sequencing methods was performed by sampling 100,000 quality trimmed, paired-end reads for each synthesized target nucleic acid and mapping this data to the desired, reference sequences. Overlapping regions from the pair-end reads were removed before synthesis accuracy was determined.

As shown in FIG. 13, the phosphoramidite synthesis was performed by synthesizing two standard desalted 162 nucleotide long phosphoramidite oligonucleotides. The two oligonucleotides were then hybridized at a complementary 24 nucleotide region located at the 3' ends of the oligonucleotides. Following hybridization, the two oligonucleotides were extended using the high-fidelity Q5 DNA polymerase to generate double-stranded DNA, referred to herein as phosphoramidite HAE. This approach is also comparable to polymerase cycling assembly (PCA), which is a commonly used gene assembly method.

### Example 3A - target nucleic acid with GC content ranging from 40% to 60% GC (40% → 60% GC target)

Phosphoramidite synthesis and the geometric synthesis methods of the present disclosure were used to synthesize a target nucleic acid that had a GC content that increased from 40% to 60% along the length of the target as measured using a sliding window of 50 nucleotides (herein referred to as a 40% → 60% GC target). As shown in Table 6 and FIG. 10A, only 28.3% of the phosphoramidite HAE synthesized product were full length sequences, whereas 82.9% of the geometric synthesis product was the correct length In Table 6, Alignment % refers to the percent of concordantly aligned sequences from 100,000 quality trimmed paired-end reads, Full-length Read % refers to the percent of full-length concordantly aligned sequences from 100,000 quality trimmed paired-end reads and Coupling Efficiency % refers to the equivalent nucleotide coupling efficiency based on the yield of full-length sequences, wherein yield = coupling efficiency ^ (length-1). As shown in Table 6 and FIG. 1 0B, plots of sequence coverage versus sequence position reveal that for the phosphoramidite HAE product, the greatest sequence coverage was at the center of the 300 nucleotide-long target nucleic acid and gradually tailed off toward the ends. Without wishing to be bound by theory, these results are consistent with the fact that the central position of the target nucleic acid corresponds to the 3' end of the two different phosphoramidite oligonucleotides, which is the most accurate area. The gradual decrease in coverage reflects phosphoramidite synthesis errors and the accumulation of truncated sequences. In contrast, as shown in Table 6 and FIG. 10B, the sequence coverage for the geometric synthesis methods of the present disclosure remained high throughout all positions of the target nucleic acid, which is consistent with a higher accuracy and coupling efficiency. These results indicate that the geometric synthesis methods of the present disclosure outperform the standard phosphoramidite methods.

**Table 6. Geometric synthesis methods of the present disclosure vs. Phosphoramidite synthesis**

| **300mer Target Nucleic acid** | **Geometric Synthesis methods of the present disclsoure** | | | **Phosphoramidite HAE synthesis** | | |
|---|---|---|---|---|---|---|
| | **Alignment %** | **Full-length Read %** | **Coupling Efficiency %** | **Alignment %** | **Full-length Read %** | **Coupling Efficiency %** |
| 40% → 60% GC target | 99.6 | 82.9 | >99.9 | 97.8 | 28.3 | 99.6 |
| T & C homopolymer target | 99.8 | 89.2 | >99.9 | 94.4 | 12.5 | 99.3 |
| N₁ to N₆ target | 99.6 | 83.8 | >99.9 | 97.0 | 27.5 | 99.6 |
| **Overall** | 99.7 ± 0.1 | 85.3 ± 3.4 | >99.9 | 96.4 ± 1.7 | 22.7 ± 8.9 | 99.5 ± 0.17 |

### Example 3B target nucleic acid containing 10-nucleotide long T and C homopolymeric regions (T & C homopolymer target)

Phosphoramidite synthesis and the geometric synthesis methods of the present disclosure were used to synthesize a target nucleic acid that contained 10-nucleotide long T and C homopolymeric regions (herein referred to as a T & C homopolymer target). As shown in Table 6 and FIG. 11A, only 12.5% of the phosphoramidite HAE synthesized product were full length sequences, whereas 89.2% of the geometric synthesis product was the correct length. As shown in Table 6 and FIG. 11B, the sequence coverage of the phosphoramidite HAE product was significantly lower than the phosphoramidite product of the 40% → 60% GC target described in *Example* 3A. Without wishing to be bound by theory, this difference is consistent with the known difficulties in synthesizing homopolymers. In contrast, as shown in Table 6 and FIG. 11B, sequence coverage of the geometric synthesis product for the T & C homopolymer target remained high across all positions, demonstrating that the geometric synthesis methods of the present disclosure can accurately produce problematic sequences.

### Example 3C - target nucleic acid containing six variable nucleotides N₁ to N₆ (N₁ to N₆ target)

Phosphoramidite synthesis and the geometric synthesis methods of the present disclosure were used to synthesize a target nucleic acid that contained six variable nucleotides N₁ to N₆ at specific locations within the target sequence (herein referred to as a N₁ to N₆ target). As shown in Table 6 and FIG. 12A, only 27.5% of the phosphoramidite HAE synthesized product were full length sequences, whereas 83.8% of the geometric synthesis product was the correct length. Furthermore, as shown in FIG. 12B and Table 7, the geometric synthesis methods of the present disclosure demonstrated a greater and more even distribution of A, C, G and T nucleotides at the degenerate N₁ to N₆ nucleotide positions as compared to the phosphoramidite methods, demonstrating that the geometric synthesis methods of the present disclosure are superior to standard phosphoramidite methods for the synthesis of target nucleic acids with degenerate nucleotide positions. In Table 7, overall coverage refers to the number of times nucleotide Ni to N₆ were covered from the initial 100,000 quality trimmed paired-ends reads. Coverage for A, C, G or T refers to the number of times each nucleotide was called from the overall coverage and the corresponding percentage. For the target synthesized with phosphoramidite synthesis, nucleotides N₁, N₂, N₃, N₄, N₅, and N₆ were located at positions 21, 22, 23, 278, 279 and 280 of the target sequence. For the target synthesized with geometric synthesis, nucleotides N₁, N₁, N₃, N₄, N₅, and N₆ were located at positions 21, 22, 23, 278, 279 and 280 of the target sequence.

**Table 7. Geometric Synthesis vs. Phosphoramidite synthesis for degenerate nucleotide positions**

| **Method** | **Nucleotide** | **N1** | **N2** | **N3** | **N4** | **N5** | **N6** |
|---|---|---|---|---|---|---|---|
| **Geometric Synthesis** | **Overall Coverage (%)** | 93,156 (100) | 93,264 (100) | 93,409 (100) | 95,568 (100) | 95,529 (100) | 95,479 (100) |
| | **A Coverage (%)** | 22,002 (23.6) | 21,984 (23.6) | 22,262 (23.8) | 22,171 (23.2) | 24,801 (26.0) | 25,171 (26.4) |
| | **C Coverage (%)** | 21,059 (22.6) | 20,445 (21.9) | 21,022 (22.5) | 27,911 (29.2) | 22,544 (23.6) | 23,487 (24.6) |
| | **G Coverage (%)** | 27,716 (29.8) | 28.691 (30.8) | 28,564 (30.6) | 21,938 (23.0) | 23,944 (25.1) | 23,086 (24.2) |
| | **T Coverage (%)** | 22,307 (23.9) | 22,132 (23.7) | 21,555 (23.1) | 23,280 (14.4) | 24,221 (25.4) | 23,729 (24.9) |
| | **Ins/Del Coverage (%)** | 72 (0.1) | 12 (0.0) | 6 (0.0) | 268 (0.3) | 19 (0.0) | 6 (0.0) |
| **Phosphoramidite HAE** | **Overall Coverage (%)** | 57,380 (100) | 57,633 (100) | 57,968 (100) | 67,826 (100) | 67,584 (100) | 67,337 (100) |
| | **A Coverage (%)** | 14,135 (24.6) | 14,225 (24.7) | 14,259 (24.6) | 20,923 (30.8) | 21,605 (32.0) | 21,595 (32.1) |
| | **C Coverage (%)** | 9,878 (17.2) | 9,908 (17.2) | 9,832 (17.0) | 15,509 (22.9) | 16,416 (24.3) | 16,228 (24.1) |
| | **G Coverage (%)** | 13,376 (23.3) | 13,525 (23.5) | 14,630 (25.2) | 12,609 (18.6) | 12,258 (18.1) | 12,217 (18.1) |
| | **T Coverage (%)** | 19,293 (33.6) | 19.923 (24.6) | 19,195 (33.1) | 17,425 (25.7) | 17,165 (25.4) | 17,208 (25.6) |
| | **Ins/Del Coverage (%)** | δ98 (1.2) | 52 (0.1) | 52 (0.1) | 1,360 (2.0) | 140 (0.2) | 89 (0.1) |

### Summary of Examples 3A 3C

As shown in Table 6, on average, 99.7 ± 0.1% of the products of the geometric synthesis methods of the present disclosure aligned to their reference (target) sequences as compared to only 96.4 ± 1.7% of the phosphoramidite HAE products. Furthermore, 85.3 ± 3.4% of the geometric synthesis products were the correct full-length, while only 22.7 ± 8.9% of the phosphoramidite HAE products were full-length. The yields of 85.3% and 22.7% full-length product indicated a coupling efficiency of >99.9% and 99.5% for geometric synthesis and phosphoramidite synthesis, respectively, indicating that the analysis was robust. Thus, these results indicate that the geometric synthesis methods of the present disclosure are superior to the standard phosphoramidite synthesis methods.

### Example 3D - target nucleic acid with GC content ranging from 10% to 90% GC (10% → 90% GC target)

Phosphoramidite synthesis and the geometric synthesis methods of the present disclosure were used to synthesize a target nucleic acid that had a GC content that increased from 10% to 90% along the length of the target as measured using a sliding window of 50 nucleotides (herein referred to as a 10% → 90% GC target), as shown in FIG. 15A. As shown in FIG. 15A, Phosphoramidite HAE synthesis was used to synthesize a 10% → 90% GC target that is herein referred to as "1090_Seq01", a first geometric synthesis reaction was used to synthesize a 10% → 90% GC target that is herein referred to as "1090 _Seq02" and a second geometric synthesis reaction was used to synthesize a 10% → 90% GC target that is herein referred to as "1090_Seq03". Next generation sequencing analysis of the products of the Phosphoramidite HAE synthesis, the first geometric synthesis reaction and the second geometric synthesis reaction are shown in Table 8-10.

**Table 8**

| **Target Name** | **Phosphoramidite Synthesis** | | | | |
|---|---|---|---|---|---|
| | **# of Reads** | **Alignment (%)** | **Avg. Overlap (bp)** | **Full-length Reads (%)** | **Coupling Efficiency (%)** |
| **1090** | 186,462 | 98.2 | 40.89 | 33.7 | 99.6 |
| **2080** | 197,740 | 98.9 | 38.3 | 46.9 | 99.7 |
| **3070** | 261,513 | 97.8 | 41.8 | 40.6 | 99.7 |
| **4060** | 150,592 | 97.7 | 55.1 | 28.4 | 99.6 |
| **5050** | 155,267 | 98.6 | 52.1 | 32.8 | 99.6 |
| **NNN** | 235,907 | 96.7 | 55.0 | 27.9 | 99.6 |
| **awkAG** | 156,695 | 95.8 | 67.1 | 20.3 | 99.5 |
| **awkTC** | 234,754 | 94.8 | 55.9 | 15.0 | 99.4 |
| **rep** | 205,788 | 84.7 | 63.4 | 32.2 | 99.6 |

**Table 9**

| **Target Name** | **First geometric synthesis reaction** | | | | |
|---|---|---|---|---|---|
| | **# of Reads** | **Alignment (%)** | **Avg. Overlap (bp)** | **Full-length Reads (%)** | **Coupling Efficiency (%)** |
| **1090** | 292,109 | 91.6 | 29.0 | 17.5 | 99.4 |
| **2080** | 49,012 | 97.5 | 30.4 | 3.1 | 98.8 |
| **3070** | N/A | N/A | N/A | N/A | N/A |
| **4060** | 126.359 | 99.5 | 9.9 | 83.1 | 99.9 |
| **5050** | 150,227 | 99.1 | 23.8 | 62.5 | 99.8 |
| **NNN** | 204,275 | 87.5 | 47.7 | 1.7 | 98.6 |
| **awkAG** | 6,531 | 6.26 | 59.6 | 30.6 | 99.6 |
| **awkTC** | 115,220 | 98.9 | 7.9 | 89.7 | >99.9 |
| **rep** | 195,653 | 19.5 | 35.3 | 40.2 | 99.7 |

**Table 10**

| **Target Name** | **Second geometric synthesis reaction** | | | | |
|---|---|---|---|---|---|
| | **# of Reads** | **Alignment (%)** | **Avg. Overlap (bp)** | **Full-length Reads (%)** | **Coupling Efficiency (%)** |
| **1090** | 1,441 | 88.1 | 94.5 | 2.2 | 98.7 |
| **2080** | 4,162 | 50.4 | 118.7 | 0.0 | 0.0 |
| **3070** | 602,988 | 99.8 | 7.1 | 86.9 | >99.9 |
| **4060** | 82,339 | 99.4 | 7.1 | 89.6 | >99.9 |
| **5050** | 68,758 | 96.5 | 50.1 | 2.2 | 98.7 |
| **NNN** | 133,321 | 99.4 | 10.9 | 84.0 | 99.9 |
| **awkAG** | 26,614 | 86.5 | 53.8 | 48.5 | 99.8 |
| **awkTC** | 160,092 | 98.8 | 15.4 | 78.7 | 99.9 |
| **rep** | 65,313 | 78.4 | 96.0 | 10.4 | 99.2 |

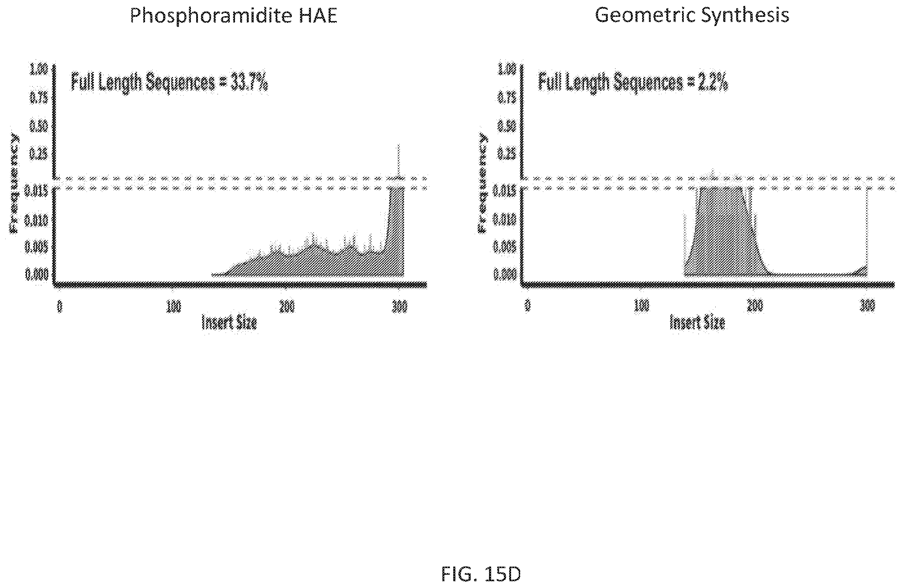

In Tables 8-10, the Number of Reads refers to the number of quality trimmed paire-end reads (Trim_Galore), which were then used in the alignments; the Alignment % refers to the percent of concordantly aligned sequences from quality trimmed paired-end reads (Bowtie 2); the Average Overlap (bp) refers to the number of base pairs (bp) on average which overlapped (and were removed) from the aligned paired-end reads (clipOverlap); the Full-length Reads % refers to the percent of aligned reads with the target size of 300 nucleotides; and the Coupling Efficiency % refers to the equivalent nucleotide coupling efficiency based on yield of full-length sequences, where yield==(coupling efficiency^(length-1)). FIG. 15B shows the frequency of products of varying lengths in the Phosphoramidite HAE synthesis reaction and first geometric synthesis reaction and FIG. 15C shows the sequence coverage over each position of the target nucleic acid in the products of the Phosphoramidite HAE synthesis reaction and first geometric synthesis reaction. FIG. 15D shows the frequency of products of varying lengths in the Phosphoramidite HAE synthesis reaction and second geometric synthesis reaction and FIG. 15E shows the sequence coverage over each position of the target nucleic acid in the products of the Phosphoramidite HAE synthesis reaction and second geometric synthesis reaction. FIG. 14 shows agarose gel analysis of the products of the Phosphoramidite HAE synthesis reaction, the first geometric synthesis reaction and the second geometric synthesis reaction.

### Example 3E - target nucleic acid with GC content rangingfrom 20% to 80% GC (20% → 80% GC target)

Phosphoramidite synthesis and the geometric synthesis methods of the present disclosure were used to synthesize a target nucleic acid that had a GC content that increased from 20% to 80% along the length of the target as measured using a sliding window of 50 nucleotides (herein referred to as a 20% → 80% GC target), as shown in FIG. 16A. As shown in FIG. 16A, Phosphoramidite HAE synthesis was used to synthesize a 20% -> 80% GC target that is herein referred to as "2080_Seq01", a first geometric synthesis reaction was used to synthesize a 20% → 80% GC target that is herein referred to as "2080_Seq02" and a second geometric synthesis reaction was used to synthesize a 20% → 80% GC target that is herein referred to as "2080_Seq03". Next generation sequencing analysis of the products of the Phosphoramidite HAE synthesis, the first geometric synthesis reaction and the second geometric synthesis reaction are shown in Tables 8-10. FIG. 16B shows the frequency of products of varying lengths in the Phosphoramidite HAE synthesis reaction and first geometric synthesis reaction and FIG. 16C shows the sequence coverage over each position of the target nucleic acid in the products of the Phosphoramidite HAE synthesis reaction and first geometric synthesis reaction. FIG. 16D shows the frequency of products of varying lengths in the Phosphoramidite HAE synthesis reaction and second geometric synthesis reaction and FIG. 16E shows the sequence coverage over each position of the target nucleic acid in the products of the Phosphoramidite HAE synthesis reaction and second geometric synthesis reaction. FIG. 14 shows agarose gel analysis of the products of the Phosphoramidite HAE synthesis reaction, the first geometric synthesis reaction and the second geometric synthesis reaction.

### Example 3F - target nucleic acid with GC content ranging from 30% to 70% GC (30% 4 70% GC target)

Phosphoramidite synthesis and the geometric synthesis methods of the present disclosure were used to synthesize a target nucleic acid that had a GC content that increased from 30% to 70% along the length of the target as measured using a sliding window of 50 nucleotides (herein referred to as a 30% → 70% GC target), as shown in FIG. 17A. As shown in FIG. 17A, Phosphoramidite HAE synthesis was used to synthesize a 30% → 70% GC target that is herein referred to as "3070_Seq01", a first geometric synthesis reaction was used to synthesize a 30% → 70% GC target that is herein referred to as "3070_Seq02" and a second geometric synthesis reaction was used to synthesize a 30% → 70% GC target that is herein referred to as "3070_Seq03". Next generation sequencing analysis of the products of the Phosphoramidite HAE synthesis, the first geometric synthesis reaction and the second geometric synthesis reaction are shown in Tables 8-10. FIG. 17B shows the frequency of products of varying lengths in the Phosphoramidite HAE synthesis reaction and second geometric synthesis reaction and FIG. 17C shows the sequence coverage over each position of the target nucleic acid in the products of the Phosphoramidite HAE synthesis reaction and second geometric synthesis reaction. FIG. 14 shows agarose gel analysis of the products of the Phosphoramidite HAE synthesis reaction, the first geometric synthesis reaction and the second geometric synthesis reaction.

### Example 3G target nucleic acid with GC content ranging from 40% to 60% GC (40% → 60% GC target)

Phosphoramidite synthesis and the geometric synthesis methods of the present disclosure were used to synthesize a target nucleic *acid* that had a GC *content* that increased from 40% to 60% along the length of the target as measured using a sliding window of 50 nucleotides (herein referred to as a 40% → 60% GC target), as shown in FIG. 18A. As shown in FIG. 18A, Phosphoramidite HAE synthesis was used to synthesize a 40% → 60% GC target that is herein referred to as "4060_Seq01", a first geometric synthesis reaction was used to synthesize a 40% → 60% GC target that is herein referred to as "4060_Seq02" and a second geometric synthesis reaction was used to synthesize a 40% → 60% GC target that is herein referred to as "4060_Seq03". Next generation sequencing analysis of the products of the Phosphoramidite HAE synthesis, the first geometric synthesis reaction and the second geometric synthesis reaction are shown in Tables 8-10. FIG. 18B shows the frequency of products of varying lengths in the Phosphoramidite HAE synthesis reaction and first geometric synthesis reaction and FIG. 18C shows the sequence coverage over each position of the target nucleic acid in the products of the Phosphoramidite HAE synthesis reaction and first geometric synthesis reaction. FIG. 18D shows the frequency of products of varying lengths in the Phosphoramidite HAE synthesis reaction and second geometric synthesis reaction and FIG. 18E shows the sequence coverage over each position of the target nucleic acid in the products of the Phosphoramidite HAE synthesis reaction and second geometric synthesis reaction. FIG. 14 shows agarose gel analysis of the products of the Phosphoramidite HAE synthesis reaction, the first geometric synthesis reaction and the second geometric synthesis reaction.

### Example 3H - target nucleic acid with GC content ranging from 50% to 50% GC (50% → 50% GC target)

Phosphoramidite synthesis and the geometric synthesis methods of the present disclosure were used to synthesize a target nucleic acid that had a GC content that increased from 50% to 50% along the length of the target as measured using a sliding window of 50 nucleotides (herein referred to as a 50% → 50% GC target), as shown in FIG. 19A. As shown in FIG 19A, Phosphoramidite HAE synthesis was used to synthesize a 50% → 50% GC target that is herein referred to as "5050_Seq01", a first geometric synthesis reaction was used to synthesize a 50% → 50% GC target that is herein referred to as "5050_Seq02'' and a second geometric synthesis reaction was used to synthesize a 50% → 50% GC target that is herein referred to as "5050_Seq03". Next generation sequencing analysis of the products of the Phosphoramidite HAE synthesis, the first geometric synthesis reaction and the second geometric synthesis reaction are shown in Tables 8-10. FIG. 19B shows the frequency of products of varying lengths in the Phosphoramidite HAE synthesis reaction and first geometric synthesis reaction and FIG. 19C shows the sequence coverage over each position of the target nucleic acid in the products of the Phosphoramidite HAF, synthesis reaction and first geometric synthesis reaction FIG. 19D shows the frequency of products of varying lengths in the Phosphoramidite HAE synthesis reaction and second geometric synthesis reaction and FIG. 19E shows the sequence coverage over each position of the target nucleic acid in the products of the Phosphoramidite HAE synthesis reaction and second geometric synthesis reaction. FIG. 14 shows agarose gel analysis of the products of the Phosphoramidite HAE synthesis reaction, the first geometric synthesis reaction and the second geometric synthesis reaction.

### Example 3I - target nucleic acid containing six variable nucleotides N₁ to N₆ and 50% GC content (N₁ to N₆ target)

Phosphoramidite synthesis and the geometric synthesis methods of the present disclosure were used to synthesize a target nucleic acid that contained six variable nucleotides N₁ to N₆ and had a GC content that of about 50% along the length of the target as measured using a sliding window of 50 nucleotides (herein referred to as a N₁ to N₆ target), as shown in FIG. 20A. As shown in FIG. 20A, Phosphoramidite HAE synthesis was used to synthesize a N₁ to N₆ target that is herein referred to as "NNN_Seq01", a first geometric synthesis reaction was used to synthesize a N₁ to N₆ target that is herein referred to as "NNN_Seq02" and a second geometric synthesis reaction was used to synthesize a N₁ to N₆ target that is herein referred to as "NNN_Seq03". Next generation sequencing analysis of the products of the Phosphoramidite HAE synthesis, the first geometric synthesis reaction and the second geometric synthesis reaction are shown in Tables 8-10. FIG. 20B shows the frequency of products of varying lengths in the Phosphoramidite HAE synthesis reaction and first geometric synthesis reaction and FIG. 20C shows the sequence coverage over each position of the target nucleic acid in the products of the Phosphoramidite HAE synthesis reaction and first geometric synthesis reaction. FIG. 20D shows the frequency of products of varying lengths in the Phosphoramidite HAE synthesis reaction and second geometric synthesis reaction and FIG. 20E shows the sequence coverage over each position of the target nucleic acid in the products of the Phosphoramidite HAE synthesis reaction and second geometric synthesis reaction. FIG. 14 shows agarose gel analysis of the products of the Phosphoramidite HAE synthesis reaction, the first geometric synthesis reaction and the second geometric synthesis reaction.

### Example 3J - target nucleic acid containing 10-nucleotide long A and G homopolymeric regions and 50% GC content (A & G homopolymer target)

Phosphoramidite synthesis and the geometric synthesis methods of the present disclosure were used to synthesize a target nucleic acid that contained 10-nucleotide long A and G homopolymeric regions and had a GC content that of about 50% along the length of the target as measured using a sliding window of 50 nucleotides (herein referred to as an A & G homopolymer target), as shown in FIG. 21A. As shown in FIG. 21A, Phosphoramidite HAE synthesis was used to synthesize an A & G homopolymer target that is herein referred to as "awkAG_Seq01", a first geometric synthesis reaction was used to synthesize an A & G homopolymer target that is herein referred to as "awkAG_Seq02" and a second geometric synthesis reaction was used to synthesize an A & G homopolymer target that is herein referred to as "awkAG_Seq03". Next generation sequencing analysis of the products of the Phosphoramidite HAE synthesis, the first geometric synthesis reaction and the second geometric synthesis reaction are shown in Tables 8-10. FIG. 21B shows the frequency of products of varying lengths in the Phosphoramidite HAE synthesis reaction and first geometric synthesis reaction and FIG. 21C shows the sequence coverage over each position of the target nucleic acid in the products of the Phosphoramidite HAE synthesis reaction and first geometric synthesis reaction. FIG. 21D shows the frequency of products of varying lengths in the Phosphoramidite HAE synthesis reaction and second geometric synthesis reaction and FIG. 21E shows the sequence coverage over each position of the target nucleic acid in the products of the Phosphoramidite HAE synthesis reaction and second geometric synthesis reaction. FIG. 14 shows agarose gel analysis of the products of the Phosphoramidite HAE synthesis reaction, the first geometric synthesis reaction and the second geometric synthesis reaction.

### Example 3K - target nucleic acid containing 10-nucleotide long T and C homopolymeric regions and 50% GC content (T & C homopolymer target)

Phosphoramidite synthesis and the geometric synthesis methods of the present disclosure were used to synthesize a target nucleic acid that contained 10-nucleotide long A and G homopolymeric regions and had a GC content that of about 50% along the length of the target as measured using a sliding window of 50 nucleotides (herein referred to as a T & C homopolymer target), as shown in FIG. 22A. As shown in FIG. 22A, Phosphoramidite HAE synthesis was used to synthesize a T & C homopolymer target that is herein referred to as "awkTC_Seq01", a first geometric synthesis reaction was used to synthesize a T & C homopolymer target that is herein referred to as "awkTC_Seq02" and a second geometric synthesis reaction was used to synthesize a T & C homopolymer target that is herein referred to as "awkTC_Seq03" Next generation sequencing analysis of the products of the Phosphoramidite HAE synthesis, the first geometric synthesis reaction and the second geometric synthesis reaction are shown in Tables 8-10. FIG. 22B shows the frequency of products of varying lengths in the Phosphoramidite HAE synthesis reaction and first geometric synthesis reaction and FIG. 22C shows the sequence coverage over each position of the target nucleic acid in the products of the Phosphoramidite HAE synthesis reaction and first geometric synthesis reaction. FIG. 22D shows the frequency of products of varying lengths in the Phosphoramidite HAE synthesis reaction and second geometric synthesis reaction and FIG. 22E shows the sequence coverage over each position of the target nucleic acid in the products of the Phosphoramidite HAE synthesis reaction and second geometric synthesis reaction. FIG. 14 shows agarose gel analysis of the products of the Phosphoramidite HAE synthesis reaction, the first geometric synthesis reaction and the second geometric synthesis reaction.

### Example 3L - target nucleic acid containing repetitious sequences and 50% GC content (repetitious target)

Phosphoramidite synthesis and the geometric synthesis methods of the present disclosure were used to synthesize a target nucleic acid that contained repetitious sequences had a GC content that of about 50% along the length of the target as measured using a sliding window of 50 nucleotides (herein referred to as a T & C homopolymer target), as shown in FIG. 23A. As shown in FIG. 23A, Phosphoramidite HAE synthesis was used to synthesize a repetitious target that is herein referred to as "rep_Scq01", a first geometric synthesis reaction was used to synthesize a repetitious target that is herein referred to as "rep_Seq02" and a second geometric synthesis reaction was used to synthesize an repetitious target that is herein referred to as "rep_Seq03". Next generation sequencing analysis of the products of the Phosphoramidite HAE synthesis, the first geometric synthesis reaction and the second geometric synthesis reaction are shown in Tables 8-10. FIG. 23B shows the frequency of products of varying lengths in the Phosphoramidite HAE synthesis reaction and first geometric synthesis reaction and FIG. 23C shows the sequence coverage over each position of the target nucleic acid in the products of the Phosphoramidite HAE synthesis reaction and first geometric synthesis reaction. FIG. 23D shows the frequency of products of varying lengths in the Phosphoramidite HAE synthesis reaction and second geometric synthesis reaction and FIG. 23E shows the sequence coverage over each position of the target nucleic acid in the products of the Phosphoramidite HAE synthesis reaction and second geometric synthesis reaction. FIG. 14 shows agarose gel analysis of the products of the Phosphoramidite HAE synthesis reaction, the first geometric synthesis reaction and the second geometric synthesis reaction.

### Example 4 - synthesis and assembly of whole plasmids using the double-stranded geometric synthesis methods of the present disclosure

The following is an example describing the use of the double-stranded geometric synthesis methods of the present disclosure to *de novo* synthesis an entire 2.7 kb plasmid.

The double-stranded geometric synthesis methods of the present disclosure were used to *de novo* synthesis the plasmid pUC19, which is a high-copy number plasmid used in bacteria. The pUC19 plasmid included an ampicillin resistance gene and a multiple cloning site that spans the LacZ gene, permitting the screening of bacteria that contain the pUC19 plasmid using blue-white screening to determine plasmids that contain DNA within the multiple cloning site. As part of the double-stranded synthesis, a coding sequence encoding for the amino acids CAMENA were added.

FIG. 24 shows agarose gel analysis of the products of each round of ligation in the double-stranded geometric synthesis assembly. FIG. 24 shows that the sequential rounds of the double-stranded geometric synthesis assembly produced exponentially longer double-stranded DNA fragments, eventually leading to the highly pure production of the entire pUC19 plasmid. The produced plasmid was then transformed into DH5a bacteria cells, which were grown overnight on an LB-Amp plate. Formation of blue bacterial colonies was observed, indicating the pUC19 plasmid produced using the double-stranded geometric synthesis method contain the desired sequence. Additionally, the products of the double-stranded geometric synthesis method were sequenced. Of the 62 bacterial colonies that were chosen for sequencing, 100% of the colonies contained plasmids with the correct CAMENA sequence.

The results described in this example demonstrate that, unlike existing DNA assembly methods, the double-stranded geometric synthesis methods of the present disclosure can be used to generate gene-length DNA fragments, including those as long as 2.7 kB with high fidelity and high purity.

### Example 3 - 4-mer triplets and quintuplets for used in 5' overhangs

The following example describes the use of highly specific 4-mer overhangs in the double-stranded geometric synthesis methods of the present disclosure, and the ability of these 4-mer overhangs to ensure high fidelity of individual ligation reactions within the entire assembly reaction. By ensuring high fidelity of the individual ligation reactions, these specific 4-mer overhangs allow the geometric synthesis methods of the present disclosure to be used to make DNA molecules whose lengths are comparable to the lengths of human genes, which is not currently feasible using existing DNA assembly methodologies or existing phosphoramidite synthesis methodologies

To determine the optimal 4-mer overhangs for use in the methods of the present disclosure, double-stranded geometric synthesis assemblies of the pUC19 plasmid were analyzed.

To generate the pUC19 plasmid using the double-strand geometric synthesis methods of the present disclosure, the pUC19 plasmid was divided into double-stranded fragments comprising two, 5' overhangs. Each 5' overhang comprised a 4 nucleotide, "4-mer" sequence. The 4-mer sequences of the overhangs were selected to exclude self-recognizing sites such as ACGT.

During the assembly process, the results of each ligation was analyzed using agarose gels to score the outcome of each sub-assembly after two sequential rounds of ligation. Thus, each experiments considers four double-stranded nucleic acid fragments, A, B, C and D, which are initially ligated to form AB and CD, and then are ligated to form the new ABCD fragment (FIG. 25). The products of each ligation were scored as followed: Good (expected product, approximately 100 bp), Short (incomplete product, less than 100 bp), Single (extra band at approximately 150 bp), Double (extra band at approximately 200 bp) and Concatemer (large band greater than 200 bp) (*see* FIG. 26). The 'Good' outcomes are desired as they correspond to the desired ligation product. Other outcomes are not effective for assembly.

To determine the best 4-mers for using the 5' overhangs of the fragments being ligated, the outcomes of 247 different experiments were analyzes, covering 170 different 4-mer sites. The 4-mers were analyzed in sets of 5, called "4-mer quintuplets", as each set of two-round ligations (as shown in FIG. 25) require 5 unique 4-mer sites for the 5' overhangs on the four double-stranded nucleic acid fragments that are to be ligated together.

The results of the analysis showed that of the 247 different experiments analyzed, 123 of the 4-mer quintuplets resulted in a "Good" outcome. Further analysis showed that these 123 experiments comprised 58 unique 4-mer quintuplets. Of these 58 unique 4-mer quintuplets, only 27 of the 4-mer quintuplets exhibited only "Good" outcomes (see Table 11).

**Table 1**

| **4-mer Quintuplet Identifier No. ('Good' Outcome)** | **4-mer #1** | **4-mer #2** | **4-mer #3** | **4-mer #4** | **4-mer #5** | **Matched 4-mer Quintuplets with 'Good' Outcomes** | **Matched 4-mer Quintuplets with 'Concatmer' Outcomes** | **Matched 4-mer Quintuplets with 'Double' Outcomes** | **Matched 4-mer Quintuplets with 'Single' Outcomes** | **Matched** 4-**mer Quintuplets with 'Short' Outcomes** |
|---|---|---|---|---|---|---|---|---|---|---|
| 238 | AAAC | AAGG | TGAC | TCGT | GTAG | 211, 184, 157, 19 | | | | |
| 139 | ACGC | CGTT | CTGG | GGAA | CGCA | 1 | | | | |
| 92 | AGCC | CACC | GCAA | ACTG | TCCC | | | | | |
| 234 | AGCC | CACC | GCAA | TGGC | TCCC | 207, 180, 153, 91 | | | | |
| 95 | AGCC | GACA | GCAA | ACTG | TCCC | | | | | |
| 94 | AGCC | GACA | GCAA | TGGC | TCCC | | | | | |
| 246 | ATCC | TACC | ACCG | CCGA | GAGG | 219, 192, 165 | | | | |
| 204 | CAAC | TTTT | TGAT | ATGT | TGAC | | | | | |
| 231 | CGAG | AACA | AGTT | TGAT | TGTG | | | | | |
| 220 | GAGG | ACGC | CGTT | CTGG | CGCA | | | | | |
| 247 | GAGG | ACGC | CGTT | CTGG | GGAA | | | | | |
| 193 | GAGG | ACGC | CGTT | GGAA | CGCA | | | | | |
| 166 | GAGG | ACGC | CTGG | GGAA | CGCA | | | | | |
| 224 | GAGG | CCCA | TGGC | CTCC | TCAC | 197, 170, 143, 5 | | | | |
| 245 | GCTC | ATGG | CGGT | TTGC | ATCC | 218, 191, 164, 26 | | | | |
| 228 | GGAA | GTTT | ATCC | ATGC | AAGG | 201, 174, 9, 147 | | | | |
| 126 | GTAG | TCTG | CTGC | TCTT | ACGA | | | | | |
| 128 | GTAG | TCTG | CTGC | TGTC | ACGA | | | | | |
| 123 | GTAG | TCTG | CTGC | TCTT | ACGA | | | | | |
| 124 | GTAG | TCTG | CTGC | TGTC | ACGA | | | | | |
| 177 | GTTT | ATGA | ATGT | TTGA | GGTC | | | | | |
| 46 | TCAC | TCTG | AACG | CACC | CGCC | | | | | |
| 48 | TCAC | TGCT | AACG | GACA | CGCC | | | | | |
| 50 | TCAC | TGCT | AAAA | CACC | CGCC | 49, 45 | | | | |
| 47 | TCAC | TGCT | AAAA | GACA | CGCC | | | | | |
| 43 | TCAC | TGCT | AACG | CACC | CGCC | | | | | |
| 44 | TCAC | TGCT | AACG | GACA | CGCC | | | | | |
| 202 | AAGG | TTCC | TTGC | TTGC | CGATG | 175, 148, 10 | 229 | | | |
| 227 | ACCG | GCCT | AGGT | AGAC | GGAA | 146, 8 | | 200 | | |
| 215 | ACGA | GGTC | GCAC | AACG | TACC | 161, 23 | 242 | | | |
| 240 | ACTC | CAGC | CTGT | AGGC | GTAG | 159, 21 | | | | 213 |
| 244 | CGCA | AAAC | CCTG | GACT | GCTC | 217, 163, 25 | | 190 | | |
| 194 | CGCA | AGGC | ATGC | GGAA | TCAC | 167 | 221 | 140 | | |
| 226 | CGCC | CGGC | TGTG | TGTG | ACCG | | 7 | 172 | 199 | |
| 223 | CGTC | GGAA | ATCG | TCGC | GAGG | 142, 4 | 196 | | 169 | |
| 182 | CGG | CGCG | GGCC | TATC | GGCA | 155, 103 | | 209 | 236 | |
| 106 | CTGG | CGCG | GGCC | TCGT | GGCA | 105 | 101 | | | |
| 230 | GATG | CGAG | CAAC | GTTT | GATG | 203 | 176 | | | |
| 150 | GATG | GTGG | TTGA | CGGT | TGAC | 71 | | | | |
| 74 | GATG | GTGG | TTGA | GTCG | TGAC | 73 | | 69 | | |
| 107 | GGCA | TCGC | CATT | CTCA | AAAC | | | | | |
| 237 | GGCA | TCGC | CATT | TACT | AAAC | 156, 108 | 183 | 210 | | |
| 239 | GTAG | CTGC | AAAC | GTTT | ACTC | 212, 185 | | | | 158 |
| 241 | GTAG | TCTG | TGCT | TGTC | ACGA | 214, 187, 160 | | | | |
| 130 | GT AG | TGCT | TGCT | TCTT | ACGA | 129 | 125 | | | |
| 216 | TACC | AAAG | AGGC | GCGG | CGCA | 162, 136 | 243 | | | |
| 134 | TACC | AAAG | AGGC | GGCA | CGCA | | | | | |
| 133 | TACC | AGCG | AAA G | GGCA | CGCA | | 138 | | | |
| 131 | TACC | AGCG | AGGC | GGCA | CGCA | | | | | |
| 42 | TCAC | CGCC | GGTC | ACCG | CGTC | 41 | | | | 37 |
| 36 | TCAC | CGCC | TCGA | GTAC | CGTC | | | | | |
| 144 | TCAC | CTGC | AAAC | ACAC | CGCC | 198 | 225 | 6 | | |
| 222 | TCAC | TACG | GTCG | ACCG | CGTC | 195 | | 168 | | |
| 38 | TCAC | TACG | TCGA | ACCG | CGTC | | | | | |
| 40 | TCAC | TACG | TCGA | GTAC | CGTC | | | | | |
| 16 | TCCC | GGAT | GGAC | CGGC | CTGG | 154, 208, 181 | | 235 | | |
| 232 | TGAC | CACC | TGA C | GAGT | TGAC | 178 | 205 | | | |
| 179 | TGAC | GGAG | ATGG | ATCG | AGCC | 233 | 206 | 14 | | |

That is, in all of the experiments that used one of these 27 4-mer quintuplets, each of the experiments resulted in the generation of the proper product. These 27 4-mer quintuplets are shown in Table 2. The remaining 31 unique 4-mer quintuplets (58-27=31) exhibited either "short", "single", "double" or "concatemer" outcomes when tested in other experiments.

In addition to positive outcome producing combination of sites, the data also reveal unsatisfactory site combinations. Analysis of the four not-'Good' outcome classes shows, firstly that there are proportionally fewer negative outcome combinations. For the 'Short' outcome we found that of 16 original experiments, 12 were unique and 6 were 'Short'-only, three of the remaining 6 had 'Good' matches as quintuplets and the final 3 had many 'Good' matches as triplets. For the 'Single' outcome we found that of the 22 original experiments, 20 were unique and 5 were 'Single' -only. For the 'Double ' outcome we found that of the 32 original experiments, 25 were unique and 7 were 'Double'-only. Finally, for the 'Concatemer' outcome, which is the most abundant negative outcome, there were 38 unique experiments of the 54 total and there were 22 'Concatemer'-only quintuplets (*see* Tables 12-15).

**Table 12**

| **4-mer Quintuplet Identifier No. ('Concalamer' Outcome)** | **4-mer #1** | **4-mer #2** | **4-mer #3** | **4-mer #4** | **4-mer #5** | **Matched 4-mer Quintuplets with 'Concatmer' Outcomes** | **Matched 4-mer Quintuplets with 'Good' Outcomes** | **Matched 4-mer Quintuplets with 'Double' Outcomes** | **Matched 4-mer Quintuplets with 'Single' Outcomes** | **Matched 4-mer Quintuplets with 'Short' Outcomes** |
|---|---|---|---|---|---|---|---|---|---|---|
| 229 | AAGG | TTCC | TTGC | TTGC | GATG | | 202 | | | |
| 242 | ACGA | GGTC | GCAC | AACG | TACC | 188 | 215 | | | |
| 95 | AGCC | CACC | TGGC | ACTG | TCCC | | | | | |
| 98 | AGCC | CACC | TGGC | TGGC | TCCC | 97,93 | | | | |
| 221 | CGCA | AGGC | ATGC | GGAA | TCAC | | 194 | 140 | 199 | |
| 7 | CGCC | CGGC | TGTG | TGTG | ACCG | | 226 | 172 | 169 | |
| 196 | CGTC | GGAA | ATCG | TCGC | GAGG | | 223 | | | |
| 101 | CTGG | CGCG | GGCC | TCGT | GGCA | | 106 | | | |
| 17 | CTGG | TCGC | GCCA | ATCG | GGCA | | | | | |
| 64 | GATG | ACGA | AACA | AGTT | GATG | | | | | |
| 62 | GATG | ACGA | AACA | TTTT | GATG | | | | | |
| 72 | GATG | ATGT | GACG | CGGT | TGAC | | | | | |
| 70 | GATG | ATGT | GACG | GTCG | TGAC | | | | | |
| 176 | GATG | CGAG | CAAC | GTTT | GATG | 149,11 | 230 | | | |
| 60 | GATG | GAGT | AACA | AGTT | GATG | | | | | |
| 59 | GATG | GAGT | AACA | TTTT | GATG | | | | | |
| 63 | GATG | GAGT | TCAA | AGTT | GATG | | | | | |
| 66 | GATG | GAGT | TCAA | TTTT | GATG | 65, 61 | | | | |
| 68 | GATG | GTGG | GACG | CGGT | TGAC | | | | | |
| 67 | GATG | GTGG | GACG | GTCG | TGAC | | | | | |
| 12 | GATG | TGTG | TGAC | GGTC | TGAC | | | | | |
| 114 | GGCA | TCGC | AGCA | CTCA | AAAC | 113, 109 | | | | |
| 111 | GGCA | TCGC | AGCA | TACT | AAAC | | | | | |
| 183 | GGCA | TCGC | CATT | TACT | AAAC | | 237 | 210 | | |
| 123 | GTAG | TGCT | TGCT | TCTT | ACGA | | 130 | | | |
| 243 | TACC | AAAG | AGGC | GCGG | CGCA | 189 | 216 | | | |
| 135 | TACC | AGCG | AAA G | GCGG | CGCA | | | | | |
| 138 | TACC | AGCG | AAAG | GGCA | C GCA | 137 | 133 | | | |
| 225 | TCAC | CTGC | AAAC | ACAC | CGCC | 171 | 144 | 6 | | |
| 82 | TGAC | ACAG | ATGA | AGTG | TGAC | 81, 77 | | | | |
| 79 | TGAC | ACAG | ATGA | TGAG | TGAC | | | | | |
| 75 | TGAC | ACAG | GACA | AGTG | TGAC | | | | | |
| 76 | TGAC | ACAG | GACA | TGAG | TGAC | | | | | |
| 205 | TGAC | CACA | TGAC | GAGT | TGAC | 13, 151 | 232 | | | |
| 87 | TGAC | GAGC | CATG | GATC | AGCC | | | | | |
| 206 | TGAC | GGAG | ATGG | ATCG | AGCC | | 179 | 14 | | |
| 78 | TGAC | TCAC | GACA | AGTG | TGAC | | | | | |
| 80 | TGAC | TCAC | GACA | TGAG | TGAC | | | | | |

**Table 13**

| **4-mer Quintuplet Identifier No. ('Single' Outcome)** | **4-mer #1** | **4-mer #2** | **4-mer #3** | **4-mer #4** | **4-mer #5** | **Matched 4-mer Quintuplets with 'Single' Outcomes** | **Matched 4-mer Quintuplets with 'Good' Outcomes** | **Matched 4-mer Quintuplets with 'Concatmer' Outcomes** | **Matched 4-mer Quintuplets with 'Short' Outcomes** | **Matched 4-mer Quintuplets witti 'Double' Outcomes** |
|---|---|---|---|---|---|---|---|---|---|---|
| 15 | AGCC | ACAC | GGCA | CTGG | TCCC | | | | | |
| 56 | CGCC | CCGG | GTGA | ATGT | ACCG | | | | | |
| 54 | CGCC | CCGG | GTGA | GTGT | ACCG | | | | | |
| 199 | CGCC | CGGC | TGTG | TGTG | ACCG | 145 | | | | |
| 55 | CGCC | GGCA | CTGT | ATGT | ACCG | | 226 | 7 | | 172 |
| 58 | CGCC | GGCA | CTGT | CTGT | ACCG | 57 | | | | |
| 169 | CGTC | GGAA | ATCG | TCGC | GAGG | | 223 | 197 | | 53 |
| 100 | CTGG | CGCG | CCAG | TATC | GGCA | | | | | |
| 99 | CTGG | CGCG | CCAG | TCGT | GGCA | | | | | |
| 236 | CTGG | CGCG | GGCC | TATC | GGCA | | 182 | | | 209 |
| 104 | CTGG | CTCG | CCAG | TATC | GGCA | | | | | |
| 102 | CTGG | CTCG | CCAG | TCGT | GGCA | | | | | |
| 110 | GGCA | GATC | CATT | CTCA | AAAC | | | | | |
| 112 | GGCA | GATC | CATT | TACT | AAAC | | | | | |
| 22 | GTAG | CTGC | GCTG | GTCT | ACGA | | | | | |
| 132 | TACC | AGCG | AGGC | GCAGG | CGCA | | | | | |
| 3 | TCAC | ACGC | GTCG | TACC | CGTC | | | | | |
| 141 | TCAC | TACG | GGTC | ACCG | CGTC | | | | | |
| 88 | TGAC | AGGA | TGGG | GATC | AGCC | | | | | |
| 84 | TGAC | GAGC | TGGG | GATC | AGCC | | | | | |

**Table 14**

| **4-mer Quintuplet Identifier No. ('Double' Outcome)** | **4-mer #1** | **4-mer #2** | **4-mer #3** | **4-mer #4** | **4-mer #5** | **Matched 4-mer Quintuplets with 'Double' Outcomes** | **Matched 4-mer Quintuplets with 'Good' Outcomes** | **Matched 4-mer Quintuplets with 'Concatmer' Outcomes** | **Matched 4-mer Quintuplets with 'Single' Outcomes** | **Matched 4-mer Quintuplets with 'Short' Outcomes** |
|---|---|---|---|---|---|---|---|---|---|---|
| 200 | ACCG | GCCT | AGGT | AGAC | GGAA | 173 | 227 | | | |
| 190 | CGCA | AAAC | CCTG | GACT | GCTC | | 244 | | | |
| 140 | CGCA | AGGC | ATGC | GGAA | TCAC | 2 | 194 | 221 | | |
| 34 | CGCA | GGCA | TATG | GAAT | TCAC | 33, 29 | | | | |
| 31 | CGCA | GGCA | TATG | TGGA | TCAC | | | | | |
| 27 | CGCA | GGCA | TGCT | GAAT | TCAC | | | | | |
| 28 | CGCA | GGCA | TGCT | TGGA | TCAC | | | | | |
| 30 | CGCA | TAGG | TGCT | GAAT | TCAC | | | | | |
| 32 | CGCA | TAGG | TGCT | TGGA | TCAC | | | | | |
| 172 | CGCC | CGGC | TGTG | TGTG | ACCG | | 226 | 7 | 199 | |
| 53 | CGCC | GGCA | CTGT | GTGT | ACCG | | | | 58 | |
| 52 | CGCC | GGCA | GTGA | ATGT | ACCG | | | | | |
| 51 | CGCC | GGCA | GTGA | GTGT | ACCG | | | | | |
| 209 | CTGG | CGCG | GGCC | TATC | GGCA | | 182 | | 236 | |
| 69 | GATG | GTGG | TTGA | GTCG | TGAC | | 74 | | | |
| 18 | GGCA | ATCG | GCAT | ACTC | AAAC | | | | | |
| 210 | GGCA | TCGC | CATT | TACT | AAAC | | 237 | 183 | | |
| 24 | TACC | AAGC | AAGG | CGGC | CGCA | | | | | |
| 6 | TCAC | CTGC | AAAC | ACAC | CGCC | | 144 | 225 | | |
| 168 | TCAC | TACG | GTCG | ACCG | CGTC | | 222 | | | |
| 235 | TCCC | GGAT | GGAC | CGGC | CTGG | | 16 | | | |
| 86 | TGAC | AGGA | TGGG | TCGT | AGCC | | | | | |
| 90 | TGAC | GAGC | CATG | TCGT | AGCC | 89, 85 | | | | |
| 83 | TGAC | GAGC | TGGG | TCGT | AGCC | | | | | |
| 14 | TGAC | GGAG | ATGG | ATCG | AGCC | 152 | 179 | 206 | | |

**Table 15**

| **4-mer Quintuplet Identifier No. ('Short' Outcome)** | **4-mer #1** | **4-mer #2** | **4-mer #3** | **4-mer #4** | **4-mer #5** | **Matched 4-mer Quintuplets with 'Short' Outcomes** | **Matched 4-mer Quintuplets with 'Good' Outcomes** | **Matched 4-mer Quintuplets with 'Concatmer' Outcomes** | **Matched 4-mer Quintuplets with 'Single' Outcomes** | **Matched 4-mer Quintuplets with 'Double' Outcomes** |
|---|---|---|---|---|---|---|---|---|---|---|
| 213 | ACTC | CAGC | CTGT | AGGC | GTAG | 186 | 240 | | | |
| 158 | GTAG | CTGC | AAAC | GTTT | ACTC | 20 | 239 | | | |
| 120 | GTAG | TCTG | AACC | TGTT | ACTC | | | | | |
| 118 | GTAG | TCTG | AACC | TTTG | ACTC | | | | | |
| 119 | GTAG | TGCG | AAAA | TGTT | ACTC | | | | | |
| 122 | GTAG | TGCG | AAAA | TTTG | ACTC | 121, 117 | | | | |
| 116 | GTAG | TGCG | AACC | TGTT | ACTC | | | | | |
| 115 | GTAG | TGCG | AACC | TTTG | ACTC | | | | | |
| 127 | GTAG | TGCT | TGCT | TGTC | ACGA | | | | | |
| 37 | TCAC | CGCC | GGTC | ACCG | CGTC | | 42 | | | |
| 39 | TCAC | CGCC | GGTC | GTAC | CGTC | | | | | |
| 35 | TCAC | CGCC | TGCGA | ACCG | CGTC | | | | | |

In the two rounds of ligation that are shown in FIG. 25, there are three relatively independent pair-wise ligations, each of which require a set of three 4-mer sites, called a "4-mer triplet". Further analysis of the data revealed that the **4-mer** triplets listed in Table 1 produced the most "Good" outcomes, and are therefore the most effective in promoting ligations that result in the formation of the desired products.

Thus, the results of this example demonstrate that sets of double-stranded nucleic acid fragments comprising 5' overhangs that comprise the 4-mer quintuplets listed in Table 2 or the **4-mer** triplets listed in **Table** 1 display unexpected and superior in that they can be used in highly efficient and highly accurate ligations reactions within a double-stranded geometric assembly reaction.

### Example 6-4-mer sequences for use in the compositions and methods of the present disclosure

The following example describes the derivation of optimal 4-mers for use in the geometric synthesis methods of the present disclosure.

To determine 4-mers that demonstrate increased fidelity (*i.e.* the percentage of the time the 4-mer correctly hybridizes and ligates to another nucleic acid molecule comprising the complementary 4-mer as opposed to a fragment comprising a mismatched 4-mer) and yield (*i.e.* the frequency of ligation events) in ligation reactions in the geometric synthesis methods of the present disclosure, the large-scale ligation data presented in Potapov et al. ("Comprehensive Profiling of Four Base Overhang Ligation Fidelity by T4 DNA Ligase and Application to DNA assembly," ACS Synthetic Biology, 2018, 7, 11, 2665-2674) was further analyzed. As shown in Table 16, for 256 different 4-mers tested, the number of ligation events was analyzed to determine how many of these ligation events were matched (*i.e.* to a fragment with a complementary 4-mer overhang; 'Total Matched Ligations Observed') and how many mismatched ligation evens were observed (*i.e.* to a fragment with a non-complementary 4-mer overhang; 'Total Mismatch Ligations observed'). A fidelity percentage was then determined by 'Total Matched Ligations Observed' by the 'Total Ligations Observed' (matched + mismatched). Additionally, for each of the 4-mers, the top three non-complementary 4-mers that the 4-mer mismatched with were determined, along with the percentage of the mismatches that corresponded to each of the top three 4-mer mismatches (*see* **Table** 17).

The 4-mers that demonstrated high fidelity and/or yield were further selected for use in the methods of the present disclosure. These 4-mers are presented in Tables 3, 4 and 5.

**Table 16**

| **4-mer** | **Total Ligations Observed** | **Total Matched Ligations observed** | **Total Mismatch Ligations observed** | **Yield (%)** | **Fidelity (%)** |
|---|---|---|---|---|---|
| AAAA | 57 | 57 | 0 | 14.3 | 100.00 |
| CCCC | 1240 | 1235 | 5 | 310.0 | 99.60 |
| AAAG | 331 | 327 | 4 | 82.8 | 98.79 |
| CAAA | 22 4 | 221 | 3 | 56.0 | 98.66 |
| CAGA | 647 | 635 | 12 | 161.8 | 98.15 |
| CTAA | 139 | 136 | 3 | 34.8 | 97.84 |
| GAAA | 324 | 317 | 7 | 81.0 | 97.84 |
| AAAC | 1156 | 1129 | 27 | 289.0 | 97.66 |
| ATAA | 79 | 77 | 2 | 19.8 | 97.47 |
| ACCC | 2333 | 2273 | 60 | 583.3 | 97.43 |
| AATA | 77 | 75 | 2 | 19.3 | 97.40 |
| AAGC | 2108 | 2048 | 60 | 527.0 | 97.15 |
| CAAC | 1842 | 1787 | 55 | 460.5 | 97.01 |
| CCCG | 2039 | 1977 | 62 | 509.8 | 96.96 |
| CAGC | 2521 | 2442 | 79 | 630.3 | 96.87 |
| ATCC | 2140 | 2069 | 71 | 535.0 | 96.68 |
| ATAG | 419 | 405 | 14 | 104.8 | 96.66 |
| CAAG | 419 | 405 | 14 | 104.8 | 96.66 |
| CACC | 2385 | 2305 | 80 | 596.3 | 96.65 |
| CCAA | 742 | 717 | 25 | 185.5 | 96.63 |
| CTCC | 1892 | 1826 | 66 | 473.0 | 96.51 |
| CCGC | 2165 | 2089 | 76 | 541.3 | 96.49 |
| GCAA | 1418 | 1368 | 50 | 354.5 | 96.47 |
| AGAA | 224 | 216 | 8 | 56.0 | 96.43 |
| AACC | 2334 | 2250 | 84 | 583.5 | 96.40 |
| CTGC | 2412 | 2319 | 93 | 603.0 | 96.14 |
| GAAG | 1181 | 1134 | 47 | 295.3 | 96.02 |
| AAGG | 1081 | 1037 | 44 | 270.3 | 95.93 |
| CTCG | 1248 | 1197 | 51 | 312.0 | 95.91 |
| CGCA | 2001 | 1919 | 82 | 500.3 | 95.90 |
| CTAC | 315 | 302 | 13 | 78.8 | 95.87 |
| GAAC | 2081 | 1994 | 87 | 520.3 | 95.82 |
| GAGA | 851 | 815 | 36 | 212.8 | 95.77 |
| AAGA | 210 | 201 | 9 | 52.5 | 95.71 |
| CCAC | 2193 | 2099 | 94 | 548.3 | 95.71 |
| CGAA | 803 | 768 | 35 | 200.8 | 95.64 |
| CGCC | 2314 | 2212 | 102 | 578.5 | 95.59 |
| CCAG | 1700 | 1624 | 76 | 425.0 | 95.53 |
| CAGG | 1661 | 1586 | 75 | 415.3 | 95.48 |
| GATC | 2328 | 2222 | 106 | 582.0 | 95.45 |
| CCCA | 1499 | 1430 | 69 | 374.8 | 95.40 |
| AATG | 539 | 514 | 25 | 134.8 | 95.36 |
| AATC | 709 | 676 | 33 | 177.3 | 95.35 |
| GTAA | 377 | 359 | 18 | 94.3 | 95.23 |
| ATGC | 2195 | 2088 | 107 | 548.8 | 95.13 |
| TTTT | 60 | 57 | 3 | 1.5.0 | 95.00 |
| CATG | 1055 | 1002 | 53 | 263.8 | 94.98 |
| CATC | 1876 | 1781 | 95 | 469.0 | 94.94 |
| AATT | 177 | 168 | 9 | 44.3 | 94.92 |
| ATCA | 442 | 418 | 24 | 110.5 | 94.57 |
| ACAA | 384 | 363 | 21 | 96.0 | 94.53 |
| TTAG | 144 | 136 | 8 | 36.0 | 94.44 |
| AGCC | 2666 | 2517 | 149 | 666.5 | 94.41 |
| GGAA | 1188 | 1120 | 68 | 297.0 | 94.28 |
| AGAG | 929 | 875 | 54 | 232.3 | 94.19 |
| GATA | 461 | 434 | 27 | 115.3 | 94.14 |
| CACA | 1038 | 977 | 61 | 259.5 | 94.12 |
| TTTA | 17 | 16 | 1 | 4.3 | 94.12 |
| ATCG | 1670 | 1568 | 102 | 417.5 | 93.89 |
| GATG | 1898 | 178 1 | 1 17 | 474.5 | 93.84 |
| GTCC | 2846 | 2669 | 177 | 711.5 | 93.78 |
| AACG | 1452 | 1361 | 91 | 363.0 | 93.73 |
| GTCA | 1423 | 1333 | 90 | 355.8 | 93.68 |
| AACA | 471 | 439 | 32 | 117.8 | 93.2.1 |
| ATTA | 88 | 82 | 6 | 22.0 | 93.18 |
| CGTC | 2621 | 2441 | 180 | 655.3 | 93.13 |
| CTTG | 43 5 | 405 | 30 | 108.8 | 93.10 |
| CTAC | 1302 | 1212 | 90 | 325.5 | 93.09 |
| CATA | 289 | 269 | 20 | 72.3 | 93.08 |
| ATAC | 906 | 843 | 63 | 226.5 | 93.05 |
| CACG | 2082 | 1935 | 147 | 520.5 | 92.94 |
| CGAG | 1288 | 1197 | 91 | 322.0 | 92.93 |
| ATTC | 920 | 855 | 65 | 230.0 | 92.93 |
| GTAG | 1305 | 1212 | 93 | 326,3 | 92.87 |
| CGAC | 2521 | 2341 | 180 | 630.3 | 92.86 |
| GTAC | 2121 | 1968 | 153 | 530.3 | 92.79 |
| CATT | 554 | 514 | 40 | 138.5 | 92.78 |
| ACCG | 2730 | 2532 | 198 | 682.5 | 92.75 |
| AGAC | 1861 | 1724 | 137 | 465.3 | 92.64 |
| CTCA | 668 | 618 | 50 | 167.0 | 92.51 |
| AGTC | 1797 | 1660 | 137 | 449.3 | 92.38 |
| GACC | 2640 | 2438 | 202 | 660.0 | 92.35 |
| GAGC | 2739 | 2529 | 210 | 684.8 | 92.33 |
| TTCG | 832 | 768 | 64 | 208.0 | 92.31 |
| TGAG | 670 | 618 | 52 | 167.5 | 92.24 |
| CGGA | 1949 | 1797 | 152. | 487.3 | 92.20 |
| CGTA | 908 | 837 | 71 | 227.0 | 92.18 |
| CTTA | 166 | 153 | 13 | 41.5 | 92.17 |
| TAAG | 166 | 153 | 13 | 41.5 | 92.17 |
| ACAG | 1322 | 1218 | 104 | 330.5 | 92.13 |
| TATG | 292 | 269 | 23 | 73.0 | 92.12 |
| AAGT | 495 | 456 | 39 | 123.8 | 92.12 |
| GATT | 734 | 676 | 58 | 183.5 | 92.10 |
| GCTA | 1245 | 1146 | 99 | 311.3 | 92.05 |
| GCAC | 2841 | 2615 | 226 | 710.3 | 92.05 |
| GTTC | 2167 | 1994 | 173 | 541.8 | 92.02 |
| CGCG | 2572 | 2366 | 206 | 643.0 | 91.99 |
| GCAG | 2522 | 2319 | 203 | 630.5 | 91.95 |
| TTGC | 1490 | 1368 | 122 | 372.5 | 91.81 |
| AGTG | 1001 | 919 | 82 | 250.3 | 91.81 |
| CGGC | 2930 | 2689 | 241 | 732.5 | 91.77 |
| CTTC | 1236 | 1134 | 102 | 309.0 | 91.75 |
| TTTG | 241 | 221 | 20 | 60.3 | 91.70 |
| GTGA | 1390 | 1274 | 116 | 347.5 | 91.65 |
| CCTG | 1731 | 1586 | 145 | 432.8 | 91.62 |
| GCCA | 2422 | 2219 | 203 | 605.5 | 91.62 |
| GCCC | 2660 | 2436 | 224 | 665.0 | 91.58 |
| CTGG | 1775 | 1624 | 151 | 443.8 | 91.49 |
| CTGT | 1332 | 1218 | 114 | 333.0 | 91.44 |
| CAGT | 919 | 840 | 79 | 229.8 | 91.40 |
| AGCA | 1311 | 1198 | 113 | 327.8 | 91.38 |
| GACA | 1424 | 1300 | 124 | 356.0 | 91.29 |
| TCGC | 2559 | 2336 | 223 | 639.8 | 91.29 |
| GACG | 2676 | 2441 | 235 | 669.0 | 91.22 |
| CGTG | 2125 | 1935 | 190 | 531.3 | 91.06 |
| TCCC | 2173 | 1977 | 196 | 543.3 | 90.98 |
| ATGA | 387 | 352 | 35 | 96.8 | 90,96 |
| AGGC | 2669 | 2427 | 242 | 667.3 | 90.93 |
| ATGG | 1568 | 1425 | 143 | 392.0 | 90.88 |
| ACAC | 2255 | 2049 | 206 | 563.8 | 90.86 |
| ATTT | 228 | 207 | 21 | 57.0 | 90.79 |
| CCTC | 1999 | 1814 | 185 | 499.8 | 90.75 |
| TTCC | 1235 | 1120 | 115 | 308.8 | 90.69 |
| AGGA | 895 | 811 | 84 | 223.8 | 90.61 |
| ATTG | 649 | 588 | 61 | 162.3 | 90.60 |
| GGTA | 1653 | 1497 | 156 | 413.3 | 90.56 |
| CGTT | 1503 | 1361 | 142 | 375.8 | 90.55 |
| GAGT | 1617 | 1464 | 153 | 404.3 | 90.54 |
| CCCT | 1687 | 1523 | 104 | 421.8 | 90.28 |
| GCTC | 2802 | 2529 | 273 | 700.5 | 90.26 |
| ATAT | 215 | 194 | 21 | 53.8 | 90.23 |
| GTTA | 439 | 396 | 43 | 109.8 | 90.21 |
| TCCG | 1993 | 1797 | 196 | 498.3 | 90,17 |
| CGCT | 2463 | 2220 | 243 | 615.8 | 90.13 |
| GAAT | 949 | 855 | 94 | 237.3 | 90.09 |
| CCAT | 1584 | 1425 | 159 | 396.0 | 89.96 |
| GCAT | 2321 | 2088 | 233 | 580.3 | 89.96 |
| CTGA | 616 | 554 | 62 | 154.0 | 89.94 |
| GTTG | 1987 | 1787 | 200 | 496.8 | 89.93 |
| GTTT | 1256 | 1129 | 127 | 314.0 | 89.89 |
| GTCG | 2606 | 2341 | 265 | 651.5 | 89.83 |
| GCTG | 2720 | 2442 | 278 | 680.0 | 89.78 |
| ACGC | 2619 | 2350 | 269 | 654.8 | 89.73 |
| AGTT | 759 | 681 | 78 | 189.8 | 89.72 |
| GCGA | 2608 | 2336 | 272 | 652.0 | 89.57 |
| GGTT | 2512 | 2250 | 262 | 628.0 | 89.57 |
| GTGC | 2920 | 2615 | 305 | 730.0 | 89.55 |
| GCTT | 2287 | 2048 | 239 | 571.8 | 89.55 |
| AGTA | 363 | 325 | 38 | 90.8 | 89.53 |
| TTGG | 801 | 717 | 84 | 200.3 | 89.51 |
| AACT | 761 | 681 | 80 | 190.3 | 89.49 |
| ATGT | 878 | 785 | 93 | 219.5 | 89.41 |
| ACCA | 1358 | 1214 | 144 | 339.5 | 89.40 |
| CAAT | 658 | 588 | 70 | 164.5 | 89.36 |
| CCGA | 1874 | 1673 | 201 | 468.5 | 89.27 |
| CGAT | 1759 | 1568 | 191 | 43 9. S | 89.14 |
| AGCG | 2491 | 2220 | 271 | 622.8 | 89.12 |
| CTTT | 367 | 327 | 40 | 91.8 | 89.10 |
| TAAA | 18 | 16 | 2 | 4.5 | 88.89 |
| AAAT | 233 | 207 | 26 | 58.3 | 88.84 |
| CGGT | 2851 | 2532 | 319 | 712.8 | 88.81 |
| GTAT | 951 | 843 | 108 | 237.8 | 88.64 |
| TTAT | 87 | 77 | 10 | 21.8 | 88.51 |
| GCCG | 3039 | 2689 | 350 | 759.8 | 88.48 |
| GTGT | 2316 | 2049 | 267 | 579.0 | 88.47 |
| GAGG | 2051 | 1814 | 237 | 512.8 | 88,44 |
| TATA | 43 | 38 | 5 | 10.8 | 88.37 |
| ATCT | 684 | 604 | 80 | 171.0 | 88.30 |
| TGCC | 2690 | 2375 | 315 | 672.5 | 88.29 |
| TATT | 85 | 75 | 10 | 21.3 | 88.24 |
| CCGT | 2446 | 2158 | 288 | 611.5 | 88.23 |
| AGGT | 1939 | 1709 | 230 | 484.8 | 88.14 |
| CTCT | 993 | 875 | 118 | 248.3 | 88.12 |
| TCAG | 631 | 554 | 77 | 157.8 | 87.80 |
| CCTA | 578 | 507 | 71 | 144.5 | 87.72 |
| AGAT | 689 | 604 | 85 | 172.3 | 87.66 |
| CTAT | 462 | 405 | 57 | 115.5 | 87.66 |
| GGAT | 2364 | 2069 | 295 | 591.0 | 87.52 |
| TTTC | 363 | 317 | 46 | 90.8 | 87.33 |
| ACTG | 963 | 840 | 123 | 240.8 | 87.23 |
| TATC | 499 | 434 | 65 | 124.8 | 86.97 |
| TAGC | 1318 | 1146 | 172 | 329.5 | 86.95 |
| TCAA | 145 | 126 | 19 | 36.3 | 86.90 |
| GTCT | 1984 | 1724 | 260 | 496.0 | 86.90 |
| AGCT | 1777 | 1544 | 233 | 444.3 | 86.89 |
| TCTG | 733 | 635 | 98 | 183.3 | 86.63 |
| GCCT | 2806 | 2427 | 379 | 701.5 | 86.49 |
| TTGT | 420 | 363 | 57 | 105.0 | 86.43 |
| CCTT | 1201 | 1037 | 164 | 300.3 | 86.34 |
| TCGA | 825 | 712 | 113 | 206.3 | 86.30 |
| TTAC | 416 | 359 | 57 | 104.0 | 86.30 |
| GGAG | 2117 | 1826 | 291 | 529.3 | 86.25 |
| GGAC | 3098 | 2669 | 429 | 774.5 | 86.15 |
| TCCA | 657 | 566 | 91 | 164.3 | 86.15 |
| ACGA | 1410 | 1213 | 197 | 352.5 | 86.03 |
| TACC | 1742 | 1497 | 245 | 435.5 | 85.94 |
| GACT | 1932 | 1660 | 272 | 483.0 | 85.92 |
| ACAT | 914 | 785 | 12.9 | 228.5 | 85.89 |
| TACG | 975 | 837 | 138 | 2.43.8 | 85.85 |
| ACTC | 1706 | 1464 | 242 | 426.5 | 85.81 |
| TGTC | 1515 | 1300 | 215 | 378.8 | 85.81 |
| TAGA | 119 | 102 | 17 | 29.8 | 85.71 |
| ACCT | 1995 | 1709 | 286 | 498. 8 | 85.66 |
| TCAC | 1489 | 1274 | 215 | 372.3 | 85.56 |
| TCGT | 1419 | 1213 | 206 | 354.8 | 85.48 |
| GGTC | 2862 | 2438 | 424 | 715.5 | 85.19 |
| TGAA | 135 | 115 | 20 | 33.8 | 85.19 |
| TTCA | 135 | 115 | 20 | 33.8 | 85.19 |
| TAGG | 596 | 507 | 89 | 149.0 | 85.07 |
| TGTG | 1150 | 977 | 173 | 2R7.5 | 84.96 |
| TGCG | 2261 | 1919 | 342 | 565.3 | 84.87 |
| ACTT | 538 | 456 | 82 | 134.5 | 84.76 |
| TCGG | 1977 | 1673 | 304 | 494.3 | 84.62 |
| TGGC | 2628 | 2219 | 409 | 657.0 | 84.44 |
| ACGT | 1970 | 1576 | 294 | 467.5 | 84.28 |
| CCGG | 2769 | 2324 | 445 | 692.3 | 83.93 |
| TACA | 230 | 193 | 37 | 57.5 | 83.91 |
| TCTA | 122 | 102 | 20 | 30.5 | 83.61 |
| GGCA | 2841 | 2375 | 466 | 710.3 | 83.60 |
| TGAC | 1595 | 1333 | 262 | 398.8 | 83.57 |
| TTAA | 24 | 20 | 4 | 6.0 | 83.33 |
| AGGG | 1839 | 1523 | 316 | 459.8 | 82.82 |
| TTCT | 261 | 216 | 45 | 65.3 | 52.76 |
| CGGG | 2389 | 1977 | 412 | 597.3 | 82.75 |
| GGGA | 2391 | 1977 | 414 | 597.8 | 82.69 |
| TAAC | 479 | 396 | 83 | 119.8 | 82.67 |
| GCGC | 2855 | 2350 | 505 | 713.8 | 82.31 |
| TCTC | 992 | 815 | 177 | 248.0 | 82.16 |
| TGGG | 1750 | 1430 | 320 | 437.5 | 81.71 |
| TCTT | 246 | 201 | 45 | 61.5 | 81.71 |
| GTGG | 2569 | 2099 | 470 | 642.3 | 81.70 |
| TCAT | 432 | 352 | 80 | 108.0 | 81.48 |
| TGTT | 539 | 439 | 100 | 134.8 | 81.45 |
| GCGT | 2897 | 2350 | 547 | 724.3 | 81.12 |
| ACTA | 266 | 215 | 51 | 66.5 | 80.83 |
| ACGG | 2679 | 2158 | 521 | 669.8 | 80.55 |
| TGGA | 705 | 566 | 139 | 176.3 | 80.28 |
| GGCT | 3146 | 2517 | 629 | 786.5 | 80.01 |
| TAGT | 269 | 215 | 54 | 67.3 | 79.93 |
| GGCC | 3323 | 2654 | 669 | 830.8 | 79.87 |
| TGTA | 242 | 193 | 49 | 60.5 | 79.75 |
| GGTG | 2899 | 2305 | 594 | 724.8 | 79.51 |
| TGCA | 902 | 714 | 188 | 225.5 | 79.16 |
| TGAT | 530 | 418 | 112 | 132.5 | 78.87 |
| TCCT | 1032 | 811 | 221 | 258.0 | 78,59 |
| TTGA | 161 | 126 | 35 | 40.3 | 78.26 |
| TGGT | 1552 | 1214 | 338 | 388.0 | 78.22 |
| CACT | 1183 | 919 | 264 | 295.8 | 77.68 |
| TACT | 424 | 325 | 99 | 106.0 | 76.65 |
| TGCT | 1571 | 1198 | 373 | 392.8 | 76.26 |
| GGCG | 2921 | 2212 | 709 | 730.3 | 75.73 |
| GGGC | 3234 | 2436 | 798 | 808.5 | 75.32 |
| GGGT | 3190 | 2273 | 917 | 797.5 | 71.25 |
| GCGG | 2948 | 2089 | 859 | 737.0 | 70.86 |
| TAAT | 118 | 82 | 36 | 29.5 | 69.49 |
| GGGG | 2043 | 1235 | 808 | 510.8 | 60.4.5 |

**Table 17**

| **4-mer** | **#1 Mismatch 4-mer** | **% #1 Mismatch 4-mer** | **#2 Mismatch 4-mer** | **% #2 Mismatch 4-mer** | **#3 Mismatch 4-mer** | **% #3 Mismatch 4-mer** |
|---|---|---|---|---|---|---|
| AAAA | N/A | - | N/A | - | N/A | - |
| CCCC | GAGG | 40.0 | GGGT | 20.00 | GGTG | 20.0 |
| AAAG | CTTG | 50.0 | CGTT | 25.00 | CTGT | 25.0 |
| CAAA | TGTG | 66.7 | GTTG | 33.33 | N/A | - |
| CAGA | GCTG | 50.0 | TCTT | 8.33 | TCCG | 8.3 |
| CTAA | GTAG | 66.7 | TTGG | 33.33 | N/A | - |
| GAAA | GTTC | 28.6 | TTGC | 28.57 | TTTT | 14.3 |
| AAAC | GGTT | 48.1 | GTTG | 29.63 | GTGT | 14.8 |
| ATAA | TTGT | 100.0 | N/A | - | N/A | - |
| ACCC | GGGG | 73.3 | GGGC | 10.00 | GGTT | 6.7 |
| AATA | TGTT | 100.0 | N/A | - | N/A | - |
| AAGC | GGTT | 31.7 | GCTG | 25.00 | GCGT | 16.7 |
| CAAC | GGTG | 58.2 | GTGG | 12.73 | GTCG | 7.3 |
| CCCG | TGGG | 69.4 | GGGG | 8.06 | AGGG | 6.5 |
| CAGC | GATG | 27.8 | GGTG | 26.58 | GTTG | 22.8 |
| ATCC | GGGT | 70.4 | GGAG | 9.86 | GGTT | 5.6 |
| ATAG | CTGT | 28.6 | CGAT | 28.57 | TTAT | 21.4 |
| CAAG | TTTG | 50.0 | ATTG | 14.29 | CTGG | 14.3 |
| CACC | GGGG | 77.5 | GGCG | 5.00 | GATG | 3.8 |
| CCAA | GTGG | 72.0 | TGGG | 12.00 | TAGG | 8.0 |
| CTCC | GGGG | 77.3 | GGTG | 12.12 | GGCG | 4.5 |
| CCGC | GTGG | 36.8 | GGGG | 25.00 | ACGG | 13.2 |
| GCAA | TTGT | 32.0 | GTGC | 28.00 | TGGC | 16.0 |
| AGAA | TTTT | 25.0 | GTCT | 25.00 | TGCT | 12.5 |
| AACC | GGGT | 59.5 | GGTG | 23.81 | GGCT | 4.8 |
| CTGC | GCGG | 54.8 | GTAG | 16.13 | GCTG | 9.7 |
| GAAG | CTTT | 34.0 | TTTC | 29.79 | CGTC | 12.8 |
| AAGG | TCTT | 29.5 | CCTG | 20.45 | CATT | 11.4 |
| CTCG | CGGG | 35.3 | TGAG | 35.29 | CGTG | 13.7 |
| CGCA | GGCG | 37.8 | TGTG | 30.49 | TGGG | 9.8 |
| CTAG | CTGG | 53.8 | TTAG | 46.15 | N/A | - |
| GAAC | GTTT | 55.2 | GGTC | 17.24 | GTGC | 10.3 |
| GAGA | TCTT | 41.7 | TTTC | 13.89 | TCTA | 13.9 |
| AAGA | TCTG | 33.3 | GCTT | 22.22 | TCGT | 22.2 |
| CCAC | GGGG | 89.4 | GCGG | 6.38 | GAGG | 3.2 |
| CGAA | GTCG | 37.1 | TGCG | 25.71 | TTTG | 22.9 |
| CGCC | GGTG | 50.0 | GGGG | 20.59 | GACG | 7.8 |
| CCAG | TTGG | 44.7 | GTGG | 19.74 | ATGG | 18.4 |
| CAGG | TCTG | 36.0 | CGTG | 18.67 | CTTG | 9.3 |
| GATC | GGTC | 64.2 | GATT | 16.04 | GAGC | 14.2 |
| CCCA | GGGG | 82.6 | CGGG | 7.25 | TGTG | 4.3 |
| AATG | CGTT | 60.0 | TATT | 20.00 | CACT | 8.0 |
| AATC | GGTT | 90.9 | GAGT | 3.03 | GATA | 3.0 |
| GTAA | TTGC | 33.3 | GTAC | 33.33 | TGAC | 11.1 |
| ATGC | GCGT | 57.0 | GCAG | 12.15 | GTAT | 11.2 |
| TTTT | AGAA | 66.7 | GAAA | 33.33 | TTTT | - |
| CATG | CGTG | 52.8 | TATG | 26.42 | CAGG | 9.4 |
| CATC | GGTG | 75.8 | GAGG | 14.74 | ATTG | 3.2 |
| AATT | AGTT | 55.6 | GATT | 33.33 | AAGT | 11.1 |
| ATCA | TGGT | 45.8 | GGAT | 33.33 | TGCT | 8.3 |
| ACAA | TTGG | 33.3 | GTGT | 19.05 | CTGT | 9.5 |
| TTAG | CTAG | 75.0 | CTGA | 25.00 | TTTT | - |
| AGCC | GGGT | 28.2 | GGTT | 25.50 | GGAT | 20.8 |
| GGAA | TTTC | 35.3 | TTCT | 19.12 | GTCC | 10.3 |
| AGAG | TTCT | 25.9 | CTTT | 22.22 | CGCT | 11.1 |
| GATA | TGTC | 59.3 | TAGC | 14.81 | GATA | 7.4 |
| CACA | TGGG | 72.1 | TGCG | 13.11 | GGT G | 6.6 |
| TTTA | GAAA | 100.0 | N/A | - | N/A | - |
| ATCG | TGAT | 42.2 | CGGT | 22.55 | CGAG | 13.7 |
| GATG | TATC | 29.1 | CGTC | 25.64 | CAGC | 18.8 |
| GTCC | GGAT | 49.7 | GGGC | 36.16 | GGAA | 4.0 |
| AACG | TGTT | 38.5 | CGGT | 30.77 | CGTG | 16.5 |
| GTCA | TGGC | 35.6 | TGAT | 28.89 | GGAC | 18.9 |
| AACA | TGGT | 46.9 | TGTG | 31.25 | TGTA | 9.4 |
| ATTA | TGAT | 50.0 | TAGT | 33.33 | GAAT | 16.7 |
| CGTC | GGCG | 62.2 | GATG | 16.67 | GAGG | 4.4 |
| CTTG | CGAG | 53.3 | CAGG | 23.33 | TAAG | 16.7 |
| CTAC | GGAG | 48.9 | GTGG | 33.33 | GTTG | 11.1 |
| CATA | TGTG | 75.0 | GATG | 10.00 | TAGG | 10.0 |
| ATAC | GTGT | 44.4 | GGAT | 30.16 | GTAG | 12.7 |
| CACG | CGGG | 49.0 | TGTG | 36.05 | CACG | 2.7 |
| CGAG | TTCG | 30.8 | CTTG | 17.58 | ATCG | 15.4 |
| ATTC | GGAT | 87.7 | GAGT | 6.15 | GTAT | 3.1 |
| GTAG | CTAT | 38.7 | TTAC | 23.66 | CTGC | 16.1 |
| CGAC | GGCG | 69.4 | GTTG | 17.78 | GTGG | 5.6 |
| GTAC | GTAT | 36.6 | GGAC | 24.18 | GTGC | 22.9 |
| CATT | GATG | 40.0 | AGTG | 30.00 | AAGG | 12.5 |
| ACCG | TGGT | 44.9 | CGGG | 37.37 | GGGT | 9.1 |
| AGAC | GGCT | 56.9 | GTTT | 17.52 | GTAT | 8.0 |
| CTCA | TGGG | 44.0 | GGAG | 32.00 | TGTG | 12.0 |
| AGTC | GGCT | 63.5 | GATT | 15.33 | GAGT | 5.1 |
| GACC | GGGC | 51.0 | GGTT | 23.27 | GGTA | 8.4 |
| GAGC | GCTT | 36.2 | GGTC | 15.71 | GCTA | 15.2 |
| TTCG | CGAG | 43.8 | CGGA | 23.44 | CGAT | 15.6 |
| TGAG | CTCG | 34.6 | CTCT | 23.08 | CTTA | 13.5 |
| CGGA | GCCG | 30.9 | TCTG | 28.95 | TACG | 11.2 |
| CGTA | TGCG | 38.0 | GACG | 30.99 | TATG | 9.9 |
| CTTA | TGAG | 53.8 | GAAG | 30.77 | TAGG | 7.7 |
| TAAG | CTTT | 46.2 | CTTG | 38.46 | CGTA | 15.4 |
| ACAG | CTGG | 54.8 | TTGT | 15.38 | GTGT | 8.7 |
| TATG | CATG | 60.9 | CGTA | 30.43 | CATT | 4.3 |
| AAGT | GCTT | 66.7 | AGTT | 10.26 | ACTG | 7.7 |
| GATT | AGTC | 36.2 | GATC | 29.31 | AAGC | 8.6 |
| GCTA | TGGC | 45.5 | GAGC | 32.32 | TAGT | 13.1 |
| GCAC | GTGT | 40.7 | GGGC | 38.05 | GTGA | 8.0 |
| GTTC | GAAT | 42.8 | GGAC | 41.62 | GAGC | 5.2 |
| CGCG | TGCG | 50.0 | CGTG | 20.39 | CGGG | 9.7 |
| GCAG | CTGT | 31.0 | TTGC | 29.56 | CTGA | 18.7 |
| TTGC | GCAG | 49.2 | GCGA | 28.69 | GCAT | 8.2 |
| AGTG | TACT | 31.7 | CGCT | 18.29 | CATT | 14.6 |
| C GGC | GACG | 29.5 | GCTG | 25.31 | GGCG | 18.7 |
| CTTC | GGAG | 83.3 | GAGG | 6.86 | GCAG | 3.9 |
| TTTG | CGAA | 40.0 | CAAG | 35.00 | TAAA | 10.0 |
| GTGA | TCAT | 32.8 | TCGC | 21.55 | GCAC | 15.5 |
| CCTG | CGGG | 44.8 | TAGG | 31.72 | AAGG | 6.2 |
| GCCA | TGGT | 43.8 | GGGC | 28.57 | TGGA | 17.2 |
| GCCC | GGGT | 80.8 | GGGA | 12.50 | GAGC | 18 |
| CTGG | ACAG | 37.7 | TCAG | 29.80 | CCGG | 13.9 |
| CTGT | GCAG | 55.3 | ACGG | 20.18 | ACTG | 4.6 |
| CAGT | GCTG | 72.2 | ATTG | 6.33 | AGTG | 6.3 |
| AGCA | TGCG | 22.1 | TGTT | 20.35 | GGCT | 18.6 |
| GACA | TGGC | 56.5 | TGTT | 16.94 | TGTA | 8.9 |
| TCGC | GCGG | 70.9 | GTGA | 11.21 | GCGT | 8.5 |
| GACG | TGTC | 31.9 | CGGC | 30.21 | CGTT | 16.6 |
| CGTG | TACG | 32.1 | CGCG | 22.11 | CATG | 14.7 |
| TCCC | GGGG | 81.1 | GGGT | 14.29 | GGTA | 1.5 |
| ATGA | GCAT | 40.0 | TCGT | 37.14 | TTAT | 5.7 |
| AGGC | GACT | 25.6 | GCTT | 24.79 | GTCT | 12.4 |
| ATGG | ACAT | 23.8 | CCGT | 23.08 | TCAT | 19.6 |
| A CAC | GTGG | 49 5 | GGGT | 36.89 | GTGC | 7.3 |
| ATTT | GAAT | 38.1 | AGAT | 28.57 | TAAT | 23.8 |
| CCTC | GGGG | 85.4 | GTGG | 5.41 | GCGG | 5.4 |
| TTCC | GGAG | 40.0 | GGGA | 38.26 | GGAT | 11.3 |
| AGGA | GCCT | 33.3 | TCTT | 15.48 | TCCG | 13.1 |
| ATTG | CGAT | 49.2 | TAAT | 24.59 | CAGT | 8.2 |
| GGTA | TGCC | 28.8 | TACT | 28.21 | TATC | 16.7 |
| CGTT | GACG | 27.5 | AGCG | 25.35 | TACG | 24.6 |
| GAGT | GCTC | 60.1 | ACTT | 18.30 | AGTC | 4.6 |
| CCCT | GGGG | 73.2 | TGGG | 20.73 | CGGG | 3.0 |
| GCTC | GGGC | 59.0 | GAGT | 33.70 | GTGC | 2.2 |
| ATAT | GTAT | 38.1 | ATGT | 33.33 | AGAT | 9.5 |
| GTTA | TGAC | 39.5 | TAAT | 25.58 | TAGC | 11.6 |
| TCCG | CGGG | 55.6 | TGGA | 24.49 | CGGT | 7.7 |
| CGCT | GGCG | 63.0 | TGCG | 17.70 | AGTG | 6.2 |
| GAAT | GTTC | 78.7 | ATTT | 8.51 | AGTC | 4.3 |
| CCAT | GTGG | 76.7 | AGGG | 14.47 | TTGG | 6.3 |
| GCAT | GTGC | 58.8 | ATGT | 18.03 | AGGC | 9.0 |
| CTGA | GCAG | 61.3 | TCGG | 24.19 | TCTG | 8.1 |
| GTTG | CAAT | 30.5 | TAAC | 28.00 | CGAC | 16.0 |
| GTTT | GAAC | 37.8 | AGAC | 18.90 | AAAT | 17.3 |
| GTCG | CGAT | 36.6 | TGAC | 30.57 | CGGC | 14.7 |
| GCTG | TAGC | 35.3 | CGGC | 21.94 | CAGT | 20.5 |
| ACGC | GCGG | 75.1 | GGGT | 9.29 | GCGC | 5.9 |
| AGTT | AGCT | 37.2 | GACT | 25.64 | TACT | 17.9 |
| GCGA | GCGC | 35.3 | TCGT | 28.68 | TCGA | 13.2 |
| GGTT | AACT | 21.0 | GACC | 17.94 | AGCC | 14.5 |
| GTGC | GCAT | 44.9 | GCGC | 19.02 | GTAC | 11.5 |
| GCTT | GAGC | 31.8 | AGGC | 25.10 | TAGC | 21.8 |
| AGTA | TGCT | 39.5 | GACT | 26.32 | TATT | 7.9 |
| TTGG | CCAG | 40.5 | CCGA | 23.81 | CCAT | 11.9 |
| AACT | GGTT | 68.8 | TGTT | 7.50 | AGGT | 7.5 |
| ATGT | GCAT | 45.2 | ACGT | 18.28 | ATAT | 7.5 |
| ACCA | TGGG | 45.1 | GGGT | 41.67 | CGGT | 4.9 |
| CAAT | GTTG | 87.1 | AGTG | 4.29 | ATGG | 2.9 |
| CCGA | GCGG | 79.6 | TTGG | 9.95 | CCGG | 2.5 |
| CGAT | GTCG | 50.8 | AGCG | 20.42 | ATTG | 15.7 |
| AGCG | TGCT | 41.3 | CGAT | 14.39 | CGGT | 13.7 |
| CTTT | GAAG | 40.0 | AGAG | 30.00 | TAAG | 15.0 |
| TAAA | TTTG | 100.0 | N/A | - | N/A | - |
| AAAT | GTTT | 84.6 | ATTG | 7.69 | GGTT | 3.8 |
| CGGT | GCCG | 37.9 | ACTG | 22.57 | AGCG | 11.6 |
| GTAT | GTAC | 51.9 | TTAC | 12.04 | ATGC | 11.1 |
| TTAT | ATAG | 30.0 | ATGA | 20.00 | ATGG | 10.0 |
| GCCG | CGGT | 34.6 | TGGC | 33.71 | CGGA | 13.4 |
| GTGT | GCAC | 34.5 | ACAT | 27.72 | TCAC | 10.9 |
| GAGG | CCTT | 33.8 | TCTC | 27.43 | CCTA | 12.7 |
| TATA | TATG | 20.0 | GATA | 20.00 | CATA | 20.0 |
| ATCT | GGAT | 57.5 | AGGT | 18.75 | TGAT | 16.3 |
| TGCC | GGCG | 54.9 | GGCT | 20.95 | GGTA | 14.3 |
| TATT | AATG | 50.0 | AGTA | 30.00 | GATA | 10.0 |
| CCGT | GCGG | 77.1 | ATGG | 11.46 | TCGG | 5.2 |
| AGGT | GCCT | 44.8 | ACTT | 13.04 | AGCT | 7.8 |
| CTCT | GGAG | 61.0 | AGGG | 22.88 | TGAG | 1.0.2 |
| TCAG | C TGG | 58.4 | TTGA | 18.18 | CTGT | 7.8 |
| CCTA | TGGG | 49.3 | GAGG | 42.25 | AAGG | 4.2 |
| AGAT | GTCT | 58.8 | AGCT | 14.12 | ATTT | 7.1 |
| CTAT | GTAG | 63.2 | ATGG | 24.56 | AGAG | 10.5 |
| GGAT | GTCC | 29.8 | ATTC | 19.32 | ATCT | 15.6 |
| TTTC | GGAA | 52.2 | GAAG | 30.43 | GAGA | 10.9 |
| ACTG | CGGT | 58.5 | TAGT | 15.45 | CTGT | 8.9 |
| TATC | GATG | 52.3 | GGTA | 40.00 | GATT | 4.6 |
| TAGC | GCTG | 57.0 | GCTT | 30.23 | GTTA | 2.9 |
| TCAA | TTGG | 47.4 | GTGA | 26.32 | TTGT | 10.5 |
| GTCT | GGAC | 43.1 | TGAC | 20.00 | AGAT | 19.2 |
| AGCT | GGCT | 40.8 | TGCT | 20.17 | AGTT | 12.4 |
| TCTG | CGGA | 44.9 | CAGG | 27.55 | TAGA | 13.3 |
| GCCT | GGGC | 39.1 | AGGT | 27.18 | TGGC | 21.4 |
| TTGT | ACAG | 28.1 | GCAA | 28.07 | ACGA | 21.1 |
| CCTT | GAGG | 48.8 | AGGG | 31.10 | TAGG | 15.2 |
| TCGA | TCGG | 45.1 | GCGA | 31.86 | TCGT | 13.3 |
| TTAC | GTAG | 38.6 | GTGA | 24.56 | GTAT | 22.8 |
| GGAG | CTTC | 29.2 | CTCT | 24.74 | TTCC | 15.8 |
| GGAC | GGCC | 34.0 | CTCT | 26.11 | GTTC | 16.8 |
| TCCA | TGGG | 56.0 | GGGA | 31.87 | TGGT | 7.7 |
| ACGA | TCGG | 53 3 | GCGT | 32.99 | TTGT | 6.1 |
| TACC | GGTG | 67.3 | GGTT | 15.51 | GGGA | 10.6 |
| GACT | GGTC | 50.4 | AGGC | 22.79 | AGTT | 7.4 |
| ACAT | GTGT | 57.4 | ATGG | 26.36 | AGGT | 7.0 |
| TACG | CGTG | 44.2 | CGTT | 25.36 | CGGA | 12.3 |
| ACTC | GGGT | 86.0 | GAGG | 4.55 | GAGC | 4.1 |
| TGTC | GGCA | 42.8 | GACG | 34.88 | GATA | 7.4 |
| TAGA | TCTG | 76.5 | TCTT | 17.65 | TGTA | 5.9 |
| ACCT | GGGT | 53.8 | AGGG | 23.43 | TGGT | 18.9 |
| TCAC | GTGG | 53.5 | GGGA | 26.98 | GTGT | 13.5 |
| TCGT | GCGA | 37.9 | ACGG | 32.52 | ACGT | 9.2 |
| GGTC | GGCC | 34.0 | GACT | 32.31 | GATC | 16.0 |
| TGAA | TTCG | 35.0 | TGCA | 20.00 | TTCT | 15.0 |
| TTCA | TGAG | 35.0 | TGGA | 30.00 | GGAA | 15.0 |
| TAGG | CCTG | 51.7 | CCTT | 28.09 | CGTA | 3.4 |
| TGTG | CACG | 30.6 | CGCA | 14.45 | TACA | 12.7 |
| TGCG | CGCG | 30.1 | TGCA | 24.85 | CGCT | 12.6 |
| ACTT | AGGT | 36.6 | GAGT | 34.15 | TAGT | 12.2 |
| TCGG | ACGA | 34.5 | CCGG | 32.24 | TCGA | 16.8 |
| TGGC | GCCG | 28.9 | GCCT | 19.80 | GACA | 17.1 |
| ACGT | ACGG | 43.5 | GCGT | 34.01 | TCGT | 6.5 |
| CCGG | ACGG | 61.8 | TCGG | 22.02 | CGGG | 4.9 |
| TACA | TGTG | 59.5 | TGGA | 16.22 | TGTT | 13.5 |
| TCTA | TGGA | 40.0 | GAGA | 25.00 | TTGA | 15.0 |
| GGCA | TGCT | 29.2 | TGAC | 20.39 | TGTC | 19.7 |
| TGAC | GGCA | 36.3 | GTCG | 30.92 | GTCT | 19.8 |
| TTAA | TTGA | 75.0 | TTAT | 25.00 | TTTT | - |
| AGGG | TCCT | 24.4 | ACCT | 21.20 | CCTT | 16.1 |
| TTCT | AGAG | 31.1 | GGAA | 28.89 | AGAT | 13.3 |
| CGGG | TCCG | 26.5 | ACCG | 17.96 | CACG | 17.5 |
| GGGA | TCTC | 24.2 | TCCT | 22.22 | TCAC | 14.0 |
| TAAC | GTTG | 67.5 | GTTT | 24.10 | GGTA | 6.0 |
| GCGC | GCGT | 49.3 | GCGA | 19.01 | GTGC | 11.5 |
| TCTC | GGGA | 56.5 | GAGG | 36.72 | GAGT | 2.8 |
| TGGG | ACCA | 20.3 | TCCA | 15.94 | CACA | 13.8 |
| TCTT | GAGA | 33.3 | AAGG | 28.89 | AGGA | 28.9 |
| GTGG | CCAT | 26.0 | TCAC | 24.47 | ACAC | 21.7 |
| TCAT | GTGA | 47.5 | ATGG | 35.00 | ATGT | 8.8 |
| TGTT | AACG | 35.0 | AGCA | 23.00 | GACA | 21.0 |
| GCGT | GCGC | 45.5 | ACGT | 18.28 | ACGA | 11.9 |
| ACTA | TGGT | 70.6 | GAGT | 13.73 | TAGG | 5.9 |
| ACGG | CCGG | 52.8 | ACGT | 24.57 | TCGT | 12.9 |
| TGGA | TCCG | 34.5 | GCCA | 25.18 | TCCT | 12.3 |
| GGCT | GGCC | 32.6 | AGCT | 15.10 | AGTC | 13.8 |
| TAGT | ACTG | 35.2 | GCTA | 24.07 | ACTT | 18.5 |
| GGCC | GGCT | 30.6 | GGAC | 21.82 | GGTC | 21.5 |
| TGTA | TACG | 30.6 | TGCA | 26.53 | GACA | 22.4 |
| GGTG | CACT | 33.0 | TACC | 27.78 | CATC | 12.1 |
| TGCA | TGCG | 45.2 | GGCA | 23.40 | TGCT | 16.5 |
| TGAT | ATCG | 38.4 | GTCA | 23.21 | AGCA | 14.3 |
| TCCT | GGGA | 41.6 | AGGG | 34.84 | TGGA | 14.0 |
| TTGA | TCAG | 40.0 | TCGA | 20.00 | GCAA | 14.3 |
| TGGT | ACCG | 26.3 | GCCA | 26.33 | ACCT | 16.0 |
| CACT | GGTG | 74.2 | AGGG | 12.88 | TGTG | 8.0 |
| TACT | GGTA | 44.4 | AGTG | 26.26 | AGTT | 14.1 |
| TGCT | GGCA | 36.5 | AGCG | 30.03 | AGCT | 12.6 |
| GGCG | TGCC | 24.4 | CGCT | 21.58 | CGAC | 17.6 |
| GGGC | GCTC | 20.2 | GCCT | 18.55 | GACC | 12.9 |
| GGGT | ACTC | 22.7 | GCCC | 19.74 | ACCT | 16.8 |
| GCGG | CCGT | 25.8 | ACGC | 23.52 | CCGA | 18.6 |
| TAAT | ATTG | 41.7 | GTTA | 30.56 | ATTT | 13.9 |
| GGGG | TCCC | 19.7 | CCTC | 19.55 | CCCT | 14.9 |

The invention can be further defined with reference to the following embodiments or clauses:
1. A composition comprising a first partially double-stranded nucleic acid molecule and an at least second partially double-stranded nucleic acid molecule,
   wherein the first partially double-stranded nucleic acid molecule comprises a first 5' overhang and a second 5' overhang,
   wherein the at least second partially double-stranded nucleic acid molecule comprises a third 5' overhang and fourth 5' overhang,
   wherein the second 5' overhang and third 5' overhang are complementary to each other,
   wherein the first 5' overhang, the third 5' overhang and the fourth 5' overhang each comprise one of the 4-mer sequences, or complement thereof, of a 4-mer triplet,
   wherein the 4-mer triplet comprises three 4-mer sequences, which yield a single fragment with at least 90% purity upon ligation of the first partially double-stranded nucleic acid molecule and the at least second partially double-stranded nucleic acid molecule, and
   wherein the first 5' overhang, the third 5' overhang and the fourth 5' overhang comprise a different 4-mer sequence.
2. The composition of clause 1, wherein the 4-mer triplet is selected from the 4-mer triplets recited in Table 1.
3. The composition of clause 1 or clause 2, wherein at least one of the first 5' overhang, the second 5' overhang, the third 5' overhang and the fourth 5' overhang is 4 nucleotides in length.
4. The composition of clause 3, wherein the first 5' overhang, the second 5' overhang, the third 5' overhang and the fourth 5' overhang are each 4 nucleotides in length.
5. The composition of any one of the preceding clauses, wherein the first and the at least second partially double-stranded nucleic acid molecules comprise RNA, XNA, DNA or a combination thereof.
6. The composition of any one of the preceding clauses, wherein the first and the at least second partially double-stranded nucleic acid molecules comprise DNA.
7. The composition of any one of the preceding clauses, wherein at least one of the first partially double-stranded nucleic acid molecule and the at least second partially double-stranded nucleic acid molecule comprises at least one modified nucleic acid.
8. The composition of any one of the preceding clauses, wherein at least one of the first partially double-stranded nucleic acid molecule and the at least second partially double-stranded nucleic acid molecule is at least about 15 nucleotides in length.
9. The composition of any one of the preceding clauses, wherein at least one of the first partially double-stranded nucleic acid molecule and the at least second partially double-stranded nucleic acid molecule comprises a double-stranded portion that is at least 30 bp in length.
10. The composition of clause 9, wherein at least one of the first partially double-stranded nucleic acid molecule and the at least second partially double-stranded nucleic acid molecule comprises a double-stranded portion that is at least 250 bp in length.
11. A method of producing a target nucleic acid molecule, the method comprising:
   a) hybridizing the first and the at least second partially double-stranded nucleic acid molecules of any of the preceding clauses by hybridizing the second 5' overhang of first partially double-stranded nucleic acid molecule and the third 5' overhang of the at least second partially double-stranded nucleic acid molecule; and
   b) ligating the hybridized first partially double-stranded nucleic acid molecule and the at least second partially double-stranded nucleic acid molecule, thereby producing the target nucleic acid molecule.
12. The method of clause 9, wherein ligating comprises contacting the hybridized first and at least second partially double-stranded nucleic acid molecules and a ligase.
13. A composition comprising a first partially double-stranded nucleic acid molecule, a second partially double-stranded nucleic acid molecule, a third partially double-stranded nucleic acid molecule and an at least fourth partially double-stranded nucleic acid molecule,
   wherein the first partially double-stranded nucleic acid molecule comprises a first 5' overhang and a second 5' overhang,
   wherein the second partially double-stranded nucleic acid molecule comprises a third 5' overhang and fourth 5' overhang,
   wherein the third partially double-stranded nucleic acid molecule comprises a fifth 5' overhang and a sixth 5' overhang,
   wherein the at least fourth partially double-stranded nucleic acid molecule comprises a seventh 5' overhang and an eighth 5' overhang,
   wherein the second 5' overhang and third 5' overhang are complementary to each other,
   wherein the fourth 5' overhang and the fifth 5' overhang are complementary to each other,
   wherein the sixth 5' overhang and the seventh 5' overhang are complementary to each other,
   wherein the first 5' overhang, the third 5' overhang, the fifth 5' overhang, the seventh 5' overhang and the eighth 5' overhang each comprise one of the 4-mer sequences, or complement thereof, of a 4-mer quintuplet,
   wherein the 4-mer quintuplet comprises five 4-mer sequences, which yield a single fragment with at least 90% purity upon ligation of the first, second, third and at least fourth partially double-stranded nucleic acid molecules, and
   wherein the first 5' overhang, the third 5' overhang, the fifth 5' overhang, the seventh 5' overhang and the eighth 5' overhang comprise a different 4-mer sequence.
14. The composition of clause 13, wherein the 4-mer quintuplet is selected from the 4-mer quintuplets recited in Table 2.
15. The composition of clause 13 or 14, wherein at least one of the first 5' overhang, the second 5' overhang, the third 5' overhang, the fourth 5' overhang, the fifth 5' overhang, the sixth 5' overhang, the seventh 5' overhang and the eighth 5' overhang is 4 nucleotides in length.
16. The composition of clause 15, wherein the first 5' overhang, the second 5' overhang, the third 5' overhang, the fourth 5' overhang, the fifth 5' overhang, the sixth 5' overhang, the seventh 5' overhang and the eighth 5' overhang are each 4 nucleotides in length.
17. The composition of any one of clauses 13-16, wherein the first, the second, the third and the at least fourth partially double-stranded nucleic acid molecules comprise RNA, XNA, DNA or a combination thereof.
18. The composition of any one of clauses 13-17, wherein the wherein the first, the second, the third and the at least fourth partially double-stranded nucleic acid molecules comprise DNA.
19. The composition of any one of clauses 13-18, wherein at least one of the first partially double-stranded nucleic acid molecule, the second partially double-stranded nucleic acid molecule, the third partially double-stranded nucleic acid molecule and the fourth partially double-stranded nucleic acid molecule comprises at least one modified nucleic acid.
20. The composition of any one of the clauses 13-19, wherein at least one of the first partially double-stranded nucleic acid molecule, the second partially double-stranded nucleic acid molecule, the third partially double-stranded nucleic acid molecule and the at least fourth partially double-stranded nucleic acid molecule is at least about 15 nucleotides in length.
21. The composition of any one of clauses 13-20, wherein at least one of the first partially double-stranded nucleic acid molecule, the second partially double-stranded nucleic acid molecule, the third partially double-stranded nucleic acid molecule and the at least fourth partially double-stranded nucleic acid molecule comprises a double-stranded portion that is at least 20 bp in length.
22. The composition of clause 21, wherein at least one of the first partially double-stranded nucleic acid molecule, the second partially double-stranded nucleic acid molecule, the third partially double-stranded nucleic acid molecule and the at least fourth partially double-stranded nucleic acid molecule comprises a double-stranded portion that is at least 250 bp in length.
23. A method of producing a target nucleic acid molecule, the method comprising:
   a) hybridizing the first and the at least second partially double-stranded nucleic acid fragments of any one of clauses 13-22 by hybridizing the second 5' overhang of the first partially double-stranded nucleic acid fragment and the third 5' overhang of the second partially double-stranded nucleic acid fragment;
   b) ligating the hybridized first partially double-stranded nucleic acid fragment and the second partially double-stranded nucleic acid fragment to produce a first ligation product;
   c) hybridizing the third and the at fourth second partially double-stranded nucleic acid fragments of any one of clauses 13-22 by hybridizing the sixth 5' overhang of third partially double-stranded nucleic acid fragment and the seventh 5' overhang of the at least fourth partially double-stranded nucleic acid fragment;
   d) ligating the hybridized third partially double-stranded nucleic acid fragment and the at least fourth partially double-stranded nucleic acid fragment to produce a second ligation product;
   e) hybridizing the first ligation product from step (b) and the second ligation product of step (d) by hybridizing the fourth 5' overhang and the fifth 5' overhang; and
   f) ligating the hybridized first ligation product and second ligation product, thereby producing the target nucleic acid molecule.
24. The method of clause 23, wherein ligating comprises contacting the hybridized molecules and a ligase.
25. A method of synthesizing a target double-stranded nucleic acid molecule comprising a target nucleic acid sequence, the method comprising
   a) determining an assembly map of the desired double-stranded nucleic acid molecule,
      wherein the assembly map divides the target double-stranded nucleic acid molecule into a plurality of double-stranded nucleic acid fragments,
      wherein the double-stranded nucleic acid fragments comprise at least two 5' overhangs,
      wherein nucleic acid fragments that are adjacent within the target nucleic acid sequence comprise 5' overhangs that are complementary,
      wherein the 5' overhangs of at least one pair of nucleic acid fragments that are adjacent within the target nucleic acid sequence each comprise one of the 4-mer sequences, or complement thereof, of a 4-mer triplet,
      wherein the 4-mer triplet comprises three 4-mer sequences, which yield a single fragment with at least 90% purity upon ligation of the at least one pair of adjacent nucleic acid fragments;
   b) providing the double-stranded nucleic acid fragments determined in step (a);
   c) hybridizing a first pair of double-stranded nucleic acid fragments that are adjacent within the target nucleic acid via their complementary 5' overhangs;
   d) ligating the hybridized nucleic acid fragments from step (c) to form a double-stranded nucleic acid fragment;
   e) hybridizing a second pair of double-stranded nucleic acid fragments that are adjacent within the target nucleic acid via their complementary 5' overhangs;
   f) ligating the hybridized nucleic acid fragments from step (e) to form a double-stranded nucleic acid fragment, such that the double-stranded nucleic acid fragment is adjacent within the target nucleic acid sequence to the double-stranded nucleic acid formed in step (d);
   g) repeating steps (c)-(f) using the ligation products such that the target double-stranded nucleic acid molecule is synthesized.
26. The method of clause 25, wherein the 4-mer triplet is selected from the 4-mer triplets recited in Table 1.
27. A method of synthesizing a target double-stranded nucleic acid molecule comprising a target nucleic acid sequence, the method comprising:
   a) determining an assembly map of the desired double-stranded nucleic acid molecule,
      wherein the assembly map divides the target double-stranded nucleic acid molecule into a plurality of double-stranded nucleic acid fragments,
      wherein the double-stranded nucleic acid fragments comprise at least two 5' overhangs,
      wherein nucleic acid fragments that are adjacent within the target nucleic acid sequence comprise 5' overhangs that are complementary,
      wherein the 5' overhangs of at least one set of four nucleic acid fragments that are adjacent within the target nucleic acid sequence each comprise one of the 4-mer sequences, or complement thereof, of a 4-mer quintuplet,
      wherein the 4-mer quintuplet comprises five 4-mer sequences, which yield a single fragment with at least 90% purity upon ligation of the at least one set of four nucleic acid fragments;
   b) providing the double-stranded nucleic acid fragments determined in step (a);
   c) hybridizing a first pair of double-stranded nucleic acid fragments that are adjacent within the target nucleic acid via their complementary 5' overhangs;
   d) ligating the hybridized nucleic acid fragments from step (c) to form a double-stranded nucleic acid fragment;
   e) hybridizing a second pair of double-stranded nucleic acid fragments that are adjacent within the target nucleic acid via their complementary 5' overhangs;
   f) ligating the hybridized nucleic acid fragments from step (e) to form a double-stranded nucleic acid fragment, such that the double-stranded nucleic acid fragment is adjacent within the target nucleic acid sequence to the double-stranded nucleic acid formed in step (d);
   g) repeating steps (c)-(f) using the ligation products such that the target double-stranded nucleic acid molecule is synthesized.
28. The method of clause 27, wherein the 4-mer quintuplet is selected from the 4-mer quintuplets recited in Table 2.
29. The method of any one of clauses 25-27, wherein the assembly map divides the target double-stranded nucleic acid molecule into at least 4 double-stranded nucleic acid fragments.
30. The method of clause 29, wherein the assembly map divides the target double-stranded nucleic acid molecule into at least 50 double-stranded nucleic acid fragments.
31. The method of clause 30, wherein the assembly map divides the target double-stranded nucleic acid molecule into at least 100 double-stranded nucleic acid fragments.
32. The method of any one of clauses 25-31, wherein the target double-stranded nucleic acid molecule is at least 1000 nucleotides in length.
33. The method of clause 32, wherein the target double-stranded nucleic acid molecule is at least 2000 nucleotides in length.
34. The method of clause 33, wherein the target double-stranded nucleic acid molecule is at least 3000 nucleotides in length.
35. The method of any one of clauses 25-34, wherein the target double-stranded nucleic acid comprises at least one homopolymeric sequence, wherein the homopolymeric sequence is at 10 nucleotides in length.
36. The method of any one of clauses 25-35, wherein the target double-stranded nucleic acid has a GC content that is at least about 50%.
37. The method of any one of clauses 25-36, wherein at least one of the double-stranded nucleic acid fragments that corresponds to at least one of the termini of the target double-stranded nucleic acid molecule comprises a hairpin sequence
38. The method of clause 37, further comprising after step (g):
   h) incubating the ligation products with at least one exonuclease.
39. The method of clause 37 or clause 38 wherein the hairpin sequence comprises at least one deoxyuridine base.
40. The method of clause 39, wherein the method further comprises after step (h):
   i) removing the at least one exonuclease; and
   j) incubating the products of the exonuclease incubation with at least one enzyme that cleaves the at least one deoxyuridine base, thereby cleaving the hairpin sequence.
41. The method of clause 37, wherein the hairpin sequence comprises at least one restriction endonuclease site.
42. The method of clause 41, wherein the method further comprises after step (h):
   i) removing the at least one exonuclease; and
   j) incubating the products of the exonuclease incubation with at least one enzyme that cleaves the at least one restriction endonuclease site, thereby cleaving the hairpin sequence.
43. The method of any one of clauses 25-42, wherein the synthesized target double-stranded nucleic acid molecule has a purity of at least 80%.
44. The method of clause 43, wherein the synthesized target double-stranded nucleic acid molecule has a purity of at least 90%.

## Claims

1. A composition comprising:
a first partially double-stranded nucleic acid molecule and an at least second partially double-stranded nucleic acid molecule,
wherein the first partially double-stranded nucleic acid molecule comprises a first 3' overhang and a second 3' overhang,
wherein the at least second partially double-stranded nucleic acid molecule comprises a third 3' overhang and fourth 3' overhang,
wherein the second 3' overhang and third 3' overhang are complementary to each other,
wherein the first 3' overhang, the third 3' overhang and the fourth 3' overhang each comprise one of the 4-mer sequences, or complement thereof, of a 4-mer triplet,
wherein the 4-mer triplet comprises three 4-mer sequences, which yield a single fragment with at least 90% purity upon ligation of the first partially double-stranded nucleic acid molecule and the at least second partially double-stranded nucleic acid molecule, and
wherein the first 3' overhang, the third 3' overhang and the fourth 3' overhang comprise a different 4-mer sequence,
wherein the 4-mer triplet is selected from the 4-mer triplets recited in Table 1; and
T7 DNA ligase.

2. The composition of claim 1, wherein:
i) at least one of the first 3' overhang, the second 3' overhang, the third 3' overhang and the fourth 3' overhang is 4 nucleotides in length; or
ii) the first 3' overhang, the second 3' overhang, the third 3' overhang and the fourth 3' overhang are each 4 nucleotides in length.

3. The composition of any one of the preceding claims, wherein:
i) the first and the at least second partially double-stranded nucleic acid molecules comprise RNA, XNA, DNA or a combination thereof; or
ii) the first and the at least second partially double-stranded nucleic acid molecules comprise DNA.

4. A method of producing a target nucleic acid molecule, the method comprising:
a) hybridizing the first and the at least second partially double-stranded nucleic acid molecules of any of the preceding claims by hybridizing the second 3' overhang of first partially double-stranded nucleic acid molecule and the third 3' overhang of the at least second partially double-stranded nucleic acid molecule; and
b) ligating the hybridized first partially double-stranded nucleic acid molecule and the at least second partially double-stranded nucleic acid molecule by contacting the hybridized first and at least second partially double-stranded nucleic acid molecules and T7 DNA ligase, thereby producing the target nucleic acid molecule.

5. A composition comprising:
a first partially double-stranded nucleic acid molecule, a second partially double-stranded nucleic acid molecule, a third partially double-stranded nucleic acid molecule and an at least fourth partially double-stranded nucleic acid molecule,
wherein the first partially double-stranded nucleic acid molecule comprises a first 3' overhang and a second 3' overhang,
wherein the second partially double-stranded nucleic acid molecule comprises a third 3' overhang and fourth 3' overhang,
wherein the third partially double-stranded nucleic acid molecule comprises a fifth 3' overhang and a sixth 3' overhang,
wherein the at least fourth partially double-stranded nucleic acid molecule comprises a seventh 3' overhang and an eighth 3' overhang,
wherein the second 3' overhang and third 3' overhang are complementary to each other,
wherein the fourth 3' overhang and the fifth 3' overhang are complementary to each other,
wherein the sixth 3' overhang and the seventh 3' overhang are complementary to each other,
wherein the first 3' overhang, the third 3' overhang, the fifth 3' overhang, the seventh 3' overhang and the eighth 3' overhang each comprise one of the 4-mer sequences, or complement thereof, of a 4-mer quintuplet,
wherein the 4-mer quintuplet comprises five 4-mer sequences, which yield a single fragment with at least 90% purity upon ligation of the first, second, third and at least fourth partially double-stranded nucleic acid molecules, and
wherein the first 3' overhang, the third 3' overhang, the fifth 3' overhang, the seventh 3' overhang and the eighth 3' overhang comprise a different 4-mer sequence, wherein the 4-mer quintuplet is selected from the 4-mer quintuplets recited in Table 2; and
T7 DNA ligase.

6. The composition of claim 5, wherein:
i) at least one of the first 3' overhang, the second 3' overhang, the third 3' overhang, the fourth 3' overhang, the fifth 3' overhang, the sixth 3' overhang, the seventh 3' overhang and the eighth 3' overhang is 4 nucleotides in length; or
ii) the first 3' overhang, the second 3' overhang, the third 3' overhang, the fourth 3' overhang, the fifth 3' overhang, the sixth 3' overhang, the seventh 3' overhang and the eighth 3' overhang are each 4 nucleotides in length.

7. The composition of any one of claims 5-6, wherein:
i) the first, the second, the third and the at least fourth partially double-stranded nucleic acid molecules comprise RNA, XNA, DNA or a combination thereof; or
ii) the first, the second, the third and the at least fourth partially double-stranded nucleic acid molecules comprise DNA.

8. The composition of any one of the preceding claims, wherein at least one of the first partially double-stranded nucleic acid molecule, the second partially double-stranded nucleic acid molecule, the third partially double-stranded nucleic acid molecule and the at least fourth partially double-stranded nucleic acid molecule comprises a double-stranded portion that is:
i) at least 20 bp in length; or
ii) at least 250 bp in length.

9. A method of producing a target nucleic acid molecule, the method comprising:
a) hybridizing the first and the at least second partially double-stranded nucleic acid fragments of any one of claims 5-8 by hybridizing the second 3' overhang of the first partially double-stranded nucleic acid fragment and the third 3' overhang of the second partially double-stranded nucleic acid fragment;
b) ligating the hybridized first partially double-stranded nucleic acid fragment and the second partially double-stranded nucleic acid fragment by contacting the hybridized first and the second partially double-stranded nucleic acid molecules and T7 DNA ligase to produce a first ligation product;
c) hybridizing the third and the at fourth second partially double-stranded nucleic acid fragments of any one of claims 5-8 by hybridizing the sixth 3' overhang of third partially double-stranded nucleic acid fragment and the seventh 3' overhang of the at least fourth partially double-stranded nucleic acid fragment;
d) ligating the hybridized third partially double-stranded nucleic acid fragment and the at least fourth partially double-stranded nucleic acid fragment by contacting the hybridized third and at least fourth partially double-stranded nucleic acid molecules and T7 DNA ligase to produce a second ligation product;
e) hybridizing the first ligation product from step (b) and the second ligation product of step (d) by hybridizing the fourth 3' overhang and the fifth 3' overhang; and
f) ligating the hybridized first ligation product and second ligation product, thereby producing the target nucleic acid molecule.

10. A method of synthesizing a target double-stranded nucleic acid molecule comprising a target nucleic acid sequence, the method comprising
a) determining an assembly map of the desired double-stranded nucleic acid molecule,
wherein the assembly map divides the target double-stranded nucleic acid molecule into a plurality of double-stranded nucleic acid fragments,
wherein the double-stranded nucleic acid fragments comprise at least two 3' overhangs,
wherein nucleic acid fragments that are adjacent within the target nucleic acid sequence comprise 3' overhangs that are complementary,
wherein the 3' overhangs of at least one pair of nucleic acid fragments that are adjacent within the target nucleic acid sequence each comprise one of the 4-mer sequences, or complement thereof, of a 4-mer triplet, wherein the 4-mer triplet is selected from the 4-mer triplets recited in Table 1,
wherein the 4-mer triplet comprises three 4-mer sequences, which yield a single fragment with at least 90% purity upon ligation of the at least one pair of adjacent nucleic acid fragments;
b) providing the double-stranded nucleic acid fragments determined in step (a);
c) hybridizing a first pair of double-stranded nucleic acid fragments that are adjacent within the target nucleic acid via their complementary 3' overhangs;
d) ligating the hybridized nucleic acid fragments from step (c) using T7 DNA ligase to form a double-stranded nucleic acid fragment;
e) hybridizing a second pair of double-stranded nucleic acid fragments that are adjacent within the target nucleic acid via their complementary 3' overhangs;
f) ligating the hybridized nucleic acid fragments from step (e) using T7 DNA ligase to form a double-stranded nucleic acid fragment, such that the double-stranded nucleic acid fragment is adjacent within the target nucleic acid sequence to the double-stranded nucleic acid formed in step (d);
g) repeating steps (c)-(f) using the ligation products such that the target double-stranded nucleic acid molecule is synthesized.

11. A method of synthesizing a target double-stranded nucleic acid molecule comprising a target nucleic acid sequence, the method comprising:
a) determining an assembly map of the desired double-stranded nucleic acid molecule,
wherein the assembly map divides the target double-stranded nucleic acid molecule into a plurality of double-stranded nucleic acid fragments,
wherein the double-stranded nucleic acid fragments comprise at least two 3' overhangs,
wherein nucleic acid fragments that are adjacent within the target nucleic acid sequence comprise 3' overhangs that are complementary,
wherein the 3' overhangs of at least one set of four nucleic acid fragments that are adjacent within the target nucleic acid sequence each comprise one of the 4-mer sequences, or complement thereof, of a 4-mer quintuplet, wherein the 4-mer quintuplet is selected from the 4-mer quintuplets recited in Table 2,
wherein the 4-mer quintuplet comprises five 4-mer sequences, which yield a single fragment with at least 90% purity upon ligation of the at least one set of four nucleic acid fragments;
b) providing the double-stranded nucleic acid fragments determined in step (a);
c) hybridizing a first pair of double-stranded nucleic acid fragments that are adjacent within the target nucleic acid via their complementary 3' overhangs;
d) ligating the hybridized nucleic acid fragments from step (c) using T7 DNA ligase to form a double-stranded nucleic acid fragment;
e) hybridizing a second pair of double-stranded nucleic acid fragments that are adjacent within the target nucleic acid via their complementary 3' overhangs;
f) ligating the hybridized nucleic acid fragments from step (e) using T7 DNA ligase to form a double-stranded nucleic acid fragment, such that the double-stranded nucleic acid fragment is adjacent within the target nucleic acid sequence to the double-stranded nucleic acid formed in step (d);
g) repeating steps (c)-(f) using the ligation products such that the target double-stranded nucleic acid molecule is synthesized.

12. The method of claim 10 or claim 11, wherein the assembly map divides the target double-stranded nucleic acid molecule into:
i) at least 4 double-stranded nucleic acid fragments;
ii) at least 50 double-stranded nucleic acid fragments; or
iii) at least 100 double-stranded nucleic acid fragments.

13. The method of any one of claims 10-12, wherein the target double-stranded nucleic acid molecule is:
i) at least 1000 nucleotides in length;
ii) at least 2000 nucleotides in length;
iii) at least 3000 nucleotides in length;
iv) comprises at least one homopolymeric sequence, wherein the homopolymeric sequence is at 10 nucleotides in length; and/or
v) has a GC content that is at least about 50%.

14. The method of any one of claims 10-13, wherein at least one of the double-stranded nucleic acid fragments that corresponds to at least one of the termini of the target double-stranded nucleic acid molecule comprises a hairpin sequence, preferably wherein the hairpin sequence comprises at least one deoxyuridine base, at least one restriction endonuclease site, or a combination thereof.

15. The method of claim 14, further comprising after step (g):
h) incubating the ligation products with at least one exonuclease,
preferably wherein the method further comprises:
i) removing the at least one exonuclease; and
j) incubating the products of the exonuclease incubation with at least one enzyme that cleaves the at least one deoxyuridine base and/or the at least one restriction endonuclease site, thereby cleaving the hairpin sequence.
